# EUROPEAN PATENT APPLICATION

(11) **EP 4 834 371 A1**
(43) Date of publication of application: **04.11.2026**
(21) Application number: 24910922.4
(22) Date of filing: 19.12.2024
(51) Int. Cl.: A61K 47/68, C07K 16/30, A61K 39/395, C07D 495/14, A61P 35/00

(54) **ANTIBODY-DRUG CONJUGATE, PHARMACEUTICAL COMPOSITION THEREOF, AND USE THEREOF**

(30) Priority: 29.12.2023 CN 202311871776; 14.09.2024 CN 202411292297
(71) Applicant: Sichuan Kelun-Biotech Biopharmaceutical Co., Ltd., Chengdu, Sichuan 611138 (CN)
(72) Inventor: LIU, Jinming, Chengdu, Sichuan 611138 (CN); REN, Yun, Chengdu, Sichuan 611138 (CN); HE, Ting, Chengdu, Sichuan 611138 (CN); LONG, Hu, Chengdu, Sichuan 611138 (CN); LUO, Shuntao, Chengdu, Sichuan 611138 (CN); LEI, Dongmei, Chengdu, Sichuan 611138 (CN); TIAN, Qiang, Chengdu, Sichuan 611138 (CN); SONG, Hongmei, Chengdu, Sichuan 611138 (CN); TAN, Miao, Chengdu, Sichuan 611138 (CN); TAN, Xiangyang, Chengdu, Sichuan 611138 (CN); GE, Junyou, Chengdu, Sichuan 611138 (CN)
(74) Representative: Santarelli
(86) International application number: PCT/CN2024/140603
(87) International publication number: WO 2025/139993

(57) **Abstract**

Provided are an antibody-drug conjugate, a pharmaceutical composition thereof, and use thereof. Specifically, provided are an antibody-drug conjugate represented by formula (I), a drug linker for preparing the antibody-drug conjugate, and use of the antibody-drug conjugate in antitumor.

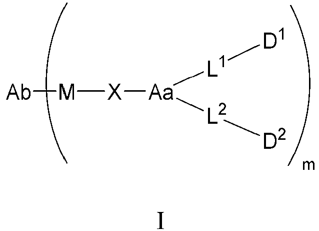

## Description

The present application is based on and claims priority to the Chinese application with application number CN 202311871776.6, filed on December 29, 2023, and Chinese application with application number CN 202411292297.3, filed on September 14, 2024. The disclosures of these Chinese patent applications are incorporated herein by reference in their entirety.

### Technical Field

The present application belongs to the field of medicine and specifically relates to an antibody-drug conjugate, pharmaceutical composition thereof, and use thereof.

### Background Art

Antibody-drug conjugates (ADCs) couple an antibody with small molecule cytotoxic drugs via a specific linker, in which the antibody acts as a carrier for targeted delivery of the small molecule drug to target cells, thereby reducing systemic exposure and improving safety. ADCs are a major focus in the research and development of targeted tumor therapy.

With the advancement of ADC technology, immune-stimulating antibody conjugates (ISACs) have been developed. ISACs are formed by conjugating an immunostimulant or immunomodulator, such as a TLR7 agonist and/or a TLR8 agonist, to an antibody via a cleavable or non-cleavable linker. Immune-stimulating antibody conjugate combines the precise targeting ability of antibodies with the immune-activating and long-lasting immune memory effects of immunostimulants, ensuring the safety of systemic administration and eliciting anti-tumor immune responses.

In anti-cancer research, there is a clear trend shifting from monotherapy to combination therapy. From a clinical perspective, ideal drug combinations can enhance efficacy, reduce toxicity, and delay or prevent the development of drug resistance. Drug combinations include, on the one hand, the combined use of two existing drugs, such as combined use of a cytotoxic drug and an immunostimulant, and on the other hand, a new single drug formed by combining two active ingredients.

Leveraging the targeting properties of antibodies, bifunctional payload immunomodulatory antibody-drug conjugates (iADCs) obtained by conjugating both cytotoxic drugs and immunostimulants to antibodies can simultaneously enter tumor cells and immune cells in the tumor microenvironment. These conjugates exert dual effects: the tumor-killing effect of the cytotoxic drug and the local immune activation effect of the immunostimulant, thereby enhancing the efficacy of antibody-drug conjugates in cancer treatment.

B7H3, also known as CD276, is an immune checkpoint molecule and a co-stimulatory/co-inhibitory immunomodulatory protein that plays a dual role in the immune system. B7H3 is a type I transmembrane protein of the B7 family and has two isoforms: 2Ig-B7H3 and 4Ig-B7H3. The membrane-bound 4Ig-B7H3 isoform is predominant in humans. B7H3 is abnormally overexpressed in various solid tumors, including head and neck cancer, renal cancer, lung cancer, breast cancer, pancreatic cancer, and melanoma. In normal tissues, B7H3 is expressed at lower levels, with low-to-moderate expression detected only in tissues such as the pancreas, liver, colon, and skin. B7H3 is involved in various biological processes related to cancer development, including promoting tumor proliferation and migration, epithelial-mesenchymal transition, tumor angiogenesis, and therapeutic resistance, leading to poor patient prognosis. Therefore, B7H3 has become a promising therapeutic target for cancer treatment.

### Contents of the present invention

The present application relates to an antibody-drug conjugate for treating a cancer associated with B7H3 expression (e.g., B7H3-positive cancer), wherein the conjugate is a bifunctional payload immune-stimulating antibody-drug conjugate obtained by conjugating both a cytotoxic drug and a TLR agonist to an antibody. This conjugate can simultaneously enter tumor cells and immune cells in the tumor microenvironment, exerting the dual effects: the cytotoxic drug's tumor-killing effect and the TLR agonist's local immune activation effect. Compared to antibody-drug conjugates formed by conjugating only a cytotoxic drug, these conjugates exhibit enhanced therapeutic efficacy against cancers.

In one aspect, the present invention provides an antibody-drug conjugate represented by Formula (I): 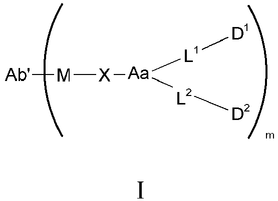 wherein,
Ab' represents an antibody or antigen-binding fragment thereof that specifically binds to B7H3;
M represents a linking site connected to the antibody or antigen-binding fragment thereof;
X represents a linker connecting M and Aa;
Aa is an amino acid fragment or a peptide fragment formed from two or more amino acids;
L¹ represents a linker connecting Aa and D¹;
L² represents a linker connecting Aa and D²;
D¹ represents a cytotoxic drug moiety;
D² represents a TLR agonist moiety;
m ranges from 1 to 10, for example, 1, 2, 3, 4, 5, 6, 7, 8, 9, or 10.

In another aspect, the present invention provides a composition of antibody-drug conjugate, which comprises the above-mentioned antibody-drug conjugate, wherein the composition has a DAR value of 1.0 to 10.0, preferably 1.0 to 5.0, for example, 1.0 to 1.5, 1.0 to 2.0, 1.0 to 2.5, 1.0 to 3.0, 1.0 to 3.5, 1.0 to 4.0, 1.0 to 4.5, 1.0 to 5.0, 2.0 to 2.5, 2.0 to 3.0, 2.0 to 3.5, 2.0 to 4.0, 2.0 to 4.5, 2.0 to 5.0, 2.5 to 3.0, 2.5 to 3.5, 2.5 to 4.0, 2.5 to 4.5, 2.5 to 5.0, 3.0 to 3.5, 3.0 to 4.0, 3.0 to 4.5, 3.0 to 5.0, 3.5 to 4.0, 3.5 to 4.5, 3.5 to 5.0, 4.0 to 4.5, 4.0 to 5.0, 4.5 to 5.0.

In another aspect, the present invention provides a compound represented by Formula (II), or a pharmaceutically acceptable salt, ester, stereoisomer, tautomer, polymorph, solvate, N-oxide, isotope-labeled compound, metabolite or prodrug thereof: 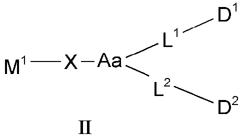 wherein,
X, Aa, L¹, L², D¹, and D² are as defined above;
M¹ is a precursor of a linking site that is attached to an antibody or antigen-binding fragment thereof;
preferably, M¹ is selected from the group consisting of the following structures: 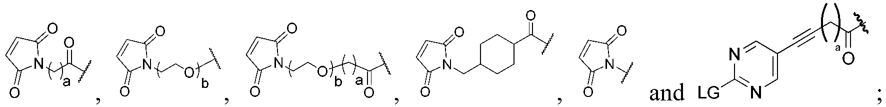
more preferably, M¹ is selected from the group consisting of the following structures: 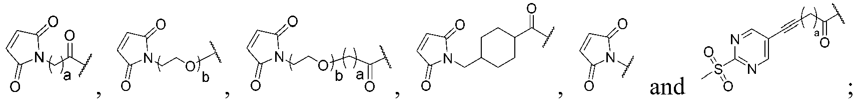
more preferably, M¹ is selected from the group consisting of the following structures: 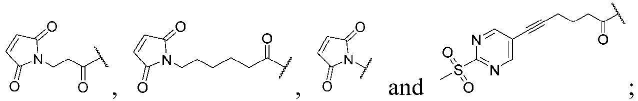
wherein, each a is independently an integer ranging from 0 to 6, and each b is an integer ranging from 1 to 10;
LG represents a leaving group, preferably, each LG is independently selected from the group consisting of halogen (e.g., F, Cl, Br, I), halogenated C₁₋₆ alkyl, C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, hydroxyl, sulfhydryl group, amino, nitro, azido, cyano, alkenyl, alkynyl, and alkynyl-containing structural fragment, and the halogenated C₁₋₆ alkyl, C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, alkenyl, alkynyl, and alkynyl-containing structural fragment are optionally substituted with one or more suitable substituents;
preferably, each LG is independently selected from the group consisting of halogen (e.g., F, Cl, Br, I), halogenated C₁₋₆ alkyl, C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, hydroxyl, sulfhydryl group, amino, nitro, azido, cyano, alkenyl, alkynyl, and alkynyl-containing structural fragment;
more preferably, LG is C₁₋₆ alkylsulfonyl, such as methylsulfonyl.

In another aspect, the present invention provides an antibody or antigen-binding fragment thereof that specifically binds to B7H3, wherein the antibody or antigen-binding fragment thereof comprises: a heavy chain variable region (VH) as set forth in SEQ ID NO: 23 or 1, or a variant thereof, and/or, a light chain variable region (VL) as set forth in SEQ ID NO: 2, or a variant thereof.

In another aspect, the present invention provides a pharmaceutical composition, which comprises the antibody-drug conjugate, the compound or the pharmaceutically acceptable salt, ester, stereoisomer, tautomer, polymorph, solvate, N-oxide, isotope-labeled compound, metabolite or prodrug thereof, the composition of antibody-drug conjugate, the antibody or antigen-binding fragment thereof as described in any one of the aforementioned items, and one or more pharmaceutical excipients.

In another aspect, the present invention provides a use of the compound or the pharmaceutically acceptable salt, ester, stereoisomer, tautomer, polymorph, solvate, N-oxide, isotope-labeled compound, metabolite or prodrug thereof, the antibody or antigen-binding fragment thereof as described in any one of the aforementioned items, in the manufacture of an antibody-drug conjugate, particularly in the manufacture of the antibody-drug conjugate as described in any one of the aforementioned items.

In another aspect, the present invention provides a use of the antibody-drug conjugate, the compound or the pharmaceutically acceptable salt, ester, stereoisomer, tautomer, polymorph, solvate, N-oxide, isotope-labeled compound, metabolite or prodrug thereof, the composition of antibody-drug conjugate, the antibody or antigen-binding fragment thereof, or the pharmaceutical composition as described in any one of the aforementioned items, in the manufacture of a medicament, particularly in the manufacture of a medicament for treating and/or preventing a cancer (e.g., a cancer associated with B7H3 expression, such as B7H3-positive cancer).

In another aspect, the present invention provides a method for treating and/or preventing a cancer (e.g., a cancer associated with B7H3 expression, such as B7H3-positive cancer), comprising administering to a subject in need thereof a therapeutically and/or prophylactically effective amount of the antibody-drug conjugate, the compound (e.g., drug-linkers, TLR agonists, or cytotoxic drugs) or the pharmaceutically acceptable salt, ester, stereoisomer, tautomer, polymorph, solvate, N-oxide, isotope-labeled compound, metabolite or prodrug thereof, the composition of antibody-drug conjugate, the antibody or antigen-binding fragment thereof, or the pharmaceutical composition as described in any one of the aforementioned items.

In another aspect, the present invention also provides an intermediate for preparing the antibody-drug conjugate, the compound or the pharmaceutically acceptable salt, ester, stereoisomer, tautomer, polymorph, solvate, N-oxide, isotope-labeled compound, metabolite or prodrug thereof as described in any one of the aforementioned items.

### Detailed Description of the Embodiments

### Definitions of terms

Unless otherwise defined below, all technical and scientific terms used herein are intended to have the same meanings as commonly understood by those skilled in the art. References to techniques used herein are intended to refer to techniques commonly understood in the art, including modifications or substitutions of equivalent techniques readily apparent to those skilled in the art. Furthermore, laboratory procedures in genomics, nucleic acid chemistry, molecular biology, and the like used herein are conventional procedures widely used in the relevant fields. While the following terms are believed to be well understood by those skilled in the art, the following definitions are provided to better illustrate the present invention.

The term "antibody" refers to immunoglobulin molecules typically composed of two pairs of polypeptide chains, each pair comprising a light chain (LC) and a heavy chain (HC). Antibody light chains can be classified as kappa (κ) and lambda (λ). Heavy chains can be classified as µ, δ, γ, α, or ε, and define the antibody isotype as IgM, IgD, IgG, IgA, and IgE, respectively. Within light and heavy chains, the variable and constant regions are connected by a "J" region of approximately 12 or more amino acids. Heavy chains also comprise a "D" region of approximately 3 or more amino acids. Each heavy chain consists of a heavy chain variable region (VH) and a heavy chain constant region (CH). The heavy chain constant region is composed of three domains (CH1, CH₂, and CH3). Each light chain is composed of a light chain variable region (VL) and a light chain constant region (CL). The light chain constant region consists of a single domain, CL. The constant domains are not directly involved in antibody-antigen binding but exhibit various effector functions, such as mediating the binding of immunoglobulins to host tissues or factors, including various cells of the immune system (e.g., effector cells) and the first component (C1q) of the classical complement system. The VH and VL regions can be further subdivided into highly variable regions called complementarity-determining regions (CDRs), interspersed with more conserved regions called framework regions (FRs). Each VH and VL consists of three CDRs and four FRs, arranged from amino-terminus to carboxyl-terminus in the following order: FR1, CDR1, FR2, CDR2, FR3, CDR3, FR4. The variable regions (VH and VL) of each heavy/light chain pair form the antigen-binding site. The assignment of amino acids to various regions or domains can follow various numbering systems known in the art.

The term "antibody" also comprises embodiments in which the heavy chain constant region comprises a C-terminal lysine, lacks a C-terminal lysine, or a C-terminal glycine-lysine dipeptide. The term also comprises embodiments in which the N-terminal amino acid of the antibody variable region has been cyclized to a pyroglutamate. Thus, in a composition comprising the antibodies disclosed herein, each antibody therein may independently comprise a C-terminal lysine, lack a C-terminal lysine, lack a C-terminal glycine-lysine, and/or, comprise an N-terminal glutamine or glutamic acid, or have the N-terminal amino acid cyclized to a pyroglutamate.

The term "complementarity determining region" or "CDR" refers to the amino acid residues in the variable region of an antibody that are responsible for antigen binding. The variable regions of the heavy and light chains each contain three CDRs, designated CDR1, CDR2, and CDR3. The precise boundaries of the CDRs can be defined according to various numbering systems known in the art, for example, as defined in the Kabat numbering system (Kabat et al., Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al. (1989) Nature 342:878-883), the IMGT numbering system (Lefranc et al., Dev. Comparat. Immunol. 27:55-77, 2003), or the AbM numbering system (Martin ACR, Cheetham JC, Rees AR (1989) Modelling antibody hypervariable loops: A combined algorithm. Proc Natl Acad Sci USA 86:9268-9272). For a given antibody, a person skilled in the art will readily identify the CDRs defined by each numbering system. Furthermore, the correspondence between different numbering systems is well known to those skilled in the art (e.g., see Lefranc et al., Dev. Comparat. Immuno.1. 27:55-77, 2003).

In the present invention, CDRs contained in antibodies or antigen-binding fragments thereof can be identified using various numbering systems known in the art, such as the Kabat, Chothia, IMGT, or AbM numbering systems. In certain embodiments, CDRs contained in antibodies or antigen-binding fragments thereof are defined using the Chothia numbering system.

The following general rules (published at www.bioinf.org.uk: Professor Andrew C.R. Martin's group) can be used to define CDRs within an antibody sequence, including amino acids that specifically interact with amino acids consisting of the antigenic epitope to which the antibody binds. In rare cases, these generally invariant features do not occur; however, Cys residues are the most conserved features.

| Loop | Kabat | AbM | Chothia¹ | Chothia² | IMGT |
|---|---|---|---|---|---|
| L1 | L24--L34 | L24--L34 | L24--L34 | L30--L36 | L27--L32 |
| L2 | L50--L56 | L50--L56 | L50--L56 | L46--L55 | L50--L52 |
| L3 | L89--L97 | L89--L97 | L89--L97 | L89--L96 | L89--L97 |
| H1 | H31--H35B (Kabat numbering system)³ | H26--H35B | H26--H32..34 | H30--H35B | H26--H35B |
| H1 | H31--H35 (Chothia numbering system) | H26--H35 | H26--H32 | H30--H35 | H26--H33 |
| H2 | H50--H65 | H50--H58 | H52--H56 | H47--H58 | H51--H56 |
| H3 | H95--H102 | H95--H102 | H95--H102 | H93--H101 | H93--H102 |
| ¹ Some of these numbering systems, particularly the Chothia numbering system, vary depending on the publications reviewed. | | | | | |
| ² Any numbering system can be used for the CDR definitions described, except for the Contact numbering system, which uses the Chothia or Martin (Extended Chothia) definitions. | | | | | |
| ³ When numbering using the Kabat numbering system, the end of the Chothia CDR-H1 loop varies between H32 and H34, depending on loop length. (This is because the insertion points are placed at H35A and H35B in the Kabat numbering system). | | | | | |
| If both H35A and H35B are absent, the loop ends at H32; | | | | | |
| If only H35A is present, the loop ends at H33; | | | | | |
| If both H35A and H35B are present, the loop ends at H34. | | | | | |

The entire amino acid sequence of the V_{H} is generally numbered according to Kabat, and the three CDRs within the variable region can be defined according to any of the above numbering systems. In certain embodiments, amino acid positions in the V_{H} may be numbered sequentially, starting from amino acid position 1, to the end of the sequence, or numbered according to Kabat. Unless otherwise indicated, the amino acid positions in the VH and VL described herein are defined according to sequential numbering.

Amino acid positions in the heavy chain constant region may be numbered sequentially, starting from amino acid position 1, to the end of the sequence, or numbered according to Eu. The amino acid sequence of the IgG1 heavy chain constant region has 330 amino acids, numbered sequentially from 1 to 330. The corresponding sequence according to Eu numbering begins at position 118 and ends at position 447. Unless otherwise indicated, amino acid positions in the heavy and light chains described herein are defined according to sequential numbering.

The term "framework region" or "FR" residues refers to those amino acid residues in the variable region of an antibody other than the CDR residues as defined above.

The term "antigen-binding fragment" of antibody refers to a polypeptide that is a fragment of antibody, such as a polypeptide that is a fragment of full-length antibody, that retains the ability to specifically bind to the same antigen as the full-length antibody and/or, competes with the full-length antibody for specific binding to the antigen, and is also referred to as an "antigen-binding portion." See generally, Fundamental Immunology, Ch. 7 (Paul, W., ed., 2nd ed., Raven Press, N.Y. (1989), which is incorporated herein by reference in its entirety for all purposes. Antigen-binding fragments of antibodies can be produced by recombinant DNA techniques or by enzymatic or chemical cleavage of intact antibodies. Non-limiting examples of antigen-binding fragments include Fab fragments, Fab' fragments, F(ab)'₂ fragments, F(ab)'₃ fragments, Fd, Fv, scFv, di-scFv, (scFv)₂, disulfide-stabilized Fv proteins ("dsFv"), single domain antibodies (sdAb, nanobody), and polypeptides that comprise at least a portion of an antibody sufficient to confer specific antigen-binding ability to the polypeptide. Engineered antibody variants are reviewed in Holliger et al., 2005; Nat Biotechnol, 23: 1126-1136.

The term "Fd" refers to an antibody fragment consisting of the VH and CH1 domains; the term "dAb fragment" refers to an antibody fragment consisting of the VH domain (Ward et al., Nature 341:544-546 (1989)); the term "Fab fragment" refers to an antibody fragment consisting of the VL, VH, CL, and CH1 domains; the term "F(ab')₂ fragment" refers to an antibody fragment comprising two Fab fragments linked by a disulfide bridge at the hinge region; the term "Fab' fragment" refers to a fragment obtained by reducing the disulfide bonds linking the two heavy chain fragments in the F(ab')₂ fragment, consisting of an intact light chain and the Fd fragment of the heavy chain (consisting of the VH and CH1 domains).

The term "Fv" refers to an antibody fragment consisting of the VL and VH domains of a single arm of an antibody. The Fv fragment is generally considered to be the smallest antibody fragment capable of forming a complete antigen-binding site. It is generally believed that the six CDRs confer antigen-binding specificity to an antibody. However, even a single variable region (e.g., an Fd fragment, which contains only three CDRs specific for an antigen) can recognize and bind antigen, although its affinity may be lower than that of the complete binding site.

The term "Fc" refers to an antibody fragment formed by binding the second and third constant regions of the first heavy chain of an antibody to the second and third constant regions of the second heavy chain via disulfide bonds. The Fc fragment of an antibody has a variety of functions but is not involved in antigen binding.

The term "scFv" refers to a single polypeptide chain comprising VL and VH domains, wherein the VL and VH domains are connected by a linker (see, for example, Bird et al., Science 242:423-426 (1988); Huston et al., Proc. Natl. Acad. Sci. USA 85:5879-5883 (1988); and Pluckthun, The Pharmacology of Monoclonal Antibodies, Vol. 113, Roseburg and Moore, eds., Springer-Verlag, New York, pp. 269-315 (1994). Such scFv molecules may have the general structure: NH₂-VL-linker-VH-COOH or NH₂-VH-linker-VL-COOH. Suitable prior art linkers consist of repeated GGGGS (SEQ ID NO: 25) amino acid sequences or variants thereof. For example, a linker having the amino acid sequence (GGGGS)₄ (SEQ ID NO: 26) may be used, but variants thereof may also be used (Holliger et al. (1993), Proc. Natl. Acad. Sci. USA 90: 6444-6448). Other linkers that can be used in the present invention are described by Alfthan et al. (1995), Protein Eng. 8:725-731, Choi et al. (2001), Eur. J. Immunol. 31: 94-106, Hu et al. (1996), Cancer Res. 56:3055-3061, Kipriyanov et al. (1999), J. Mol. Biol. 293:41-56, and Roovers et al. (2001), Cancer Immunol. In some cases, a disulfide bond may also exist between the VH and VL of scFv. In certain embodiments, the VH and VL domains may be positioned relative to each other in any suitable arrangement. For example, an scFv may comprise NH₂-VH-VH-COOH, NH₂-VL-VL-COOH.

Each of the above antibody fragments retains the ability to specifically bind to the same antigen bound by the full-length antibody and/or, competes with the full-length antibody for specific binding to the antigen.

As used herein, the term "specific binding" refers to a non-random binding reaction between two molecules, such as a reaction between an antibody and an antigen to which it targets. The strength or affinity of a specific binding interaction can be expressed as the equilibrium dissociation constant (KD) or half-maximal effect concentration (EC₅₀) of the interaction.

As used herein, the term "variant", in the context of peptides (including polypeptides), also refers to a polypeptide or peptide comprising an amino acid sequence that has been altered by the introduction of an amino acid residue substitution, deletion or addition. In some cases, the term "variant" also refers to a polypeptide or peptide that has been modified (i.e., by covalently attaching any type of molecule to the polypeptide or peptide). For example, but not limitation, a polypeptide can be modified, for example, by glycosylation, acetylation, pegylation, phosphorylation, amidation, derivatization by known protecting/blocking groups, proteolytic cleavage, attachment to cellular ligands or other proteins, and the like. Derivatized polypeptides or peptides can be produced by chemical modification using techniques known to those skilled in the art, including but not limited to specific chemical cleavage, acetylation, formylation, metabolic synthesis of tunicamycin, and the like. In addition, variants have similar, identical or improved functions as compared to the polypeptide or peptide from which they originate. In some embodiments, the variant of the present invention has a sequence identity of at least 80%, at least 85%, at least 90%, at least 91%, at least 92%, at least 93%, at least 94%, at least 95%, at least 96%, at least 97%, at least 98%, at least 99%, or 100% as compared with the sequence from which it is derived, or has a substitution, deletion, or addition of one or more amino acids (e.g., a substitution, deletion, or addition of 1, 2, 3, 4, or 5 amino acids) as compared with the sequence from which it is derived; preferably, the substitution is a conservative substitution.

As used herein, the term "identity" refers to the sequence matching between two polypeptides or between two nucleic acids. When a position in the two sequences being compared is replaced by the same base or amino acid monomer (e.g., when a certain position in each of the two DNA molecules is occupied by adenine, or a certain position in each of the two polypeptides is occupied by lysine), then the molecules are identical at that position. The "percent identity" between two sequences is a function of the number of matching positions shared by the two sequences divided by the number of positions being compared × 100. For example, if 6 out of 10 positions in two sequences match, then the two sequences have 60% identity. For example, the DNA sequences CTGACT and CAGGTT have an identity of 50% (3 out of a total of 6 positions match). Typically, two sequences are compared when they are aligned to produce maximum identity. Such an alignment can be performed, for example, by using a computer program, such as the Align program (DNAstar, Inc.) that is conveniently performed by the method of Needleman et al. (1970) J. Mol. Biol. 48: 443-453. The percent identity between two amino acid sequences can also be determined using the algorithm of E. Meyers and W. Miller (Comput. Appl Biosci., 4: 11-17 (1988)), which has been incorporated into the ALIGN program (version 2.0), using a PAM120 weight residue table, a gap length penalty of 12, and a gap penalty of 4. In addition, the percent identity between two amino acid sequences can be determined by using the algorithm of Needleman and Wunsch (J Mol Biol. 48: 444-453 (1970)), which has been incorporated into the GAP program in the GCG software package (available at www.gcg.com), using a Blossum 62 matrix or a PAM250 matrix, as well as a gap weight of 16, 14, 12, 10, 8, 6, or 4, and a length weight of 1, 2, 3, 4, 5 or 6.

As used herein, the term "conservative substitution" refers to an amino acid substitution that does not adversely affect or change the expected properties of the protein/polypeptide comprising the amino acid sequence. For example, conservative substitutions can be introduced by standard techniques known in the art, such as site-directed mutagenesis and PCR-mediated mutagenesis. Conservative amino acid substitutions include substitutions in which an amino acid residue is replaced with an amino acid residue having a similar side chain, such as an amino acid residue that is physically or functionally similar to the corresponding amino acid residue (e.g., having similar size, shape, charge, chemical properties, including the ability to form covalent bond or hydrogen bond, etc.). Families of amino acid residues with similar side chains have been defined in the art. These Families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), *β*-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative amino acid substitutions are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which is incorporated herein by reference).

Herein, unless the context clearly indicates otherwise, when referring to the term "antibody," it includes not only intact antibody but also antigen-binding fragments of antibody.

Antigen-binding fragments of antibody (e.g., the aforementioned antibody fragments) can be obtained from a given antibody (e.g., the antibody provided herein) using conventional techniques known to those skilled in the art (e.g., recombinant DNA technology or enzymatic or chemical cleavage methods), and the antigen-binding fragments can be screened for specificity in the same manner as for intact antibody.

The term "murine antibody" refers to an antibody obtained by fusing B cells from immunized mice with myeloma cells, screening for murine hybrid fusion cells that can both proliferate indefinitely and secrete antibodies, followed by screening, antibody preparation, and antibody purification; or refers to an antibody secreted by plasma cells formed by differentiation and proliferation of B cells in mice after antigen invasion.

The term "humanized antibody" refers to a genetically engineered non-human antibody whose amino acid sequence has been modified to increase sequence homology with a human antibody. Generally speaking, a humanized antibody comprises all or part of the CDR regions derived from a non-human antibody (the donor antibody), and all or part of the non-CDR regions (e.g., variable region (FR) and/or, constant region) derived from a human immunoglobulin (the recipient antibody). Humanized antibodies typically retain the desired properties of the donor antibody, including, but not limited to, antigen specificity, affinity, reactivity, ability to enhance immune cell activity, and ability to enhance immune response. The donor antibody can be a mouse, rat, rabbit, or non-human primate (e.g., cynomolgus monkey) antibody with the desired properties (e.g., antigen specificity, affinity, reactivity, ability to enhance immune cell activity, and/or, ability to enhance immune response).

The twenty conventional amino acids referred to herein are notated according to conventional usage. See, for example, Immunology-A Synthesis (2nd Edition, E. S. Golub and D. R. Gren, Eds., Sinauer Associates, Sunderland, Mass. (1991)), which is incorporated herein by reference. In the present invention, amino acids are generally represented by single-letter and three-letter abbreviations known in the art. For example, alanine can be represented by A or Ala.

The terms "comprise," "include," "have," "contain," or "involve," and their variations herein, are intended to be inclusive or open-ended and do not exclude unrecited elements or method steps.

The term "alkyl" refers to a group obtained by removing one hydrogen atom from a straight-chain or branched saturated hydrocarbonyl, such as "C₁₋₂₀ alkyl," "C₁₋₁₀ alkyl," "C₁₋₆ alkyl," "C₁₋₄ alkyl," "C₁₋₃ alkyl," and the like. Specific examples include, but are not limited to, methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, 1,2-dimethylpropyl, and the like.

The term "alkylene" refers to a group obtained by removing two hydrogen atoms from a straight-chain or branched saturated hydrocarbonyl, such as "C₁₋₂₀ alkylene," "C₁₋₁₀ alkylene," "C₃₋₁₀ alkylene," "C₅₋₈ alkylene," "C₁₋₆ alkylene," "C₁₋₄ alkylene," and "C₁₋₃ alkylene." Specific examples include, but are not limited to, methylene, ethylene, 1,3-propylene, 1,4-butylene, 1,5-pentylene, and 1,6-hexylene.

The term "cycloalkyl" refers to a saturated cyclic hydrocarbonyl, including, but not limited to, monocyclic and bicyclic alkyl groups (e.g., spiro, fused, and bridged cycloalkyl groups). The term "C₃₋₁₀ cycloalkyl" refers to a cycloalkyl having 3 to 10 ring carbon atoms, such as cyclopropyl, cyclobutyl, cyclopentyl, and cyclohexyl.

The term "heterocyclyl" refers to a saturated or partially saturated cyclic structure containing at least one ring member selected from the group consisting of nitrogen, oxygen, and sulfur. Specific examples include, but are not limited to, 3- to 12-membered heterocyclyl, 3- to 8-membered heterocyclyl, and 5- to 6-membered heterocyclyl, such as tetrahydrofuranyl, pyrrolidinyl, piperidinyl, and tetrahydropyranyl.

The term "heteroaryl" refers to an aromatic ring structure containing at least one ring member selected from the group consisting of nitrogen, oxygen, and sulfur. Specific examples include, but are not limited to, 5- to 10-membered heteroaryl and 5- to 6-membered heteroaryl, such as furanyl, thienyl, pyrrolyl, thiazolyl, isothiazolyl, thiadiazolyl, oxazolyl, isoxazolyl, oxadiazolyl, imidazolyl, pyrazolyl, 1,2,3-triazolyl, 1,2,4-triazolyl, 1,2,3-oxadiazolyl, 1,2,4-oxadiazolyl, 1,2,5-oxadiazolyl, 1,3,4-oxadiazolyl, pyridyl, pyrimidinyl, pyridazinyl, pyrazinyl, 1,2,3-triazinyl, 1,3,5-triazinyl, and 1,2,4,5-tetrazinyl.

The term "aryl" refers to a group obtained by removing a hydrogen atom from an aromatic carbon atom of an aromatic hydrocarbon molecule. For example, C₆₋₁₀ aryl groups, and specific examples include, but are not limited to, phenyl, naphthyl, and anthracenyl.

As used herein, the term "fragment" or "structural fragment" refers to a moiety of a compound molecule remaining after one or more atoms or groups of atoms have been lost. For example, a "cytotoxic drug fragment" refers to a moiety of a cytotoxic drug described herein that remains after the cytotoxic drug has lost a hydrogen atom or hydroxyl, etc., and is then linked to the linker in the antibody-drug conjugate, and is also referred to as "cytotoxic drug moiety." In some specific embodiments, the cytotoxic drug fragment/moiety is represented by D¹. Similarly, a "TLR agonist fragment" refers to a moiety of a TLR agonist described herein that remains after the TLR agonist has lost one or more atoms or groups of atoms and is then linked to the linker in the antibody-drug conjugate, and is also referred to as "TLR agonist moiety." In some specific embodiments, the TLR agonist fragment/moiety is represented by D².

If a substituent or value is described as "independently selected" from a group of groups or values, each substituent or value is selected independently of the other. Thus, each substituent or value may be the same as or different from another (other) substituent or value.

The present invention also includes all pharmaceutically acceptable isotope-labeled compounds, which are identical to the compounds of the present invention except that one or more atoms are replaced by an atom having the same atomic number but an atomic mass or mass number different from the atomic mass or mass number prevalent in nature. Examples of suitable isotopes for inclusion in the compounds of the present invention include, but are not limited to, isotopes of hydrogen (e.g., ²H, ³H, deuterium D, tritium T); isotopes of carbon (e.g., ¹¹C, ¹³C, and ¹⁴C); isotopes of chlorine (e.g., ³⁷Cl); isotopes of fluorine (e.g., ¹⁸F); isotopes of iodine (e.g., ¹²³I and ¹²⁵I); isotopes of nitrogen (e.g., ¹³N and ¹⁵N); isotopes of oxygen (e.g., ¹⁵O, ¹⁷O, and ¹⁸O); isotopes of phosphorus (e.g., ³²P); and isotopes of sulfur (e.g., ³⁵S). Certain isotope-labeled compounds of the present invention (e.g., those incorporating radioactive isotopes) are useful in studies (e.g., assays) of drug and/or, substrate tissue distribution. The radioactive isotopes tritium (i.e., ³H) and carbon-14 (i.e., ¹⁴C) are particularly useful for this purpose due to their ease of incorporation and detection. Substitution with positron-emitting isotopes (e.g., ¹¹C, ¹⁸F, ¹⁵O, and ¹³N) can be used to examine substrate receptor occupancy in positron emission tomography (PET) studies. Isotope-labeled compounds of the present invention can be prepared by methods analogous to those described in the accompanying schemes and/or, in the examples and preparations, by using an appropriate isotopically labeled reagent in place of the unlabeled reagent previously employed. Pharmaceutically acceptable solvates of the present invention include those in which the crystallization solvent is isotopically substituted, for example, D₂O, acetone-*d6*, or DMSO-*d6*.

The term "stereoisomer" refers to an isomer that is formed by at least one asymmetric center. Compounds with one or more (e.g., one, two, three, or four) asymmetric centers may produce racemic mixtures, single enantiomers, diastereomeric mixtures, and individual diastereomers. Certain individual molecules may also exist as geometric isomers (cis/trans). Similarly, the compounds of the present invention may exist as mixtures of two or more structurally distinct forms in rapid equilibrium (commonly referred to as tautomers). Representative examples of tautomers include keto-enol tautomers, phenol-keto tautomers, nitroso-oxime tautomers, imineenamine tautomers, and the like. It is to be understood that the scope of the present application encompasses all such isomers, in any proportion (e.g., 60%, 65%, 70%, 75%, 80%, 85%, 90%, 95%, 96%, 97%, 98%, 99%), or mixtures thereof.

As used herein, the term "suitable substituent" refers to a modification that one skilled in the art can make to a compound based on the desired substituent. "Suitable substituents" include oxo (=O), halogen, cyano, NR⁸R⁹, carboxyl, sulfhydryl group, hydroxyl, ester (e.g., -C₁₋₆ alkyl-C(=O)-OC₁₋₆ alkyl), C₁₋₆ alkyl, C₂₋₆ alkenyl, C₂₋₆ alkynyl, C₁₋₆ alkyl-O-C₁₋₆ alkyl, C₁₋₆ haloalkyl, C₁₋₆ alkoxy, C₃₋₆ cycloalkyl, 3- to 10-membered heterocyclyl, 5- to 10-membered heteroaryl, C₆₋₁₀ alkyl, C₁₋₆ alkyl, C₃₋₆ cycloalkyl, 3- to 10-membered heterocyclyl, 5- to 10-membered heteroaryl, C₆₋₁₀ aryl, C₁₋₆ alkoxy, C₁₋₆ haloalkyl, C₁₋₆ haloalkoxy, halogen, hydroxyl, carboxyl, and ester (e.g., -C₁₋₆ alkyl-C(=O)-OC₁₋₆ alkyl).

Whether explicitly stated or not, numerical values herein are modified by the term "about." The term "about" means within ±20%, ±10%, ±5%, or ±2% of the stated value.

Ranges provided herein should be understood as shorthand for all values within that range. For example, a range of "1 to 10" should be understood to include any number, combination of numbers, or subrange selected from that range. Specifically, "an integer ranging from 1 to 10" refers to an integer ranging from the group consisting of 1, 2, 3, 4, 5, 6, 7, 8, 9, or 10, and "an integer ranging from 0 to 6" refers to an integer ranging from the group consisting of 0, 1, 2, 3, 4, 5, or 6.

The present invention encompasses all possible crystalline forms or polymorphs of the compounds of the present invention, which may be a single polymorph or a mixture of more than one polymorph in any proportion.

It should also be understood that certain compounds of the present invention may exist in free form for therapeutic use or, where appropriate, in the form of pharmaceutically acceptable derivatives thereof. In the present invention, pharmaceutically acceptable derivatives include, but are not limited to, pharmaceutically acceptable salts, solvates, metabolites, or prodrugs that, upon administration to a patient in need thereof, are capable of directly or indirectly providing the compounds of the present invention or their metabolites or residues. Therefore, when reference is made herein to a "compound of the present invention," these various derivative forms of the compound are also intended to be encompassed.

Pharmaceutically acceptable salts of the compound of the present invention include acid addition salts and base addition salts thereof. Suitable acid addition salts are formed from acids that form pharmaceutically acceptable salts. Suitable base addition salts are formed from bases that form pharmaceutically acceptable salts. Suitable salts are reviewed in Stahl and Wermuth, "Handbook of Pharmaceutical Salts: Properties, Selection, and Use" (Wiley-VCH, 2002). Methods for preparing pharmaceutically acceptable salts of the compound of the present invention are known to those skilled in the art.

The compound of the present invention may exist as a solvate, preferably a hydrate, wherein the compound of the present invention contain a polar solvent as a structural element of the crystal lattice of the compound. The amount of polar solvent, particularly water, may be present in a stoichiometric or non-stoichiometric ratio.

One skilled in the art will appreciate that not all nitrogen-containing heterocyclic rings are capable of forming N-oxides, as nitrogen requires an available lone pair of electrons to oxidize to an oxide; one skilled in the art will recognize nitrogen-containing heterocyclic rings that are capable of forming N-oxides. One skilled in the art will also recognize that tertiary amines are capable of forming N-oxides. Synthetic methods for preparing N-oxides of heterocyclic rings and tertiary amines are well known to those skilled in the art, including oxidation of heterocyclic rings and tertiary amines with peroxyacids such as peracetic acid and m-chloroperbenzoic acid (MCPBA), hydrogen peroxide, alkyl hydroperoxides such as tert-butyl hydroperoxide, sodium perborate, and dioxiranes such as dimethyldioxirane. These methods for preparing N-oxides have been extensively described and reviewed in the literature, for example, in T. L. Gilchrist, Comprehensive Organic Synthesis, vol. 7, pp. 748-750; A. R. Katritzky and A. J. Boulton, Eds., Academic Press; and G. W. H. Cheeseman and E. S. G. Werstiuk, Advances in Heterocyclic Chemistry, vol. 22, pp. 390-392, A. R. Katritzky and A. J. Boulton, Eds., Academic Press.

Also within the scope of the present invention are metabolites of the compounds of the present invention, i.e., substances formed in the body upon administration of the compounds of the present invention. Such products may result, for example, from oxidation, reduction, hydrolysis, amidation, deamidation, esterification, enzymatic hydrolysis, and the like of the administered compound. Thus, the present invention encompasses metabolites of the compounds of the present invention, including compounds produced by contacting the compounds of the present invention with a mammal for a period of time sufficient to produce their metabolic products.

The present invention further encompasses prodrugs of the compounds of the present invention, which are derivatives of the compounds of the present invention that may themselves have little or no pharmacological activity but, when administered to or onto the body, are converted, for example, by hydrolytic cleavage, to the compounds of the present invention having the desired activity. Typically, such prodrugs will be functional group derivatives of the compounds that are readily converted in vivo to the desired therapeutically active compounds. Additional information on the use of prodrugs can be found in "Pro-drugs as Novel Delivery Systems," Volume 14, ACS Symposium Series (T. HigUchi and V. Stella) and "Bioreversible Carriers in Drug Design," Pergamon Press, 1987 (E. B. Roche, ed., American Pharmaceutical Association). The prodrugs of the present invention can be prepared, for example, by replacing appropriate functional groups present in the compounds of the present invention with certain moieties known to those skilled in the art as "pro-moieties" (for example, as described in "Design of Prodrugs," H. Bundgaard (Elsevier, 1985)).

The term "about" means within ±10%, preferably ±5%, and more preferably ±2% of the stated value.

Herein, when the targeting moiety in the antibody-drug conjugate of the present invention is an antibody or antigen-binding fragment, 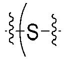 represents the specific mode of connecting the sulfhydryl group in the antibody or antigen-binding fragment to the drug-linker.

Herein, when the targeting moiety in the antibody-drug conjugate of the present invention is an antibody or antigen-binding fragment, 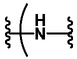 represents the specific mode of connecting the amino group in the antibody or antigen-binding fragment to the drug-linker.

The antibody-drug conjugate is also characterized by the average load factor of the drug moiety (e.g., cytotoxic drug and TLR agonist) to the antibody-binding moiety, which is often referred to as the drug-to-antibody ratio (DAR) of the conjugate sample. For example, the average DAR of an ADC can be calculated from LC-MS data of reduced and deglycosylated sample based on the average load factors of the LC and HC chains. The DAR of a given antibody-drug conjugate sample represents the average number of drug (payload) molecules attached to a tetrameric antibody containing two light chains and two heavy chains.

While the drug-to-antibody ratio has a specific value (e.g., m in Formula (I)) for a particular antibody-drug conjugate molecule, it should be understood that when it is used to describe a sample containing many molecules, this value will often be an average value due to some degree of heterogeneity typically associated with the conjugation step. The average load factor of an antibody-drug conjugate sample is referred to herein as the drug-to-antibody ratio, or "DAR."

In some embodiments, the structure of the antibody-drug conjugate of the present application can be represented by Formula I, wherein m refers to the number of small molecule drug fragments attached to per antibody molecule. During the preparation of the antibody-drug conjugate, each antibody molecule may be linked to a different number of small molecule drug fragments. Therefore, a composition of antibody-drug conjugate is generally a mixture of antibody-drug conjugates with varying drug-to-antibody conjugation ratios. In practice, DAR is often used to represent the average number of drug molecules linked to the antibodies.

In some embodiments, the DAR value (drug-to-antibody ratio of the conjugated sample) of the antibody-drug conjugate composition is 1 to 10, for example: 1 to 2, 1 to 3, 1 to 4, 1 to 5, 1 to 6, 1 to 7, 1 to 8, 1 to 9, 1 to 10,2 to 3, 2 to 4, 2 to 5, 2 to 6, 2 to 7, 2 to 8,2 to 9, 2 to 10,3 to 4, 3 to 5, 3 to 6, 3 to 7, 3 to 8, 3 to 9, 3 to 10, 4 to 5, 4 to 6, 4 to 7, 4 to 8, 4 to 9, 4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10, preferably 3 to 8, for example, 3.0 to 3.5, 3.0 to 4.0, 3.0 to 4.5, 3.0 to 5.0, 3.0 to 5.5, 3.0 to 6.0, 3.5 to 4.0, 3.5 to 4.5, 3.5 to 5.0, 3.5 to 5.5, 3.5 to 6.0, 3.5 to 6.5, 3.5 to 7.0, 3.5 to 7.5, 3.5 to 8.0, 4.0 to 4.5, 4.0 to 5.0, 4.0 to 5.5, 4.0 to 6.0, 4.0 to 6.5, 4.0 to 7.0, 4.0 to 7.5, 4.0 to 8.0, 4.5 to 5.0, 4.5 to 5.5, 4.5 to 6.0, 4.5 to 6.5, 4.5 to 7.0, 4.5 to 7.5, 4.5 to 8.0, 5.0 to 5.5, 5.0 to 6.0, 5.0 to 6.5, 5.0 to 7.0, 5.0 to 7.5, 5.0 to 8.0, 5.5 to 6.0, 5.5 to 6.5, 5.5 to 7.0, 5.5 to 7.5, 5.5 to 8.0, 6.0 to 6.5, 6.0 to 7.0, 6.0 to 7.5, 6.0 to 8.0, 6.5 to 7.0, 6.5 to 7.5, 6.5 to 8.0, 7.0 to 7.5, 7.0 to 8.0 or 7.5 to 8.0, or preferably 1.0 to 5.0, for example, 1.0 to 1.5, 1.0 to 2.0, 1.0 to 2.5, 1.0 to 3.0, 1.0 to 3.5, 1.0 to 4.0, 1.0 to 4.5, 1.0 to 5.0, 2.0 to 2.5, 2.0 to 3.0, 2.0 to 3.5, 2.0 to 4.0, 2.0 to 4.5, 2.0 to 5.0, 2.5 to 3.0, 2.5 to 3.5, 2.5 to 4.0, 2.5 to 4.5, 2.5 to 5.0, 3.0 to 3.5, 3.0 to 4.0, 3.0 to 4.5, 3.0 to 5.0, 3.5 to 4.0, 3.5 to 4.5, 3.5 to 5.0, 4.0 to 4.5, 4.0 to 5.0, 4.5 to 5.0.

In the present invention, "treatment" generally refers to the partial or complete stabilization or cure of a disease and/or, its side effects. As used herein, "treatment" encompasses any treatment of a disease in a patient, including: (a) suppressing the symptoms of the disease, i.e., arresting its development; or (b) alleviating the symptoms of the disease, i.e., causing regression of the disease or its symptoms.

In the present invention, "prevention" refers to inhibiting and delaying the onset of a disease, and includes not only prevention before the disease develops, but also prevention of recurrence of the disease after treatment.

In the present invention, "subject" refers to a vertebrate. In some embodiments, the vertebrate refers to a mammal. The mammal includes, but is not limited to, livestock (e.g., cattle), pets (e.g., cats, dogs, and horses), primates, mice, and rats. In some embodiments, the mammal refers to a human.

In the present invention, "effective amount" refers to an amount effective, at the dosage and for the duration necessary, to achieve the desired therapeutic effect. The "therapeutically effective amount" may vary depending on factors such as the individual's disease state, age, sex, and weight, and the ability of the active ingredient to elicit a desired response in the individual. A therapeutically effective amount also encompasses an amount in which any toxic or deleterious effects of the active ingredient are outweighed by the therapeutically beneficial effects. In cancer treatment, a therapeutically effective amount of a drug can reduce the number of cancer cells; shrink tumor volume; inhibit (i.e., slow down to a certain extent, preferably stop) cancer cell infiltration into surrounding organs; inhibit (i.e., slow down to a certain extent, preferably stop) tumor metastasis; inhibit tumor growth to a certain extent; and/or, alleviate one or more symptoms associated with cancer to a certain extent.

### Antibody-drug conjugates

One aspect of the present invention provides an antibody-drug conjugate as represented by Formula (I): 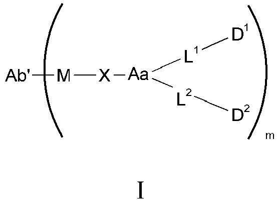 wherein,
Ab' represents an antibody or antigen-binding fragment thereof that specifically binds to B7H3;
M represents a linking site attached to the antibody or antigen-binding fragment thereof;
X represents a linker connecting M and Aa;
Aa is an amino acid fragment or a peptide fragment formed from two or more amino acids;
L¹ represents a linker connecting Aa and D¹;
L² represents a linker connecting Aa and D²;
D¹ represents a cytotoxic drug moiety;
D² represents a TLR agonist moiety;
m ranges from 1 to 10, for example, 1, 2, 3, 4, 5, 6, 7, 8, 9, or 10.

In some embodiments, m is 1 to 2, 1 to 3, 1 to 4, 1 to 5, 1 to 6, 1 to 7, 1 to 8, 1 to 9, 1 to 10, 1 to 16, 2 to 3, 2 to 4, 2 to 5, 2 to 6, 2 to 7, 2 to 8, 2 to 9, 2 to 10, 3 to 4, 3 to 5, 3 to 6, 3 to 7, 3 to 8, 3 to 9, 3 to 10, 4 to 5, 4 to 6, 4 to 7, 4 to 8, 4 to 9, 4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10.

In some embodiments, m is an integer.

In some embodiments, the antibody or antigen-binding fragment thereof comprises:
(1) the following heavy chain variable region (VH) and/or light chain variable region (VL):
   a heavy chain variable region (VH) comprising the following 3 CDRs: a CDR-H1 having a sequence as set forth in SEQ ID NO: 11 or a variant thereof, a CDR-H2 having a sequence as set forth in SEQ ID NO: 12 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(2) the following heavy chain variable region (VH) and/or light chain variable region (VL):
   a heavy chain variable region (VH) comprising the following 3 CDRs: a CDR-H1 having a sequence as set forth in SEQ ID NO: 14 or a variant thereof, a CDR-H2 having a sequence as set forth in SEQ ID NO: 15 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(3) the following heavy chain variable region (VH) and/or light chain variable region (VL):
   a heavy chain variable region (VH) comprising the following 3 CDRs: a CDR-H1 having a sequence as set forth in SEQ ID NO: 8 or a variant thereof, a CDR-H2 having a sequence as set forth in SEQ ID NO: 9 or a variant thereof, and a CDR-H3 having a sequence as set forth in SEQ ID NO: 10 or a variant thereof; and/or, a light chain variable region (VL) comprising the following 3 CDRs: a CDR-L1 having a sequence as set forth in SEQ ID NO: 3 or a variant thereof, a CDR-L2 having a sequence as set forth in SEQ ID NO: 4 or a variant thereof, and a CDR-L3 having a sequence as set forth in SEQ ID NO: 5 or a variant thereof; or
(4) the following heavy chain variable region (VH) and/or light chain variable region (VL):
   a heavy chain variable region (VH) comprising the following 3 CDRs: a CDR-H1 having a sequence as set forth in SEQ ID NO: 16 or a variant thereof, a CDR-H2 having a sequence as set forth in SEQ ID NO: 17 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof.

In some embodiments, the antibody or antigen-binding fragment thereof comprises:
(1) the following heavy chain variable region (VH) and/or light chain variable region (VL), wherein the CDRs are defined according to the Chothia numbering system:
   a heavy chain variable region (VH) comprising the following 3 CDRs: a CDR-H1 having a sequence as set forth in SEQ ID NO: 11 or a variant thereof, a CDR-H2 having a sequence as set forth in SEQ ID NO: 12 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(2) the following heavy chain variable region (VH) and/or light chain variable region (VL), wherein the CDRs are defined according to the Kabat numbering system:
   a heavy chain variable region (VH) comprising the following 3 CDRs: a CDR-H1 having a sequence as set forth in SEQ ID NO: 14 or a variant thereof, a CDR-H2 having a sequence as set forth in SEQ ID NO: 15 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(3) the following heavy chain variable region (VH) and/or light chain variable region (VL), wherein the CDRs are defined according to the IMGT numbering system:
   a heavy chain variable region (VH) comprising the following 3 CDRs: a CDR-H1 having a sequence as set forth in SEQ ID NO: 8 or a variant thereof, a CDR-H2 having a sequence as set forth in SEQ ID NO: 9 or a variant thereof, and a CDR-H3 having a sequence as set forth in SEQ ID NO: 10 or a variant thereof; and/or, a light chain variable region (VL) comprising the following 3 CDRs: a CDR-L1 having a sequence as set forth in SEQ ID NO: 3 or a variant thereof, a CDR-L2 having a sequence as set forth in SEQ ID NO: 4 or a variant thereof, and a CDR-L3 having a sequence as set forth in SEQ ID NO: 5 or a variant thereof; or
(4) the following heavy chain variable region (VH) and/or light chain variable region (VL), wherein the CDRs are defined according to the AbM numbering system:
   a heavy chain variable region (VH) comprising the following 3 CDRs: a CDR-H1 having a sequence as set forth in SEQ ID NO: 16 or a variant thereof, a CDR-H2 having a sequence as set forth in SEQ ID NO: 17 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof.

In some embodiments, the antibody or antigen-binding fragment thereof comprises: a VH as set forth in SEQ ID NO: 23 or a variant thereof, and/or, a VL as set forth in SEQ ID NO: 2 or a variant thereof;
preferably, the antibody or antigen-binding fragment thereof comprises: a heavy chain comprising a VH as set forth in SEQ ID NO: 23 or a variant thereof and a heavy chain constant region (CH) as set forth in SEQ ID NO: 18 or a variant thereof, and/or, a light chain comprising a VL as set forth in SEQ ID NO: 2 or a variant thereof and a light chain constant region (CL) as set forth in SEQ ID NO: 19 or a variant thereof;
preferably, the amino acid sequence of the heavy chain of the antibody or antigen-binding fragment thereof is set forth in SEQ ID NO: 22, and the amino acid sequence of the light chain of the antibody or antigen-binding fragment thereof is set forth in SEQ ID NO: 21.

In some embodiments, the antibody or antigen-binding fragment thereof comprises: a VH as set forth in SEQ ID NO: 1 or a variant thereof, and/or, a VL as set forth in SEQ ID NO: 2 or a variant thereof; preferably, the antibody or antigen-binding fragment thereof comprises: a heavy chain comprising a VH as set forth in SEQ ID NO: 1 or a variant thereof and a heavy chain constant region (CH) as set forth in SEQ ID NO: 18 or a variant thereof, and/or, a light chain comprising a VL as set forth in SEQ ID NO: 2 or a variant thereof and a light chain constant region (CL) as set forth in SEQ ID NO: 19 or a variant thereof; preferably, the amino acid sequence of the heavy chain of the antibody or antigen-binding fragment thereof is set forth in SEQ ID NO: 20, and the amino acid sequence of the light chain of the antibody or antigen-binding fragment thereof is set forth in SEQ ID NO: 21.

In some embodiments, the N-terminal glutamine of the heavy chain or heavy chain variable region and/or the light chain or light chain variable region undergoes cyclization to form pyroglutamic acid or pyroglutamate; and/or, the C-terminal lysine is absent from the heavy chain or heavy chain constant region (CH) or variant thereof.

In some embodiments, the variant has a sequence identity of at least 80%, at least 85%, at least 90%, at least 91%, at least 92%, at least 93%, at least 94%, at least 95%, at least 96%, at least 97%, at least 98%, at least 99%, or 100% as compared with the sequence from which it is derived, or has a substitution, deletion, or addition of one or more amino acids (e.g., a substitution, deletion, or addition of 1, 2, 3, 4, or 5 amino acids) as compared with the sequence from which it is derived; preferably, the substitution is a conservative substitution.

In some embodiments, Formula (I) is further represented by 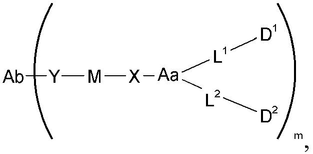 wherein Ab-(Y- represents the antibody or antigen-binding fragment thereof as described above, and -(Y- represents a linkage between an amino acid residue in the antibody or antigen-binding fragment thereof and M.

In some embodiments, the amino acid residue is a cysteine, lysine, serine, or threonine residue.

In some embodiments, Y is S, NH, or O.

In some embodiments, M is a covalent bond or is selected from the group consisting of the following structures: 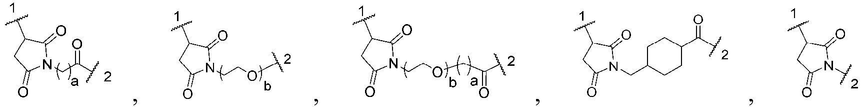 and 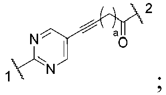 wherein, each a is independently an integer ranging from 0 to 6, b is an integer ranging from 1 to 10, and the position 1 of M is connected to Ab' and the position 2 is connected to X.

In some embodiments, each a is independently selected from the group consisting of 0, 1, 2, 3, 4, 5, and 6. In some embodiments, each a is independently selected from the group consisting of 1, 2, 3, 4, 5, and 6. In some embodiments, each a is independently selected from the group consisting of 2, 3, 4, 5, and 6.

In some embodiments, b is selected from the group consisting of 1, 2, 3, 4, 5, 6, 7, 8, 9, and 10.

In some embodiments, M is a covalent bond or is selected from the group consisting of the following structures: 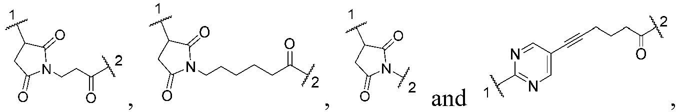 wherein, the position 1 of M is connected to Ab' and the position 2 is connected to X.

In some embodiments, X is a covalent bond or is selected from the group consisting of C₁₋₆ alkylene, -NH-(CH₂)_{c}-C(O)-, 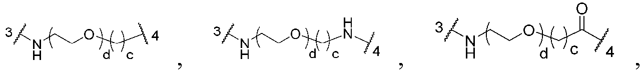 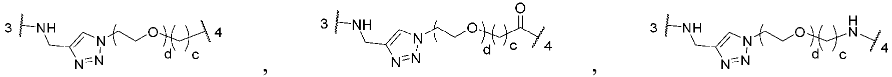 and 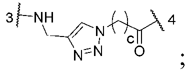 wherein, each c is independently an integer ranging from 0 to 6, and each d is independently an integer ranging from 1 to 10; the position 3 of X is connected to M, and the position 4 is connected to Aa.

In some embodiments, each c is independently selected from the group consisting of 0, 1, 2, 3, 4, 5, and 6. In some embodiments, each c is independently selected from the group consisting of 1, 2, 3, 4, 5, and 6. In some embodiments, c is 2.

In some embodiments, each d is independently selected from the group consisting of 1, 2, 3, 4, 5, 6, 7, 8, 9, and 10.

In some embodiments, X is a covalent bond or 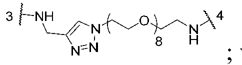 wherein the position 3 of X is connected to M and the position 4 is connected to Aa.

In some embodiments, X is a covalent bond.

In some embodiments, X is 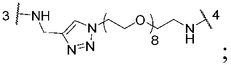 wherein the position 3 of X is connected to M and the position 4 is connected to Aa.

In some embodiments, Aa is selected from the group consisting of the following amino acid fragments: Gly, Phe, Ala, Val, Cys, Asp, Glu, Lys, Nle, and Arg.

In some embodiments, Aa is selected from the group consisting of the following amino acid fragments: Cys, Asp, Glu, Lys, and Nle.

In some embodiments, Aa is selected from the group consisting of the following structures: 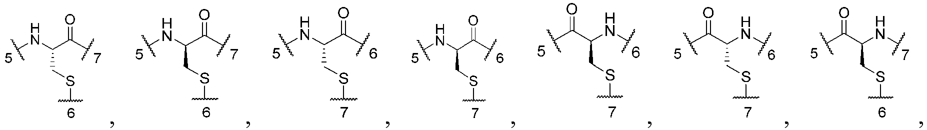 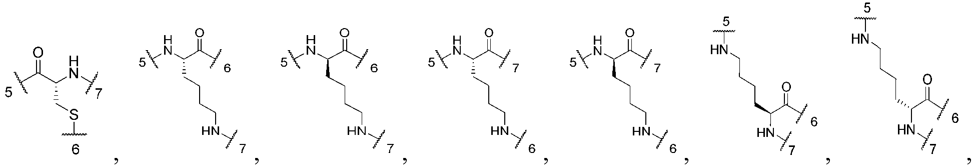 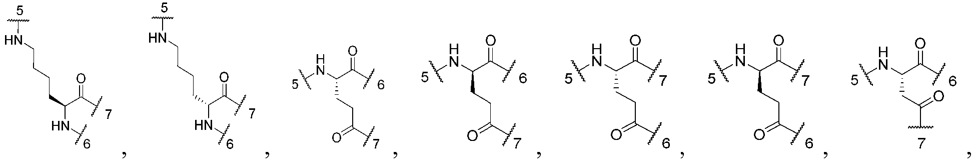 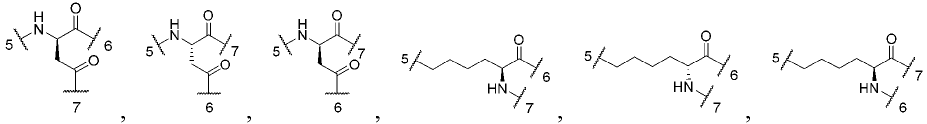 and 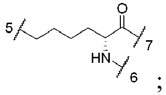 wherein, the position 5 of Aa is connected to X, the position 6 is connected to L¹, and the position 7 is connected to L².

In some embodiments, L¹ is a covalent bond or selected from the group consisting of uncleavable linkers and cleavable linkers. The cleavable linker is cleavable by an enzyme present in a pathological environment, and the enzyme is selected from the group consisting of a protease, a phosphatase, a pyrophosphatase, *β*-glucuronidase, *β*-galactosidase, and a sulfatase.

In some embodiments, L¹ is -L^{a}-L^{b}-L^{c}-, wherein:
L^{a} is a covalent bond or selected from the group consisting of C₁₋₆ alkylene, -NH-(CH₂)ₑ-C(O)-, 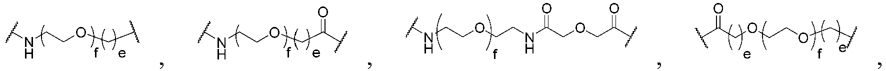 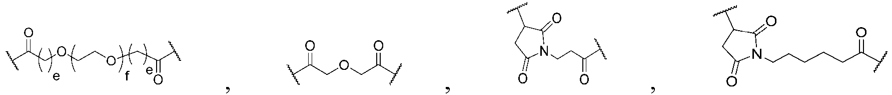 and 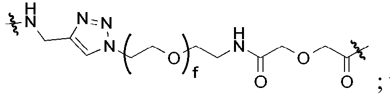 wherein, each e is independently an integer ranging from 0 to 6, and each f is independently an integer ranging from 1 to 10.
L^{b} is a covalent bond or selected from the group consisting of an amino acid fragment and a peptide fragment formed from two or more amino acids, wherein the amino acids are selected from the group consisting of Val, Cit, Glu, Lys, Arg, Phe, Leu, Gly, Ala, and Asn;
L° is a covalent bond or selected from the group consisting of -NH-CH₂- and the following structures:

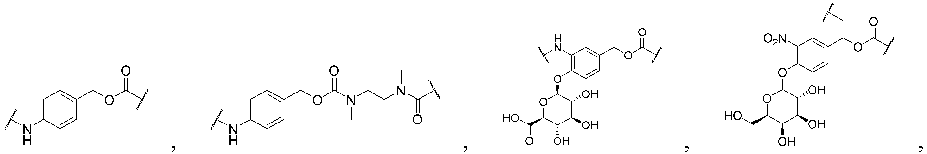

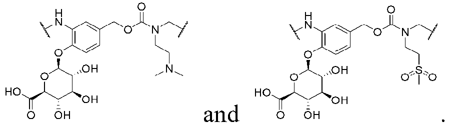

In some embodiments, L^{a} is a covalent bond or selected from the group consisting of C₁₋₆ alkylene, -NH-(CH₂)ₑ-C(O)-,

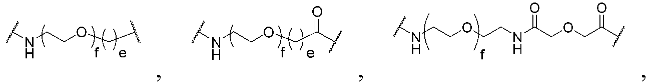

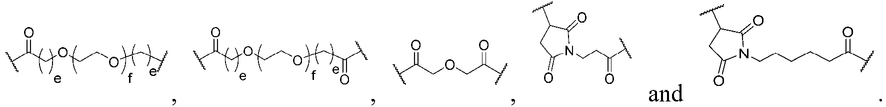

In some embodiments, L^{a} is selected from the group consisting of a covalent bond,

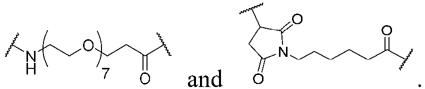

In some embodiments, L^{a} is a covalent bond.

In some embodiments, each e is independently selected from the group consisting of 0, 1, 2, 3, 4, 5, and 6. In some embodiments, each e is independently selected from the group consisting of 1, 2, 3, 4, 5, and 6. In some embodiments, e is 2.

In some embodiments, each f is independently selected from the group consisting of 1, 2, 3, 4, 5, 6, 7, 8, 9, and 10.

In some embodiments, L^{b} is selected from the group consisting of Val-Cit, Ala-Ala-Ala, Val-Lys-Gly, and Gly-Gly-Phe-Gly (SEQ ID NO: 24).

In some embodiments, L^{b} is a covalent bond or is selected from the group consisting of the following structures:

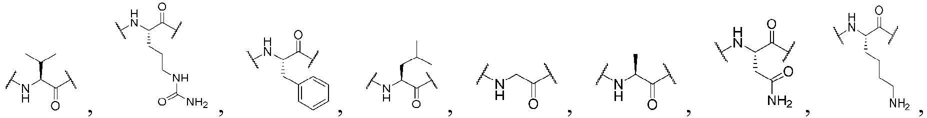

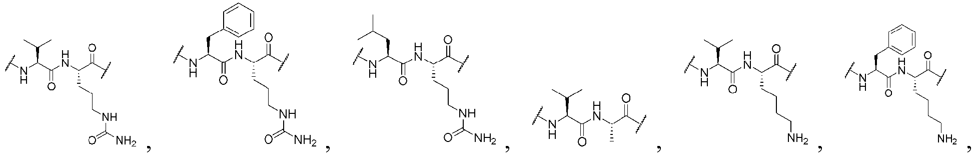

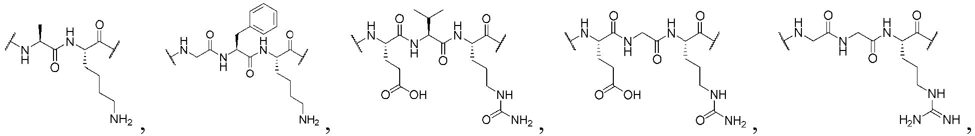

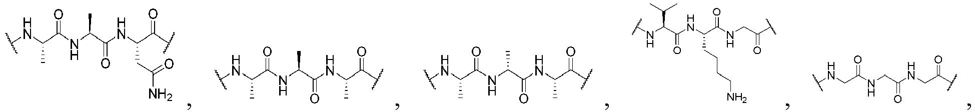

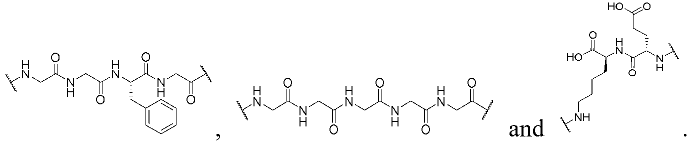

In some embodiments, L° is -NH-CH₂- or

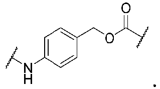

In some embodiments, L¹ is a covalent bond or is selected from the group consisting of the following structures: 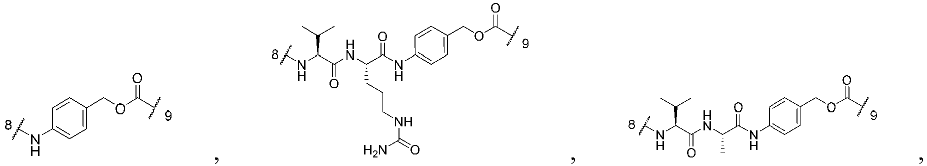 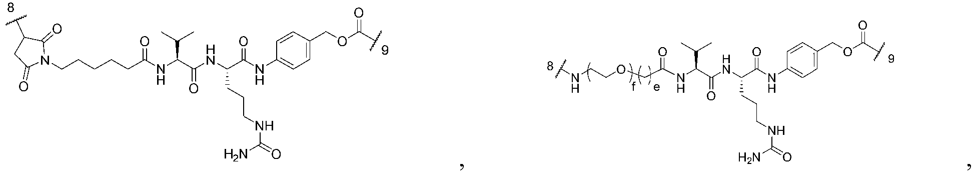 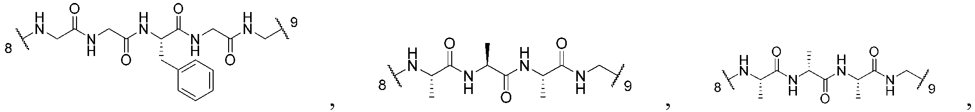 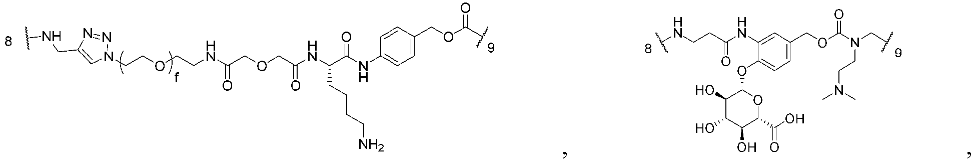 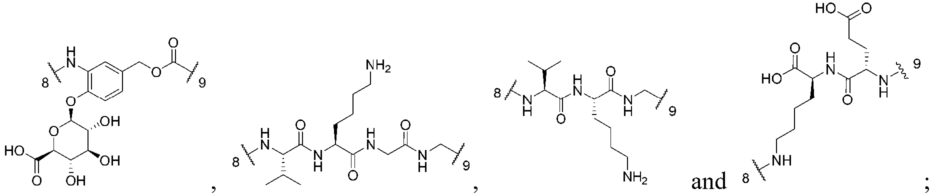 wherein, each e is independently an integer ranging from 0 to 6, and each f is independently an integer ranging from 1 to 10; the position 8 of L¹ is connected to Aa, and the position 9 is connected to D¹.

In some embodiments, L¹ is: 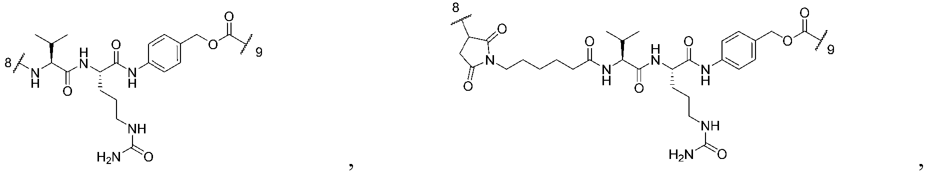 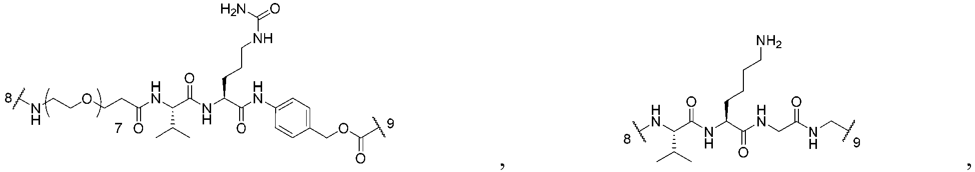 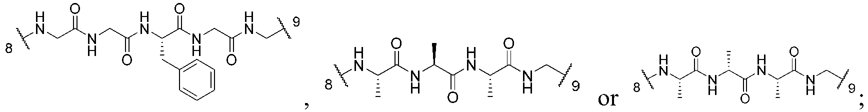 wherein, the position 8 of L¹ is connected to Aa, and the position 9 is connected to D¹.

In some embodiments, L² is selected from the group consisting of non-cleavable linkers and cleavable linkers. The cleavable linker is cleavable by an enzyme present in a pathological environment, and the enzyme is selected from the group consisting of a protease, a phosphatase, a pyrophosphatase, *β*-glucuronidase, *β*-galactosidase, and a sulfatase.

In some embodiments, L² is selected from the group consisting of the following structures: 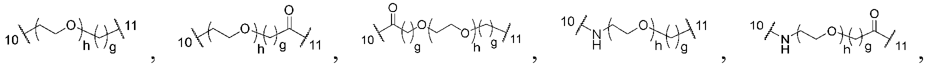 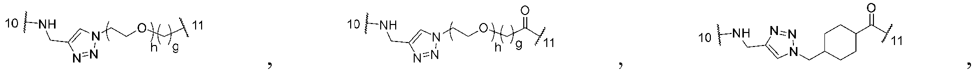 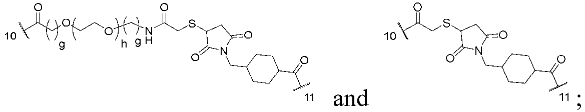 wherein each g is independently an integer ranging from 0 to 6, and each h is independently an integer ranging from 1 to 10; the 10 position of L² is connected to Aa, and the 11 position is connected to D².

In some embodiments, each g is independently selected from the group consisting of 0, 1, 2, 3, 4, 5, and 6. In some embodiments, each g is independently selected from the group consisting of 1, 2, 3, 4, 5, and 6. In some embodiments, g is 2.

In some embodiments, each h is independently selected from the group consisting of 1, 2, 3, 4, 5, 6, 7, 8, 9, and 10.

In some embodiments, L² is selected from the group consisting of the following structures: 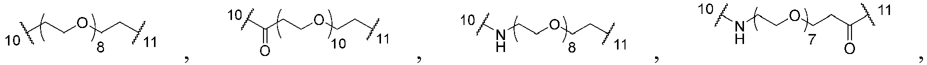 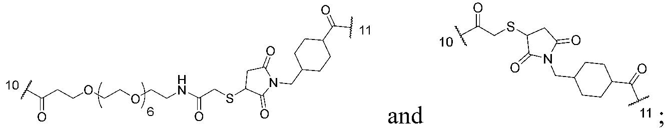 wherein, the 10 position of L² is connected to Aa, and the 11 position is connected to D².

In some embodiments, D¹ is selected from the group consisting of cytotoxic drug moieties. In some embodiments, the cytotoxic drug includes but is not limited to tubulin inhibitors, DNA damaging agents and topoisomerase inhibitors. In some embodiments, the tubulin inhibitor includes but is not limited to dolastatin and auristatins, maytansines, tubulysins and cryptomycins. In some embodiments, the tubulin inhibitor is selected from the group consisting of dolastatin 10, MMAE, MMAF, maytansine, DM1, DM3 and DM4. In some embodiments, the topoisomerase inhibitor includes but is not limited to camptothecin and its derivatives, epipodophyllotoxins, anthracyclines and anthraquinones. In some embodiments, the topoisomerase inhibitor is selected from the group consisting of camptothecin, SN-38, exatecan, topotecan, belotecan, rubitecan, diflomotecan, 10-hydroxycamptothecin, 9-aminocamptothecin, Dxd, T030, doxorubicin, epirubicin, and PNU-159682. In some embodiments, the DNA damaging agent includes but is not limited to pyrrolobenzodiazepines, duocarmycins, and calicheamicins.

In some embodiments, D¹ is selected from the group consisting of the following structures:

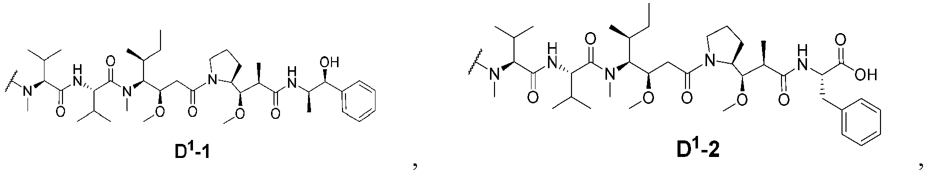

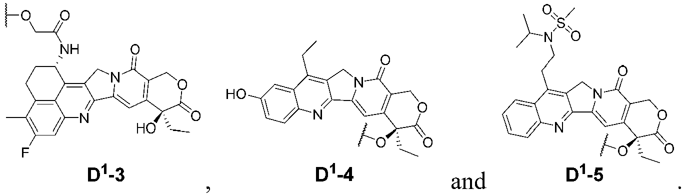

In some embodiments, D² is a TLR agonist moiety, and the TLR agonist is selected from the group consisting of a TLR2 agonist, a TLR4 agonist, a TLR6 agonist, a TLR7 agonist, a TLR8 agonist, a TLR7/8 agonist, and a TLR9 agonist. In some embodiments, the TLR agonist is selected from the group consisting of a TLR7 agonist, a TLR8 agonist, and a TLR7/8 agonist.

In some embodiments, D² is selected from the group consisting of the following structures:

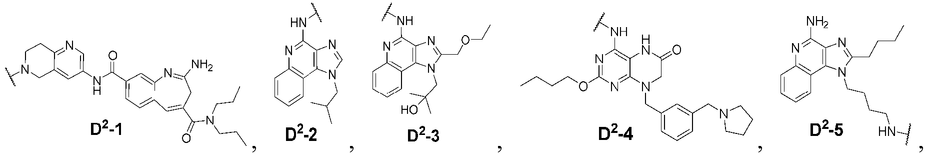

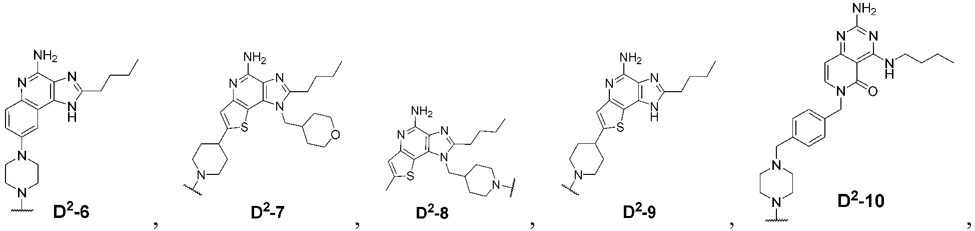

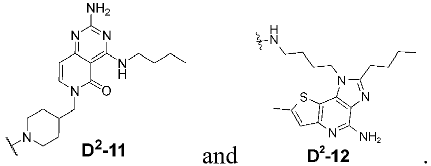

In some embodiments, 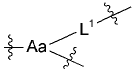 is selected from the group consisting of: 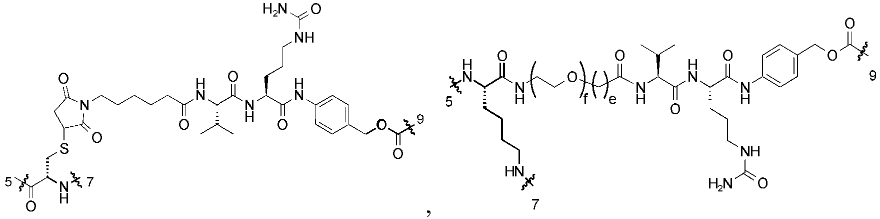 (for example, 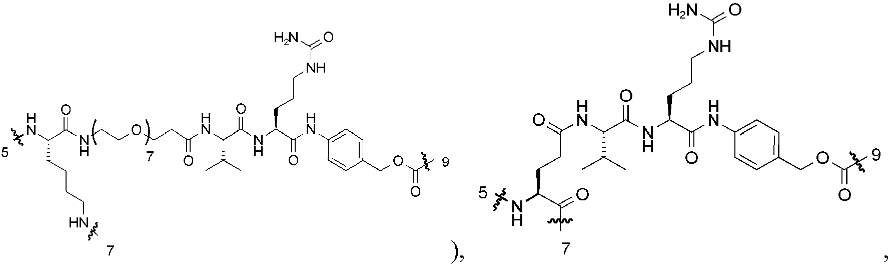 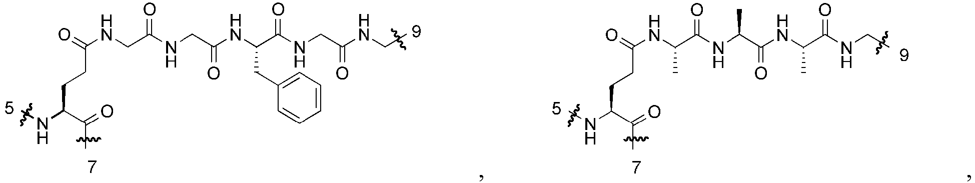 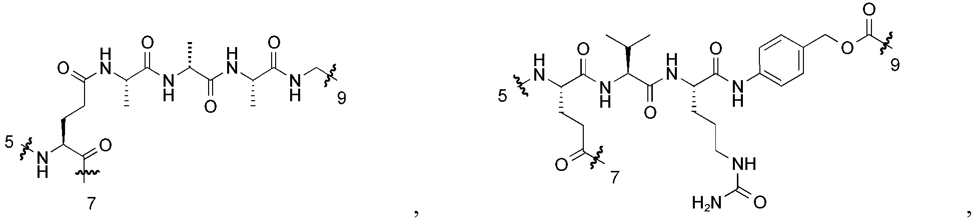 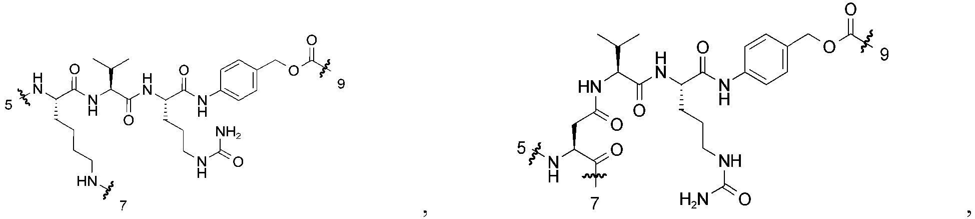 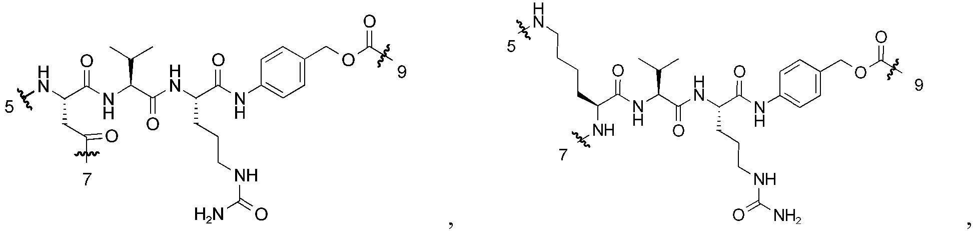 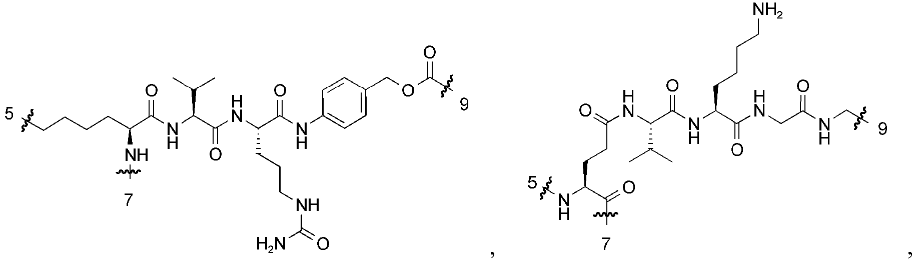 and 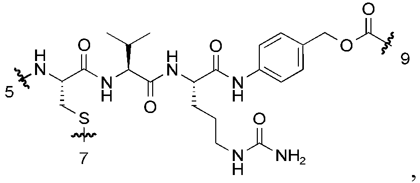 wherein, e is an integer ranging from 0 to 6, f is an integer ranging from 1 to 10; the position 5 is connected to X, the position 7 is connected to L², and the position 9 is connected to D¹.

In some embodiments, 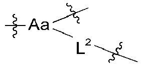 is selected from the group consisting of: 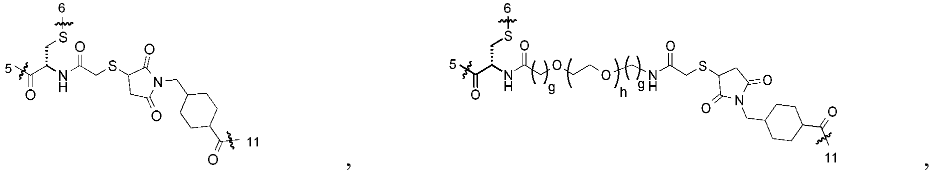 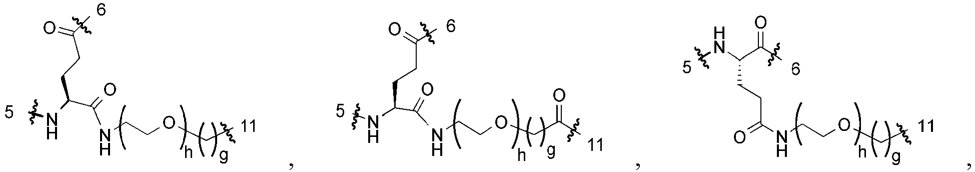 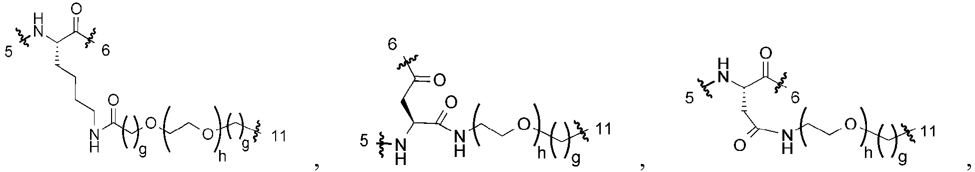 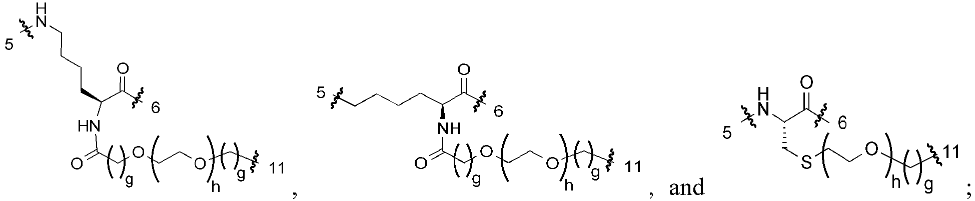 wherein, each g is independently an integer ranging from 0 to 6, each h is independently an integer ranging from 1 to 10; the position 5 is connected to X, the position 6 is connected to L¹, and the position 11 is connected to D².

In some embodiments, 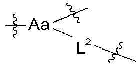 is selected from the group consisting of: 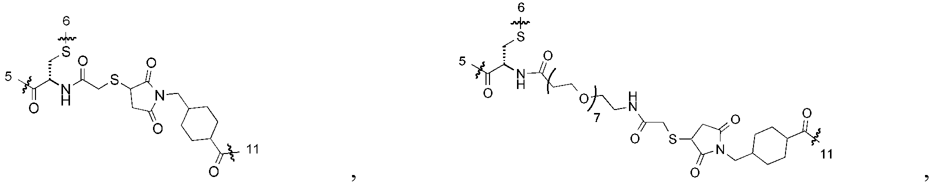 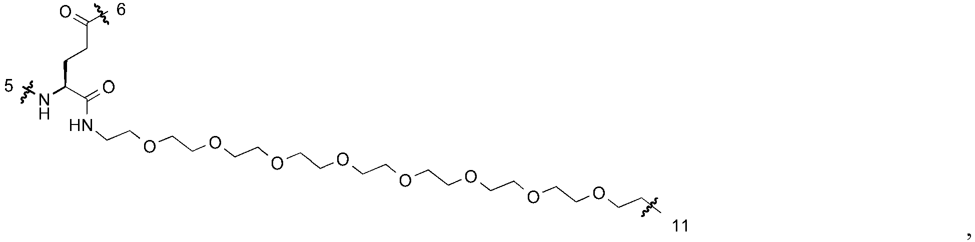 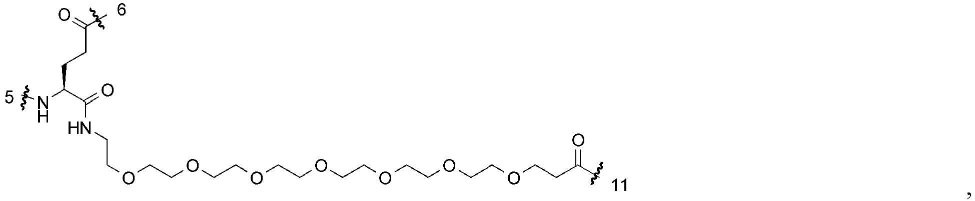 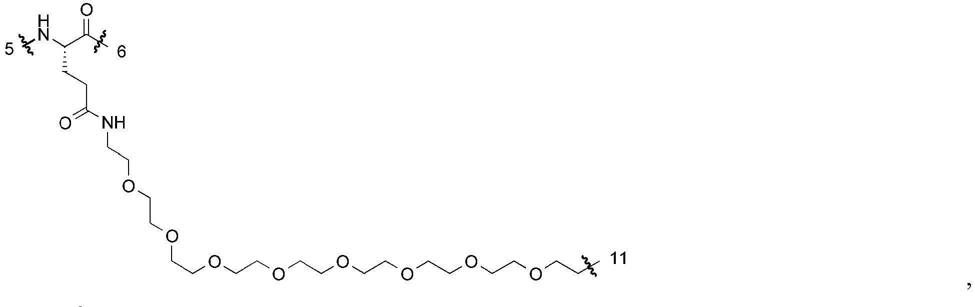 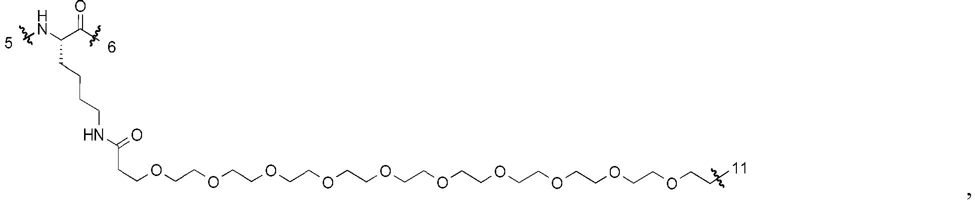 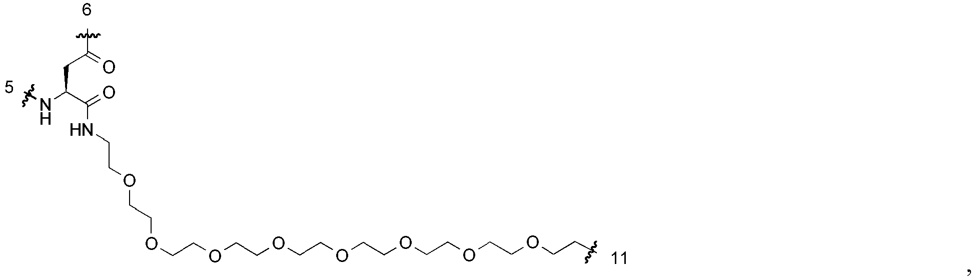 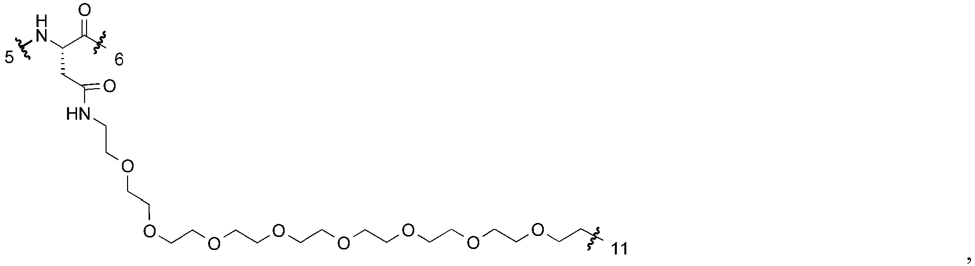 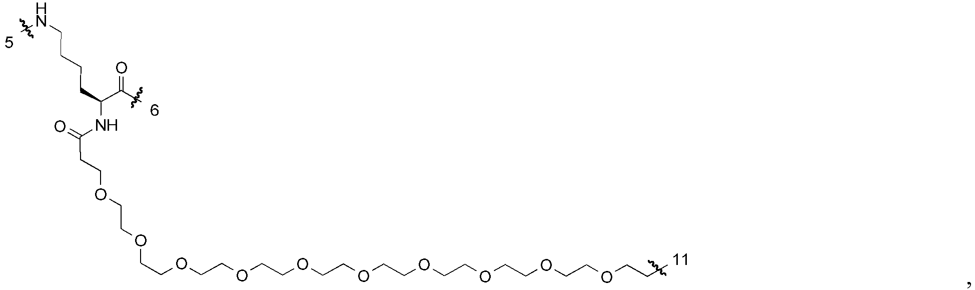 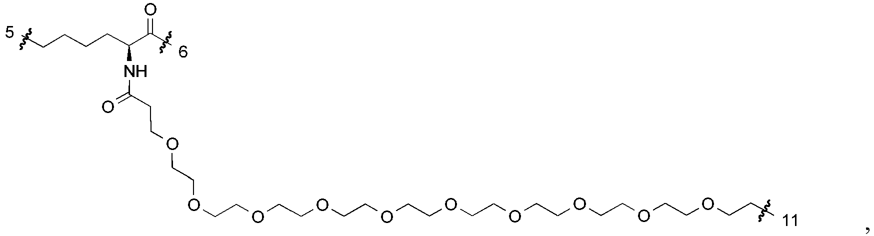 and 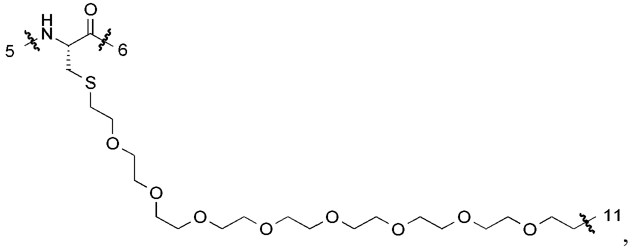 wherein, the position 5 is connected to X, the position 6 is connected to L¹, and the position 11 is connected to D².

In some embodiments, 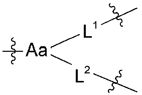 is selected from the group consisting of: 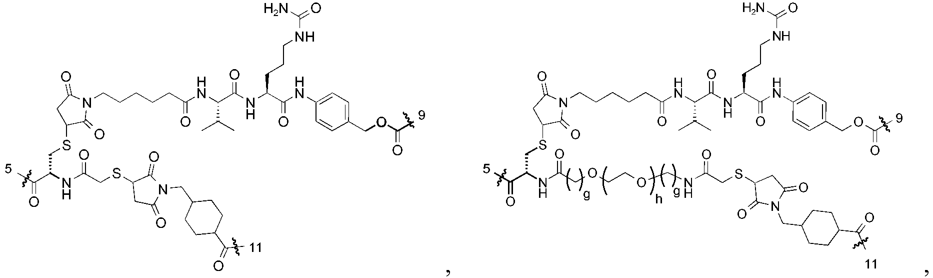 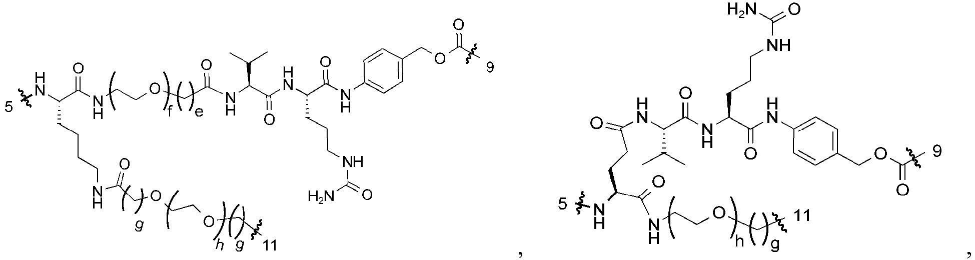 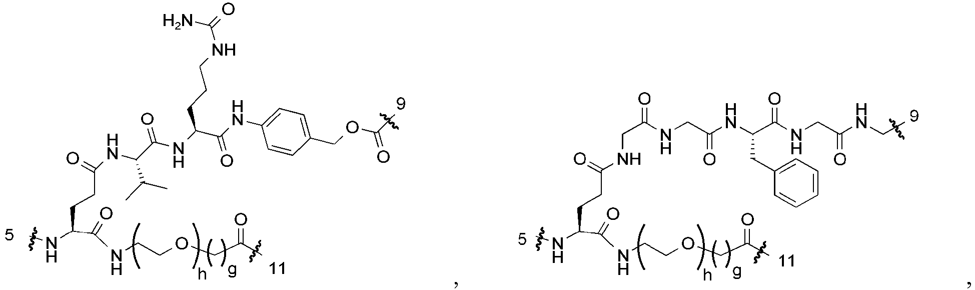 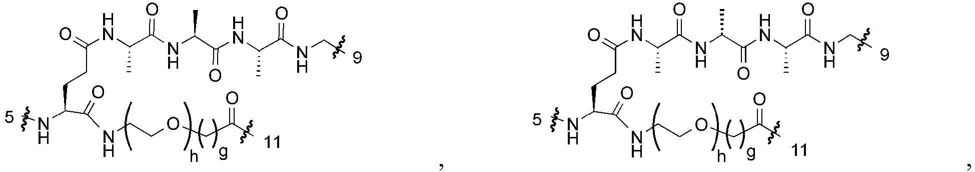 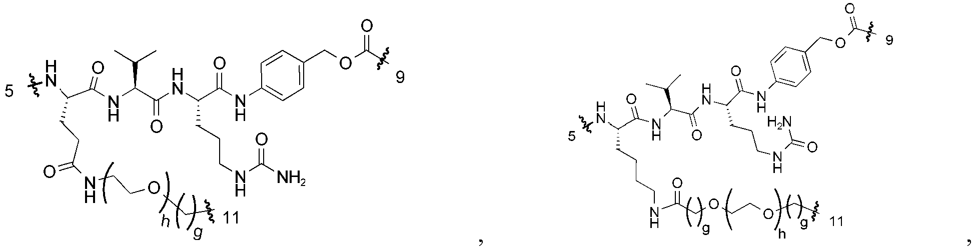 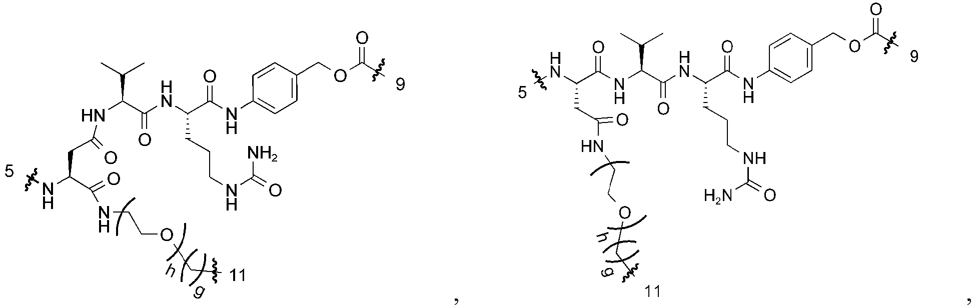 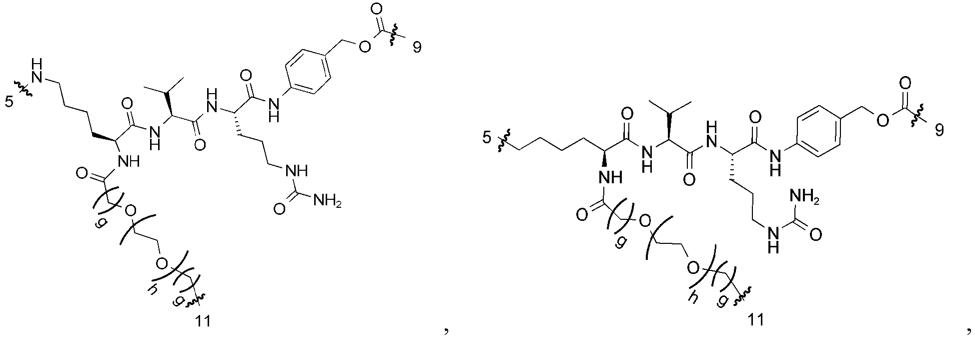 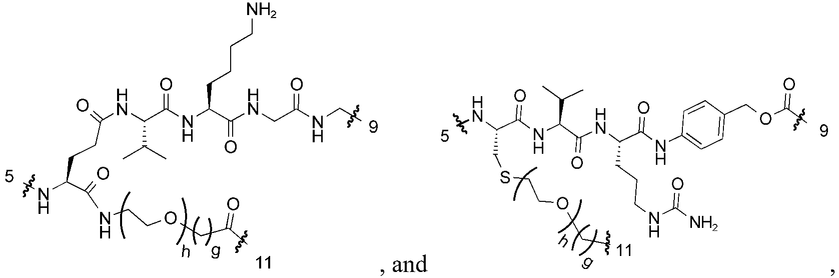 wherein, each g is independently an integer ranging from 0 to 6, each h is independently an integer ranging from 1 to 10; the position 5 is connected to X, the position 9 is connected to D¹, and the position 11 is connected to D².

In some embodiments, 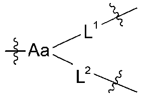 is selected from the group consisting of: 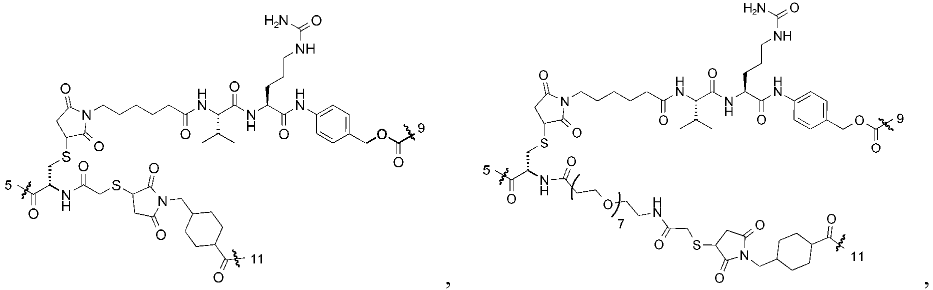 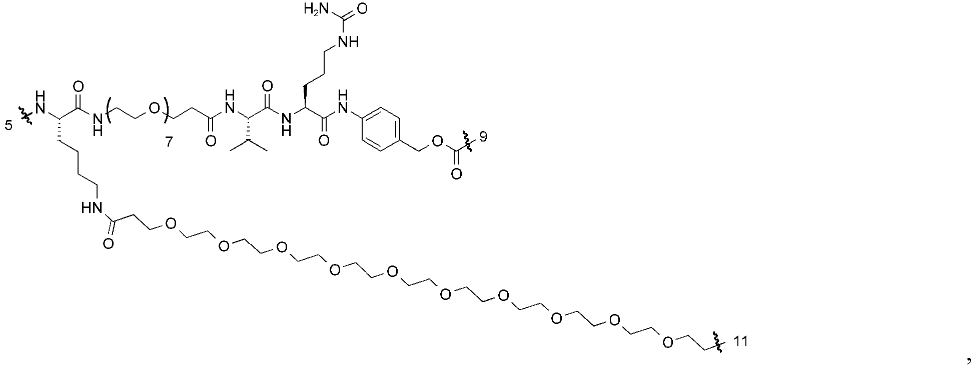 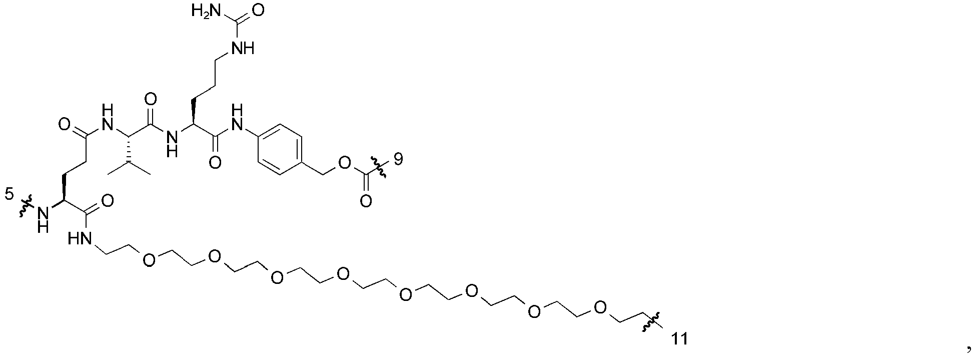 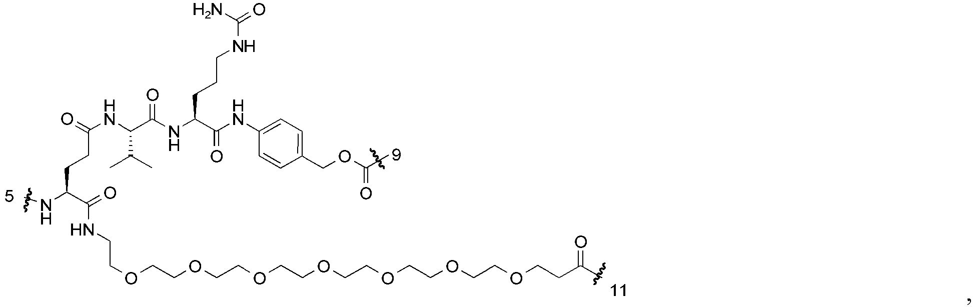 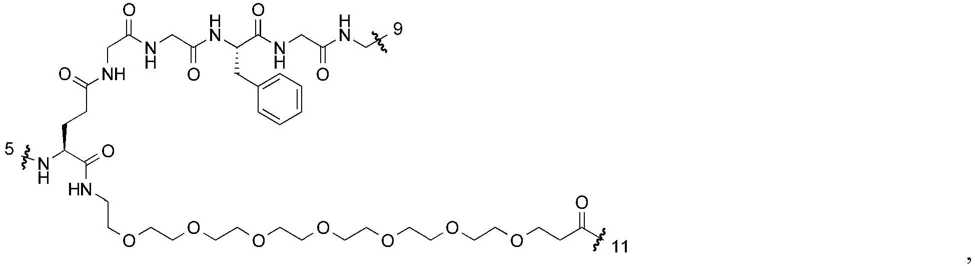 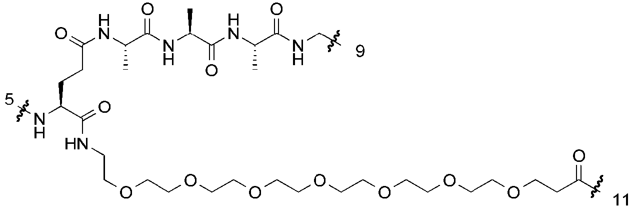 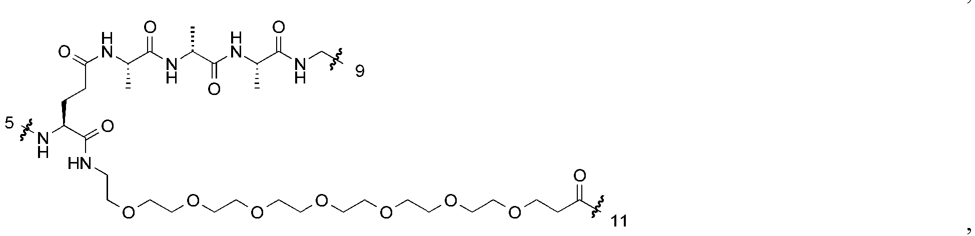 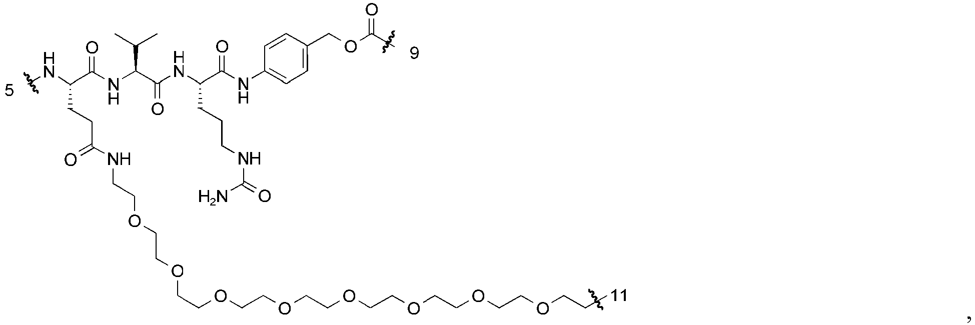 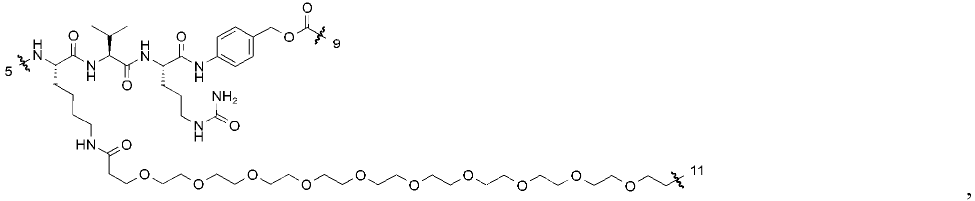 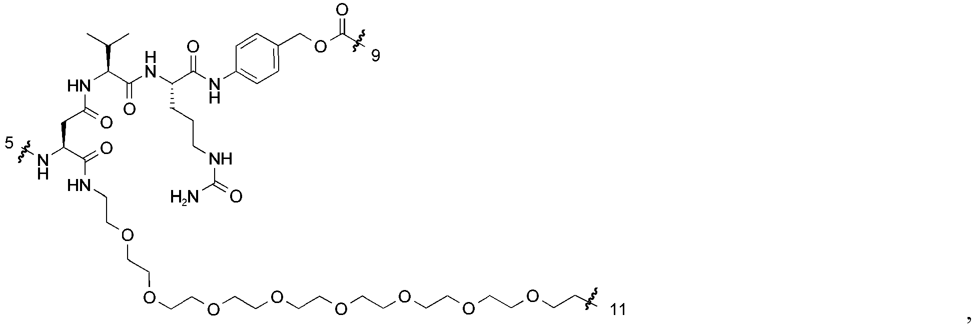 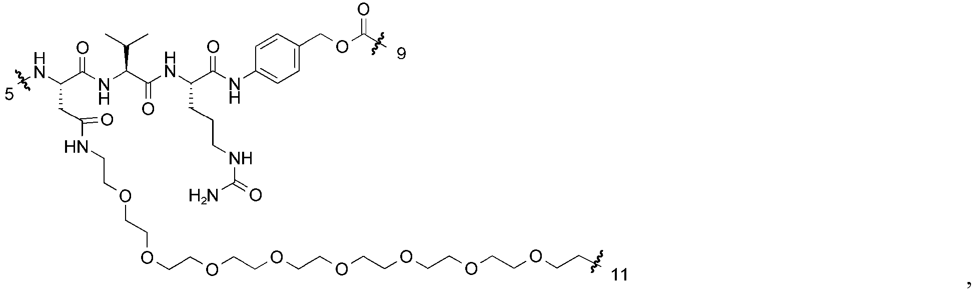 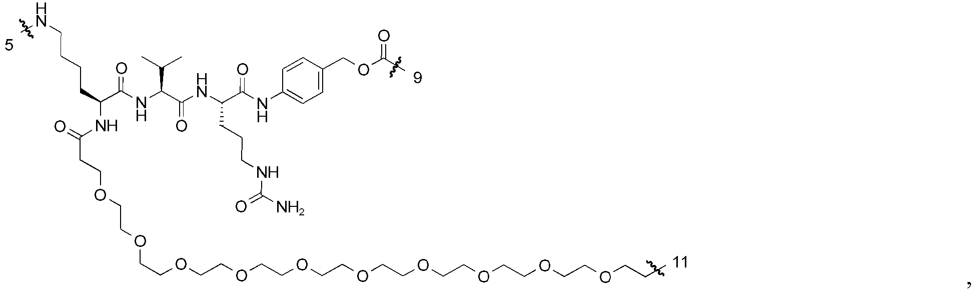 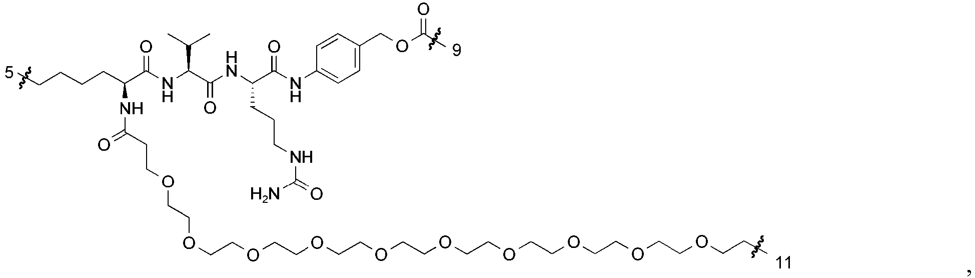 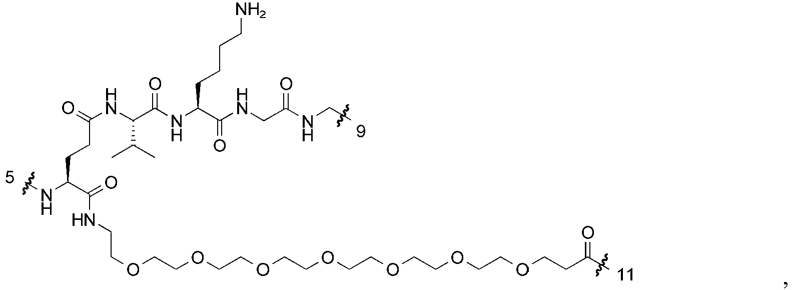 and 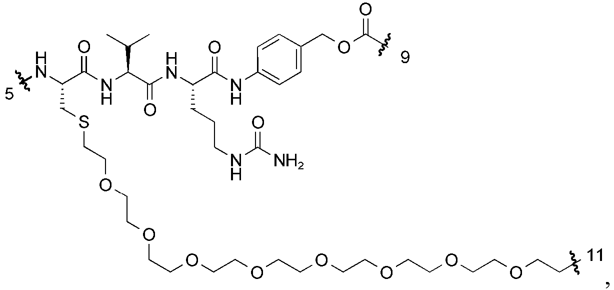 wherein the position 5 is connected to X, the position 9 is connected to D¹, and the position 11 is connected to D².

In some embodiments, 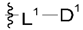 is selected from the group consisting of: 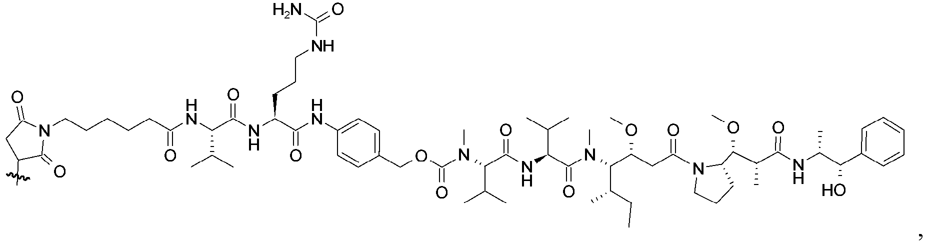 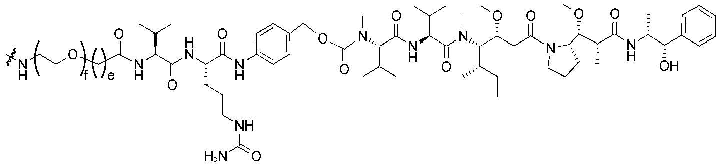 (for example, 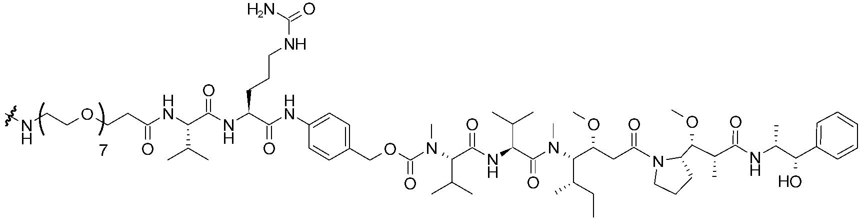 ), 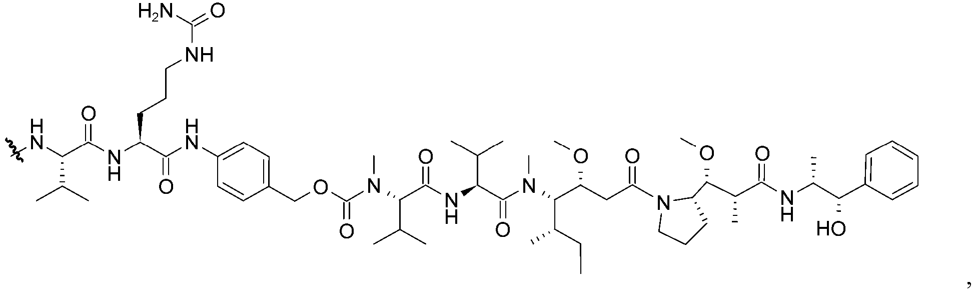 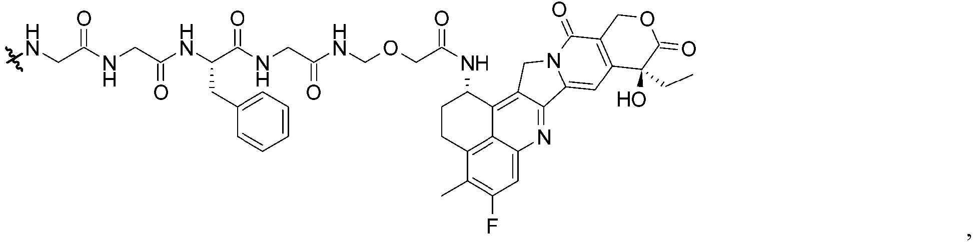 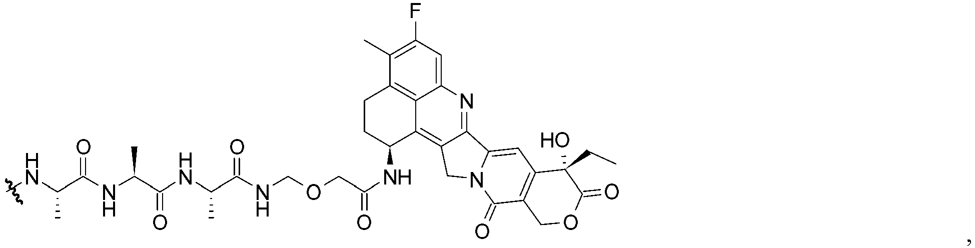 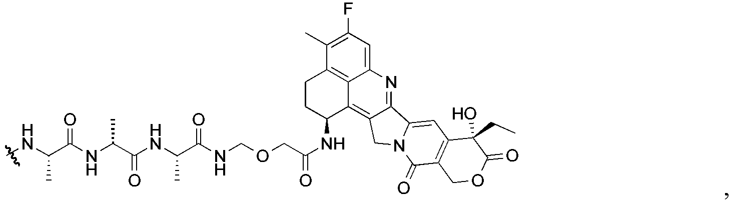 and 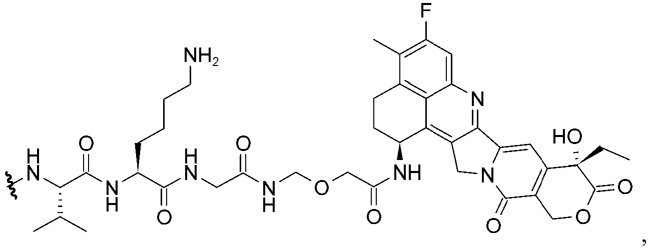 wherein, each e is independently an integer ranging from 0 to 6, and each f is independently an integer ranging from 1 to 10.

In some embodiments, 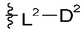 is selected from the group consisting of: 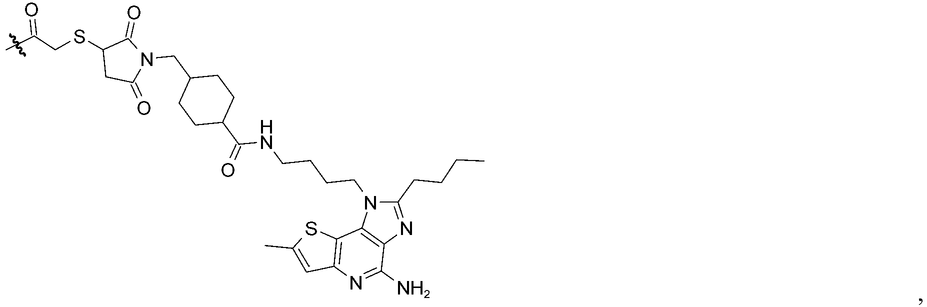 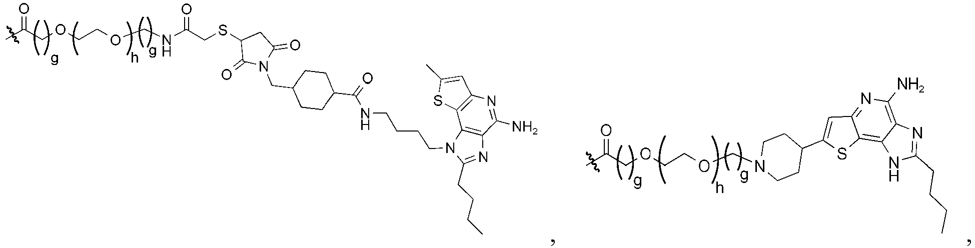 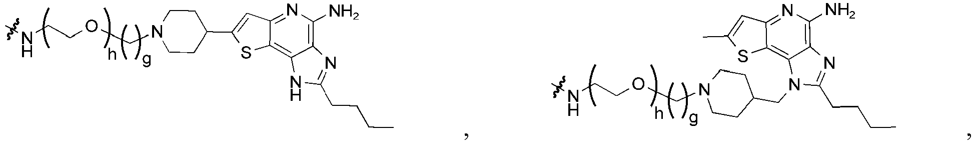 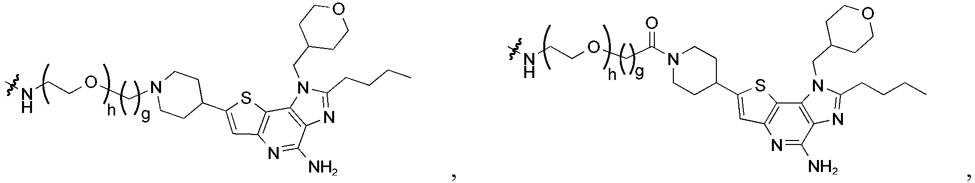 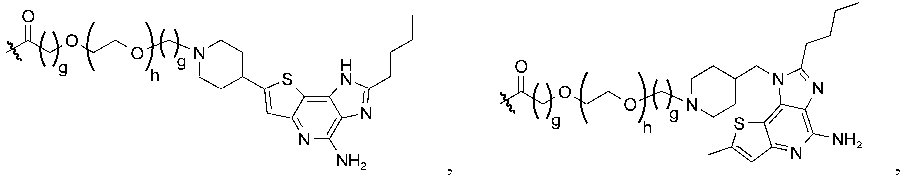 and 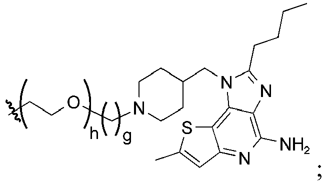 wherein, each g is independently an integer ranging from 0 to 6, and each h is independently an integer ranging from 1 to 10.

In some embodiments, 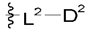 is selected from the group consisting of: 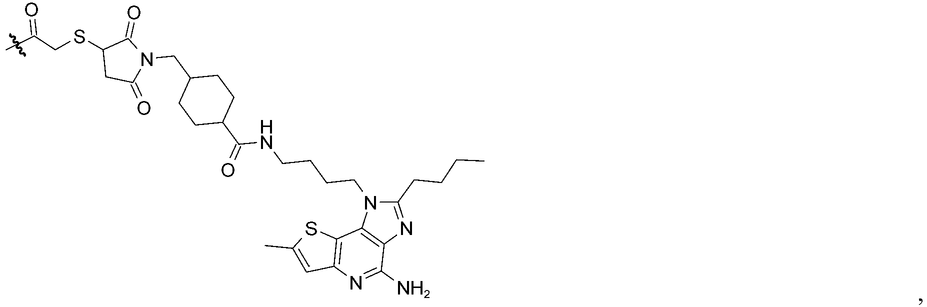 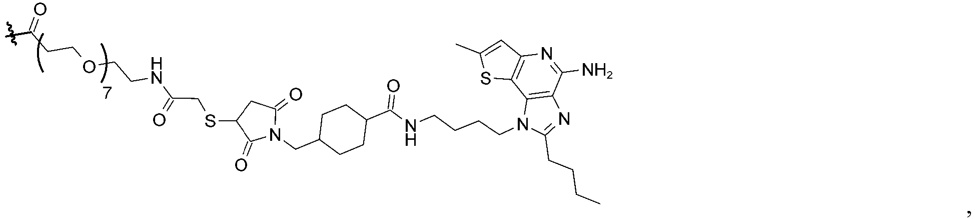 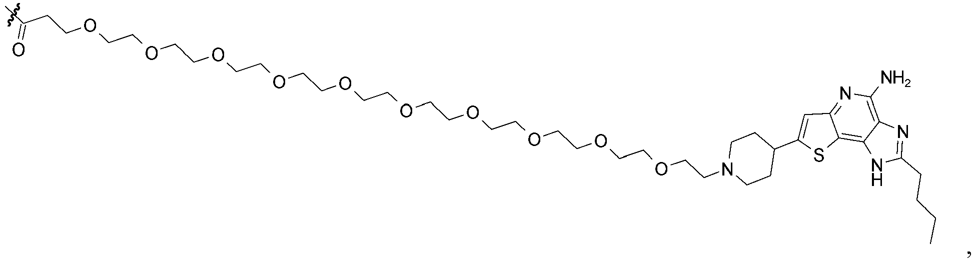 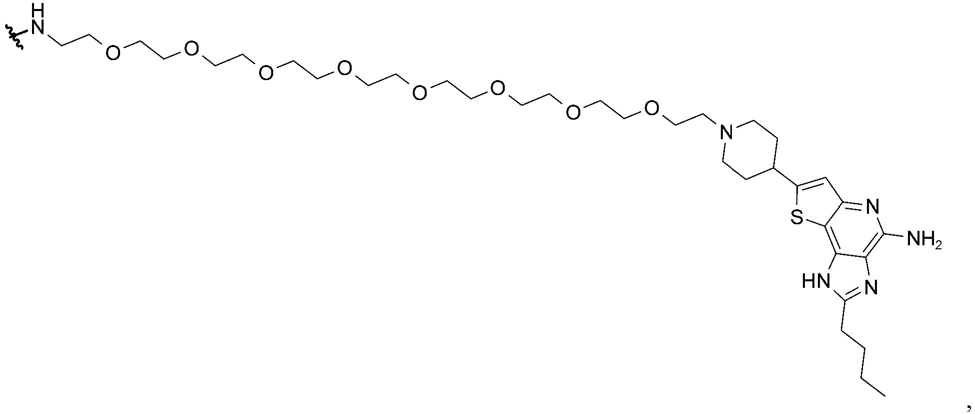 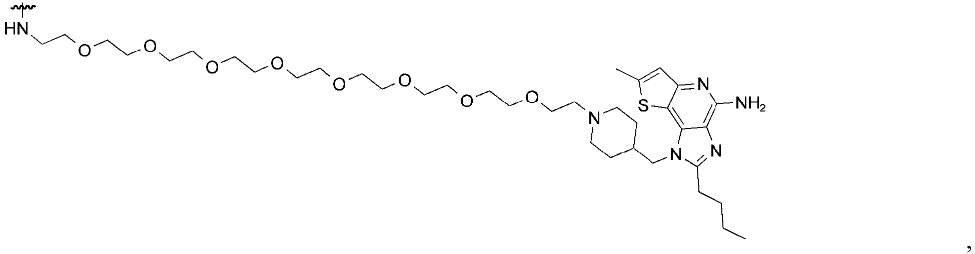 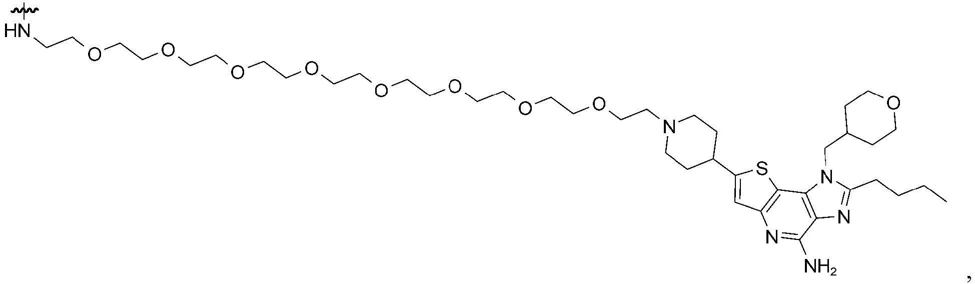 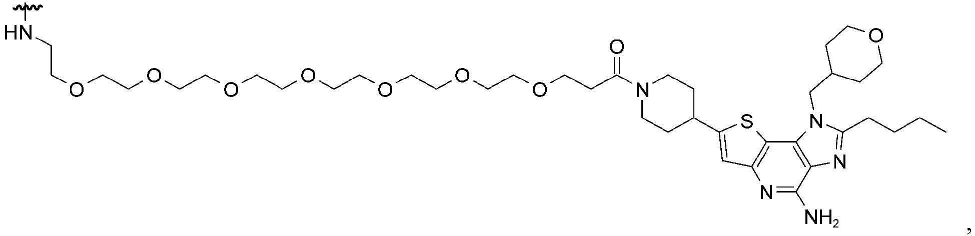 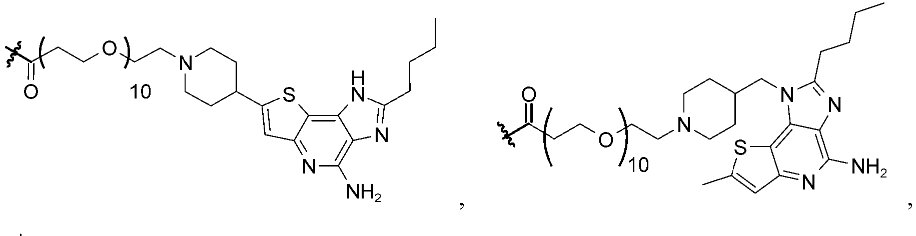 and

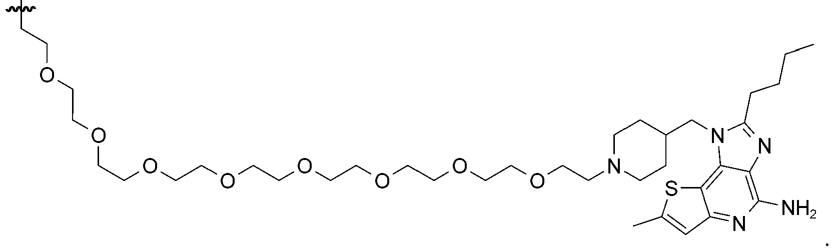

In some embodiments, 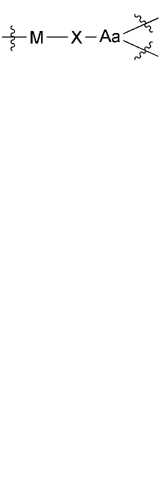 is selected from the group consisting of: 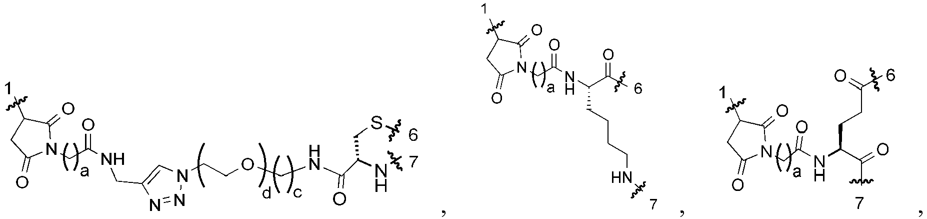 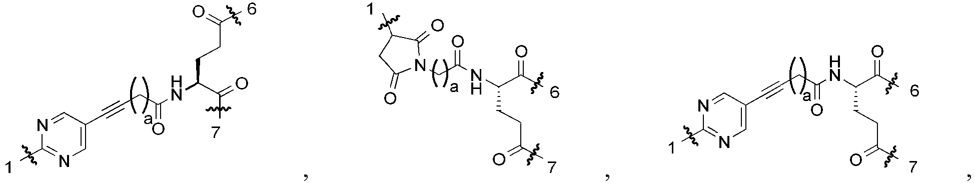 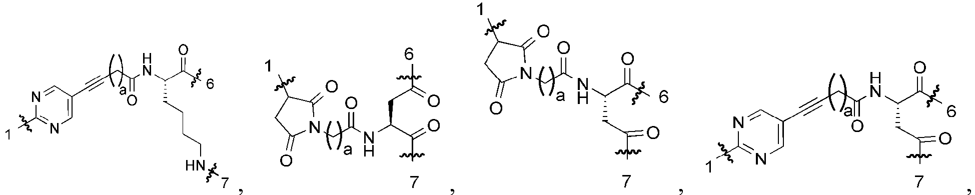 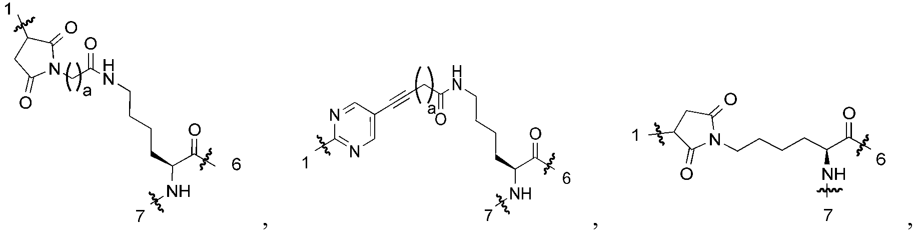 and 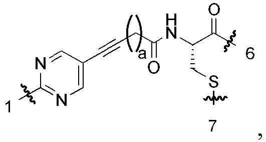 wherein, each a is independently an integer ranging from 0 to 6, each c is independently an integer ranging from 0 to 6, and each d is independently an integer ranging from 1 to 10; the position 1 is connected to Ab', the position 6 is connected to L¹, and the position 7 is connected to L².

In some embodiments, 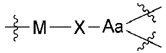 is selected from the group consisting of: 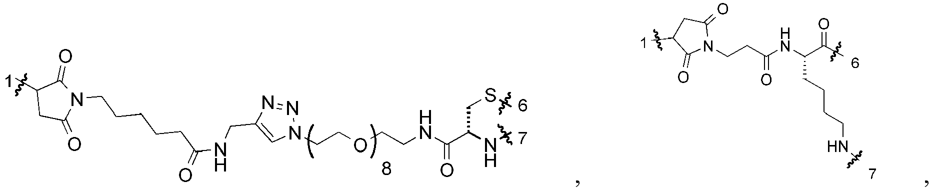 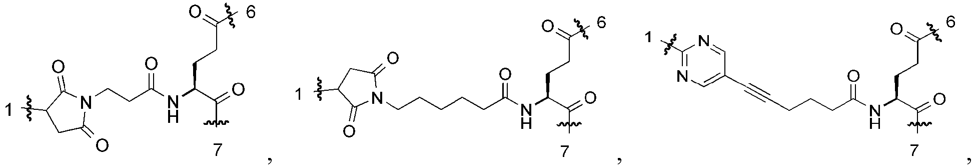 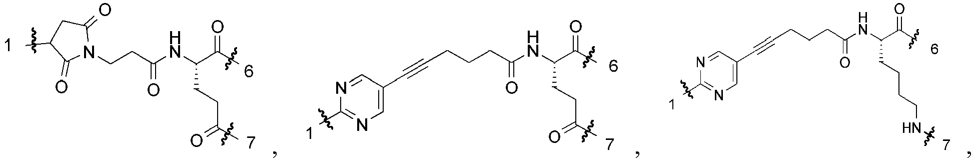 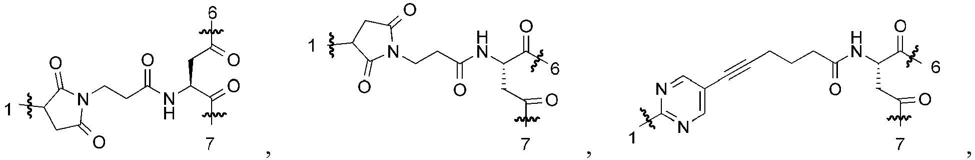 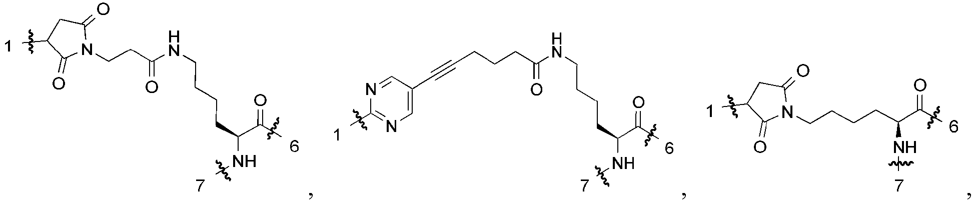 and 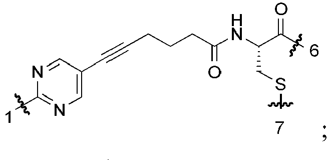 wherein, the position 1 is connected to Ab', the position 6 is connected to L¹, and the position 7 is connected to L².

In some embodiments, 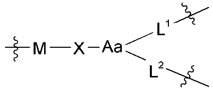 is selected from the group consisting of: 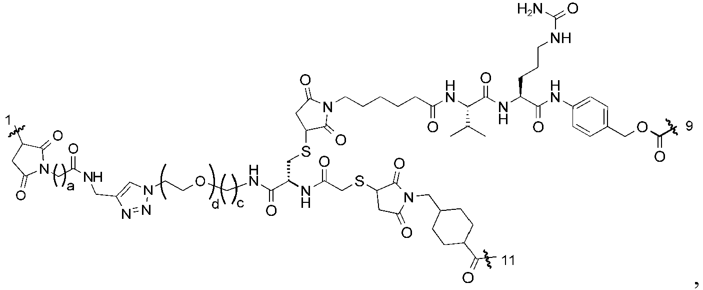 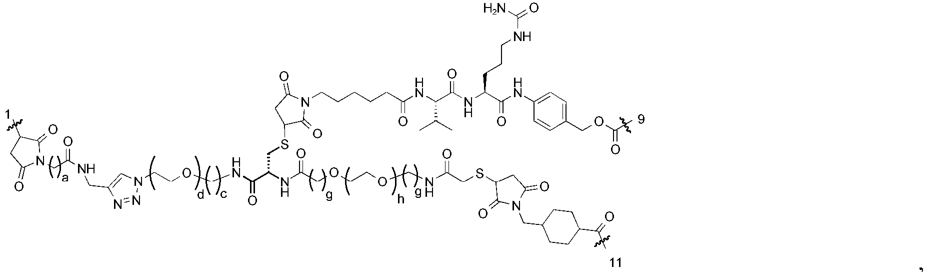 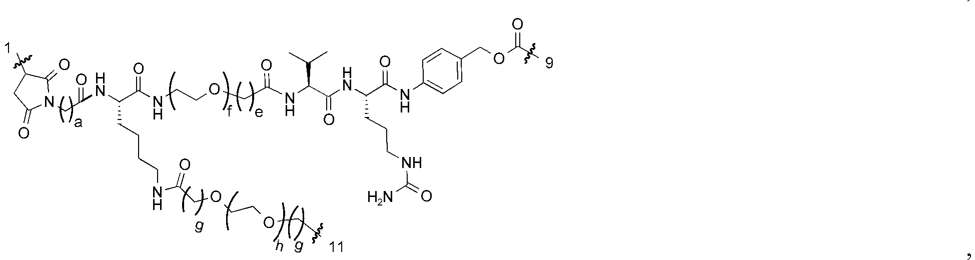 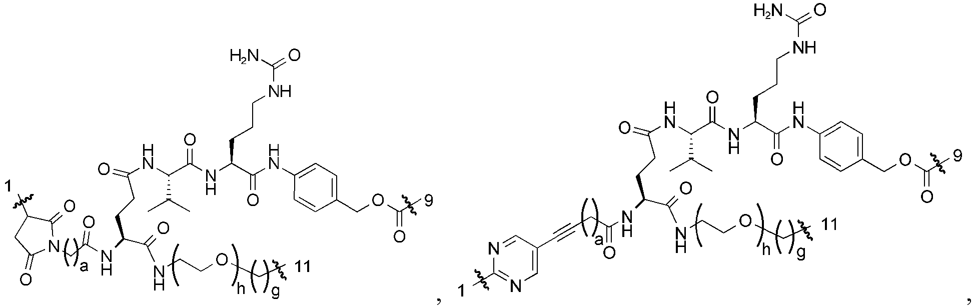 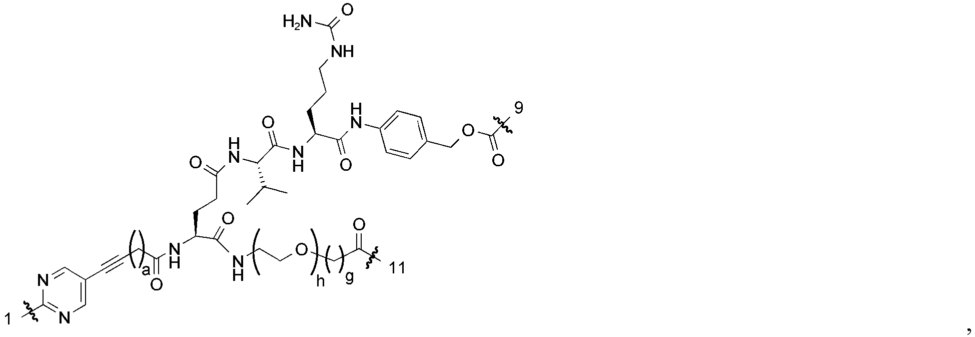 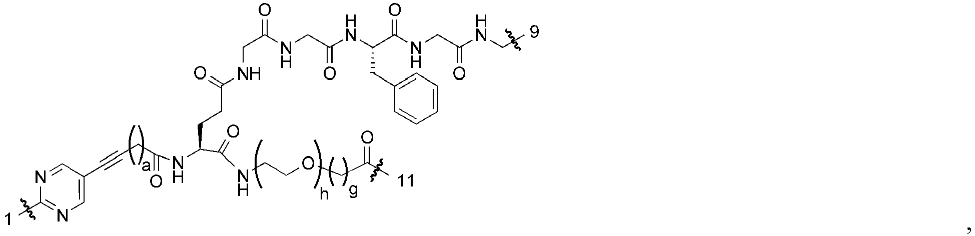 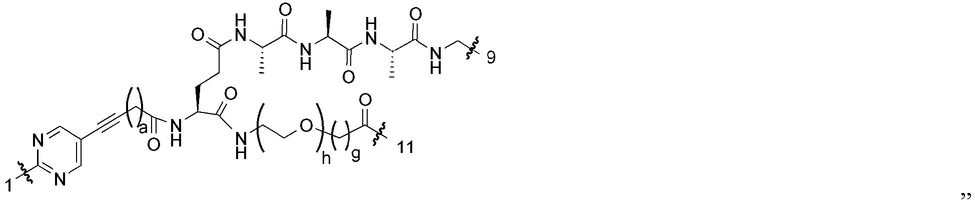 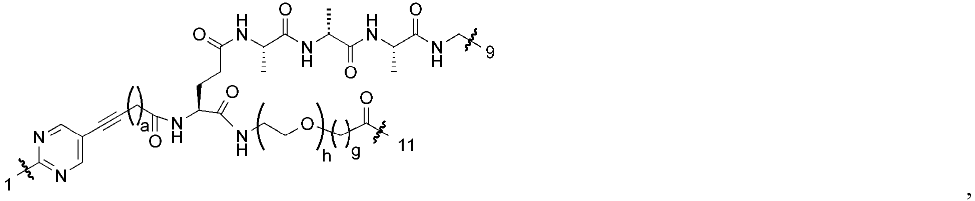 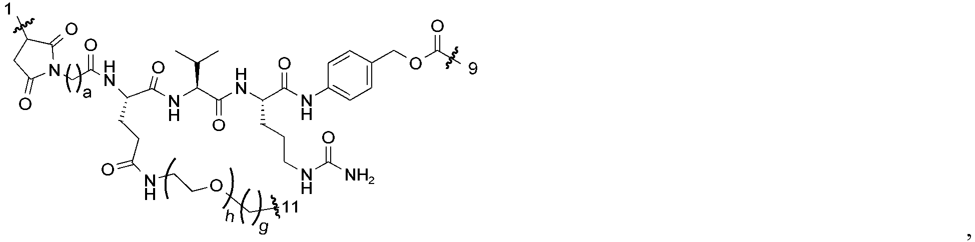 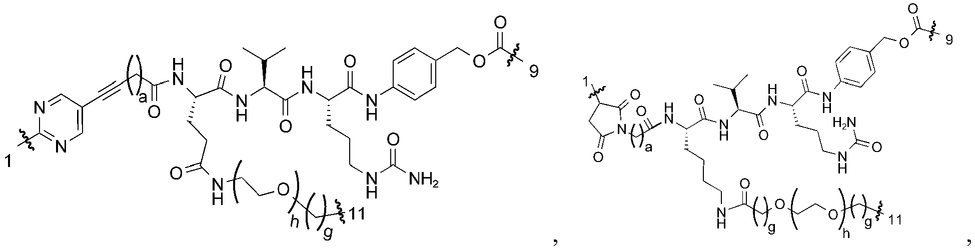 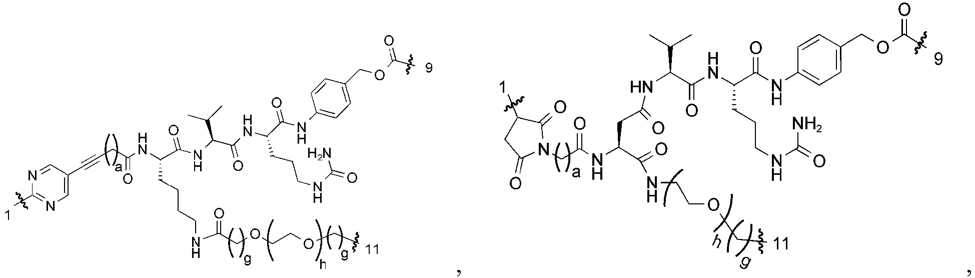 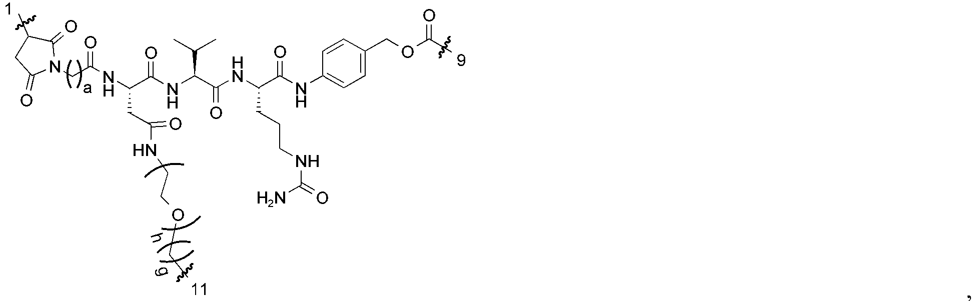 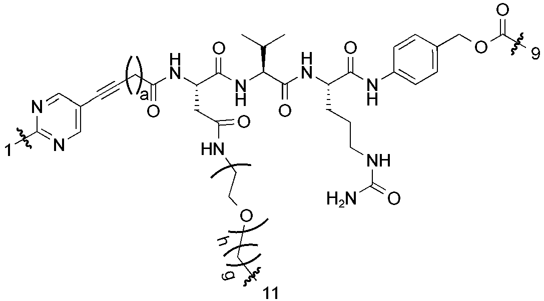 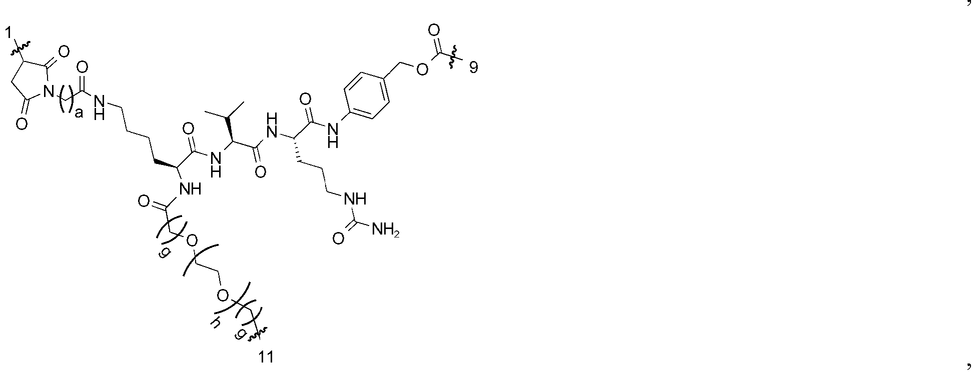 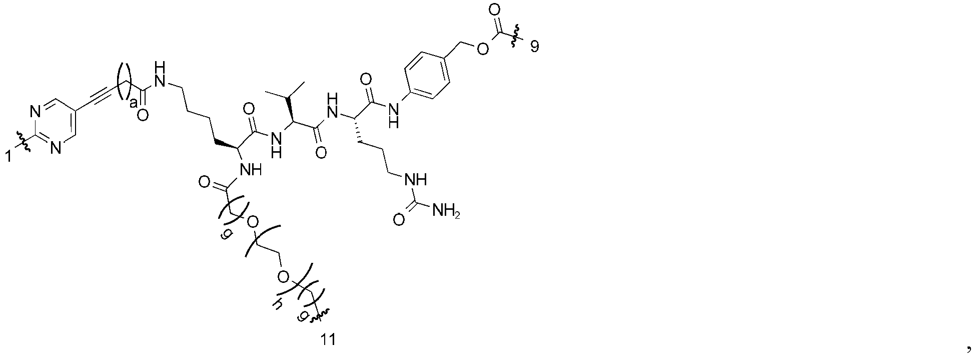 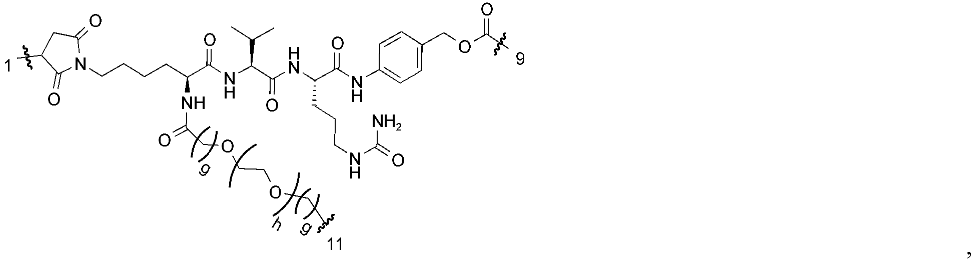 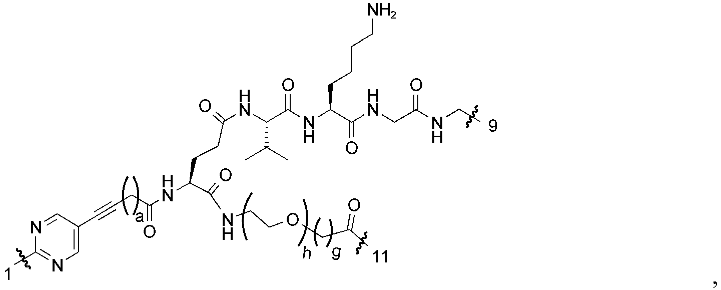 and 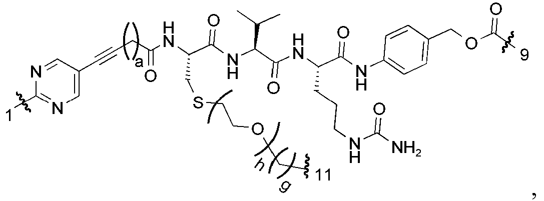 wherein, each a is independently an integer ranging from 0 to 6, each c is independently an integer ranging from 0 to 6, each d is independently an integer ranging from 1 to 10, each e is independently an integer ranging from 0 to 6, each f is independently an integer ranging from 1 to 10, each g is independently an integer ranging from 0 to 6, and each h is independently an integer ranging from 1 to 10; the position 1 is connected to Ab', the position 9 is connected to D¹, and the position 11 is connected to D².

In some embodiments, 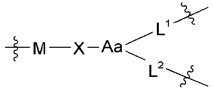 is selected from the group consisting of: 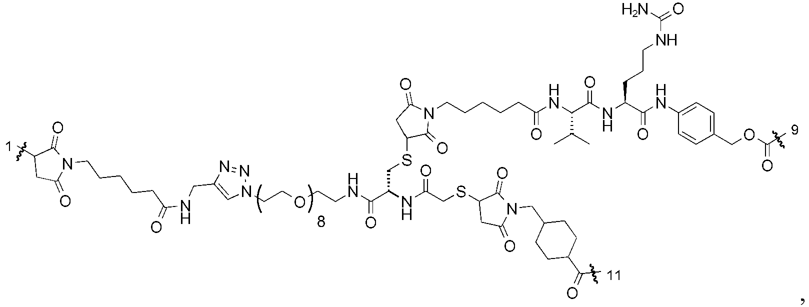 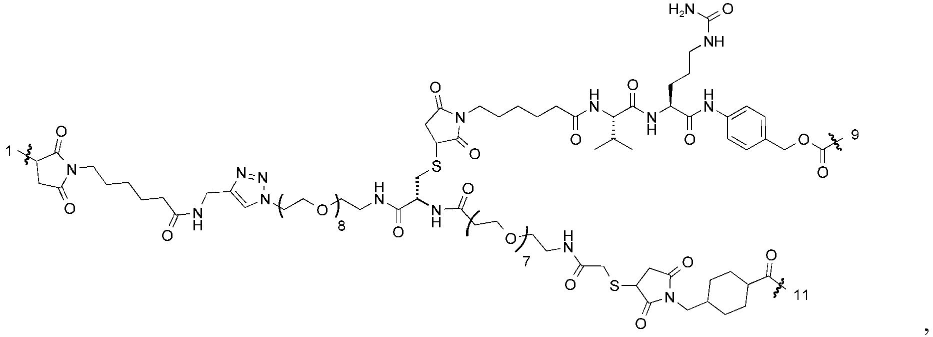 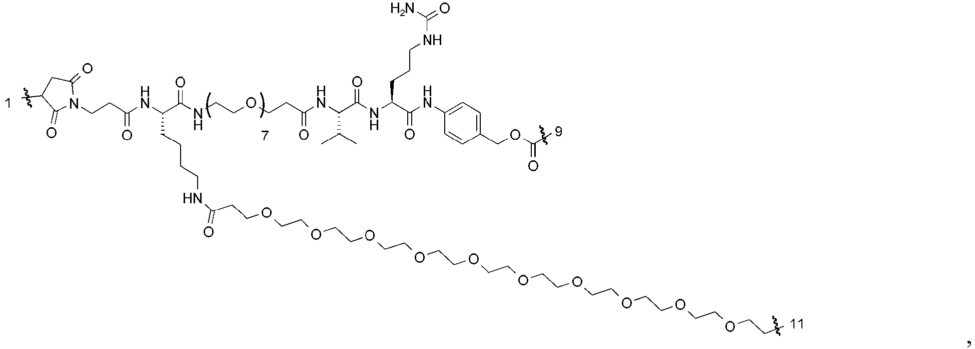 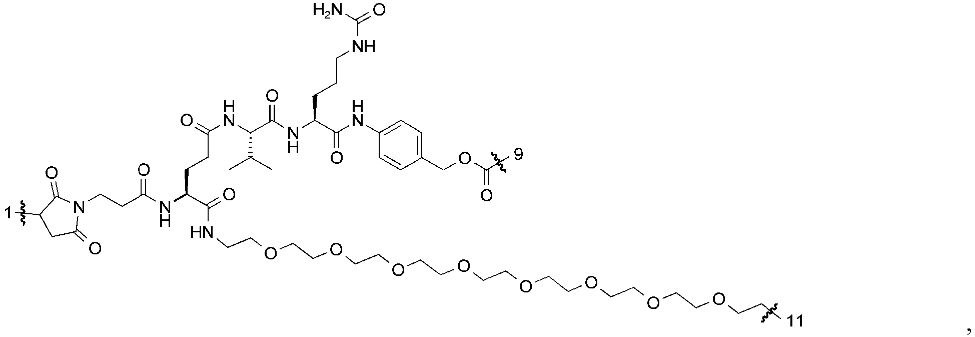 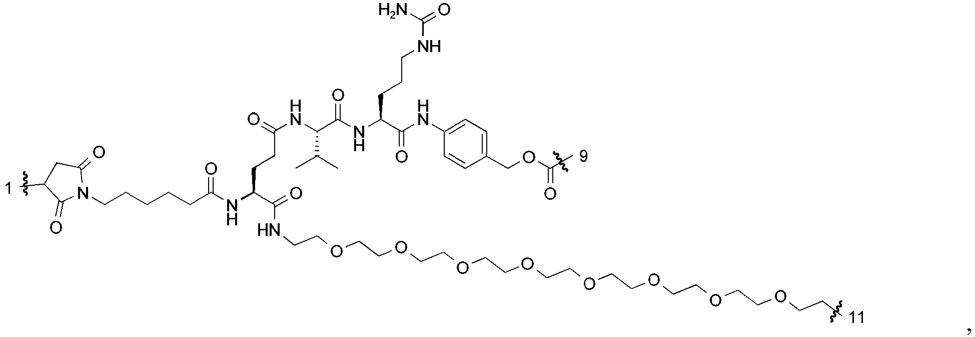 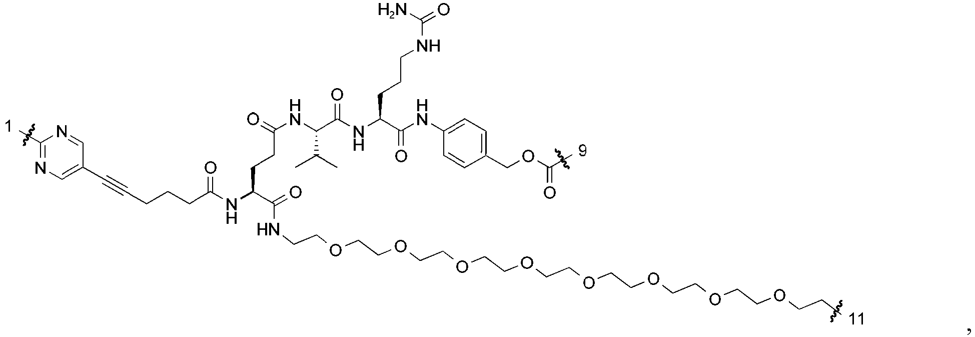 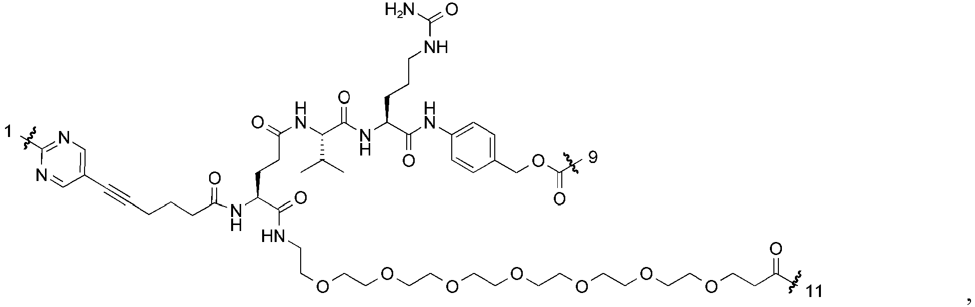 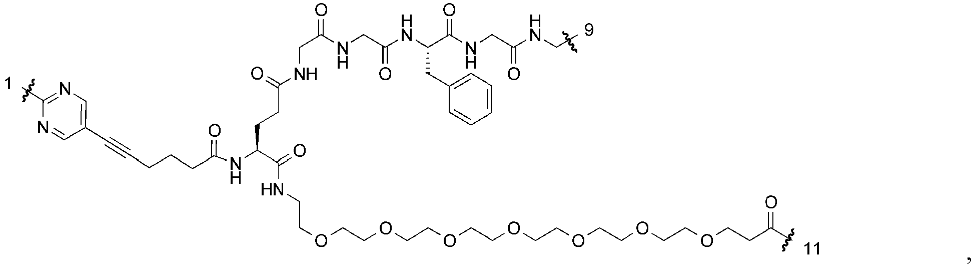 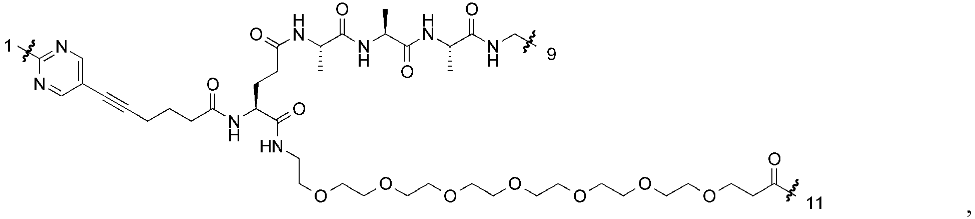 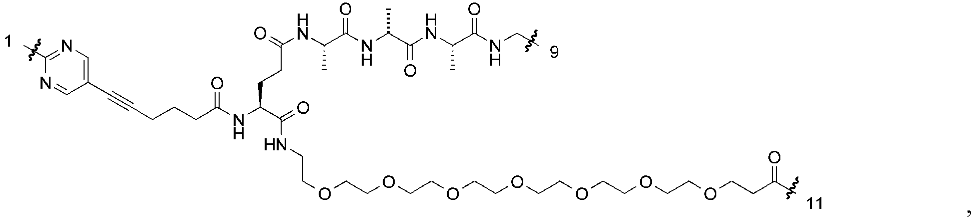 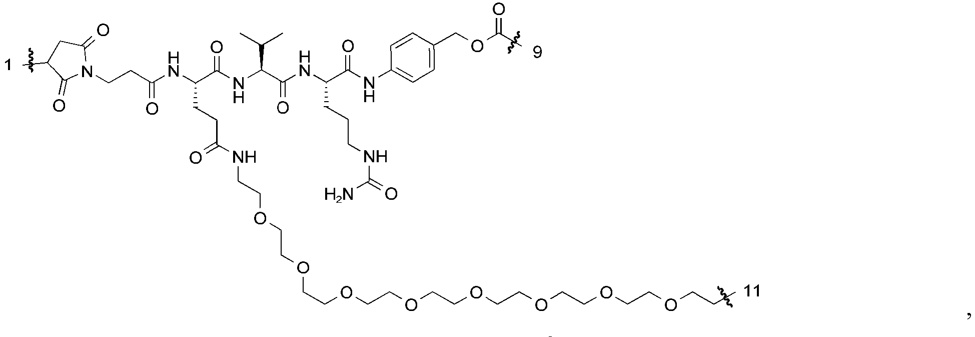 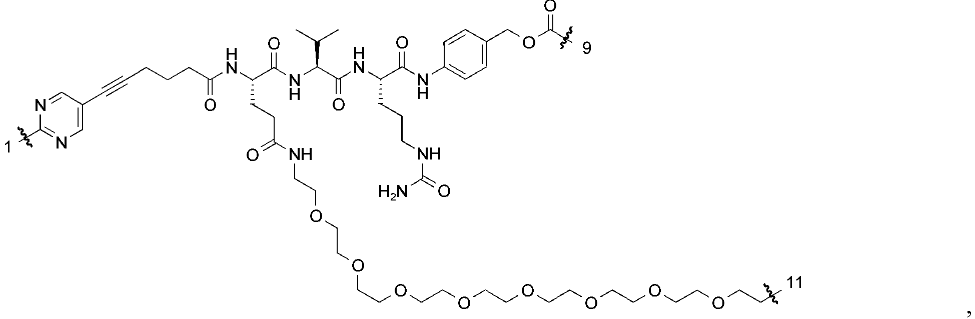 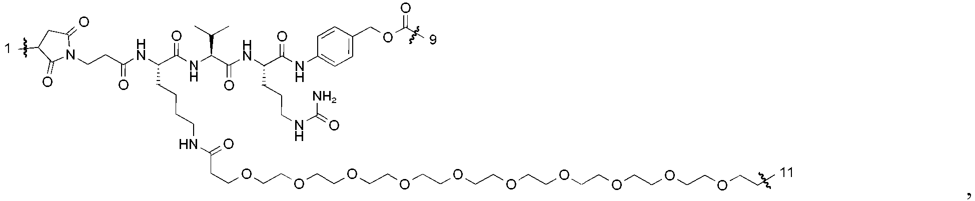 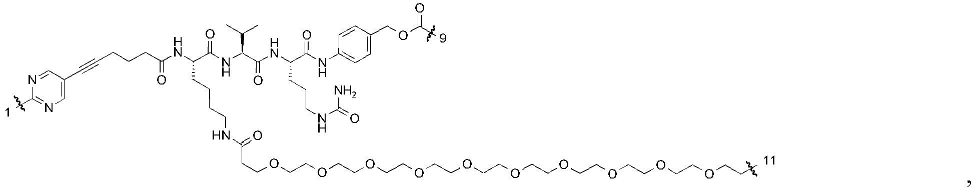 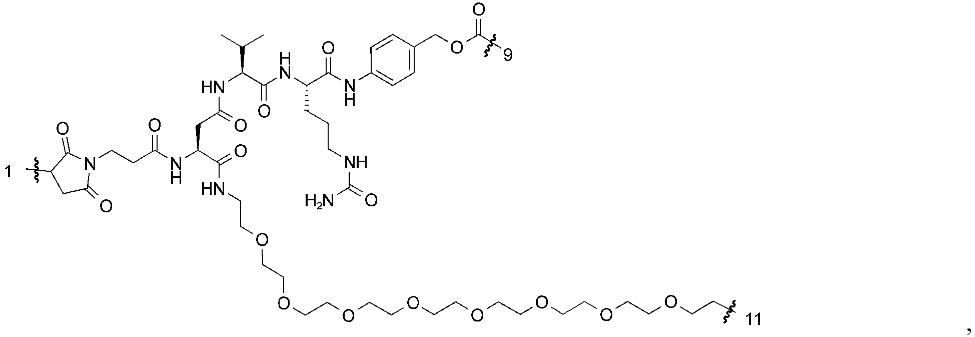 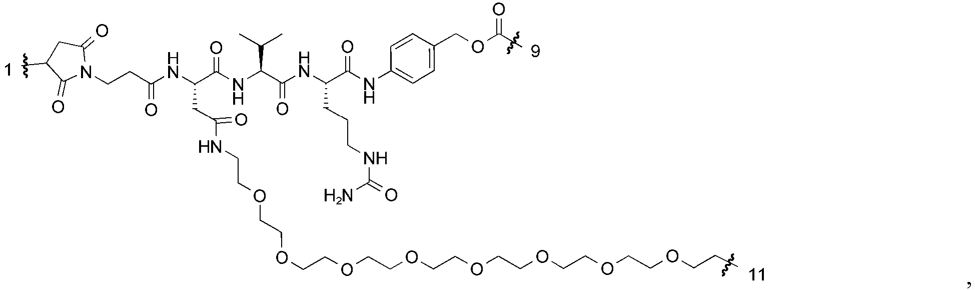 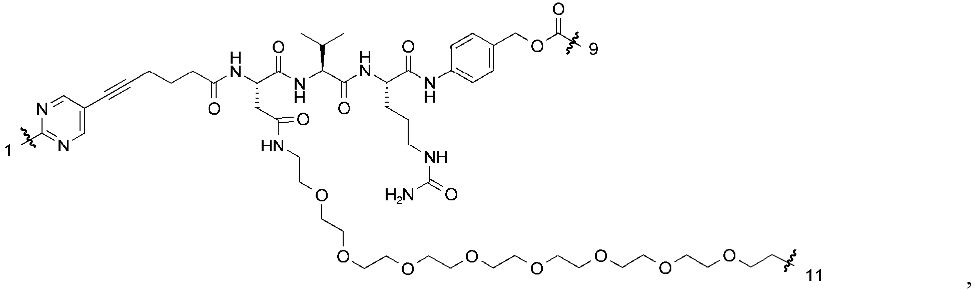 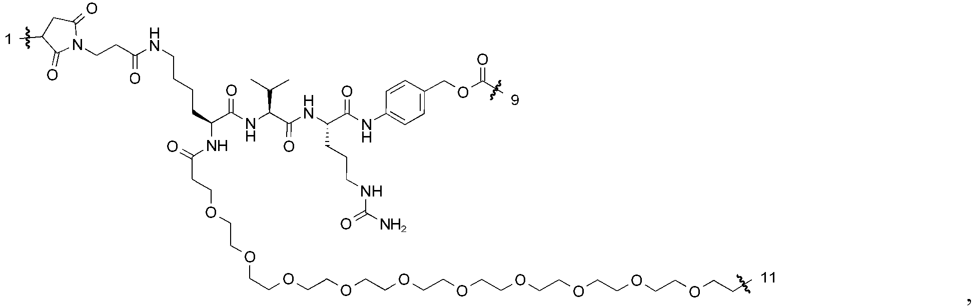 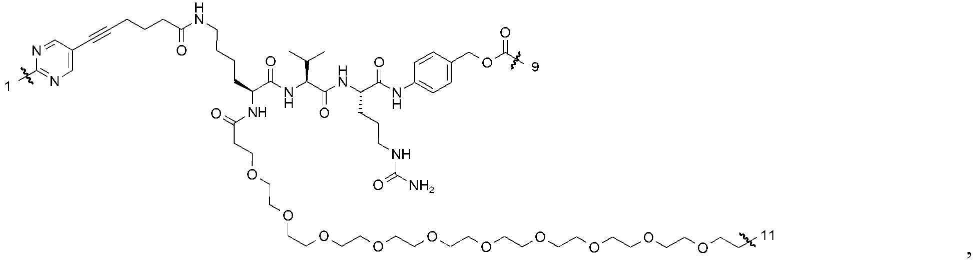 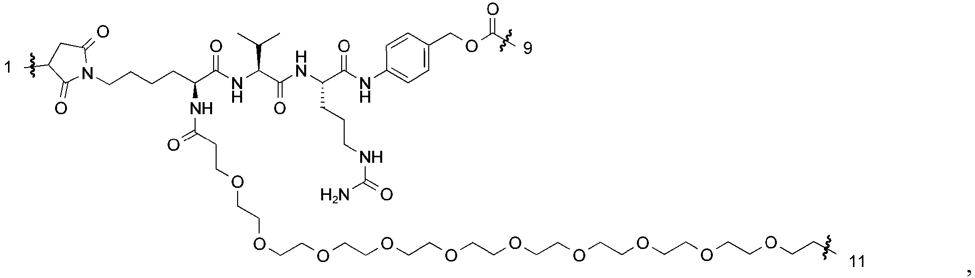 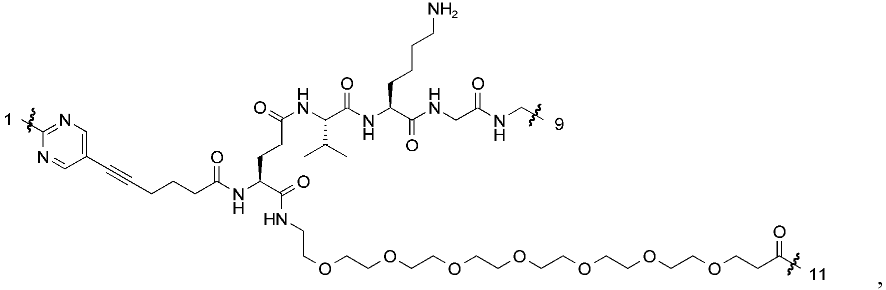 and 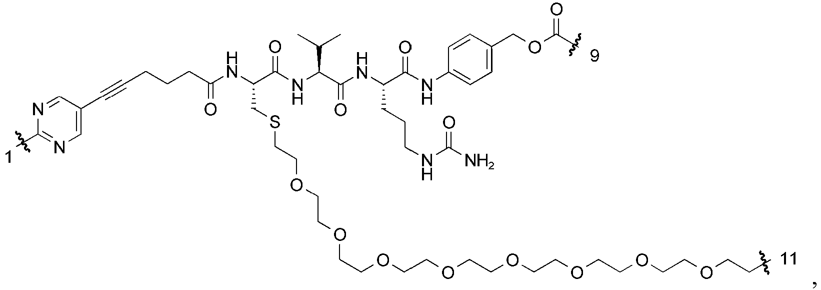 wherein, the position 1 is connected to Ab', the position 9 position is connected to D¹, and the position 11 is connected to D².

In some embodiments, 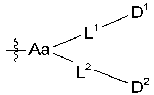 is selected from the group consisting of: 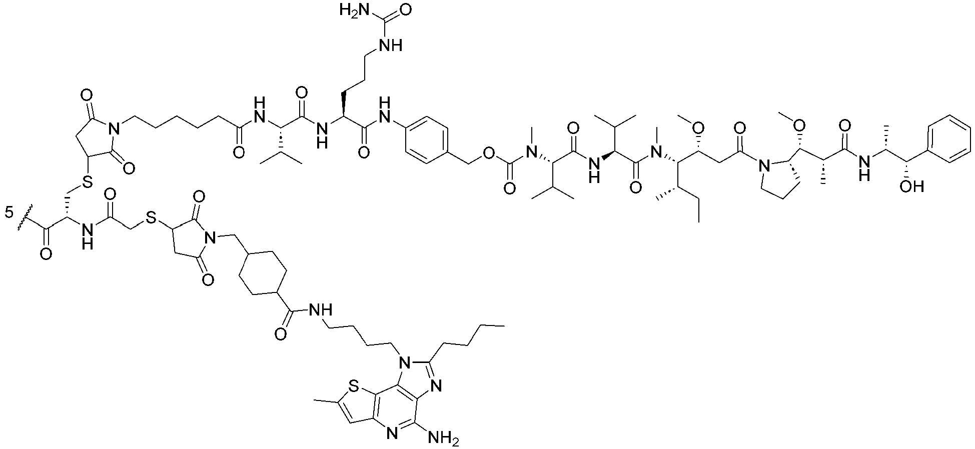 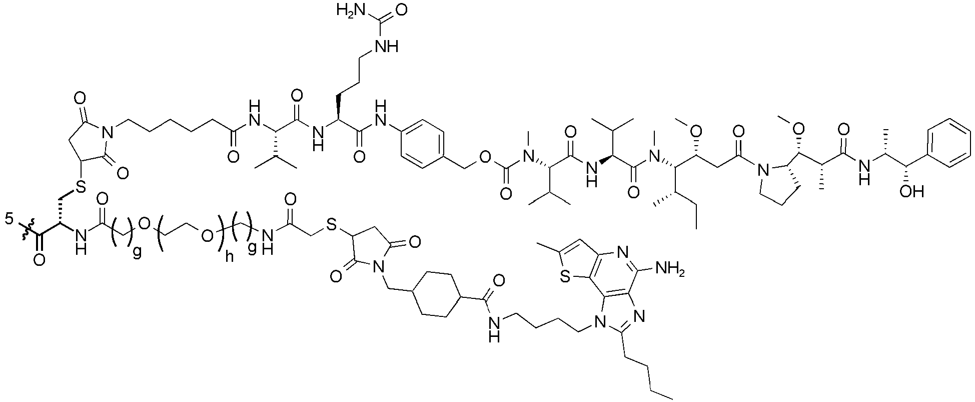 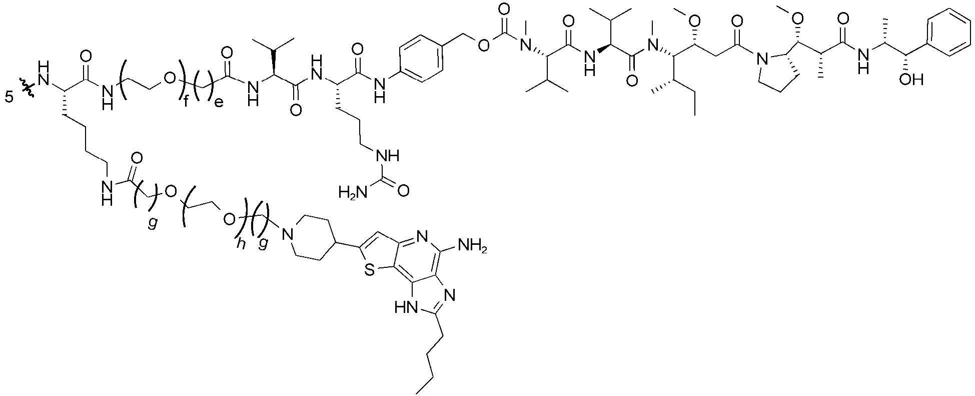 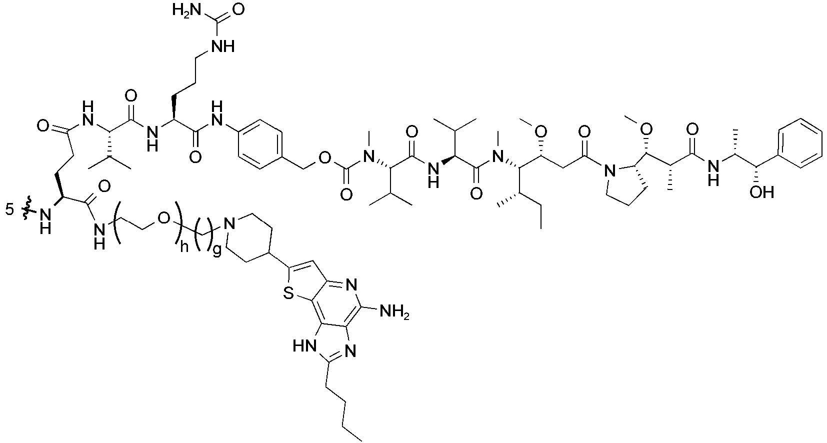 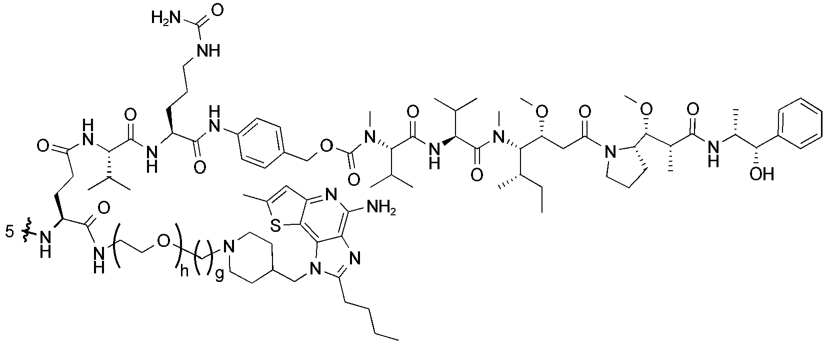 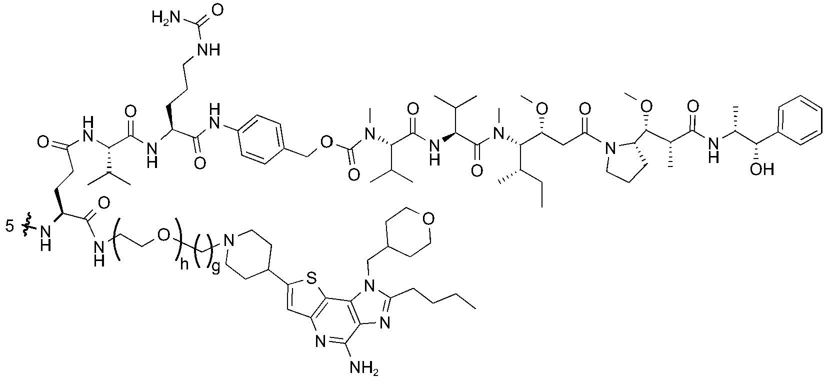 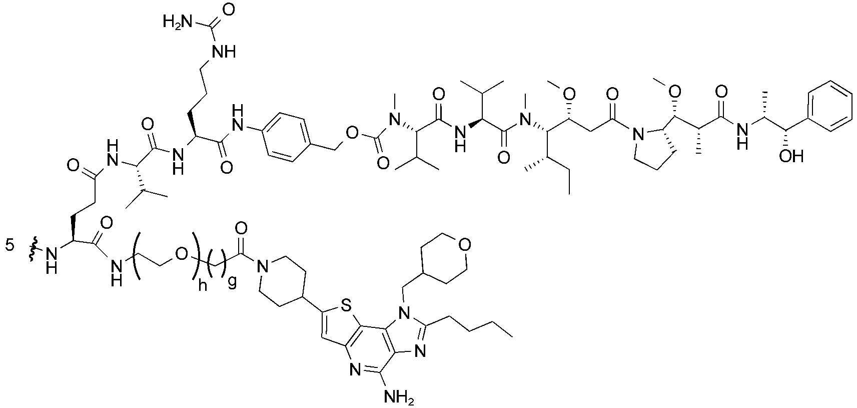 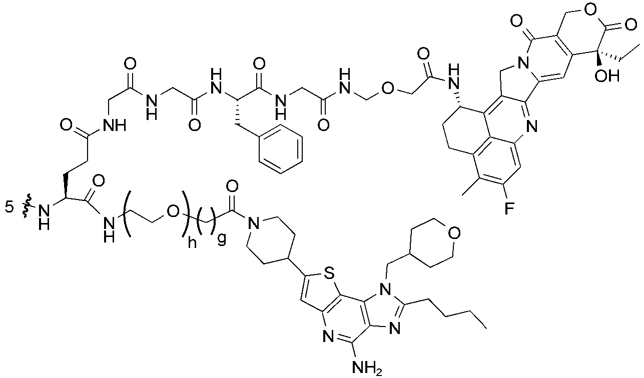 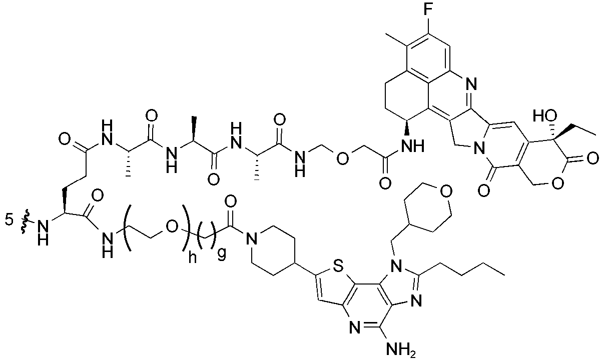 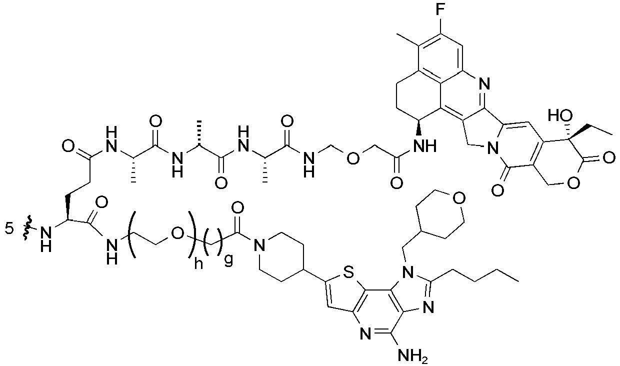 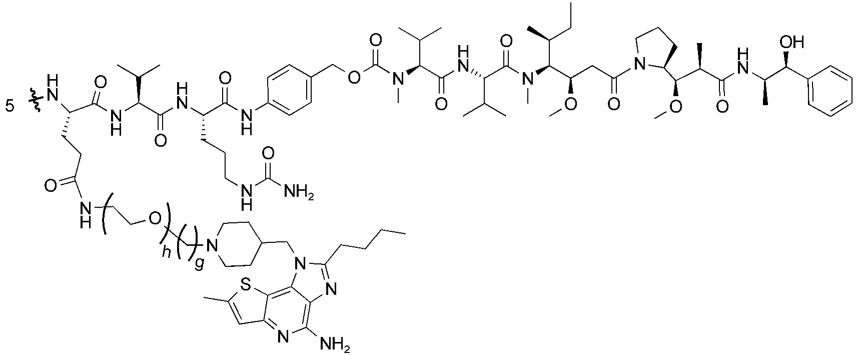 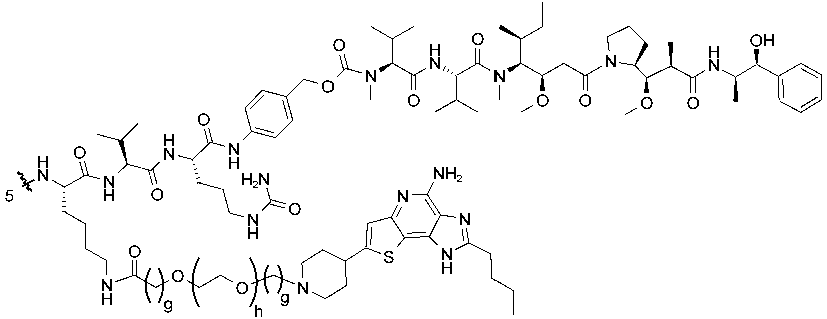 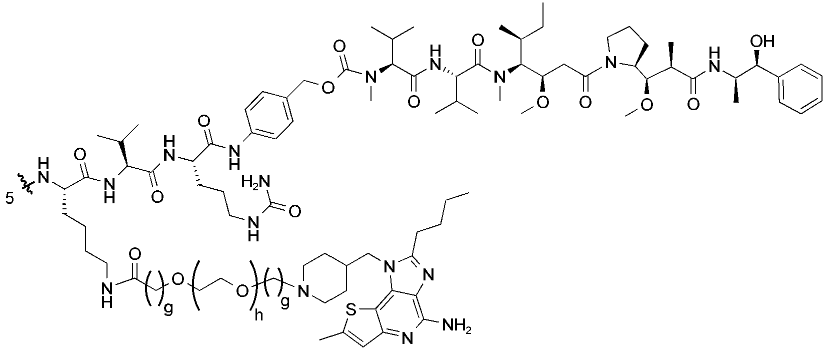 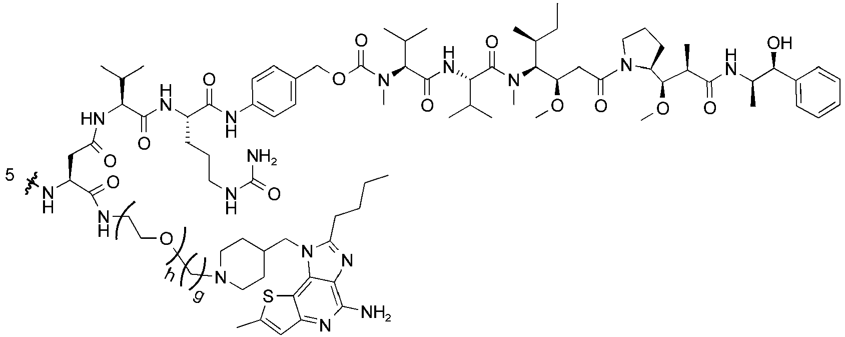 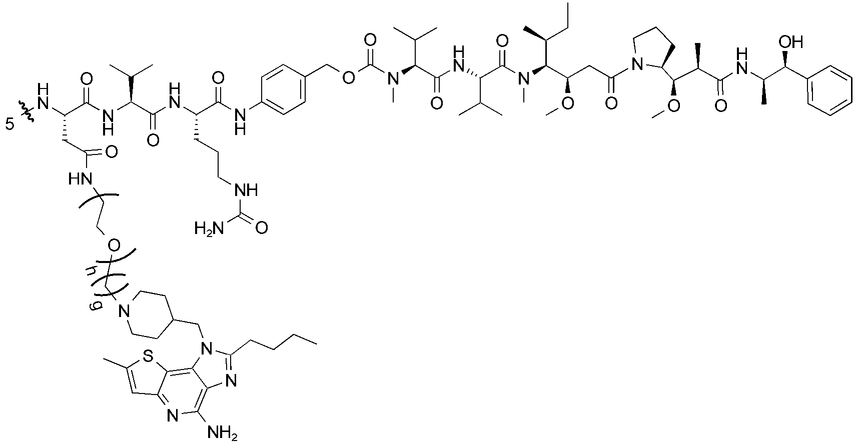 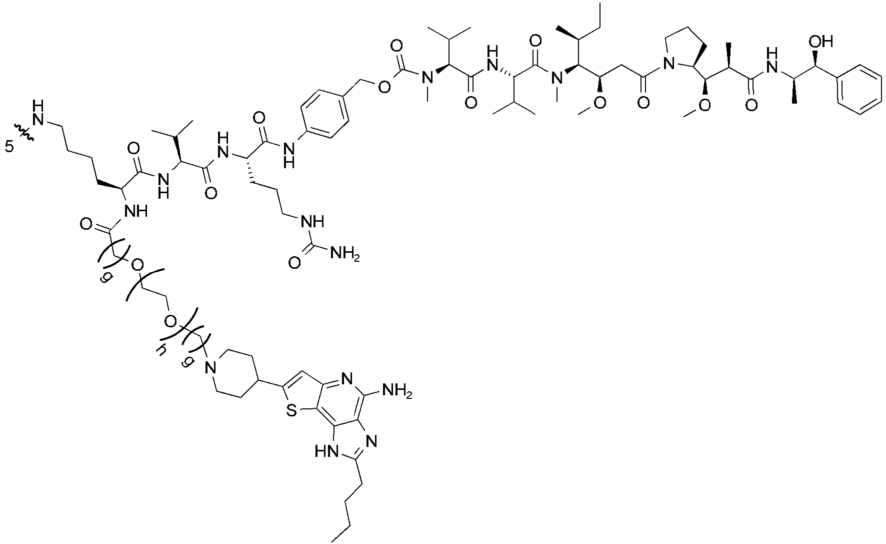 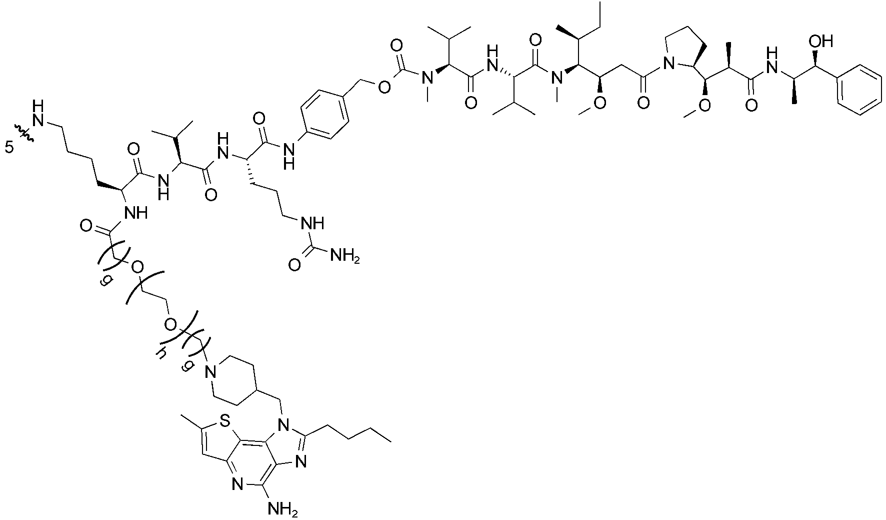 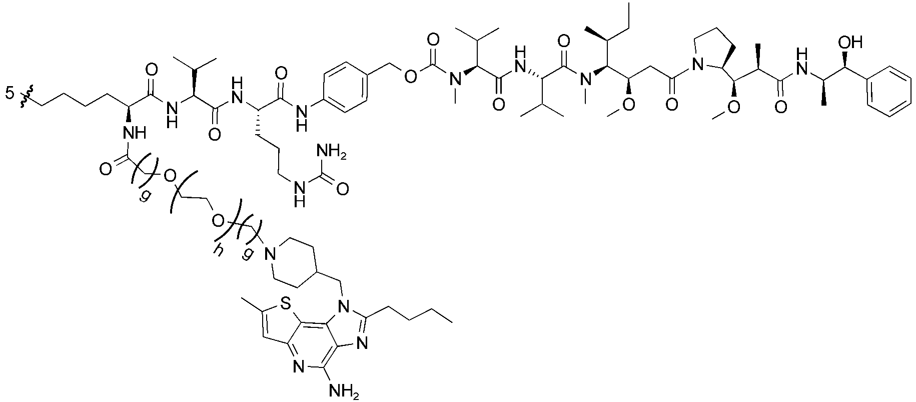 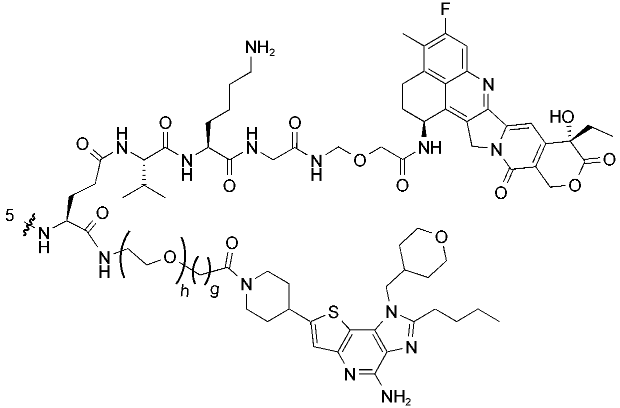 and 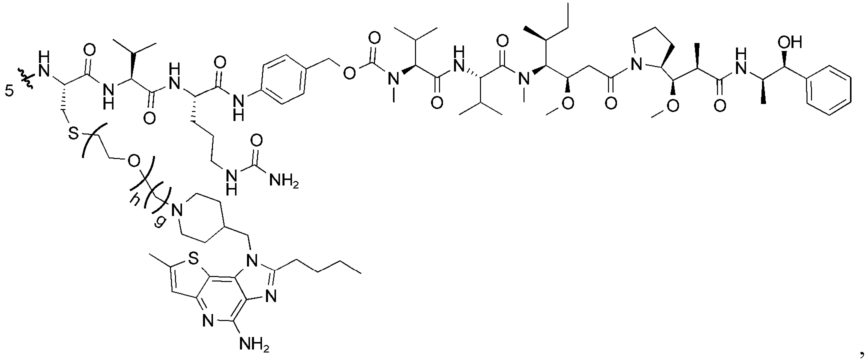 wherein, each g is independently an integer ranging from 0 to 6, and each h is independently an integer ranging from 1 to 10; the position 5 is connected to X.

In some embodiments, 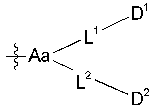 is selected from the group consisting of: 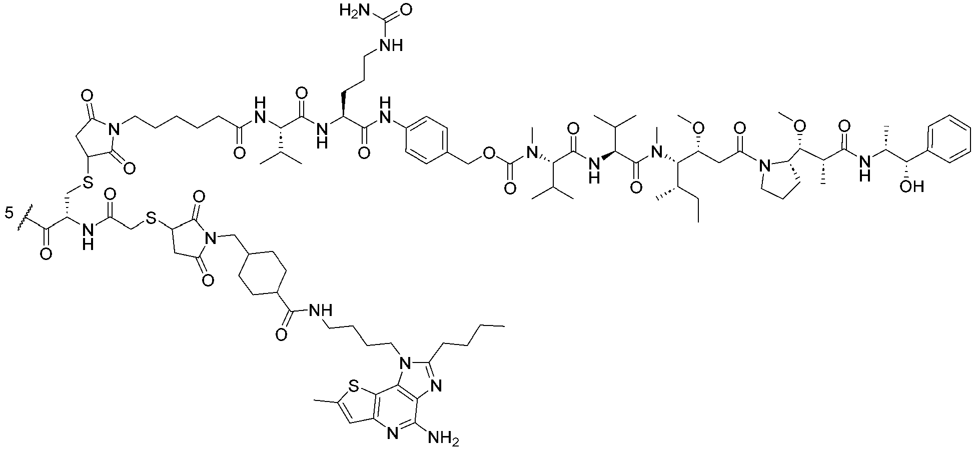 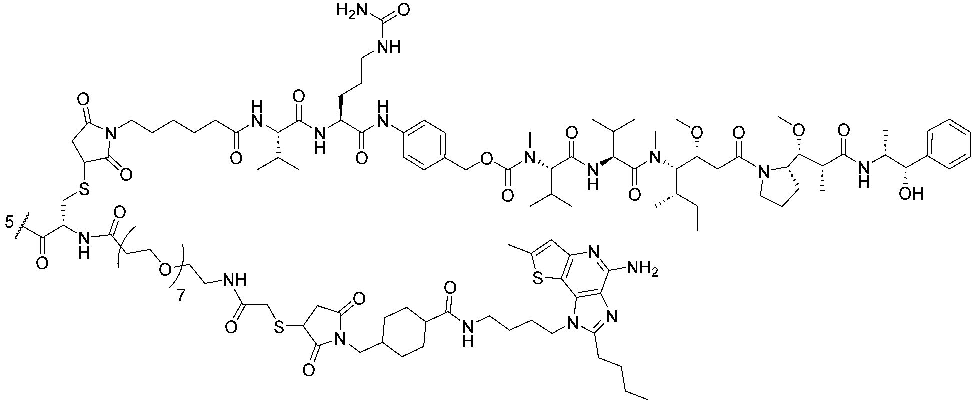 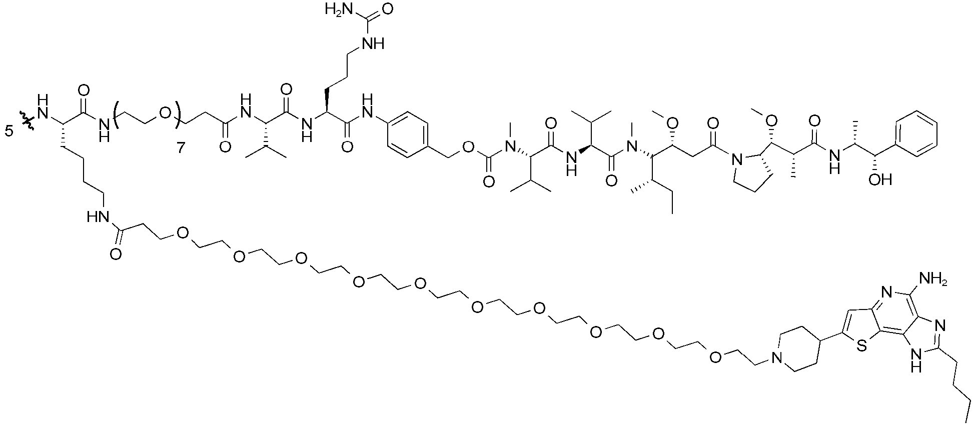 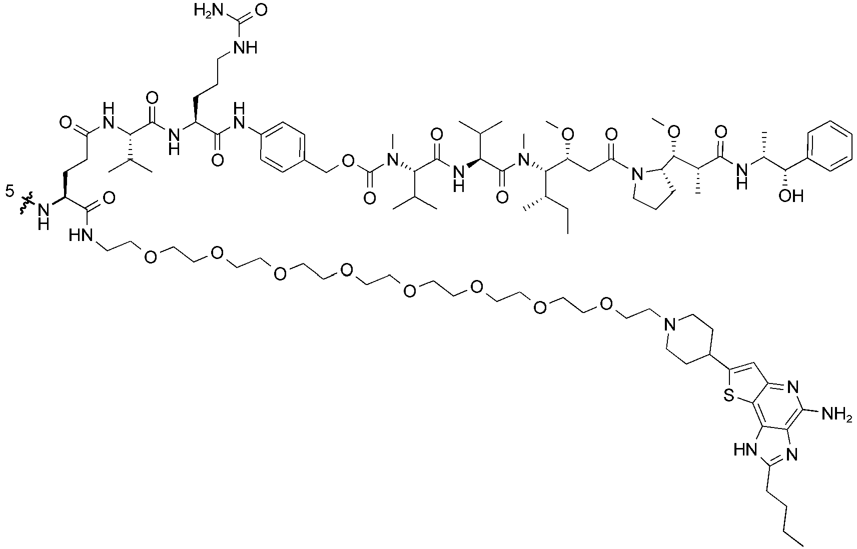 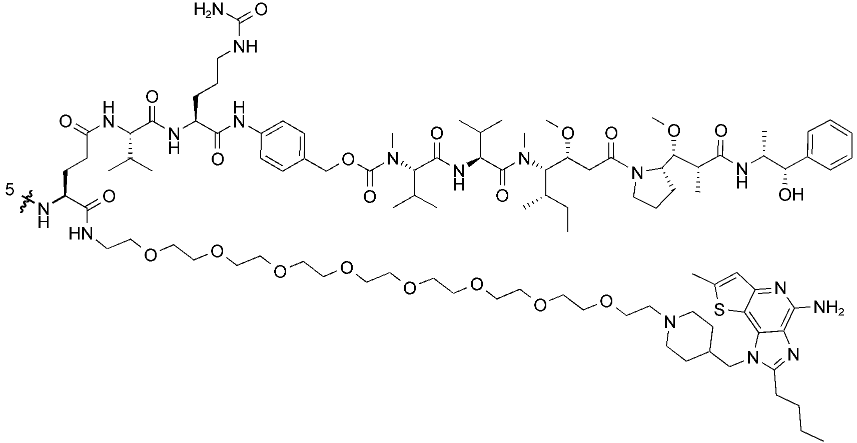 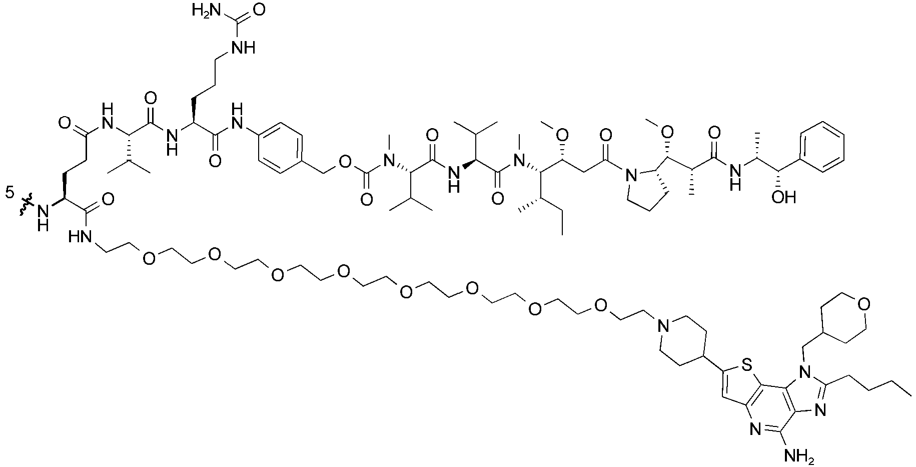 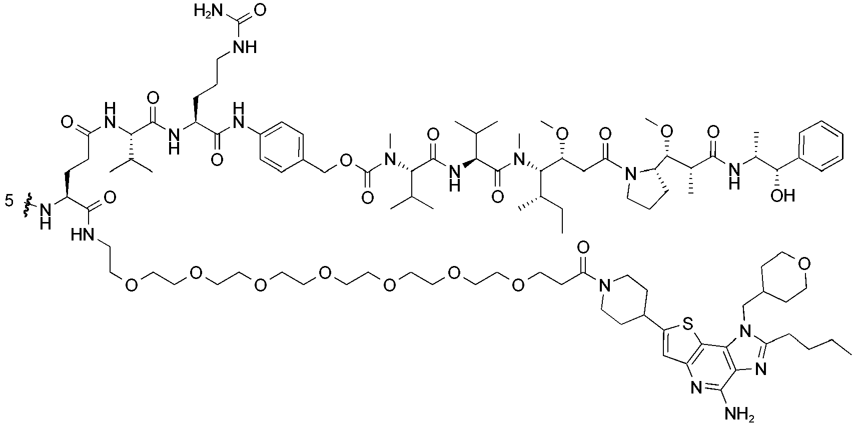 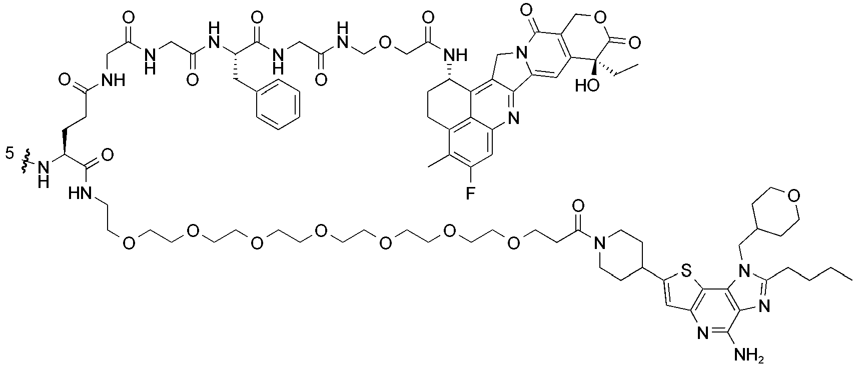 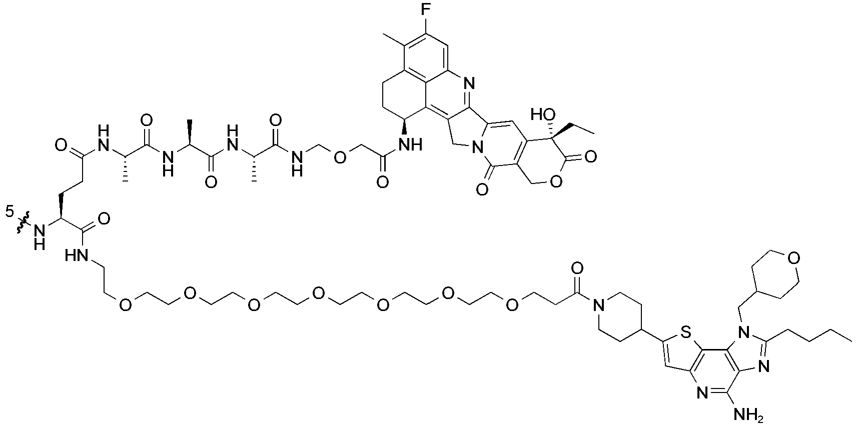 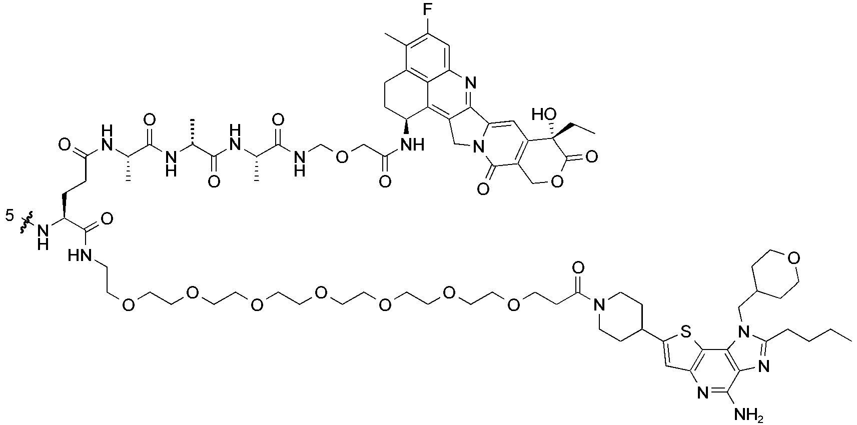 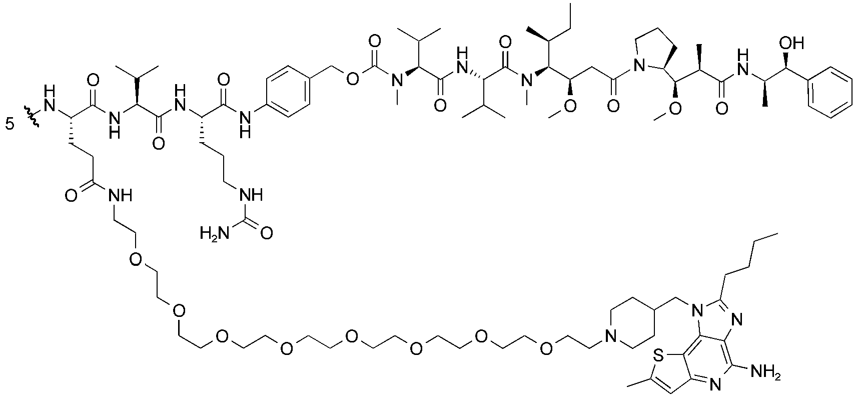 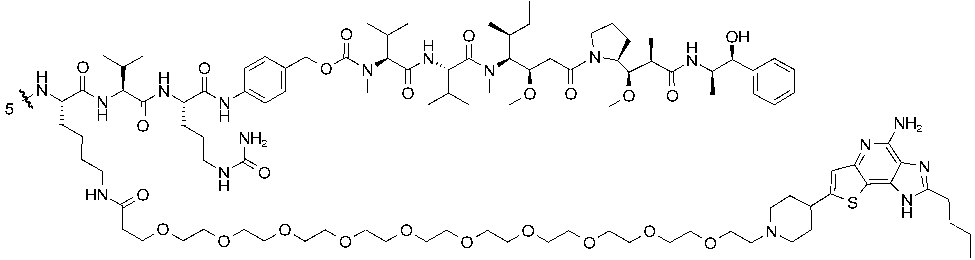 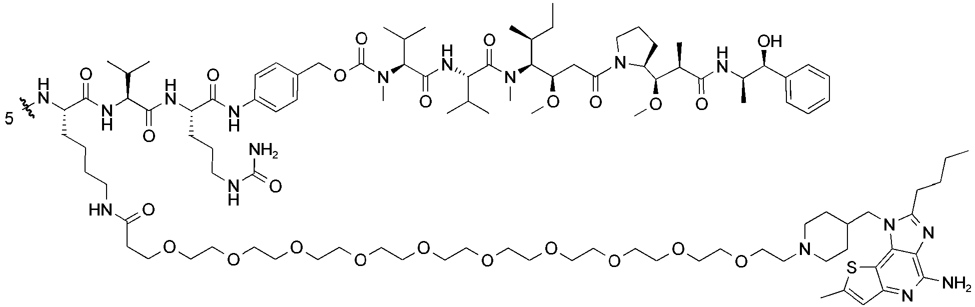 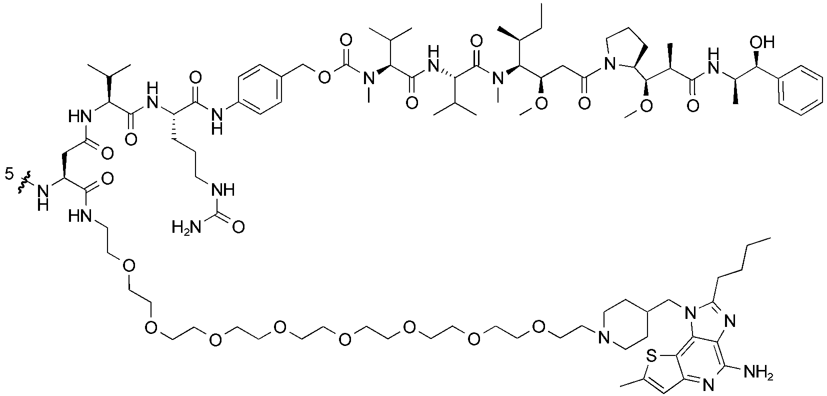 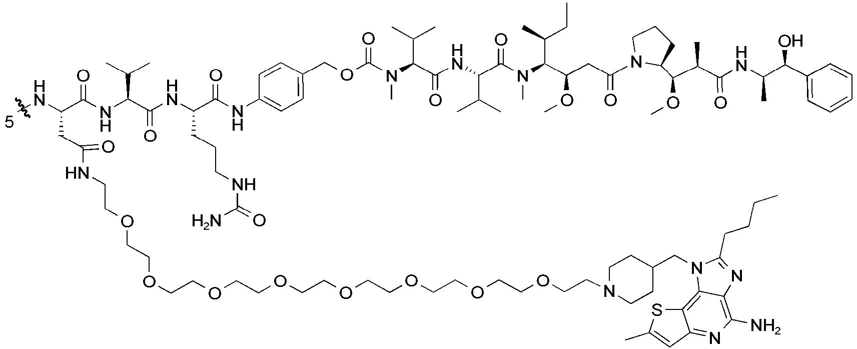 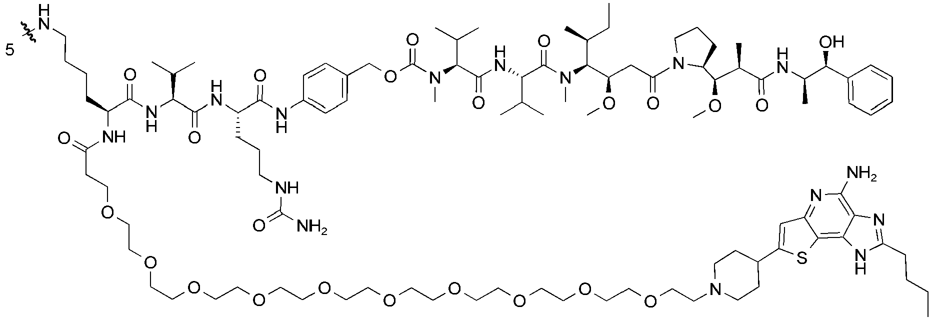 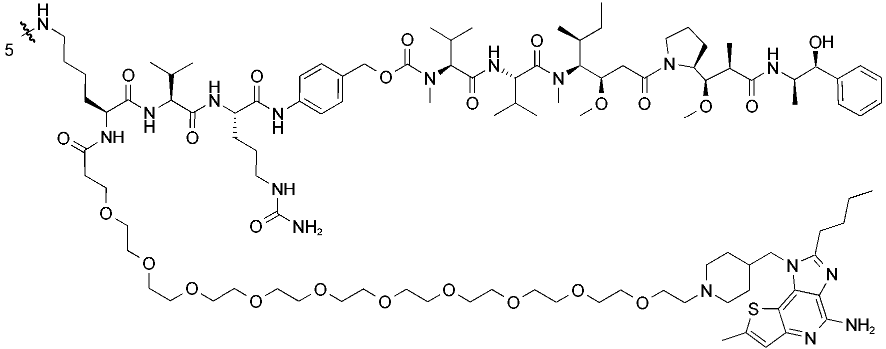 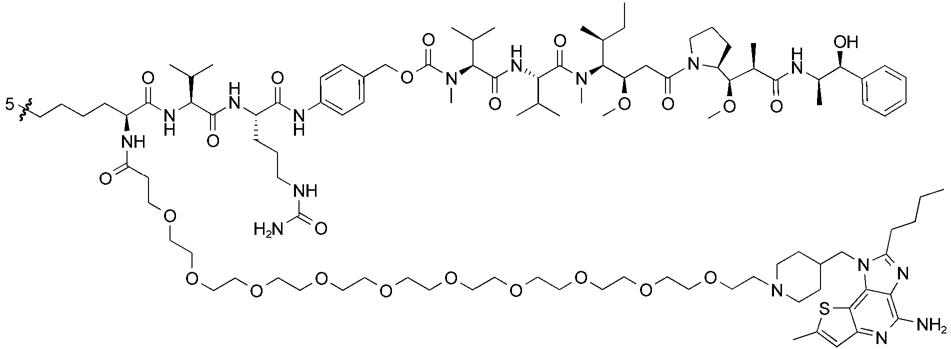 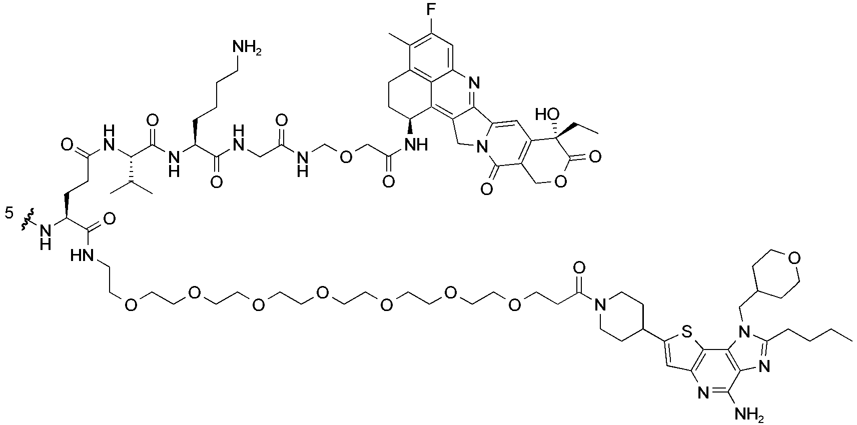 and 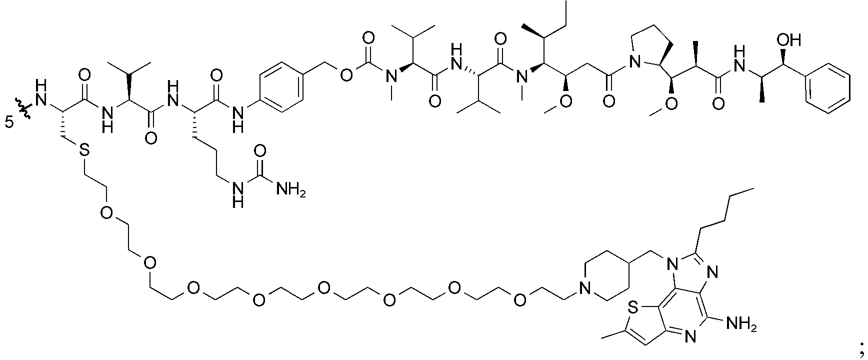 wherein, the position 5 is connected to X.

In some embodiments, the antibody-drug conjugate is selected from the group consisting of the following structures: 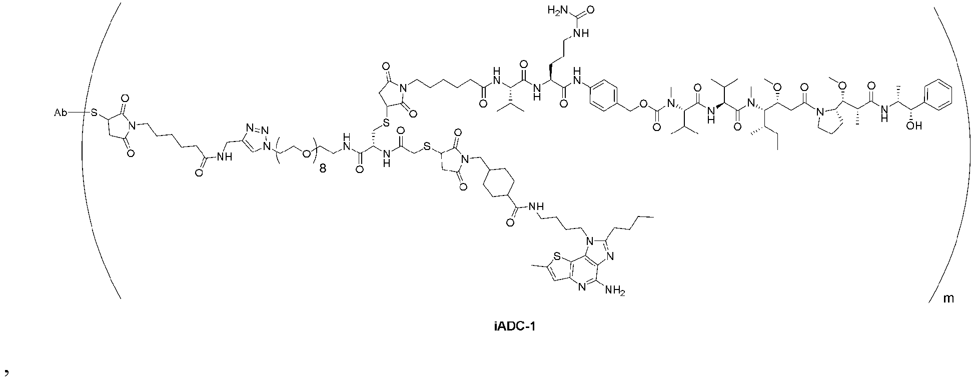 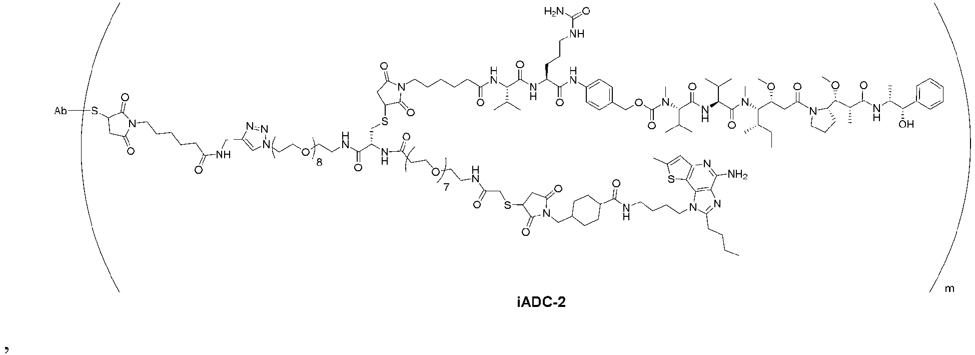 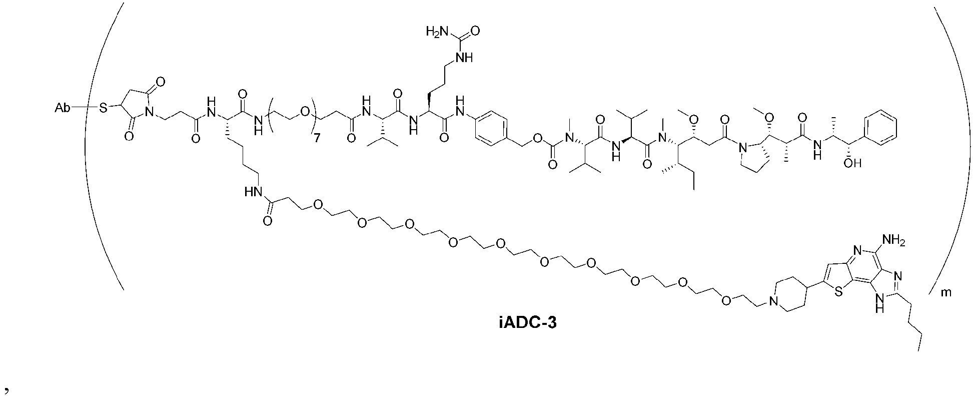 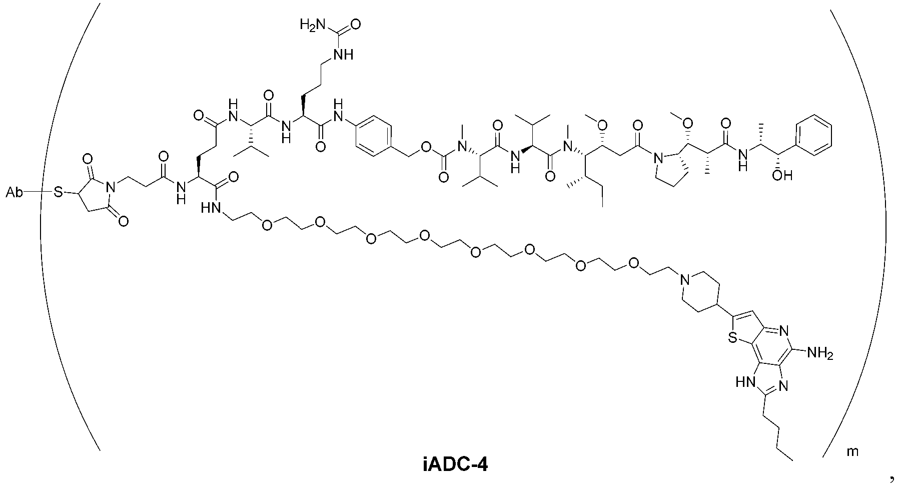 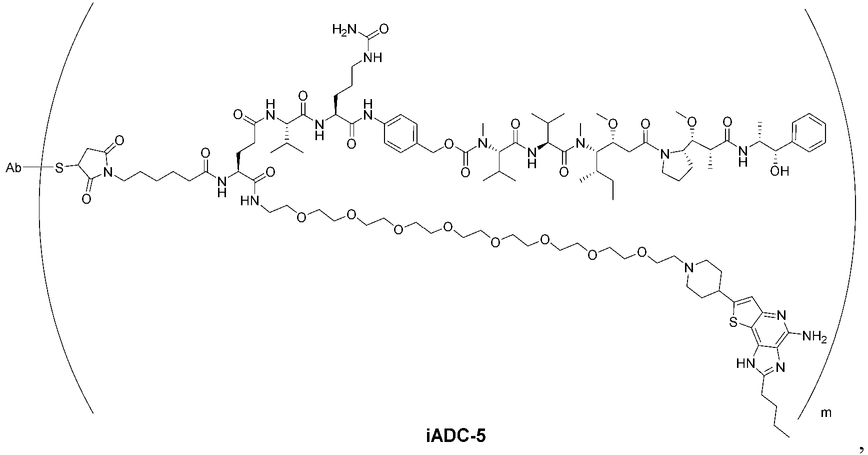 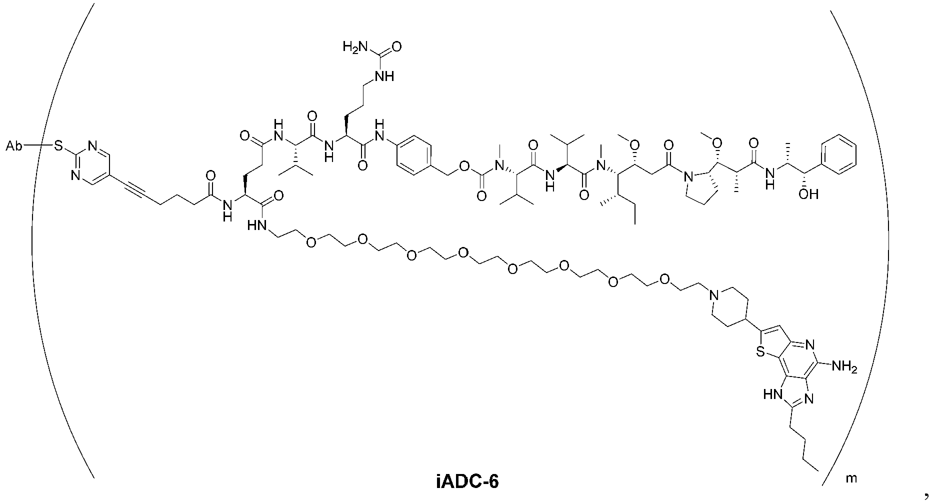 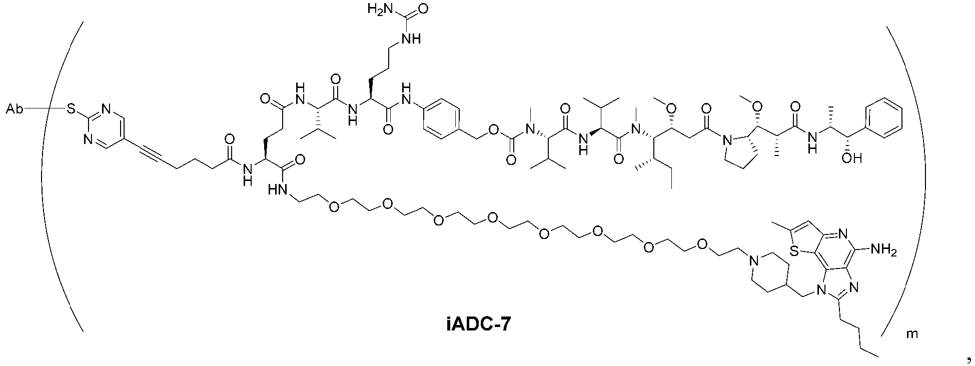 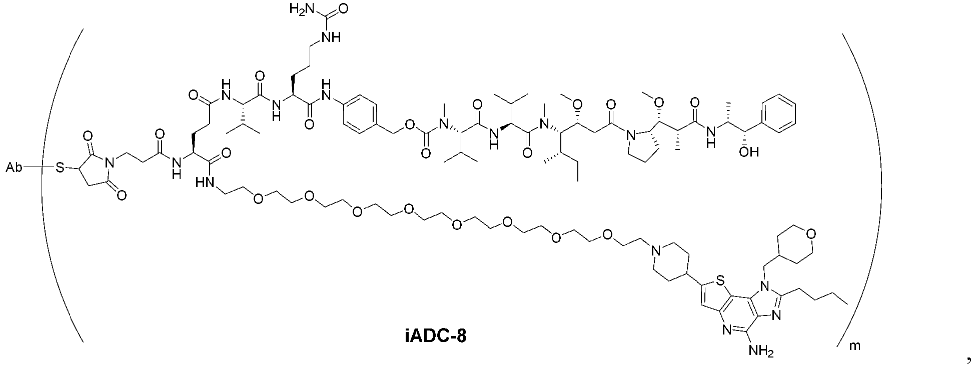 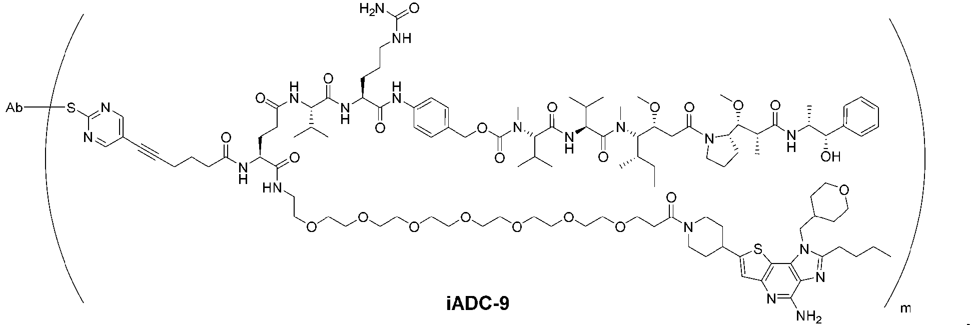 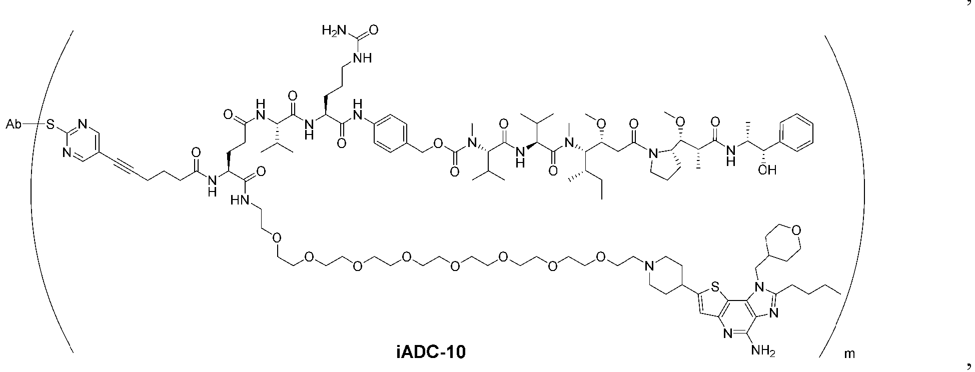 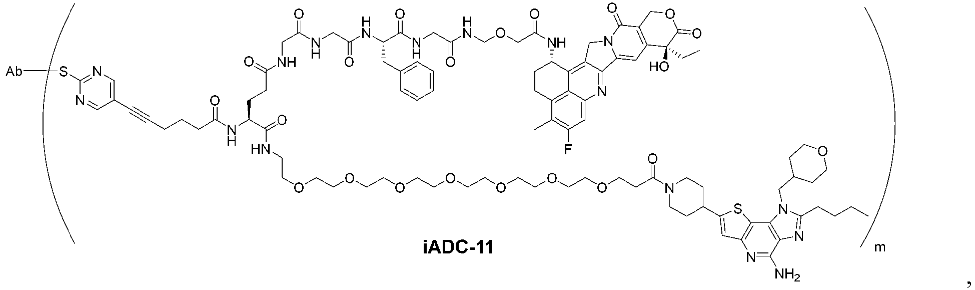 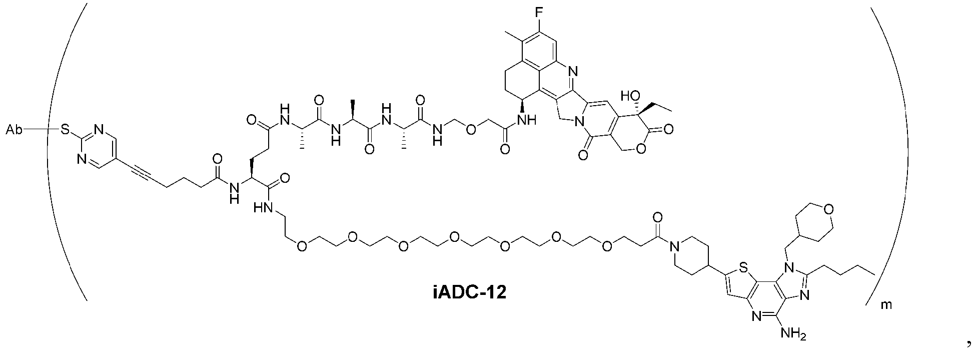 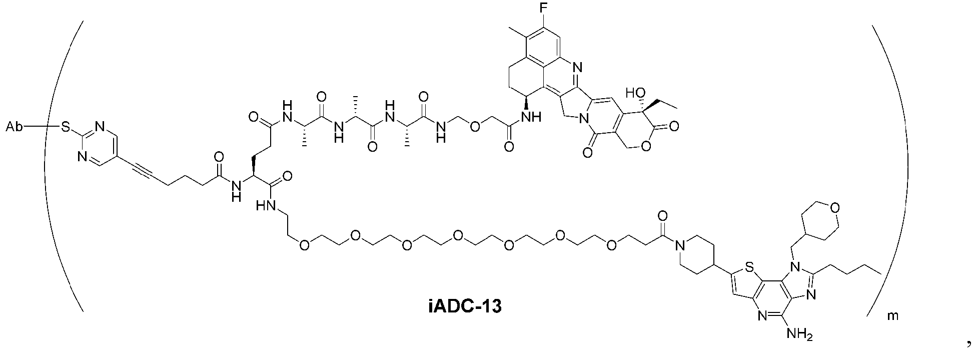 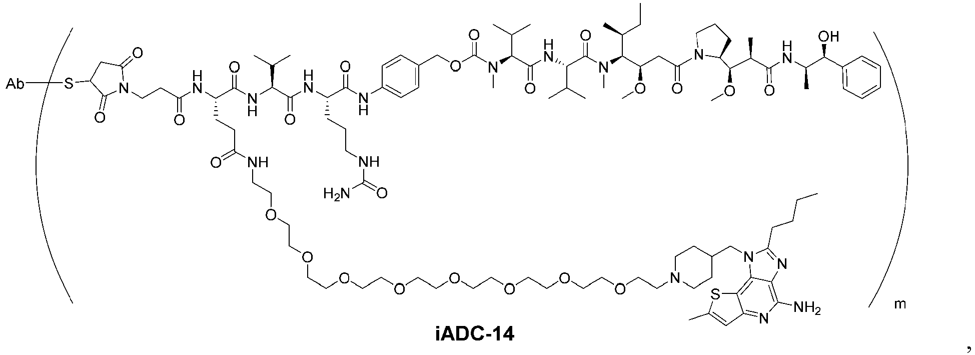 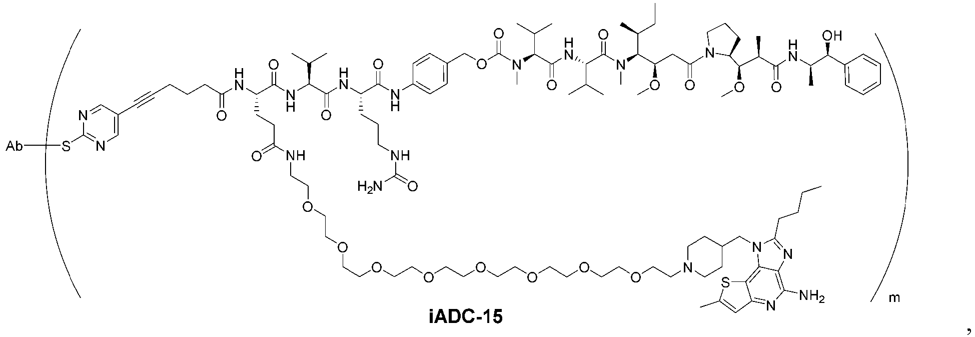 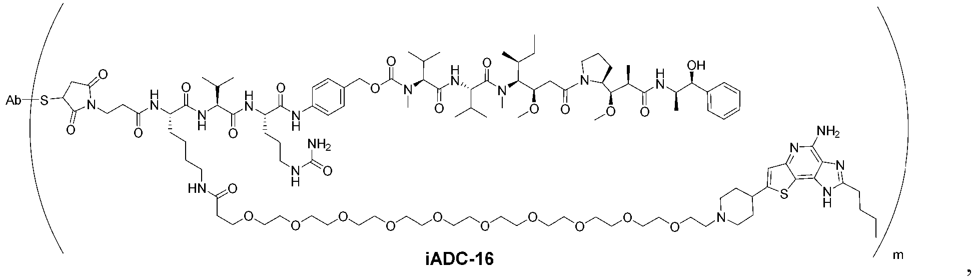 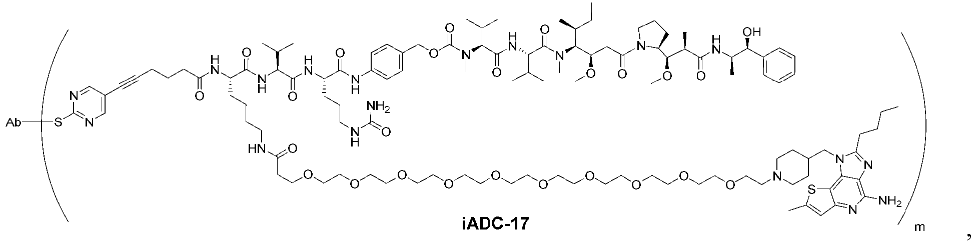 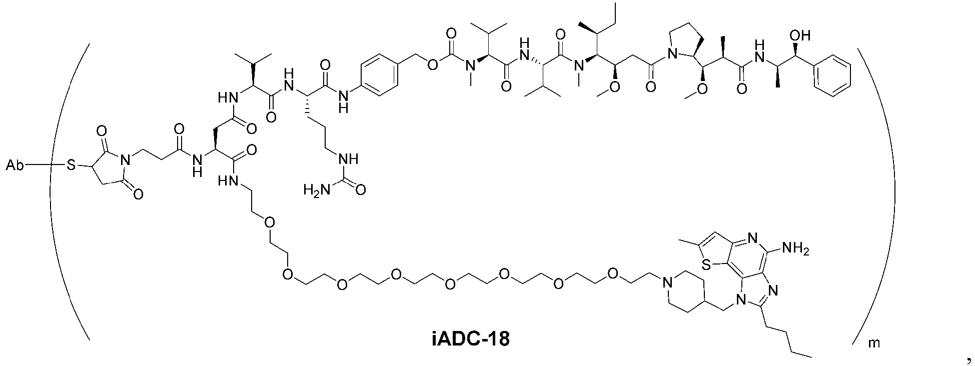 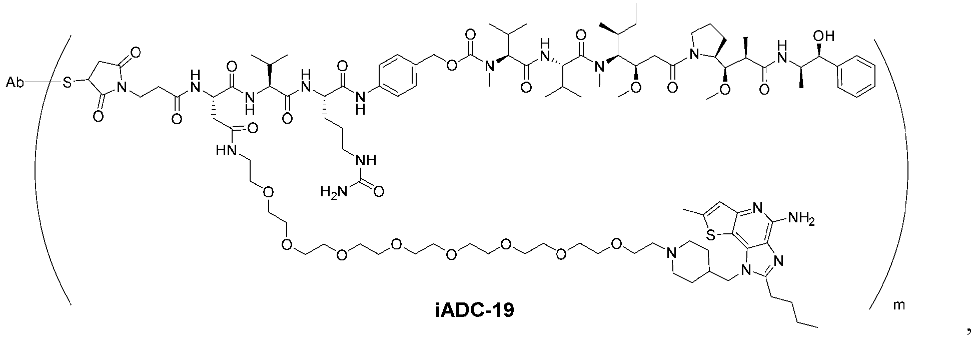 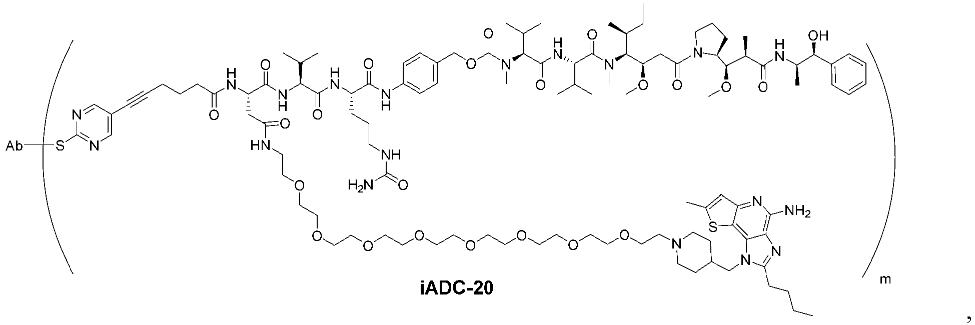 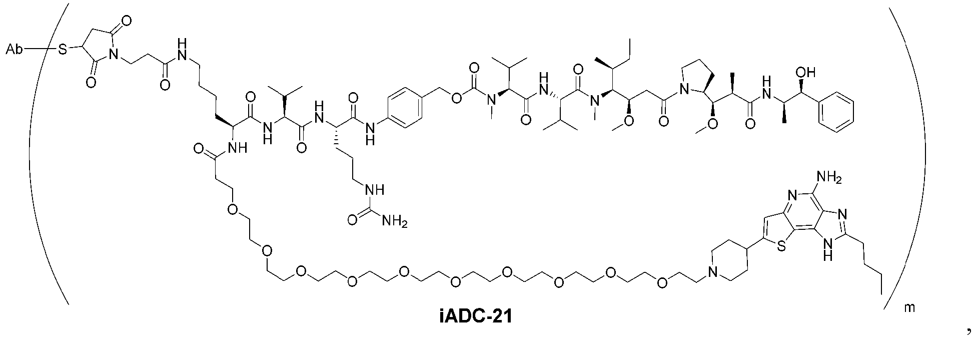 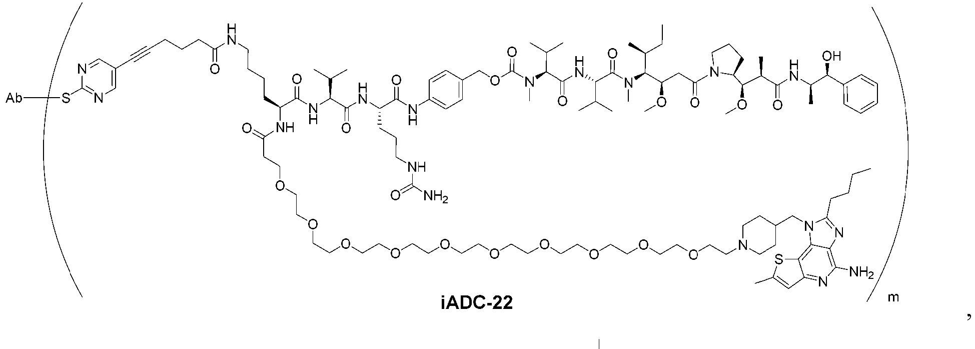 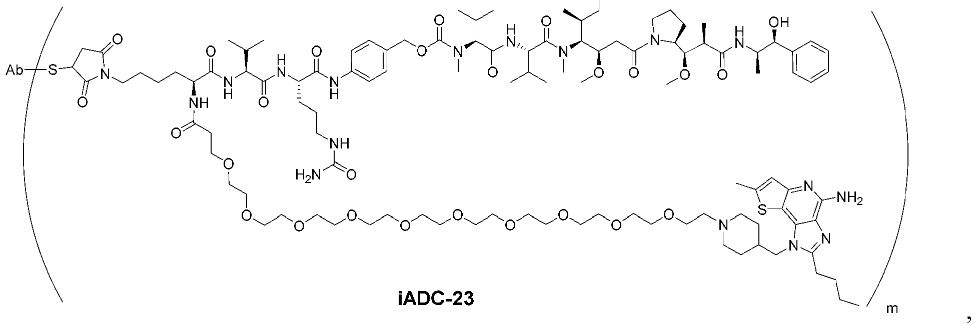 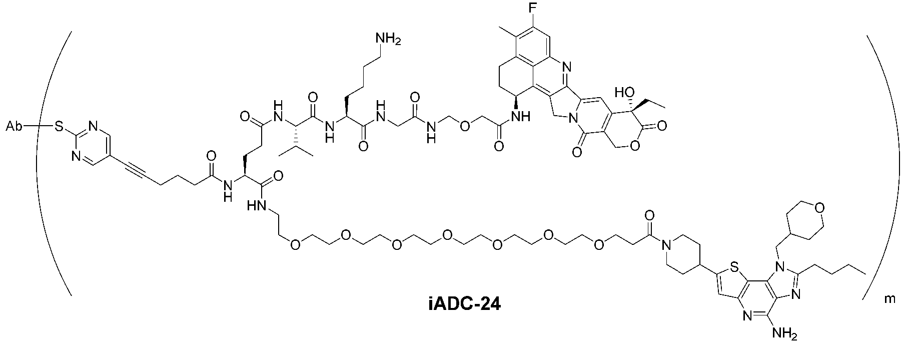 and 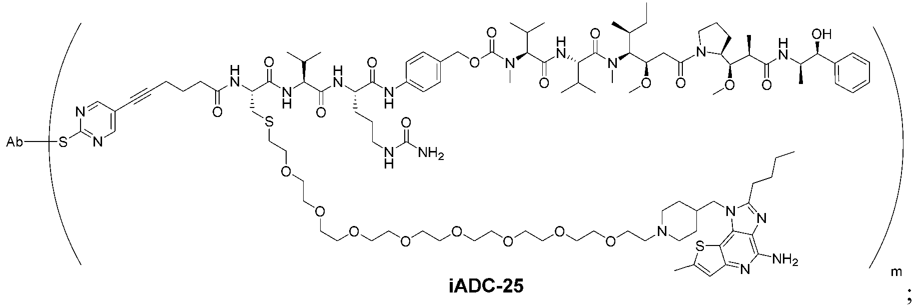 each m is independently selected from 1 to 10, for example, 1, 2, 3, 4, 5, 6, 7, 8, 9 or 10.

In some embodiments, Ab-(S- in each antibody-drug conjugate refers to the antibody or antigen-binding fragment thereof as defined in any of the previous items, and S in Ab-(S- is derived from a sulfhydryl group in the antibody or antigen-binding fragment thereof. In some embodiments, Ab-(S- in each antibody-drug conjugate is an antibody or antigen-binding fragment thereof comprising a VH having a sequence as set forth in SEQ ID NO: 23 or a VH having a sequence as set forth in SEQ ID NO: 1 and a VL having a sequence as set forth in SEQ ID NO: 2, for example, Ab-01 (which comprises a heavy chain having a sequence as set forth in SEQ ID NO: 22 and a light chain having a sequence as set forth in SEQ ID NO: 21), for example, Ab-02 (which comprises a heavy chain having a sequence as set forth in SEQ ID NO: 20 and a light chain having a sequence as set forth in SEQ ID NO: 21).

In some embodiments, the N-terminal glutamine of the heavy chain or heavy chain variable region and/or the light chain or light chain variable region of the antibody or antigen-binding fragment thereof undergoes cyclization to form a pyroglutamic acid or pyroglutamate; and/or, the heavy chain or heavy chain constant region (CH) or variant thereof lacks C-terminal lysine.

In some embodiments, the present invention provides a composition of antibody-drug conjugate, which comprises the antibody-drug conjugate as defined in any of the preceding items, wherein the composition has a DAR value of 1.0 to 10.0, preferably 1.0 to 5.0, for example, 1.0 to 1.5, 1.0 to 2.0, 1.0 to 2.5, 1.0 to 3.0, 1.0 to 3.5, 1.0 to 4.0, 1.0 to 4.5, 1.0 to 5.0, 2.0 to 2.5, 2.0 to 3.0, 2.0 to 3.5, 2.0 to 4.0, 2.0 to 4.5, 2.0 to 5.0, 2.5 to 3.0, 2.5 to 3.5, 2.5 to 4.0, 2.5 to 4.5, 2.5 to 5.0, 3.0 to 3.5, 3.0 to 4.0, 3.0 to 4.5, 3.0 to 5.0, 3.5 to 4.0, 3.5 to 4.5, 3.5 to 5.0, 4.0 to 4.5, 4.0 to 5.0, 4.5 to 5.0, for example, about 1.0, about 1.01, about 1.02, about 1. 03, about 1.04, about 1.05, about 1.06, about 1.07, about 1.08, about 1.09, about 1.1, about 1.11, about 1.12, about 1.13, about 1.14, about 1.15, about 1.16, about 1.17, about 1.18, about 1.19, about 1.2, about 1.21, about 1.22, about 1.23, about 1.24, about 1.25, about 1.26, about 1.27, about 1.28, about 1.29, about 1.3, about 1.31, about 1.32, about 1.33, about 1.34, about 1.35, about 1.36, about 1.37, about 1.38, about 1.39, about 1.4, about 1.41, about 1.42, about 1.43, about 1.44, about 1.45, about 1.46, about 1.47, about 1.48, about 1.49, about 1.5, about 1.51, about 1.52, about 1.53, about 1.54, about 1.55, about 1.56, about 1.57, about 1.58, about 1.59, about 1.6, about 1.61, about 1.62, about 1.63, about 1.64, about 1.65, about 1.66, about 1.67, about 1.68, about 1.69, about 1.7, about 1.71, about 1.72, about 1.73, about 1.74, about 1.75, about 1.76, about 1.77, about 1.78, about 1.79, about 1.8, about 1.81, about 1.82, about 1.83, about 1.84, about 1.85, about 1.86, about 1.87, about 1.88, about 1.89, about 1.9, about 1.91, about 1.92, about 1.93, about 1.94, about 1.95, about 1.96, about 1.97, about 1.98, about 1.99, about 2.0, about 2.01, about 2.02, about 2.03, about 2.04, about 2.05, about 2.06, about 2.07, about 2.08, about 2.09, about 2.1, about 2.11, about 2.12, about 2.13, about 2.14, about 2.15, about 2.16, about 2.17, about 2.18, about 2.19, about 2.2, about 2.21, about 2.22, about 2.23, about 2.24, about 2.25, about 2.26, about 2.27, about 2.28, about 2.29, about 2.3, about 2.31, about 2.32, about 2.33, about 2.34, about 2.35, about 2.36, about 2.37, about 2.38, about 2.39, about 2.4, about 2.41, about 2.42, about 2.43, about 2.44, about 2.45, about 2.46, about 2.47, about 2.48, about 2.49, about 2.5, about 2.51, about 2.52, about 2.53, about 2.54, about 2.55, about 2.56, about 2.57, about 2.58, about 2.59, about 2.6, about 2.61, about 2.62, about 2.63, about 2.64, about 2.65, about 2.66, about 2.67, about 2.68, about 2.69, about 2.7, about 2.71, about 2.72, about 2.73, about 2.74, about 2.75, about 2.76, about 2.77, about 2.78, about 2.79, about 2.8, about 2.81, about 2.82, about 2.83, about 2.84, about 2.85, about 2.86, about 2.87, about 2.88, about 2.89, about 2.9, about 2.91, about 2.92, about 2.93, about 2.94, about 2.95, about 2.96, about 2.97, about 2.98, about 2.99, about 3.0, about 3.01, about 3.02, about 3.03, about 3.04, about 3.05, about 3.06, about 3.07, about 3.08, about 3.09, about 3.1, about 3.11, about 3.12, about 3.13, about 3.14, about 3.15, about 3.16, about 3.17, about 3.18, about 3.19, about 3.2, about 3.21, about 3.22, about 3.23, about 3.24, about 3.25, about 3.26, about 3.27, about 3.28, about 3.29, about 3.3, about 3.31, about 3.32, about 3.33, about 3.34, about 3.35, about 3.36, about 3.37, about 3.38, about 3.39, about 3.4, about 3.41, about 3.42, about 3.43, about 3.44, about 3.45, about 3.46, about 3.47, about 3.48, about 3.49, about 3.5, about 3.51, about 3.52, about 3.53, about 3.54, about 3.55, about 3.56, about 3.57, about 3.58, about 3.59, about 3.6, about 3.61, about 3.62, about 3.63, about 3.64, about 3.65, about 3.66, about 3.67, about 3.68, about 3.69, about 3.7, about 3.71, about 3.72, about 3.73, about 3.74, about 3.75, about 3.76, about 3.77, about 3.78, about 3.79, about 3.8, about 3.81, about 3.82, about 3.83, about 3.84, about 3.85, about 3.86, about 3.87, about 3.88, about 3.89, about 3.9, about 3.91, about 3.92, about 3.93, about 3.94, about 3.95, about 3.96, about 3.97, about 3.98, about 3.99, about 4.0, about 4.01, about 4.02, about 4.03, about 4.04, about 4.05, about 4.06, about 4.07, about 4.08, about 4.09, about 4.1, about 4.11, about 4.12, about 4.13, about 4.14, about 4.15, about 4.16, about 4.17, about 4.18, about 4.19, about 4.2, about 4.21, about 4.22, about 4.23, about 4.24, about 4.25, about 4.26, about 4.27, about 4.28, about 4.29, about 4.3, about 4.31, about 4.32, about 4.33, about 4.34, about 4.35, about 4.36, about 4.37, about 4.38, about 4.39, about 4.4, about 4.41, about 4.42, about 4.43, about 4.44, about 4.45, about 4.46, about 4.47, about 4.48, about 4.49, about 4.5, about 4.51, about 4.52, about 4.53, about 4.54, about 4.55, about 4.56, about 4.57, about 4.58, about 4.59, about 4.6, about 4.61, about 4.62, about 4.63, about 4.64, about 4.65, about 4.66, about 4.67, about 4.68, about 4.69, about 4.7, about 4.71, about 4.72, about 4.73, about 4.74, about 4.75, about 4.76, about 4.77, about 4.78, about 4.79, about 4.8, about 4.81, about 4.82, about 4.83, about 4.84, about 4.85, about 4.86, about 4.87, about 4.88, about 4.89, about 4.9, about 4.91, about 4.92, about 4.93, about 4.94, about 4.95, about 4.96, about 4.97, about 4.98, about 4.99, about 5.0.

In some embodiments, the antibody-drug conjugate is selected from the group consisting of **iADC-1** to **iADC-25,** wherein each m ranges from 1 to 10, such as 1, 2, 3, 4, 5, 6, 7, 8, 9 or 10; Ab-(S- is an antibody or antigen-binding fragment thereof comprising a VH as set forth in SEQ ID NO: 23 or a VH as set forth in SEQ ID NO: 1 and a VL as set forth in SEQ ID NO: 2, for example, Ab-01 (which comprises: a heavy chain having a sequence as set forth in SEQ ID NO: 22, and a light chain having a sequence as set forth in SEQ ID NO: 21), for example, Ab-02 (which comprises: a heavy chain having a sequence as set forth in SEQ ID NO: 20, and a light chain having a sequence as set forth in SEQ ID NO: 21), and S in Ab-(S-) is derived from a sulfhydryl group in the antibody or antigen-binding fragment thereof, such as a sulfhydryl group of a cysteine residue in the antibody or antigen-binding fragment thereof.

In some embodiments, the antibody-drug conjugate is selected from the group consisting of **iADC-8a** to **iADC-12a:** 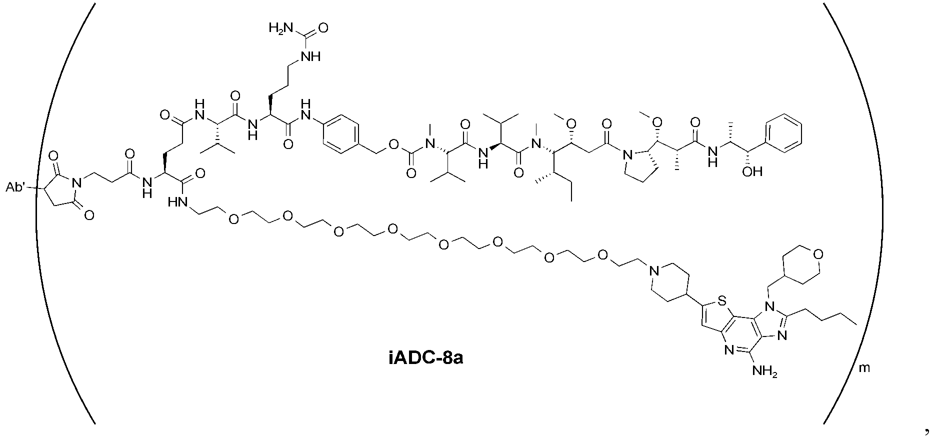 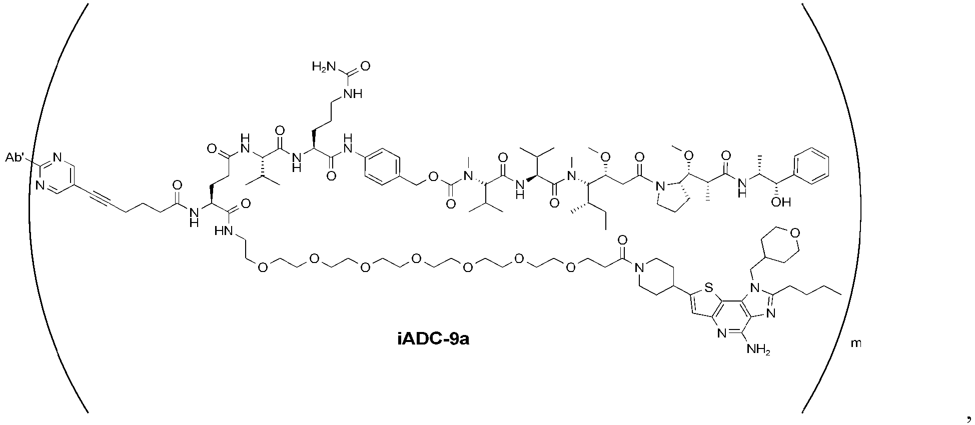 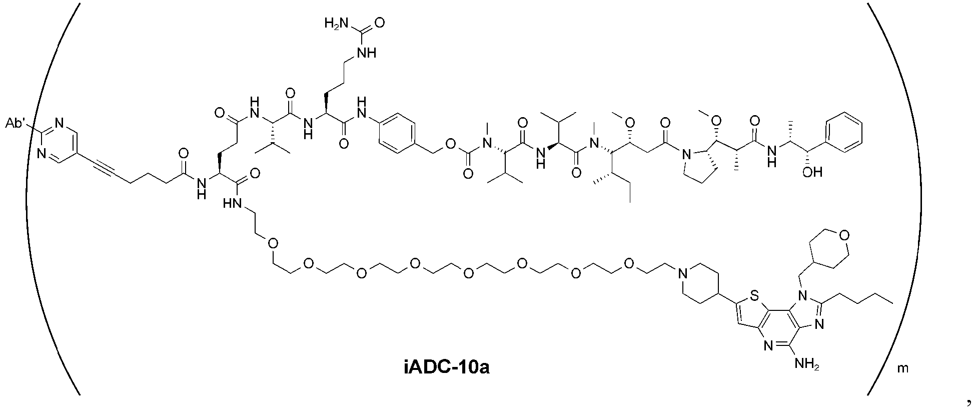 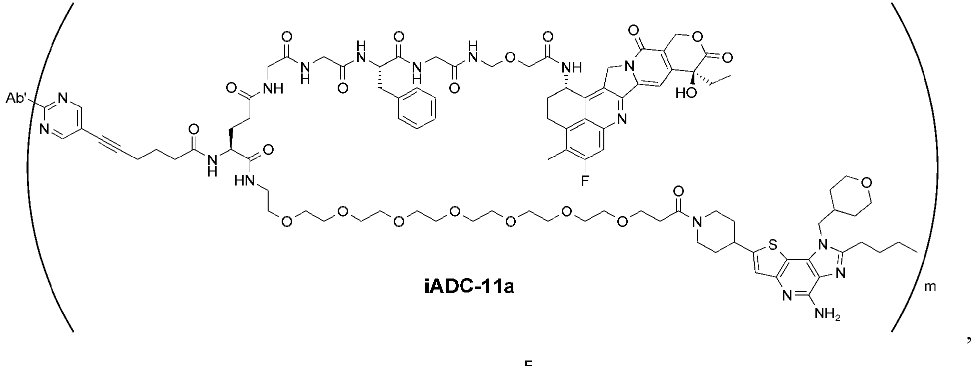 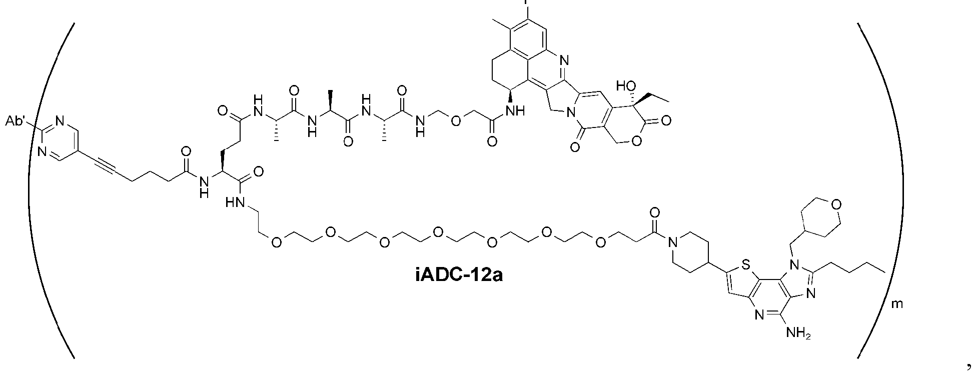 wherein Ab' represents the antibody or antibody-binding fragment thereof as described above, and each m ranges from 1 to 10, such as 1, 2, 3, 4, 5, 6, 7, 8, 9, or 10.

In some embodiments, the antibody-drug conjugate is selected from the group consisting of **iADC-8a to iADC-12a,** wherein each m ranges from 1 to 10, such as 1, 2, 3, 4, 5, 6, 7, 8, 9, or 10, and Ab' represents an antibody or antigen-binding fragment thereof comprising a VH having a sequence as set forth in SEQ ID NO: 23 or a VH having a sequence as set forth in SEQ ID NO: 1 and a VL having a sequence as set forth in SEQ ID NO: 2, for example, Ab-01 (which comprises: a heavy chain having a sequence as set forth in SEQ ID NO: 22, and a VL having a sequence as set forth in SEQ ID NO: 21), for example, Ab-02 (which comprises: a heavy chain having a sequence as set forth in SEQ ID NO: 20, and a light chain having a sequence as set forth in SEQ ID NO: 21).

In some embodiments, Ab' is linked via one or more cysteine residue sulfhydryl groups, one or more lysine residue amino groups, one or more threonine residue hydroxyl groups, or one or more serine residue hydroxyl groups in the antibody to form the conjugate.

In some embodiments, Ab' is linked via one or more cysteine residue sulfhydryl groups in the antibody to form the conjugate.

In some embodiments, Ab' is linked via one or more lysine residue amino groups in the antibody to form the conjugate.

In some embodiments, Ab' is linked via one or more threonine residue hydroxyl groups in the antibody to form the conjugate.

In some embodiments, Ab' is linked via one or more serine residue hydroxyl groups in the antibody to form the conjugate.

In some embodiments, Ab' is linked via m cysteine residue sulfhydryl groups, m lysine residue amino groups, m threonine residue hydroxyl groups, or m serine residue hydroxyl groups in the antibody to form the conjugate.

In some embodiments, Ab' is linked via m cysteine residue sulfhydryl groups in the antibody.

In some embodiments, Ab' is linked via m lysine residue amino groups in the antibody.

In some embodiments, Ab' is linked via m threonine residue hydroxyl groups in the antibody.

In some embodiments, Ab' is linked via m serine residue hydroxyl groups in the antibody.

In some embodiments, the antibody-drug conjugates of the present invention are optionally substituted with one or more suitable substituents.

In some embodiments, the antibody-drug conjugates of the present invention have improved cancer treatment effects. In some embodiments, the antibody-drug conjugates of the present invention have better physicochemical properties. In some embodiments, the antibody-drug conjugates of the present invention have better safety.

### Drug-linker

In another aspect, the present invention provides a drug-linker to which both a cytotoxic drug and a TLR agonist are conjugated, which can be used to prepare the aforementioned antibody-drug conjugates.

Specifically, the present invention provides a compound as represented by Formula (II), or a pharmaceutically acceptable salt, ester, stereoisomer, tautomer, polymorph, solvate, N-oxide, isotope-labeled compound, metabolite or prodrug thereof: 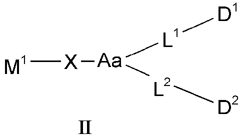 wherein,
X, Aa, L¹, L², D¹, and D² are as defined above,
M¹ represents a precursor of the linking site attached to an antibody or antigen-binding fragment thereof.

In some embodiments, the antibody or antigen-binding fragment thereof is as defined above.

In some embodiments, M¹ is selected from the group consisting of the following structures: 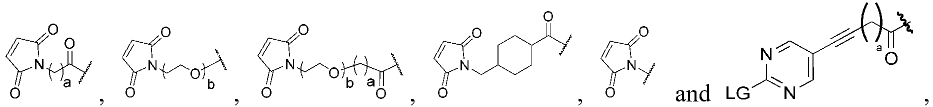 wherein, each a is independently an integer ranging from 0 to 6, b is an integer ranging from 1 to 10, and LG represents a leaving group.

In some embodiments, M¹ is selected from the group consisting of the following structures:

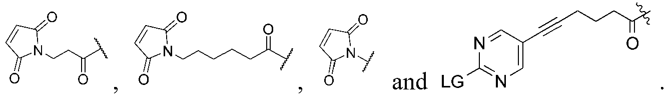

In some embodiments, LG each is independently selected from the group consisting of halogen (e.g., F, Cl, Br, I), halogenated C₁₋₆ alkyl, C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, hydroxyl, sulfhydryl group, amino, nitro, azido, cyano, alkenyl, alkynyl and alkynyl-containing structural fragment, the halogenated C₁₋₆ alkyl, C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, alkenyl, alkynyl and alkynyl-containing structural fragment are optionally substituted with one or more suitable substituents.

In some embodiments, LG is independently selected from the group consisting of halogen (e.g., F, Cl, Br, I), halogenated C₁₋₆ alkyl, C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, hydroxyl, sulfhydryl group, amino, nitro, azido, cyano, alkenyl, alkynyl, and alkynyl-containing structural fragment.

In some embodiments, LG is C₁₋₆ alkylsulfonyl, for example, methylsulfonyl.

In some embodiments, M¹ is selected from the group consisting of the following structures: 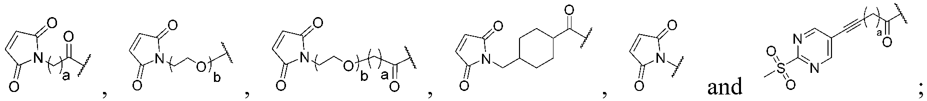 wherein each a is independently an integer ranging from 0 to 6, and b is an integer ranging from 1 to 10.

In some embodiments, M¹ is selected from the group consisting of the following structures:

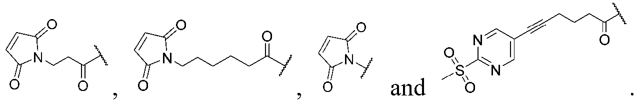

In some embodiments, M¹ is 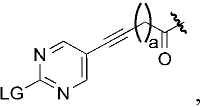 a is an integer ranging from 0 to 6;
L² is 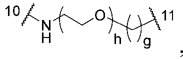 , wherein g is an integer ranging from 0 to 6, h is an integer ranging from 1 to 10; the position 10 of L² is connected to Aa, and the position 11 is connected to D²;
D² is

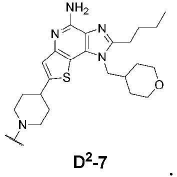

In some embodiments, M¹ is 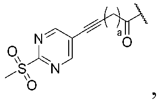 a is an integer ranging from 0 to 6.

In some embodiments, M¹ is

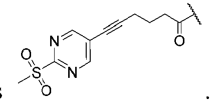

In some embodiments, L² is

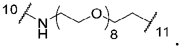

In some embodiments, 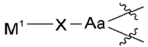 is selected from 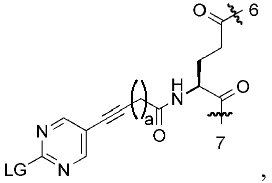 wherein, a is an integer ranging from 0 to 6; the position 6 is connected to L¹, and the position 7 is connected to L².

In some embodiments, 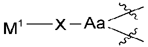 is selected from 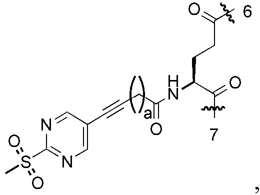 wherein a is an integer ranging from 0 to 6; the position 6 is connected to L¹, and the position 7 is connected to L².

In some embodiments, 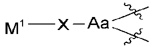 is selected from 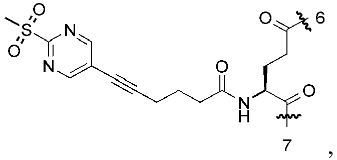 wherein a is an integer ranging from 0 to 6; the position 6 is connected to L¹, and the position 7 is connected to L².

In some embodiments, 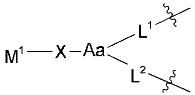 is selected from 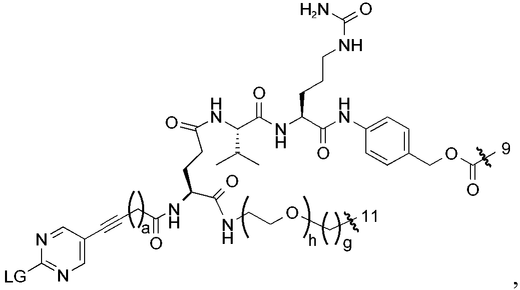 wherein a is an integer ranging from 0 to 6, g is an integer ranging from 0 to 6, and h is an integer ranging from 1 to 10; the position 9 is connected to D¹, the position 11 is connected to D², and LG is as defined in any of the preceding items.

In some embodiments, 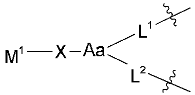 is selected from 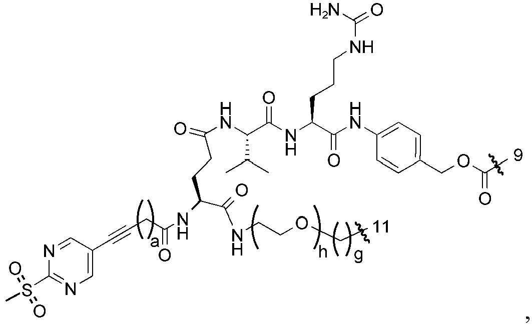 wherein a is an integer ranging from 0 to 6, g is an integer ranging from 0 to 6, and h is an integer ranging from 1 to 10; the position 9 is connected to D¹, and the position 11 is connected to D².

In some embodiments, 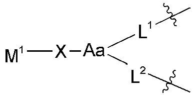 is selected from 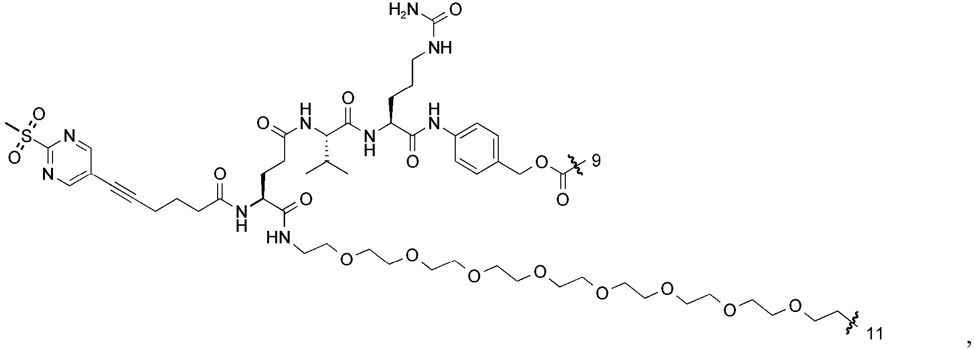 wherein the position 9 is connected to D¹, and the position 11 is connected to D².

In some embodiments, the compound represented by Formula (II) is selected from the group consisting of the following structures: 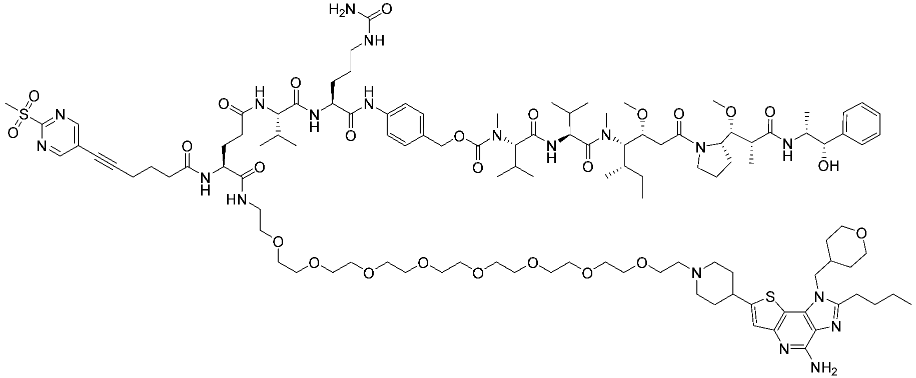 and

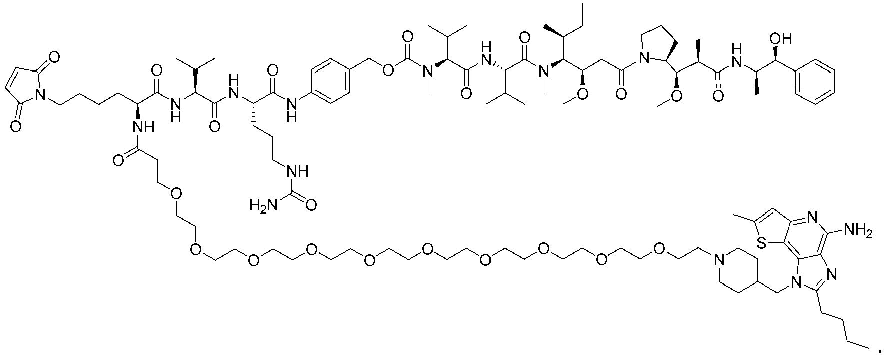

In some embodiments, the drug-linker described herein is optionally substituted with one or more suitable substituents.

In Formula I and Formula II above, the groups of all embodiments can be appropriately selected and combined to obtain different general formula ranges or specific embodiments. These ranges and embodiments are all within the present invention. The present invention covers compounds or antibody-drug conjugates obtained by any combination of the various embodiments.

### Intermediates/linker units

In some embodiments, the present invention provides a compound represented by Formula (III), or a salt, ester, stereoisomer, tautomer, polymorph, solvate, N-oxide, isotope-labeled compound, metabolite or prodrug thereof: 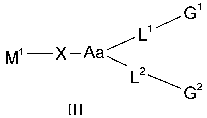 wherein,
X, Aa, L¹, L², and M¹ are as defined above;
G¹ is p-nitrophenoxy or C₁₋₆ alkanoyloxy;
G² is hydroxyl or oxo.

In some embodiments, when the structure at the position 9 of L¹ is 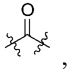 G¹ is p-nitrophenoxy.

In some embodiments, when the structure at the position 9 of L¹ is 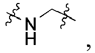 G¹ is C₁₋₆ alkanoyloxy.

In some embodiments, when the structure at the position 11 of L² is 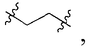 G² is oxo.

In some embodiments, when the structure at the position 11 of L² is 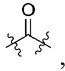 G² is hydroxyl.

In some embodiments, the compound represented by Formula (III) is selected from the group consisting of: 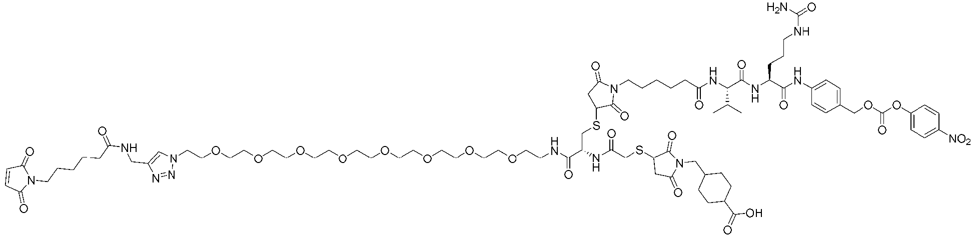 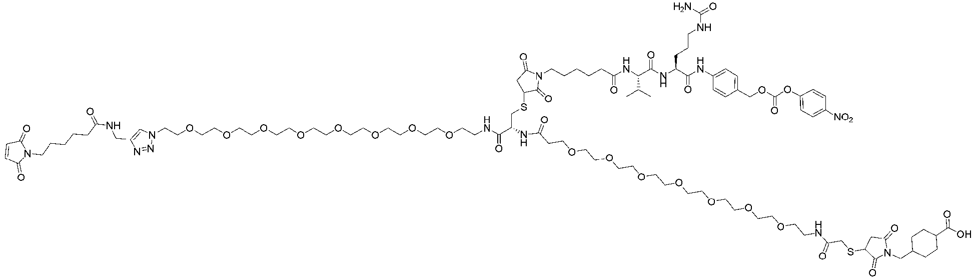 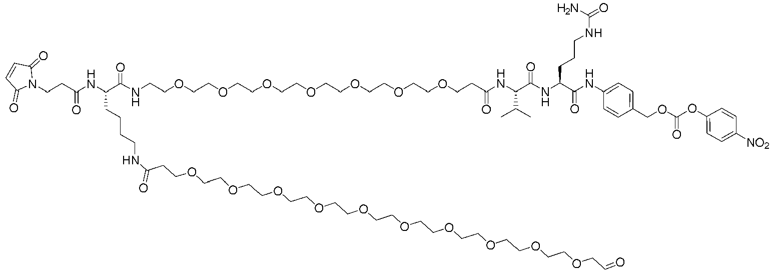 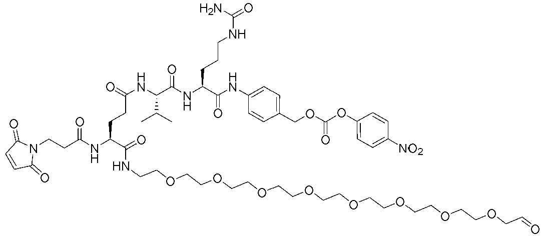 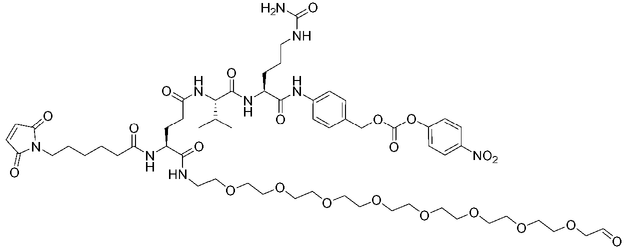 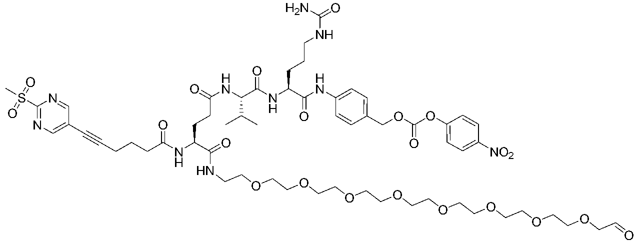 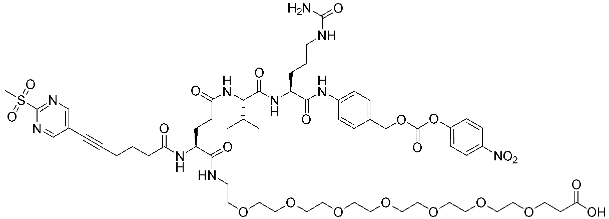 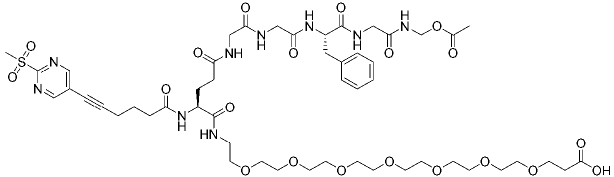 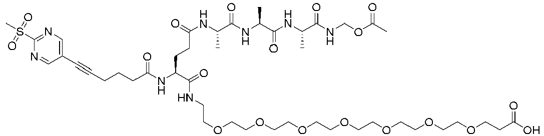 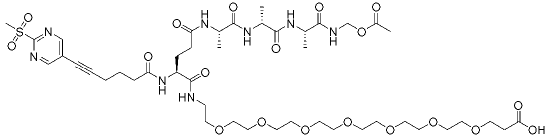 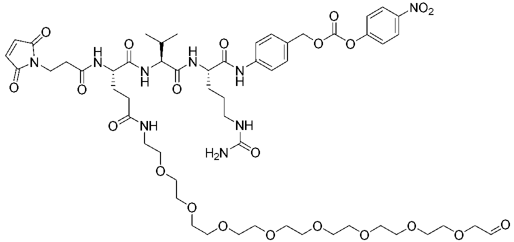 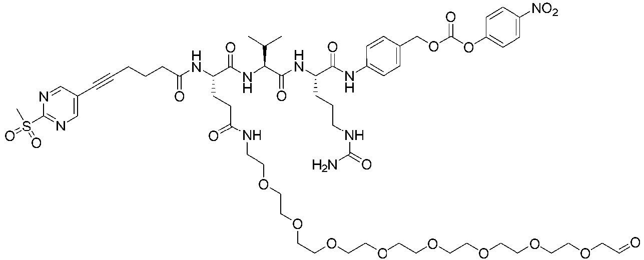 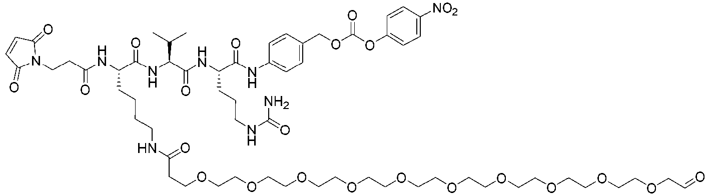 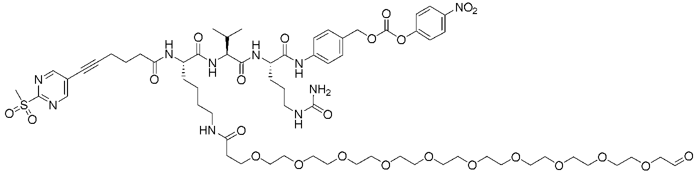 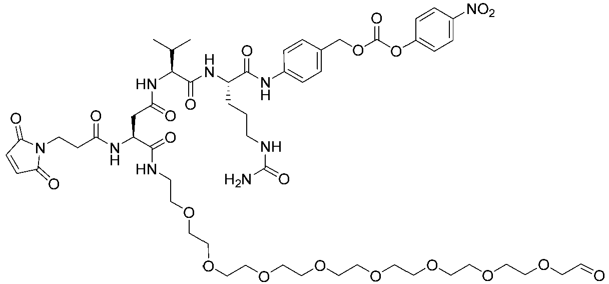 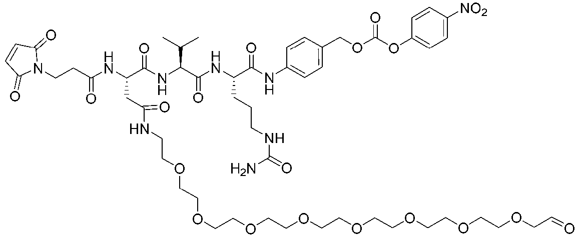 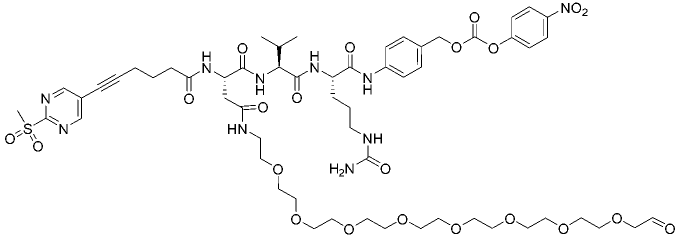 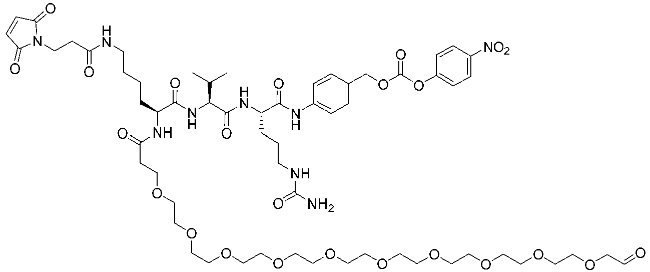 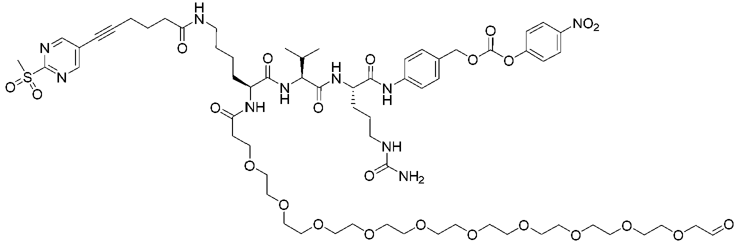 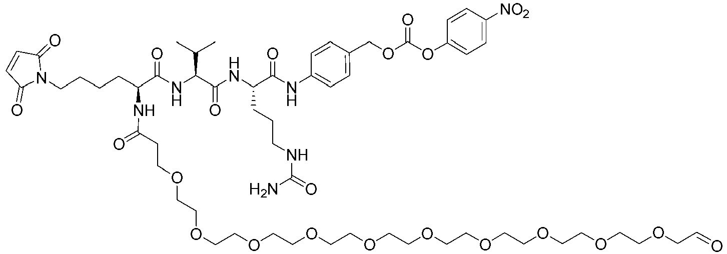 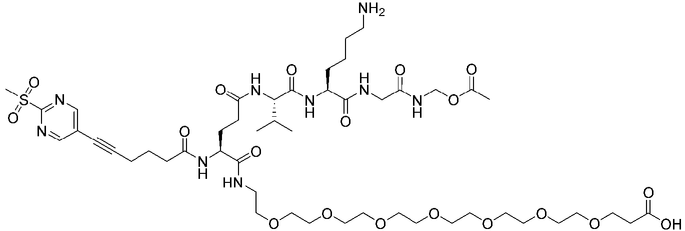 and

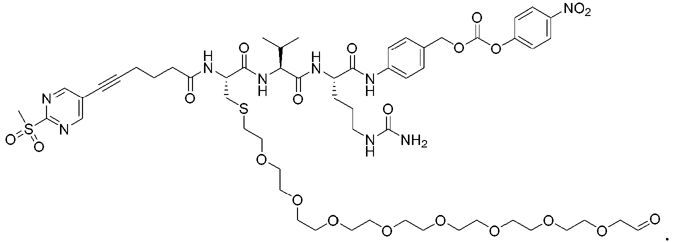

In some embodiments, the present invention provides the following compounds or their salts, esters, stereoisomers, tautomers, polymorphs, solvates, N-oxides, isotope-labeled compounds, metabolites, or prodrugs: 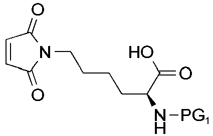 and 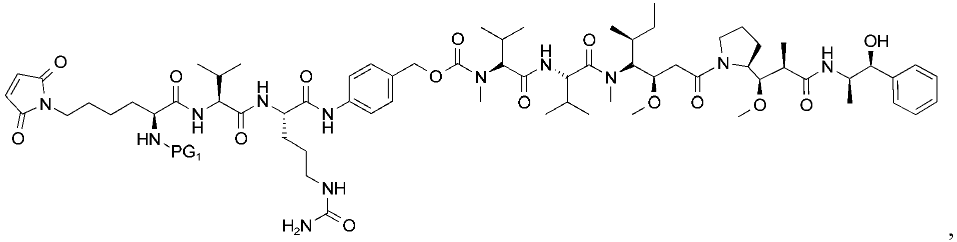 wherein, PG₁ is each independently H or an amino-protecting group, preferably an alkoxycarbonyl amino-protecting group, such as benzyloxycarbonyl (Cbz), tert-butyloxycarbonyl (Boc), methyloxycarbonyl (Fmoc), allyloxycarbonyl (Alloc), trimethylsilylethoxycarbonyl (Teoc), methyl(or ethyl)oxycarbonyl; an acyl amino-protecting group, such as phthaloyl (Pht), tosyl (Tos), trifluoroacetyl (Tfa), o-(p-)nitrobenzenesulfonyl (Ns), pivaloyl, benzoyl, tert-butyloxycarbonyl, 9-fluorenylmethyloxycarbonyl, allyloxycarbonyl, trichloroethoxycarbonyl, trimethylsilylethoxycarbonyl, benzyloxycarbonyl, tosyl, p-nitrobenzenesulfonyl, trifluoroacetyl, methoxycarbonyl, or ethoxycarbonyl; or an alkyl amino-protecting group, such as trityl (Trt), 2,4-dimethoxybenzyl (Dmb), 4-methoxybenzyl (PMB), and benzyl (Bn).

In some embodiments, the present invention provides the following compounds, or salts, esters, stereoisomers, tautomers, polymorphs, solvates, N-oxides, isotope-labeled compounds, metabolites, or prodrugs thereof: 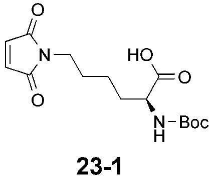 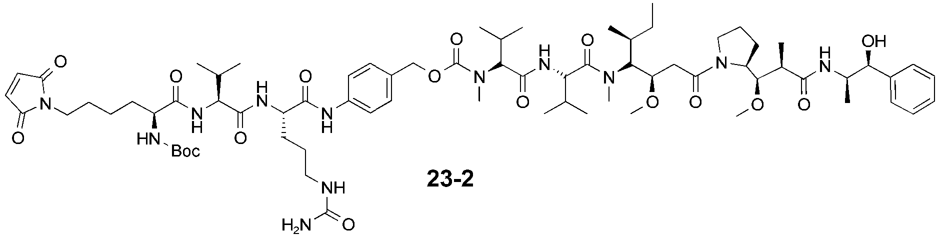 an

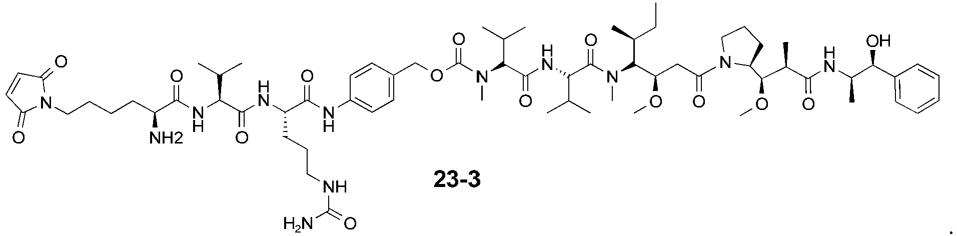

The compounds shown above, or their salts, esters, stereoisomers, tautomers, polymorphs, solvates, N-oxides, isotope-labeled compounds, metabolites, or prodrugs, can be used to prepare the drug-linker or antibody-drug conjugate as described herein.

In some embodiments, the present invention provides a linker unit, which is a compound having the structure of 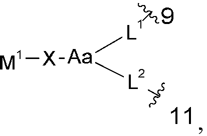 or a salt, ester, stereoisomer, tautomer, polymorph, solvate, *N*-oxide, isotope-labeled compound, metabolite or prodrug thereof, wherein X, Aa, L¹, L², and M¹ are as defined above.

In some embodiments, the compound having the structure of 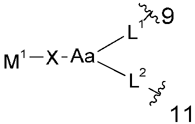 is selected from the group consisting of: 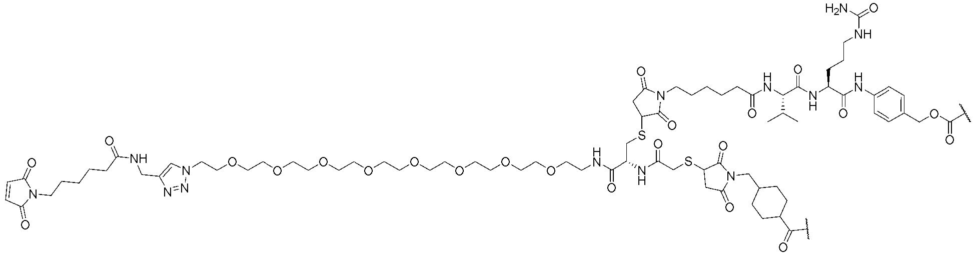 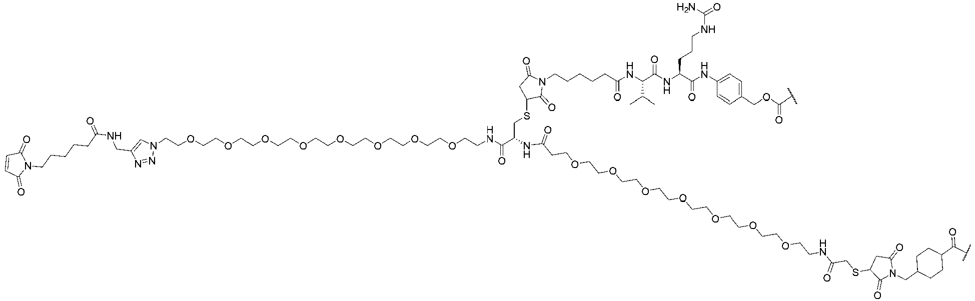 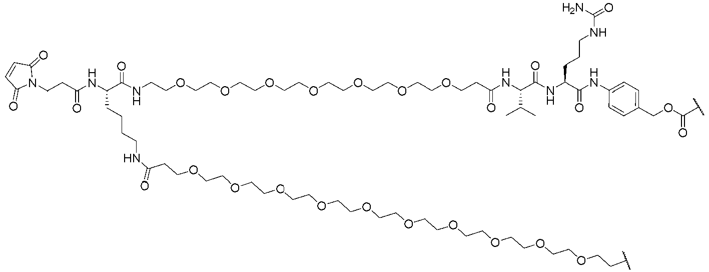 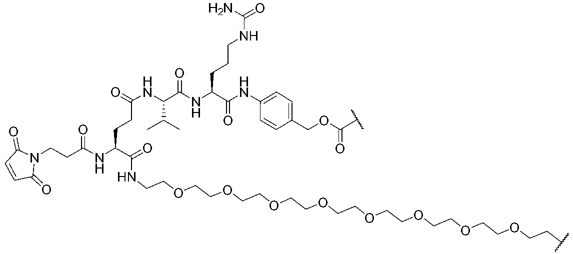 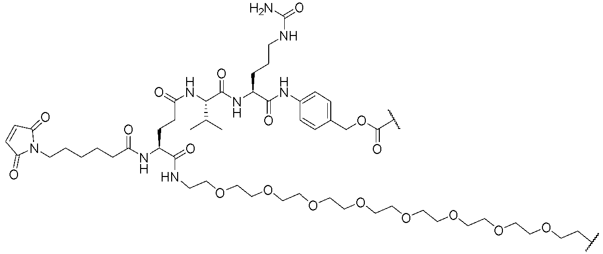 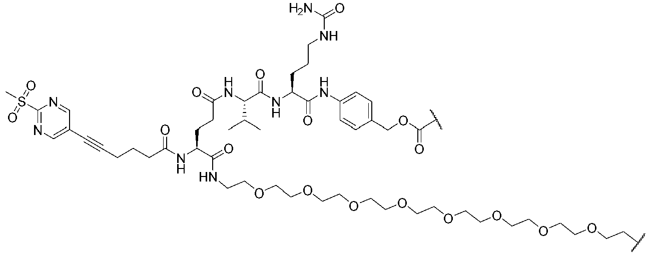 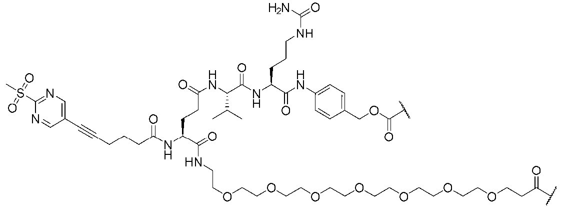 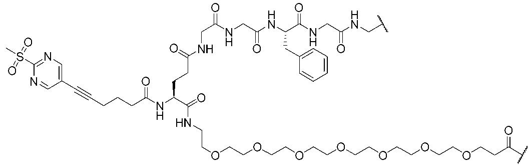 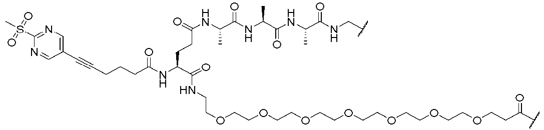 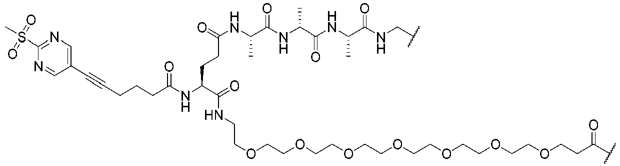 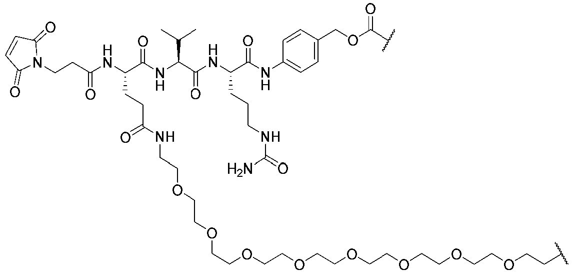 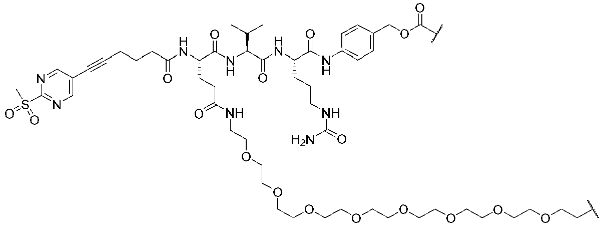 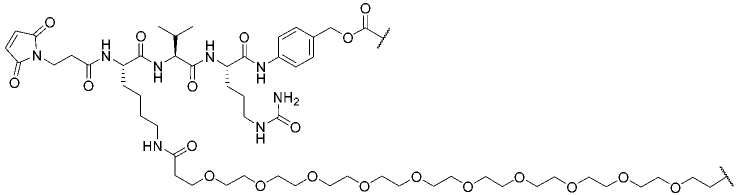 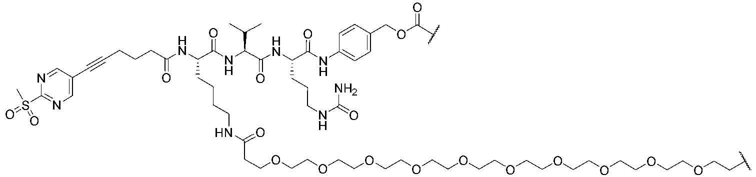 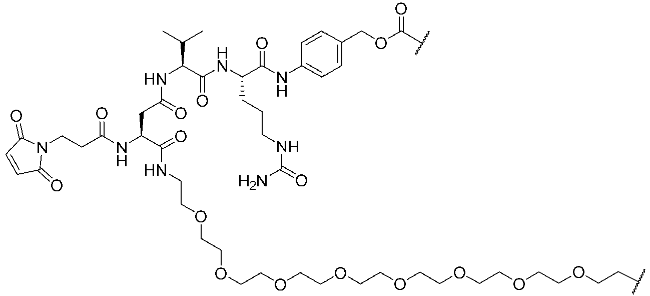 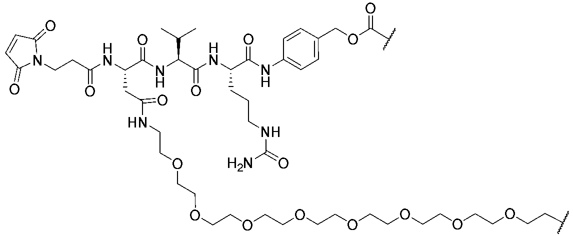 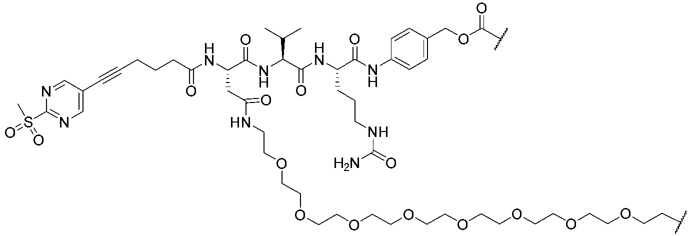 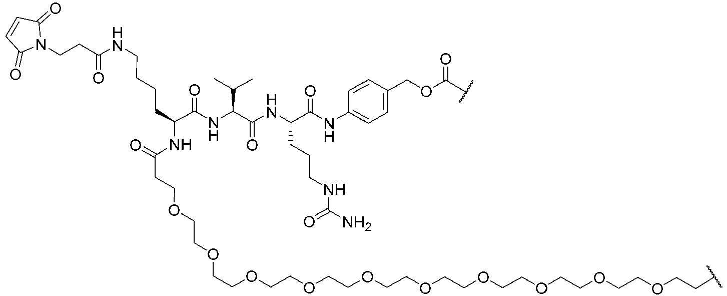 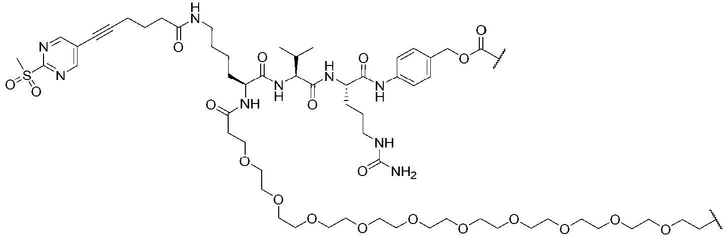 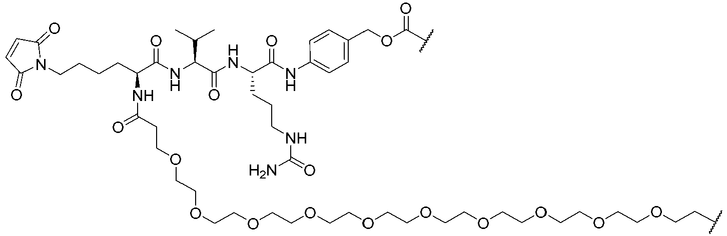 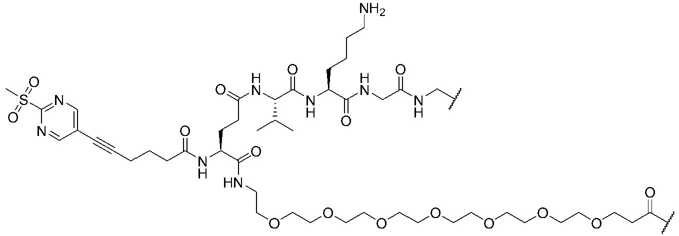 and

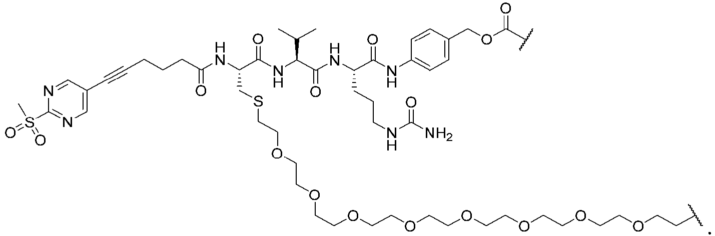

In some embodiments, the present invention provides a linker unit, which is a compound having the structure represented by Formula (IV), or a salt, ester, stereoisomer, tautomer, polymorph, solvate, N-oxide, isotope-labeled compound, metabolite or prodrug thereof: 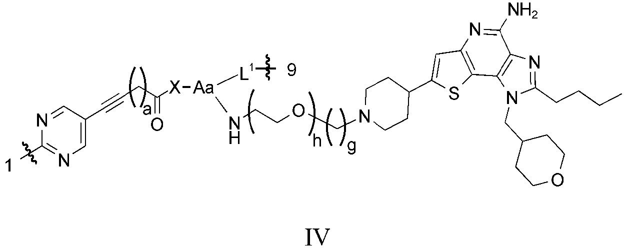 wherein X, Aa, L¹, a, g, and h are as defined above.

In some embodiments, the provided is a compound, or a pharmaceutically acceptable salt, ester, stereoisomer, tautomer, polymorph, solvate, N-oxide, isotope-labeled compound, metabolite or prodrug thereof, which comprises a linker unit having the structure represented by Formula (IV) and at least one of LG or D¹, wherein the linker unit is linked to LG or D¹, wherein D¹ is linked to the position 9 of the linker unit; and LG is linked to the position 1 of the linker unit; wherein D¹ is as defined above, and LG is as defined above.

In some embodiments, the present invention provides a linker unit, which is a compound having the structure represented by Formula (IVa), or a salt, ester, stereoisomer, tautomer, polymorph, solvate, N-oxide, isotope-labeled compound, metabolite or prodrug thereof: 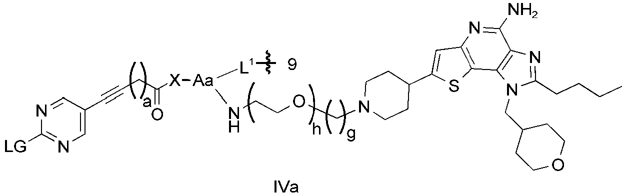 wherein LG, X, Aa, L¹, a, g, and h are as defined above.

In some embodiments, the provided is a compound, or a pharmaceutically acceptable salt, ester, stereoisomer, tautomer, polymorph, solvate, *N*-oxide, isotope-labeled compound, metabolite or prodrug thereof, which comprises a linker unit represented by Formula (IVa) and optionally D¹, wherein the linker unit is linked to D¹, wherein D¹ is linked to the position 9 of the linker unit; and D¹ is as defined above.

In some embodiments, the present invention provides a linker unit, which has the following structure: 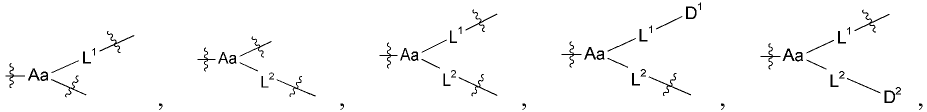 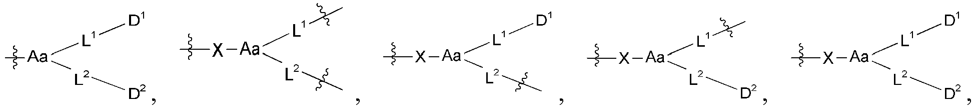 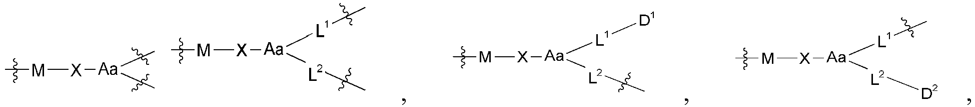 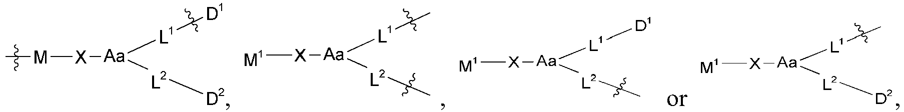 wherein, M, M¹, X, Aa, L¹, L², D¹, and D² are as defined above.

In some embodiments, the intermediates described herein are optionally substituted with one or more suitable substituents.

### Antibody or antigen-binding fragment thereof

In another aspect, the present invention provides an antibody or antigen-binding fragment thereof that specifically binds to B7H3, wherein the antibody or antigen-binding fragment thereof comprises:
(1) the following heavy chain variable region (VH) and/or light chain variable region (VL):
   a heavy chain variable region (VH) comprising the following 3 CDRs: a CDR-H1 having a sequence as set forth in SEQ ID NO: 11 or a variant thereof, a CDR-H2 having a sequence as set forth in SEQ ID NO: 12 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(2) the following heavy chain variable region (VH) and/or light chain variable region (VL):
   a heavy chain variable region (VH) comprising the following 3 CDRs: a CDR-H1 having a sequence as set forth in SEQ ID NO: 14 or a variant thereof, a CDR-H2 having a sequence as set forth in SEQ ID NO: 15 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(3) the following heavy chain variable region (VH) and/or light chain variable region (VL):
   a heavy chain variable region (VH) comprising the following 3 CDRs: a CDR-H1 having a sequence as set forth in SEQ ID NO: 8 or a variant thereof, a CDR-H2 having a sequence as set forth in SEQ ID NO: 9 or a variant thereof, a CDR-H3 having a sequence as set forth in SEQ ID NO: 10 or a variant thereof; and/or, a light chain variable region (VL) comprising the following 3 CDRs: a CDR-L1 having a sequence as set forth in SEQ ID NO: 3 or a variant thereof, a CDR-L2 having a sequence as set forth in SEQ ID NO: 4 or a variant thereof, and a CDR-L3 having a sequence as set forth in SEQ ID NO: 5 or a variant thereof; or
(4) the following heavy chain variable region (VH) and/or light chain variable region (VL):
   a heavy chain variable region (VH) comprising the following 3 CDRs: a CDR-H1 having a sequence as set forth in SEQ ID NO: 16 or a variant thereof, a CDR-H2 having a sequence as set forth in SEQ ID NO: 17 or a variant thereof, a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof.

In some embodiments, the antibody or antigen-binding fragment thereof comprises:
(1) the following heavy chain variable region (VH) and/or light chain variable region (VL), wherein the CDRs are defined according to the Chothia numbering system:
   a heavy chain variable region (VH) comprising the following 3 CDRs: a CDR-H1 having a sequence as set forth in SEQ ID NO: 11 or a variant thereof, a CDR-H2 having a sequence as set forth in SEQ ID NO: 12 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(2) the following heavy chain variable region (VH) and/or light chain variable region (VL), wherein the CDRs are defined according to the Kabat numbering system:
   a heavy chain variable region (VH) comprising the following 3 CDRs: a CDR-H1 having a sequence as set forth in SEQ ID NO: 14 or a variant thereof, a CDR-H2 having a sequence as set forth in SEQ ID NO: 15 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(3) the following heavy chain variable region (VH) and/or light chain variable region (VL), wherein the CDRs are defined according to the IMGT numbering system:
   a heavy chain variable region (VH) comprising the following 3 CDRs: a CDR-H1 having a sequence as set forth in SEQ ID NO: 8 or a variant thereof, a CDR-H2 having a sequence as set forth in SEQ ID NO: 9 or a variant thereof, and a CDR-H3 having a sequence as set forth in SEQ ID NO: 10 or a variant thereof; and/or, a light chain variable region (VL) comprising the following 3 CDRs: a CDR-L1 having a sequence as set forth in SEQ ID NO: 3 or a variant thereof, a CDR-L2 having a sequence as set forth in SEQ ID NO: 4 or a variant thereof, and a CDR-L3 having a sequence as set forth in SEQ ID NO: 5 or a variant thereof; or
(4) the following heavy chain variable region (VH) and/or light chain variable region (VL), wherein the CDRs are defined according to the AbM numbering system:
   a heavy chain variable region (VH) comprising the following 3 CDRs: a CDR-H1 having a sequence as set forth in SEQ ID NO: 16 or a variant thereof, a CDR-H2 having a sequence as set forth in SEQ ID NO: 17 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof.

In some embodiments, the antibody or antigen-binding fragment thereof comprises: a VH as set forth in SEQ ID NO: 23 or 1 or a variant thereof, and/or, a VL as set forth in SEQ ID NO: 2 or a variant thereof.

In some embodiments, the antibody or antigen-binding fragment thereof comprises: a heavy chain comprising: a VH as set forth in SEQ ID NO: 23 or 1 or a variant thereof, and a heavy chain constant region (CH) as set forth in SEQ ID NO: 18 or a variant thereof, and/or, a light chain comprising: a VL as set forth in SEQ ID NO: 2 or a variant thereof, and a light chain constant region (CL) as set forth in SEQ ID NO: 19 or a variant thereof.

In some embodiments, the amino acid sequence of the heavy chain of the antibody or antigen-binding fragment thereof is set forth in SEQ ID NO: 22 or 20, and the amino acid sequence of the light chain of the antibody or antigen-binding fragment thereof is set forth in SEQ ID NO: 21.

In some embodiments, the N-terminal glutamine of the heavy chain or heavy chain variable region and/or the light chain or light chain variable region undergoes cyclization to form a pyroglutamic acid or pyroglutamate; and/or, the C-terminal lysine is absent from the heavy chain or heavy chain constant region (CH) or variant thereof.

In some embodiments, the variant has a sequence identity of at least 80%, at least 85%, at least 90%, at least 91%, at least 92%, at least 93%, at least 94%, at least 95%, at least 96%, at least 97%, at least 98%, at least 99%, or 100% as compared with the sequence from which it is derived, or has a substitution, deletion, or addition of one or more amino acids (e.g., a substitution, deletion, or addition of 1, 2, 3, 4, or 5 amino acids) as compared with the sequence from which it is derived; preferably, the substitution is a conservative substitution.

In some embodiments, the amino acid sequence of the heavy chain of the antibody or antigen-binding fragment thereof is set forth in SEQ ID NO: 22, and the amino acid sequence of the light chain of the antibody or antigen-binding fragment thereof is set forth in SEQ ID NO: 21.

In some embodiments, the amino acid sequence of the heavy chain of the antibody or antigen-binding fragment thereof is set forth in SEQ ID NO: 20, and the amino acid sequence of the light chain of the antibody or antigen-binding fragment thereof is set forth in SEQ ID NO: 21.

In certain embodiments of the antibody or antigen-binding fragment disclosed herein, the heavy chain constant domain may comprise a C-terminal lysine, or lack a C-terminal lysine or a C-terminal glycine-lysine dipeptide. In some embodiments of the antibody or antigen-binding fragment thereof, the N-terminal amino acid of the antibody or antigen-binding fragment thereof may be cyclized to form a pyroglutamic acid. In some embodiments of the antibody or antigen-binding fragment thereof, the N-terminal amino acid of the antibody or antigen-binding fragment thereof may be cyclized to form a pyroglutamic acid.

As known to those skilled in the art, pyroglutamic acid is a conjugate acid of pyroglutamate and is in equilibrium with pyroglutamate in solution.

In certain embodiments, the present invention provides a composition comprising the antibody or antigen-binding fragment as disclosed herein, wherein each antibody or antigen-binding fragment thereof may independently comprise a C-terminal lysine, lack a C-terminal lysine, lack a C-terminal glycine-lysine dipeptide, and/or, comprises a N-terminal glutamine or glutamic acid, a N-terminal amino acid cyclized to pyroglutamic acid, or a N-terminal amino acid cyclized to pyroglutamate.

In certain embodiments, the antibody or antigen-binding fragment disclosed herein includes an antibody or antigen-binding fragment that specifically binds to an antigen, and may include a post-translational modification thereof (e.g., cleavage of the C-terminal lysine in the heavy chain) (conversion of the N-terminal glutamine or glutamic acid in the heavy or light chains to pyroglutamic acid or pyroglutamate), which may occur during recombinant expression in a host cell (e.g., CHO cells) or during purification/storage.

In certain embodiments, the N-terminal glutamine of the VH comprising a sequence as set forth in SEQ ID NO: 23 or 1 or a variant thereof undergoes cyclization to form a pyroglutamic acid or pyroglutamate; and/or, the N-terminal glutamic acid of the VL comprising a sequence as set forth in SEQ ID NO: 2 or a variant thereof undergoes cyclization to form a pyroglutamic acid or pyroglutamate.

In some embodiments, the N-terminal glutamine of the heavy chain represented by SEQ ID NO: 22 or 20 and/or the light chain represented by SEQ ID NO: 21 undergoes cyclization to form a pyroglutamic acid or pyroglutamate; and/or, the heavy chain constant region (CH) represented by SEQ ID NO: 18 or a variant thereof lacks a C-terminal lysine.

### Conjugate

In another aspect, the present application provides a conjugate, which comprises the compound (e.g., drug-linkers, TLR agonists, or cytotoxic drugs), or a pharmaceutically acceptable salt, ester, stereoisomer, tautomer, polymorph, solvate, *N*-oxide, isotope-labeled compound, metabolite or prodrug thereof as described in any of the above items, or the linker unit as described in any of the above items. In some embodiments, the conjugate is an antibody-drug conjugate, wherein the compound (e.g., drug-linkers, TLR agonists, or cytotoxic drugs) or the pharmaceutically acceptable salt, ester, stereoisomer, tautomer, polymorph, solvate, *N*-oxide, isotope-labeled compound, metabolite or prodrug thereof as described in any of the above items is linked to an antibody or antigen-binding fragment thereof.

In some embodiments, the present application provides an antibody-drug conjugate, which comprises the antibody or antigen-binding fragment thereof as described in any of the above items. In some embodiments, the antibody or antigen-binding fragment thereof is linked to a bioactive molecule (e.g., TLR agonists and/or cytotoxic drugs) via a linker.

### Pharmaceutical composition

In another aspect, the present invention provides a pharmaceutical composition, which comprises the antibody-drug conjugate, compound (e.g., drug-linkers, TLR agonists, or cytotoxic drugs) or the pharmaceutically acceptable salt, ester, stereoisomer, tautomer, polymorph, solvate, N-oxide, isotope-labeled compound, metabolite or prodrug thereof as described in any of the above items, and one or more pharmaceutical excipients.

Methods for preparing various pharmaceutical compositions containing a certain amount of active ingredient are known or will be apparent to those skilled in the art based on the disclosure of the present invention. As described in REMINGTON'S PHARMACEUTICAL SCIENCES, Martin, E.W., ed., Mack Publishing Company, 19th ed. (1995), the methods for preparing pharmaceutical compositions comprise incorporating an appropriate pharmaceutical excipient.

### Therapeutic method and use

In another aspect, the present invention provides a use of the compound (e.g., drug-linkers, TLR agonists, or cytotoxic drugs) or the pharmaceutically acceptable salt, ester, stereoisomer, tautomer, polymorph, solvate, *N*-oxide, isotope-labeled compound, metabolite or prodrug thereof as described in any of the above items, in the manufacture of an antibody-drug conjugate, particularly the manufacture of the antibody-drug conjugate as described in any of the above items.

In another aspect, the present invention provides a use of the antibody-drug conjugate, compound (e.g., drug-linkers, TLR agonists, or cytotoxic drugs) or the pharmaceutically acceptable salt, ester, stereoisomer, tautomer, polymorph, solvate, *N*-oxide, isotope-labeled compound, metabolite or prodrug thereof, composition of antibody-drug conjugate, antibody or antigen-binding fragment thereof, or pharmaceutical composition, as described in any of the above items, in the preparation of a medicament, particularly in the manufacture of a medicament for treating and/or preventing a cancer (e.g., a cancer associated with B7H3 expression).

In another aspect, the present invention provides the antibody-drug conjugate, compound (e.g., drug-linkers, TLR agonists, or cytotoxic drugs) or the pharmaceutically acceptable salt, ester, stereoisomer, tautomer, polymorph, solvate, *N*-oxide, isotope-labeled compound, metabolite or prodrug thereof, composition of antibody-drug conjugate, antibody or antigen-binding fragment thereof, or pharmaceutical composition, as described in any of the above items, for use in treating and/or preventing a disease, such as a cancer (e.g., a cancer associated with B7H3 expression).

In another aspect, the present invention provides a method for treating and/or preventing cancer (e.g., a cancer associated with B7H3 expression), comprising administering to a subject in need thereof a therapeutically and/or prophylactically effective amount of the antibody-drug conjugate, compound (e.g., drug-linkers, TLR agonists, or cytotoxic drugs) or the pharmaceutically acceptable salt, ester, stereoisomer, tautomer, polymorph, solvate, *N*-oxide, isotope-labeled compound, metabolite or prodrug thereof, composition of antibody-drug conjugate, antibody or antigen-binding fragment thereof, or pharmaceutical composition, as described in any of the above items.

In some embodiments, the cancer is selected from the group consisting of breast cancer (e.g., breast ductal carcinoma), gastric cancer, lung cancer (e.g., lung adenocarcinoma), and colon cancer.

### Examples

To further clarify the objectives and technical solutions of the present invention, embodiments of the present invention are described in detail below with reference to examples. However, those skilled in the art will appreciate that the following examples are merely illustrative of the present invention and should not be construed as limiting the scope of the present invention. In cases where specific conditions are not specified, standard conditions or conditions recommended by the manufacturers are used. Reagents or instruments used, for which the manufacturers are not specified, are conventional commercially available products.

The structures of the compounds were determined by nuclear magnetic resonance (¹H NMR) or mass spectrometry (MS). ¹H NMR measurements were performed on a JEOL Eclipse 400 NMR spectrometer. The solvents used were deuterated methanol (CD₃OD), deuterated chloroform (CDCl₃), or hexadeuterated dimethyl sulfoxide (DMSO-*d6*). The internal standard was tetramethylsilane (TMS). Chemical shifts (δ) are given in parts per million (ppm).

MS measurements were performed on an Agilent (ESI) mass spectrometer, manufacturer: Agilent, model: Agilent 6120B.

Preparation method using preparative high performance liquid chromatograph:
Instrument model: Agilent 1260, Column: Waters SunFire Prep C18 OBD (19 mm × 150 mm × 5.0 µm); Column temperature: 25°C; Flow rate: 20.0 mL/min; Detection wavelength: 214 nm; Elution gradient: (0 min: 10% A, 90% B; 16.0 min: 90% A, 10% B); Mobile phase A: acetonitrile; Mobile phase B: 0.05% formic acid in water.

Thin-layer chromatography (TLC) silica gel plate: aluminum plate (20 × 20 cm) made by Merck; TLC separation and purification were performed by using GF 254 (1 mm) produced in Yantai.

The reaction was monitored by thin-layer chromatography (TLC) or LC-MS. The developing solvent systems used comprised: the system of dichloromethane and methanol, the system of n-hexane and ethyl acetate, and the system of petroleum ether and ethyl acetate. The volume ratios of the solvents were adjusted according to the polarity of the compound or by the addition of triethylamine.

Preparation method of using reverse phase column chromatography:
Preparation method A:
   Instrument Model: Biotage fast medium-pressure preparative chromatography, Column: Agela C18 reverse phase column (Spherical; 20-35µm; 100A); Column temperature: 25°C; Flow rate: 28.0 mL/min; Detection wavelength: 220 nm; Mobile phase A: acetonitrile; Mobile phase B: water;
Preparation method B:
   Instrument Model: Biotage fast medium pressure preparative chromatography, Column: Agela C18 reverse phase column (Spherical; 20-35µm; 100A); Column temperature: 25°C; Flow rate: 28.0 mL/min; Detection wavelength: 220 nm; Mobile phase A: acetonitrile; Mobile phase B: 0.05% aqueous formic acid solution;
Preparation method C:
   Instrument Model: Biotage fast medium pressure preparative chromatography, Column: Agela C18 reverse phase column (Spherical; 20-35µm; 100A); Column temperature: 25°C; Flow rate: 28.0 mL/min; Detection wavelength: 220 nm; Mobile phase A: acetonitrile; Mobile phase B: 0.05% aqueous ammonium bicarbonate solution;
Microwave reactions were performed using a Biotage Initiator + (400 W, RT ~ 300°C) microwave reactor.

Column chromatography was generally performed by using 200~300 mesh silica gel as the stationary phase. Eluent systems included the system of dichloromethane and methanol, and the system of petroleum ether and ethyl acetate. The volume ratios of the solvents were adjusted according to the polarity of the compound, and a small amount of triethylamine could also be added for adjustment.

Unless otherwise specified in the examples, the reaction temperature was room temperature (20°C to 35°C).

Reagents used in the present invention were purchased from Acros Organics, Aldrich Chemical Company, and Teber Chemical.

In the general synthesis methods, examples, and intermediate synthesis examples, the meanings of the abbreviations are as follows.

| Abbreviation | Meaning | Abbreviation | Meaning |
|---|---|---|---|
| TLC | Thin-layer chromatography | DMSO-*d*₆ | Hexadeuterated dimethyl sulfoxide |
| LC-MS | Liquid chromatography-mass spectrometry | s | singlet |
| DMSO | Dimethyl sulfoxide | d | doublet |
| TCEP | Tris(2-carboxyethyl)phosphine | t | triplet |
| EDTA | Ethylenediaminetetraacetic acid | q | quartet |
| MS | Mass spectrometry | dd | double doublet |
| NMR | Nuclear magnetic resonance | m | multiplet |
| TMS | Tetramethylsilane | br | broad |
| CD₃OD | Deuterated methanol | *J* | Coupling constant |
| CDCl₃ | Deuterated chloroform | Hz | Hertz |
| Nle | Norleucine | | |

The information of the sequences involved in the present invention is described in the following table:

| SEQ ID NO: | Description | Sequence |
|---|---|---|
| 1 | Ab-02 VH amino acid sequence | QVQLQESGPGLVQPSGTLSLTCAVSGGSDSST NWWTWVRQSPGQGLEWIGEISPIGFTSYSPSL RSRVTISVDKSKTQFSLKLSSVTAADTAVYYC ARDRLYFAFWGQGTLVTVSS |
| 2 | Ab-01, Ab-02 VL amino acid sequence | EIVMTQSPATLSVSPGERATLSCRASQSVSSN LAWYQQKPGQAPRLLIYGASTRATGIPDRFS GSGSGTEFTLTISSLESEDFAVYYCQQYNNWP ITFGQGTRLEIK |
| 3 | Ab-01, Ab-02 CDR-L1 (IMGT definition) | QSVSSN |
| 4 | Ab-01, Ab-02 CDR-L2 (IMGT definition) | GAS |
| 5 | Ab-01, Ab-02 CDR-L3 (IMGT definition/Chothia definition/Kabat definition/AbM definition) | QQYNNWPIT |
| 6 | Ab-01, Ab-02 CDR-L1 (Chothia definition/Kabat definition/AbM definition) | RASQSVSSNLA |
| 7 | Ab-01, Ab-02 CDR-L2 (Chothia definition/Kabat definition/AbM definition) | GASTRAT |
| 8 | Ab-01, Ab-02 CDR-H1 (IMGT definition) | GGSDSSTNW |
| 9 | Ab-01, Ab-02 CDR-H2 (IMGT definition) | ISPIGFT |
| 10 | Ab-01, Ab-02 CDR-H3 (IMGT definition) | ARDRLYFAF |
| 11 | Ab-01, Ab-02 CDR-H1 (Chothia definition) | GGSDSSTN |
| 12 | Ab-01, Ab-02 CDR-H2 (Chothia definition) | SPIGF |
| 13 | Ab-01, Ab-02 CDR-H3 (Chothia definition/ Kabat definition/AbM definition) | DRLYFAF |
| 14 | Ab-01, Ab-02 CDR-H1 (Kabat definition) | STNWWT |
| 15 | Ab-01, Ab-02 CDR-H2 (Kabat definition) | EISPIGFTSYSPSLRS |
| 16 | Ab-01, Ab-02 CDR-H1 (AbM definition) | GGSDSSTNWWT |
| 17 | Ab-01, Ab-02 CDR-H2 (AbM definition) | EISPIGFTS |
| 18 | Ab-01, Ab-02 CH | |
| 19 | Ab-01, Ab-02 CL | |
| | | |
| 20 | Heavy chain amino acid sequence of the antibody Ab-02 | QVQLQESGPGLVQPSGTLSLTCAVSGGSDSST NWWTWVRQSPGQGLEWIGEISPIGFTSYSPSL RSRVTISVDKSKTQFSLKLSSVTAADTAVYYC ARDRLYFAFWGQGTLVTVSSASTKGPSVFPL APSSKSTSGGTAALGCLVKDYFPEPVTVSWNS GALTSGVHTFPAVLQSSGLYSLSSVVTVPSSSL GTQTYICNVNHKPSNTKVDKKVEPKSCDKTH TCPPCPAPELLGGPSVFLFPPKPKDTLMISRTP EVTCVVVDVSHEDPEVKFNWYVDGVEVHNA KTKPREEQYNSTYRVVSVLTVLHQDWLNGK EYKCKVSNKALPAPIEKTISKAKGQPREPQVY TLPPSRDELTKNQVSLTCLVKGFYPSDIAVEW ESNGQPENNYKTTPPVLDSDGSFFLYSKLTVD KSRWQQGNVFSCSVMHEALHNHYTQKSLSL SPGK |
| 21 | Light chain amino acid sequence of the antibodies Ab-01 and Ab-02 | EIVMTQSPATLSVSPGERATLSCRASQSVSSN LAWYQQKPGQAPRLLIYGASTRATGIPDRFS GSGSGTEFTLTISSLESEDFAVYYCQQYNNWP ITFGQGTRLEIKRTVAAPSVFIFPPSDEQLKSGT ASVVCLLNNFYPREAKVQWKVDNALQSGNS QESVTEQDSKDSTYSLSSTLTLSKADYEKHKV YACEVTHQGLSSPVTKSFNRGEC |
| 22 | Heavy chain amino acid sequence of the antibody Ab-0 1 | QVQLQESGPGLVQPSGTLSLTCAVSGGSDSST NWWTWVRQSPGQGLEWIGEISPIGFTSYSPSL RSRVTISVDKSKTQFSLNLSSVTAADTAVYYC ARDRLYFAFWGQGTLVTVSSASTKGPSVFPL APSSKSTSGGTAALGCLVKDYFPEPVTVSWNS GALTSGVHTFPAVLQSSGLYSLSSVVTVPSSSL GTQTYICNVNHKPSNTKVDKKVEPKSCDKTH TCPPCPAPELLGGPSVFLFPPKPKDTLMISRTP EVTCVVVDVSHEDPEVKFNWYVDGVEVHNA KTKPREEQYNSTYRVVSVLTVLHQDWLNGK EYKCKVSNKALPAPIEKTISKAKGQPREPQVY TLPPSRDELTKNQVSLTCLVKGFYPSDIAVEW ESNGQPENNYKTTPPVLDSDGSFFLYSKLTVD KSRWQQGNVFSCSVMHEALHNHYTQKSLSL SPGK |
| 23 | Ab-01 VH amino acid sequence | QVQLQESGPGLVQPSGTLSLTCAVSGGSDSST NWWTWVRQSPGQGLEWIGEISPIGFTSYSPSL RSRVTISVDKSKTQFSLNLSSVTAADTAVYYC ARDRLYFAFWGQGTLVTVSS |
| 24 | Linker fragment | GGFG |
| 25 | Linker subunit for antibody light and heavy chains | GGGGS |
| 26 | Linker for antibody light and heavy chains | GGGGSGGGGSGGGGSGGGGS |

### I. Compound examples

### Intermediate Preparation Example 1: Preparation of 2-((1-((4-((4-(4-amino-2-butyl-7-methyl-1H-imidazo[4,5-d]thieno[3,2-b]pyridin-1-yl)butyl)carbamoyl)cyclohexyl)methyl)-2,5-dioxopyrrolidin-3-yl)mercapto)acetic acid (Compound Int1)

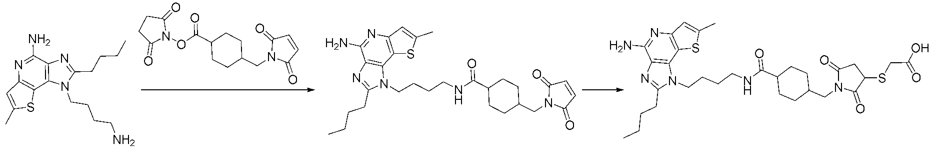

### Step 1: Preparation of N-(4-(4-amino-2-butyl-7-methyl-1H-imidazo[4,5-d]thieno[3,2-b]pyridin-1-yl)butyl)-4-((2,5-dioxo-2,5-dihydro-1H-pyrrol-1-yl)methyl)cyclohexyl-1-carboxamide

1-(4-Aminobutyl)-2-butyl-7-methyl-1*H*-imidazo[4,5-*d*]thieno[3,2-*b*]pyridin-4-amine (200 mg, 0.60 mmol) and *N,N*-diisopropylethylamine (155 mg, 1.2 mmol) were added to *N,N-*dimethylformamide (2 mL), and a solution of 2,5-dioxopyrrolidin-1-yl 4-((2,5-dioxo-2,5-dihydro-1*H*-pyrrol-1-yl)methyl)cyclohexyl-1-carboxylate (240 mg, 0.72 mmol) in dichloromethane (1 mL) was added dropwise. The reaction mixture was stirred at 25°C for 12 hours. Water was added to the reaction system, and the mixture was extracted three times with dichloromethane. The organic phase was collected, washed with saturated brine, dried over anhydrous sodium sulfate, filtered, and the filtrate was concentrated to obtain a crude product, which was purified by silica gel column chromatography (eluent: dichloromethane/methanol = 10/1 (v/v)) to obtain the title compound of this step (282 mg, yield: 85.4%).

MS m/z (ESI): 551.3 [M+H]⁺.

### Step 2: Preparation of 2-((1-((4-((4-(4-amino-2-butyl-7-methyl-1H-imidazo[4,5-d]thieno[3,2-b]pyridin-1-yl)butyl)carbamoyl)cyclohexyl)methyl)-2,5-dioxopyrrolidin-3-yl)mercapto)acetic acid (Int1)

*N*-(4-(4-amino-2-butyl-7-methyl-1*H*-imidazo[4,5-*d*]thieno[3,2-*b*]pyridin-1-yl)butyl)-4-((2,5-dioxo-2,5-dihydro-1*H*-pyrrol-1-yl)methyl)cyclohexyl-1-carboxamide (282 mg, 0.51 mmol) and mercaptoacetic acid (94 mg, 1.02 mmol) were added to dichloromethane (50 mL), and the reaction mixture was reacted under stirring at 25°C for 30 minutes. The reaction mixture was concentrated to obtain a yellow solid, which was slurried with methyl tert-butyl ether to afford the title compound (237 mg, yield: 72.3%).

MS m/z (ESI): 643.3 [M+H]⁺.

### Intermediate Preparation Example 2: Preparation of Compound Int2

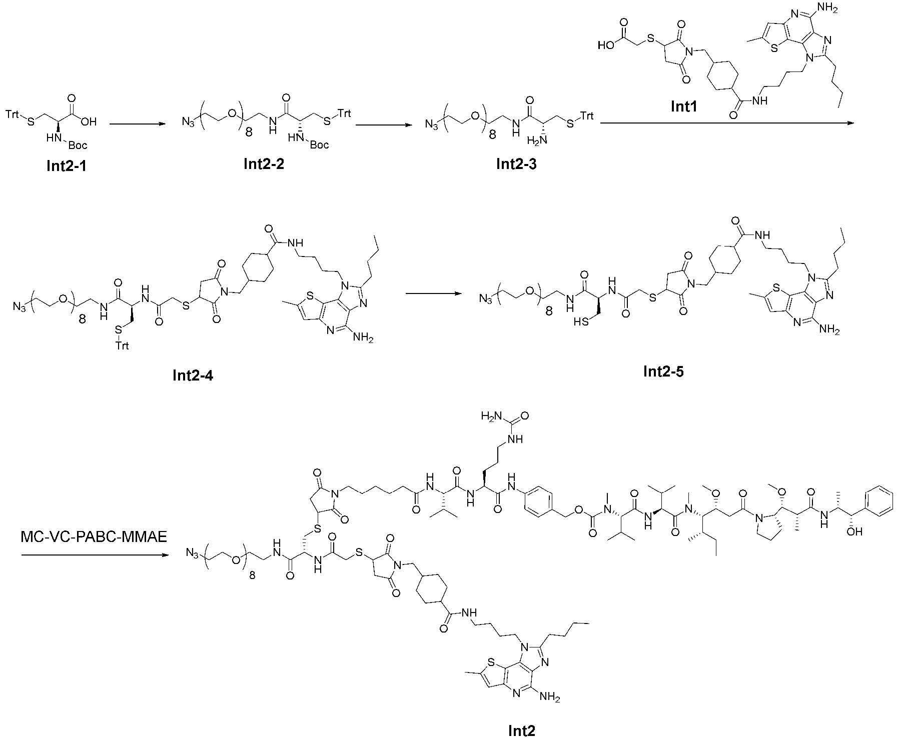

### Step 1: Preparation of Compound Int2-2

*N*-Tert-butoxycarbonyl-*S*-trityl-*L*-cysteine (384 mg, 0.83 mmol) and 26-azido-3,6,9,12,15,18,21,24-octaoxahexacosan-1-amine (435 mg, 0.99 mmol) were dissolved in *N,N-*dimethylformamide (4 mL). To this mixture under ice-cooling were added 1-hydroxybenzotriazole (167.58 mg, 1.24 mmol), *N,N*-diisopropylethylamine (320.43 mg, 2.48 mmol), and 1-(3-dimethylaminopropyl)- 3-ethylcarbodiimide hydrochloride (238 mg, 1.24 mmol). The reaction mixture was stirred at 20°C for 3 hours. The reaction mixture was added dropwise to water and extracted with dichloromethane. The organic phase was dried and concentrated to obtain the title compound of this step (0.69 g, yield: 78.4%).

MS m/z (ESI): 884.4 [M+H]⁺.

### Step 2: Preparation of Compound Int2-3

Compound **Int2-2** (0.69 g, 0.78 mmol) was dissolved in dichloromethane (10 mL). Trifluoroacetic acid (10 mL) was then added dropwise. The reaction mixture was stirred at room temperature for 2 hours, concentrated, and diluted with dichloromethane (20 mL). The organic phase was washed with sodium bicarbonate solution, dried, and concentrated to obtain the title compound of this step (0.6 g, yield: 98%).

MS m/z (ESI): 784.4 [M+H]⁺.

### Step 3: Preparation of Compound Int2-4

Compound **Int1** (136 mg, 211 mmol) and Compound **Int2-3** (182 mg, 233 mmol) were dissolved in *N,N*-dimethylformamide (4 mL). 1-Hydroxybenzotriazole (43.2 mg, 0.32 mmol), *N,N*-diisopropylethylamine (81.6 mg, 0.63 mmol) and 1-(3-dimethylaminopropyl)- 3-ethylcarbodiimide hydrochloride (61.3 mg, 0.32 mmol) were added under ice-cooling. The mixture was stirred at 20°C for 3 hours. The reaction mixture was added dropwise to water and extracted with dichloromethane. The organic phase was dried and concentrated to obtain the title compound of this step (0.23 g, yield: 95.8%).

MS m/z (ESI): 1408.6 [M+H]⁺.

### Step 4: Preparation of Compound Int2-5

Compound **Int2-4** (0.23 g, 0.16 mmol) was dissolved in dichloromethane (2 mL) and triethylsilane (0.52 mL). Trifluoroacetic acid (1 mL) was then added dropwise. The reaction mixture was stirred at room temperature for 1 hour. Then the mixture was concentrated and diluted with dichloromethane (20 mL). The organic phase was washed with sodium bicarbonate solution, dried, and concentrated to obtain the title compound of this step (0.15 g, yield: 78.9%).

MS m/z (ESI): 1166.5 [M+H]⁺.

### Step 5: Preparation of Compound Int2

Compound **Int2-5** (0.15 g, 0.13 mmol) was dissolved in *N,N*-dimethylformamide (5 mL). MC-VC-PABC-MMAE (0.17 g, 0.13 mmol) and *N,N*-diisopropylethylamine (50.3 mg, 0.39 mmol) was then added. The reaction mixture was stirred at room temperature for 1 hour, then an aqueous solution of ammonium chloride (10 mL) was added, and the mixture was extracted three times with dichloromethane (10 mL). The organic phase was dried and concentrated to obtain a crude product, which was purified on a preparative thin-layer silica gel plate (eluent: dichloromethane/methanol = 10/1 (v/v)) to obtain the title compound of this step (150 mg, yield: 47.0%).

MS m/z (ESI): 1241.7 [M/2+H]⁺.

### Intermediate Preparation Example 3: Preparation of Compound Int3

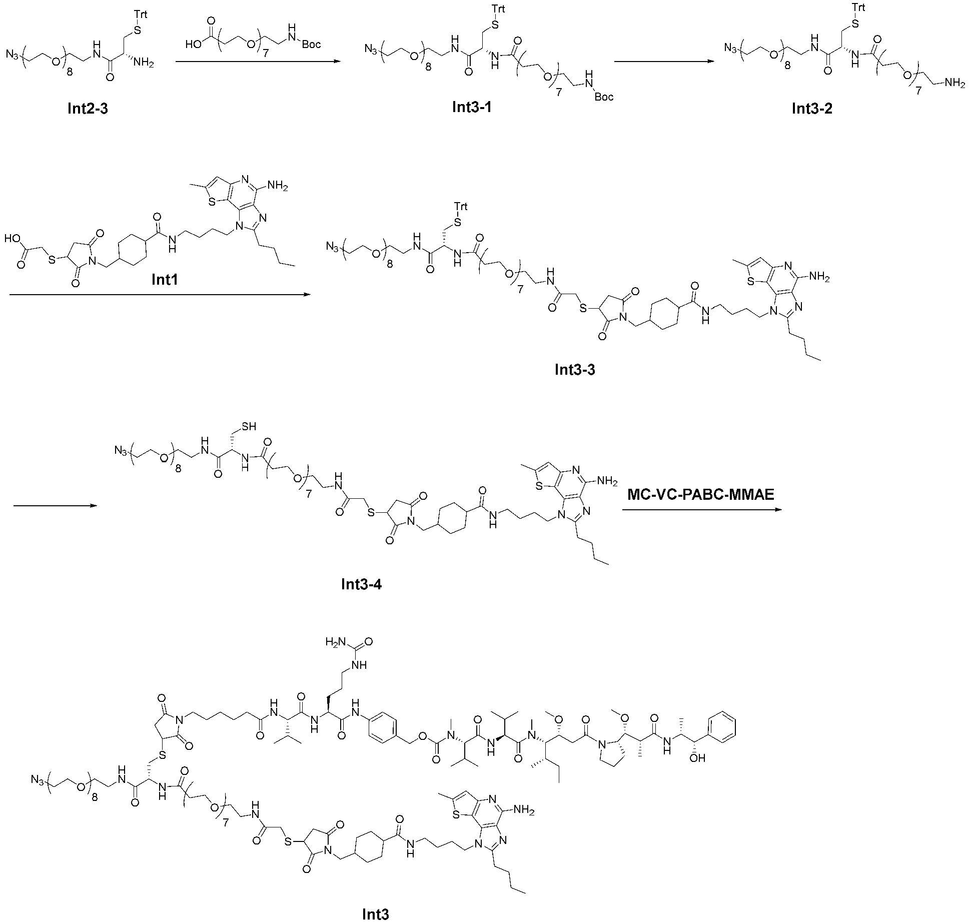

### Step 1: Preparation of Compound Int3-1

Compound **Int2-3** (192 mg, 0.244 mmol) and 2,2-dimethyl-4-oxo-3,8,11,14,17,20,23,26-octaoxa-5-nonacosane-29-oic acid (110.53 mg, 0.222 mmol) were dissolved in *N,N-*dimethylformamide (3 mL). 1-Hydroxybenzotriazole (45 mg, 0.333 mmol), *N,N-*diisopropylethylamine (85.9 mg, 0.666 mmol) and 1-(3-dimethylaminopropyl)- 3-ethylcarbodiimide hydrochloride (63.8 mg, 0.333 mmol) were added under ice-cooling. The mixture was stirred at 20°C for 6 hours. The reaction mixture was then added dropwise to water (10 mL), extracted three times with dichloromethane (10 mL). The organic phase was dried and concentrated to obtain a crude product, which was purified by silica gel column chromatography (eluent: dichloromethane/methanol = 10/1 (v/v)) to obtain the title compound of this step (145 mg, yield: 51.6%).

MS m/z (ESI): 1263.6 [M+H]⁺.

### Step 2: Preparation of Compound Int3-2

Compound **Int3-1** (145 mg, 0.11 mmol) was dissolved in dichloromethane (2 mL). Trifluoroacetic acid (1 mL) was added dropwise. The reaction mixture was stirred at room temperature for 2 hours, concentrated and diluted with dichloromethane (10 mL). The organic phase was washed with sodium bicarbonate solution, dried, and concentrated to obtain the title compound of this step (130 mg, yield: 97.0%).

MS m/z (ESI): 1163.6 [M+H]⁺.

### Step 3: Preparation of Compound Int3-3

Compound **Int1** (65 mg, 0.10 mmol) and Compound **Int3-2** (130 mg, 0.11 mmol) were dissolved in *N,N*-dimethylformamide (5 mL). 1-Hydroxybenzotriazole (20.3 mg, 0.15 mmol), *N,N*-diisopropylethylamine (38.7 mg, 0.3 mmol) and 1-(3-dimethylaminopropyl)- 3-ethylcarbodiimide hydrochloride (28.8 mg, 0.15 mmol) were added under ice-cooling, and the mixture was stirred at 20°C for 3 hours. The reaction mixture was added dropwise to water (10 mL) and extracted three times with dichloromethane (10 mL). The organic phase was dried and concentrated to obtain a crude product, which was purified by silica gel column chromatography (eluent: dichloromethane/methanol = 10/1 (v/v)) to obtain the title compound of this step (110 mg, yield: 60.8%).

MS m/z (ESI): 1787.8 [M+H]⁺.

### Step 4: Preparation of Compound Int3-4

Compound **Int3-3** (110 mg, 0.06 mmol) was dissolved in dichloromethane (2 mL) and triethylsilane (0.4 mL). Trifluoroacetic acid (1 mL) was added dropwise. The reaction mixture was stirred at room temperature for 1 hour, concentrated and diluted with dichloromethane (20 mL). The organic phase was washed with sodium bicarbonate solution, dried, and concentrated to obtain the title compound of this step (95 mg, yield: 100.0%).

MS m/z (ESI): 1545.7 [M+H]⁺.

### Step 5: Preparation of Compound Int3

Compound **Int3-4** (95 mg, 0.06 mmol) was dissolved in *N,N*-dimethylformamide (25 mL). MC-VC-PABC-MMAE (79 mg, 0.06 mmol) and *N*,*N*-diisopropylethylamine (23.2 mg, 0.18 mmol) was added. The reaction mixture was stirred at room temperature for 1 hour, then an aqueous solution of ammonium chloride (10 mL) was added, and the mixture was extracted three times with dichloromethane (10 mL). The organic phase was dried and concentrated to obtain a crude product, which was purified on a preparative thin-layer silica gel plate (eluent: dichloromethane/methanol = 8/1 (v/v)) to obtain the title compound of this step (73 mg, yield: 41.4%).

MS m/z (ESI): 1431.3 [M/2+H]⁺.

### Intermediate Preparation Example 4: Preparation of Compound Int4

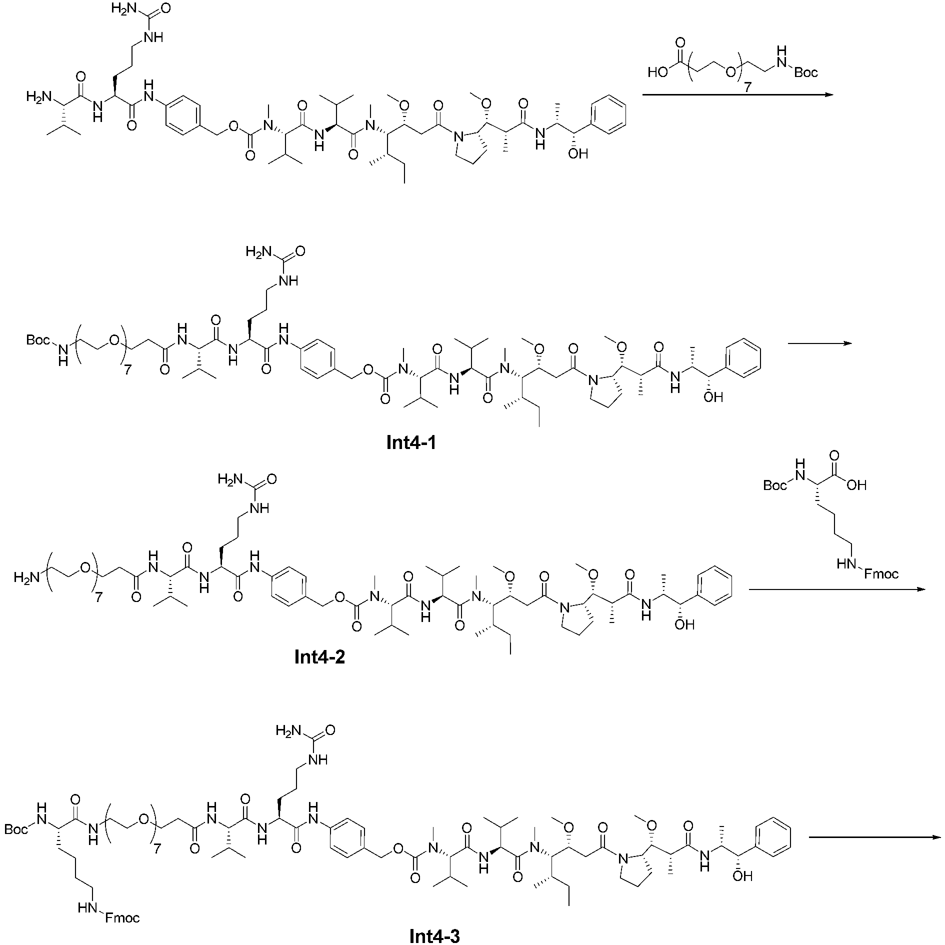

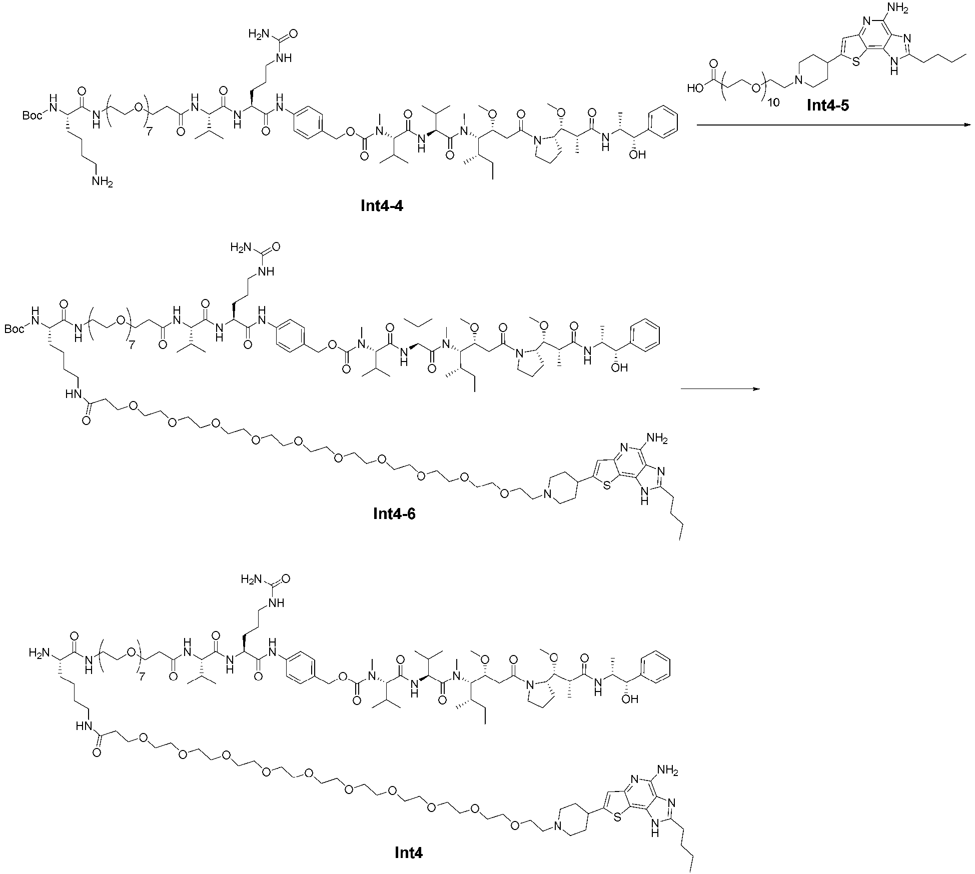

### Step 1: Preparation of Compound Int4-1

VC-PABC-MMAE (155 mg, 131.07 µmol) and 2,2-dimethyl-4-oxo-3,8,11,14,17,20,23,26-octaoxa-5-nonacosane-29-oic acid (68.65 mg, 131.07 µmol) were dissolved in anhydrous *N,N-*dimethylformamide (4 mL). *N,N*-diisopropylethylamine (42.78 mg, 327.68 µmol) and 2-(7-azobenzotriazole)-*N,N,N,N*-tetramethyluronium hexafluorophosphate (75.53 mg, 196.61 µmol) was added, and the reaction system was reacted at 25°C for 5 hours. The reaction mixture was concentrated to obtain a residue, which was purified by silica gel column chromatography (eluent: dichloromethane/methanol = 20/1 (v/v)) to yield the title compound (193 mg, yield: 87.3%).

MS m/z (ESI): 1603.0 [M+H]⁺.

### Step 2: Preparation of Compound Int4-2

Under the protection of nitrogen atmosphere, Compound **Int4-1** (165 mg, 97.79 µmol) and zinc bromide (139.08 mg, 586.72 µmol) were added sequentially to anhydrous dichloromethane (2 mL), and reacted at 25°C for 2 hours. The reaction mixture was concentrated to obtain the crude title compound, which was used directly in the next step.

MS m/z (ESI): 1502.9 [M+H]⁺.

### Step 3: Preparation of Compound Int4-3

Compound **Int4-2** (114 mg, 72.06 µmol) and *N⁶*-(((9*H*-fluoren-9-yl)methoxy)carbonyl)- *N²-*(tert-butoxycarbonyl)-L-lysine (37.43 mg, 72.06 µmol) were dissolved in anhydrous *N,N-*dimethylformamide (2 mL). *N,N*-diisopropylethylamine (23.52 mg, 180.16 µmol) and 2-(7-azobenzotriazole)-*N*,*N*,*N*',*N*'-tetramethyluronium hexafluorophosphate (41.53 mg, 108.09 µmol) was added, and the reaction system was reacted at 25°C for 5 hours. The reaction mixture was concentrated to obtain a residue, which was purified by silica gel column chromatography (eluent: dichloromethane/methanol = 50/1 (v/v)) to yield the title compound of this step (110 mg, yield: 70.4%).

MS m/z (ESI): 1953.1 [M+H]⁺.

### Step 4: Preparation of Compound Int4-4

Compound **Int4-3** (100 mg, 46.07 µmol) was dissolved in dichloromethane (3 mL). Diethylamine (67.40 mg, 921.47 µmol) was added, and the reaction system was reacted at 20°C for 10 hours. The reaction mixture was concentrated to obtain a residue, which was purified by silica gel column chromatography (eluent: dichloromethane/methanol = 20/1 (v/v)) to obtain the title compound of this step (40 mg, yield: 42.8%).

MS m/z (ESI): 1731.1 [M+H]⁺.

### Step 5: Preparation of Compound Int4-6

Compound **Int4-4** (100 mg, 54.88 µmol) and Compound **Int4-5** (46.64 mg, 54.88 µmol) were added to anhydrous *N,N*-dimethylformamide (2 mL). *N,N*-diisopropylethylamine (10.75 mg, 82.31 µmol) and 2-(7-azobenzotriazole)-*N,N,N,N*-tetramethyluronium hexafluorophosphate (25.29 mg, 65.85 µmol) was added, and the reaction system was reacted at 25°C for 5 hours. The reaction mixture was purified by preparative HPLC to obtain the title compound of this step (60 mg, yield: 40.7%).

MS m/z (ESI): 1277.8 [M/2+H]⁺.

### Step 4: Preparation of Compound Int4

Under the protection of nitrogen atmosphere, Compound **Int4-6** (45 mg, 7.04 µmol) and zinc bromide (6.68 mg, 28.18 µmol) were added sequentially to anhydrous dichloromethane (2 mL), and reacted at 25°C for 2 hours. The reaction mixture was concentrated to obtain the crude title compound, which was used directly in the next step.

MS m/z (ESI): 1227.8 [M/2+H]⁺.

### Intermediate Preparation Example 5: Preparation of Compound Int5

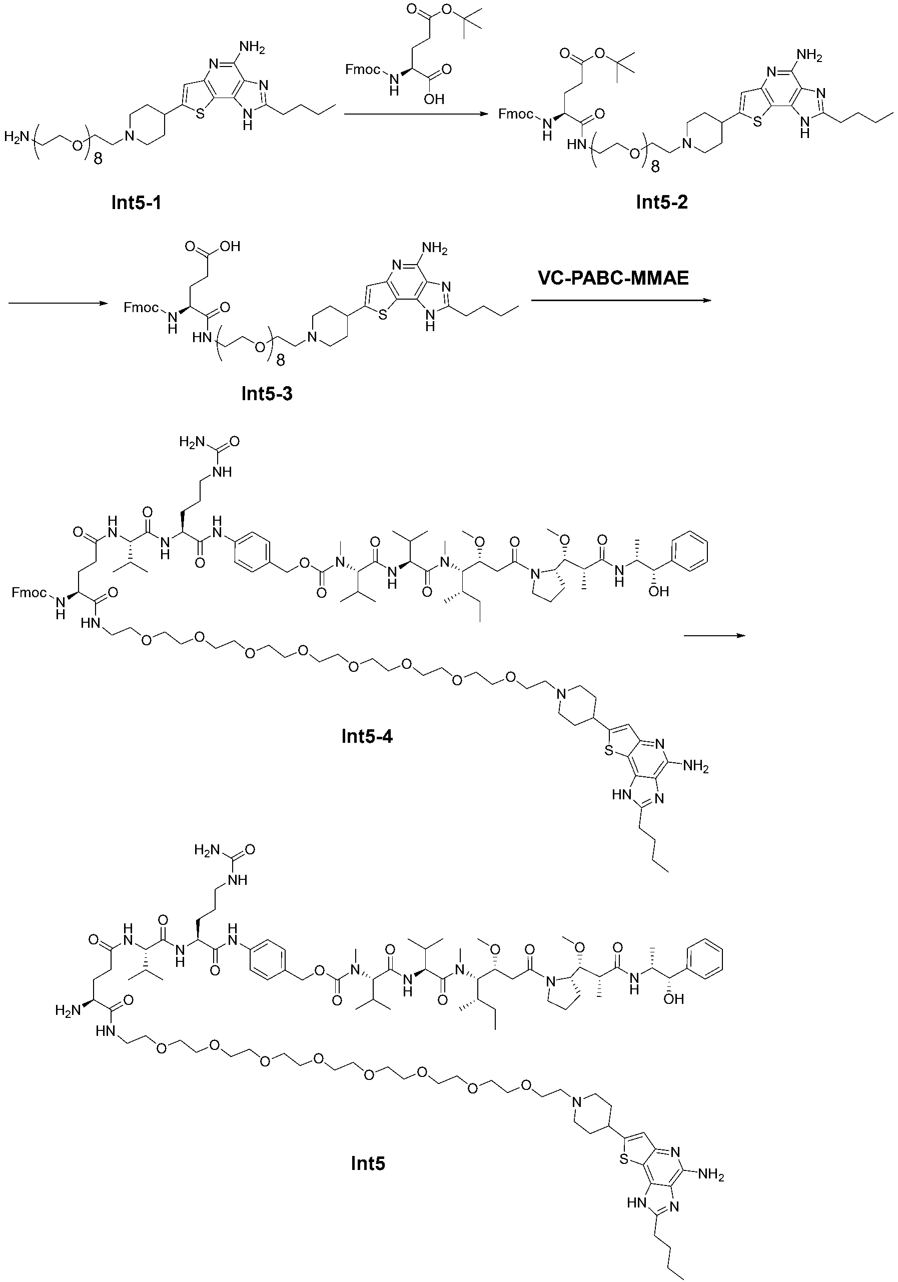

### Step 1: Preparation of Compound Int5-2

Compound **Int5-1** (186 mg, 243.74 µmol), (*S*)-2-((((9*H*-fluoren-9-yl)methoxy)carbonyl) amino)-5-(tert-butyloxy)-5-oxopentanoic acid (131 mg, 292.49 µmol), *N,N*-diisopropylethylamine (66.32 mg, 487.48 µmol) and 2-(7-azobenzotriazole)-*N,N,N',N'*-tetramethyluronium hexafluorophosphate (136.58 mg, 341.24 µmol) were added sequentially to anhydrous *N,N-*dimethylformamide (3 mL), and reacted at 0°C for 1 hour. The reaction mixture was directly purified by reverse-phase column chromatography (Preparation method A, elution gradient: A% = 25% to 35%) to obtain the title compound of this step (270 mg, yield: 92.9%).

MS m/z (ESI): 1132.6 [M+H]⁺.

### Step 2: Preparation of Compound Int5-3

Compound **Int5-2** (340 mg, 285.23 µmol) and trifluoroacetic acid (2 mL) were added to dichloromethane (6 mL), and reacted at 25°C for 2 hours. The reaction mixture was concentrated to obtain a residue, which was purified by silica gel column chromatography (eluent: dichloromethane/methanol = 4/1 (v/v)) to obtain the title compound of this step (250 mg, yield: 77.4%).

MS m/z (ESI): 1076.6 [M+H]⁺.

### Step 3: Preparation of Compound Int5-4

Compound **Int5-3** (150 mg, 132.40 µmol), VC-PABC-MMAE (156.57 mg, 132.40 µmol), *N,N*-diisopropylethylamine (25.93 mg, 198.60 µmol), and 2-(7-azobenzotriazole)-*N,N,N',N'-*tetramethyluronium hexafluorophosphate (61.02 mg, 158.88 µmol) were added sequentially to anhydrous *N,N*-dimethylformamide (5 mL), and reacted at 0°C for 1 hour. The reaction mixture was directly purified by reverse-phase column chromatography (Preparation method B, elution gradient: A% = 35% to 45%) to obtain the title compound of this step (250 mg, yield: 82.2%).

MS m/z (ESI): 1091.6 [M/2+H]⁺.

### Step 4: Preparation of Compound Int5

Compound **Int5-4** (250 mg, 8.86 µmol) and piperidine (37.45 mg, 435.44 µmol) were added to *N,N*-dimethylformamide (4 mL), and reacted at 25°C for 2 hours. The reaction mixture was directly purified by reverse-phase column chromatography (Preparation method A, elution gradient: A% = 20% to 30%) to obtain the title compound of this step (185 mg, yield: 82.4%).

MS m/z (ESI): 980.6 [M/2+H]⁺.

### Intermediate Preparation Example 6: Preparation of Compound Int6

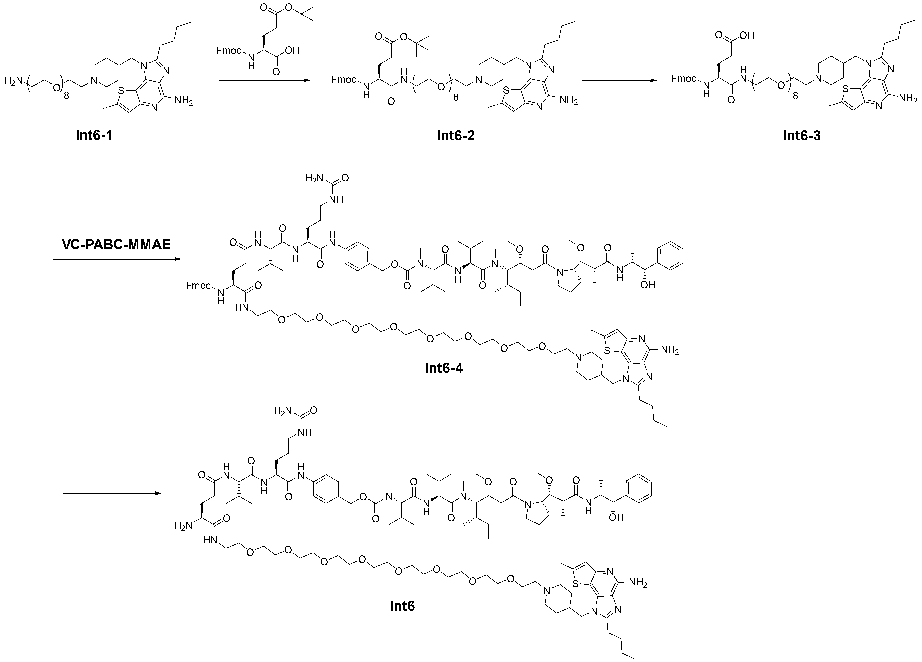

### Step 1: Preparation of Compound Int6-2

(*S*)-2-((((9*H*-Fluoren-9-yl)methoxy)carbonyl)amino)-5-(tert-butyloxy)-5-oxopentanoic acid (85.29 mg, 200.46 µmol), *N,N*-diisopropylethylamine (45.2 mg, 346.26 µmol), 2-(7-azobenzotriazole)-*N*,*N*,*N*',*N*'-tetramethyluronium hexafluorophosphate (103.94 mg, 270.22 µmol), and Compound **Int6-1** (150 mg, 182.24 µmol) were added sequentially to anhydrous *N,N-*dimethylformamide (4 mL), and reacted at 0°C for 2 hours. The reaction mixture was directly purified by reverse-phase column chromatography (Preparation method B, elution gradient: A% = 25% to 35%) to obtain the title compound of this step (170 mg, yield: 79.8%).

MS m/z (ESI): 1160.6 [M+H]⁺.

### Step 2: Preparation of Compound Int6-3

Compound **Int6-2** (170 mg, 138.15 µmol) was dissolved in a mixed solvent of anhydrous dichloromethane (6 mL) and trifluoroacetic acid (3 mL), and reacted at 25°C for 2 hours. The reaction mixture was concentrated to obtain the title compound of this step (133 mg, yield: 86.3%).

MS m/z (ESI): 1104.6 [M+H]⁺.

### Step 3: Preparation of Compound Int6-4

Compound **Int6-3** (51 mg, 46.19 µmol), VC-PABC-MMAE (51.88 mg, 46.19 µmol), *N,N-*diisopropylethylamine (24.37 mg, 187.03 µmol), and 2-(7-azobenzotriazole)-*N,N,N',N'-*tetramethyluronium hexafluorophosphate (19.14 mg, 49.88 µmol) were added sequentially to *N,N-*dimethylformamide (1 mL), and reacted at room temperature for 1.5 hours. The reaction system was poured into ice water (10 mL) and extracted with ethyl acetate (5 mL x 3). The organic phases were combined and backwashed with saturated brine (3 mL x 2). The organic phase was dried over anhydrous sodium sulfate and concentrated to obtain the title compound of this step (86 mg, yield: 84.3%).

MS m/z (ESI): 1105.7 [M/2+H]⁺.

### Step 4: Preparation of Compound Int6

Compound **Int6-4** (86 mg, 38.93 µmol) and piperidine (5.36 mg, 62.27 µmol) were added to *N,N*-dimethylformamide (1 mL), and reacted at room temperature for 2 hours. The reaction mixture was directly purified by reverse-phase column chromatography (Preparation method B, elution gradient: A% = 20%-25%) to obtain the title compound of this step (47 mg, yield: 60.8%).

MS m/z (ESI): 1988.2 [M+H]⁺

### Intermediate Preparation Example 7: Preparation of Compound Int7

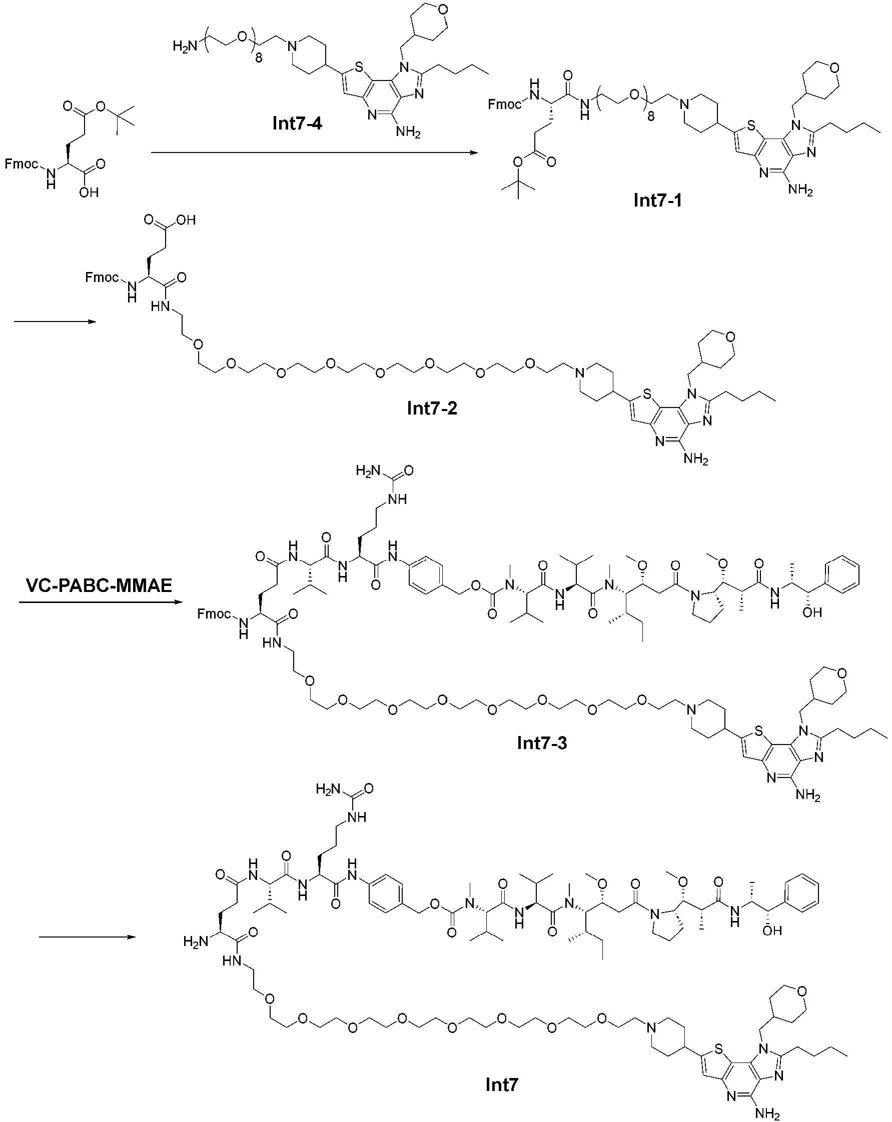

### Step 1: Preparation of Compound Int7-1

(*S*)-2-((((9*H*-Fluoren-9-yl)methoxy)carbonyl)amino)-5-(tert-butyloxy)-5-oxopentanoic acid (91.93 mg, 216.06 µmol), Compound **Int7-4** (148.2 mg, 180.05 µmol), N,N-diisopropylethylamine (46.54 mg, 360.11 µmol) and 2-(7-azabenzotriazole)-*N,N,N',N'*-tetramethyluronium hexafluorophosphate (75.26 mg, 198.06 µmol) were added to *N,N*-dimethylformamide (2 mL), and stirred at 25°C for 2 hours. The reaction mixture was purified by reverse-phase column chromatography (Preparation method B, elution gradient: A% = 30% to 40%) to obtain the title compound of this step (152 mg, yield: 68.6%).

MS m/z (ESI): 1230.7 [M+H]⁺.

### Step 2: Preparation of Compound Int7-2

Compound **Int7-1** (133 mg, 108.08 µmol) was dissolved in dichloromethane (3 mL). Trifluoroacetic acid (1 mL) was added, and the reaction mixture was stirred at 25°C for 2 hours. Then the reaction mixture was concentrated to obtain the crude title compound, which was used directly in the next step.

MS m/z (ESI): 1174.6 [M+H]⁺.

### Step 3: Preparation of Compound Int7-3

Compound **Int7-2** (47.5 mg, 40.44 µmol), VC-PABC-MMAE (54.52 mg, 48.53 µmol), *N,N-*diisopropylethylamine (10.45 mg, 80.89 µmol), and 2-(7-azabenzotriazole)-*N,N,N',N'-*tetramethyluronium hexafluorophosphate (16.91 mg, 44.49 µmol) were added to *N,N-*dimethylformamide (3 mL), and stirred at 25°C for 2 hours. The reaction mixture was diluted with *N,N*-dimethylformamide and then purified by reverse-phase column chromatography (Preparation method B, elution gradient: A% = 40%-50%) to obtain the title compound of this step (57.8 mg, 62.7% yield).

MS m/z (ESI): 1140.7 [M/2+H]⁺.

### Step 4: Preparation of Compound Int7

Compound **Int7-3** (57.8 mg, 25.35 µmol) was dissolved in *N*,*N*-dimethylformamide (2 mL). Then diethylamine (9.27 mg, 126.76 µmol) was added and stirred at 25°C for 2 hours. The reaction mixture was directly purified by reverse-phase column chromatography (Preparation method A, elution gradient: A% = 40%-50%) to obtain the title compound of this step (39.6 mg, yield: 75.9%).

MS m/z (ESI): 1029.6 [M/2+H]⁺.

### Intermediate Preparation Example 8: Preparation of Compound Int8

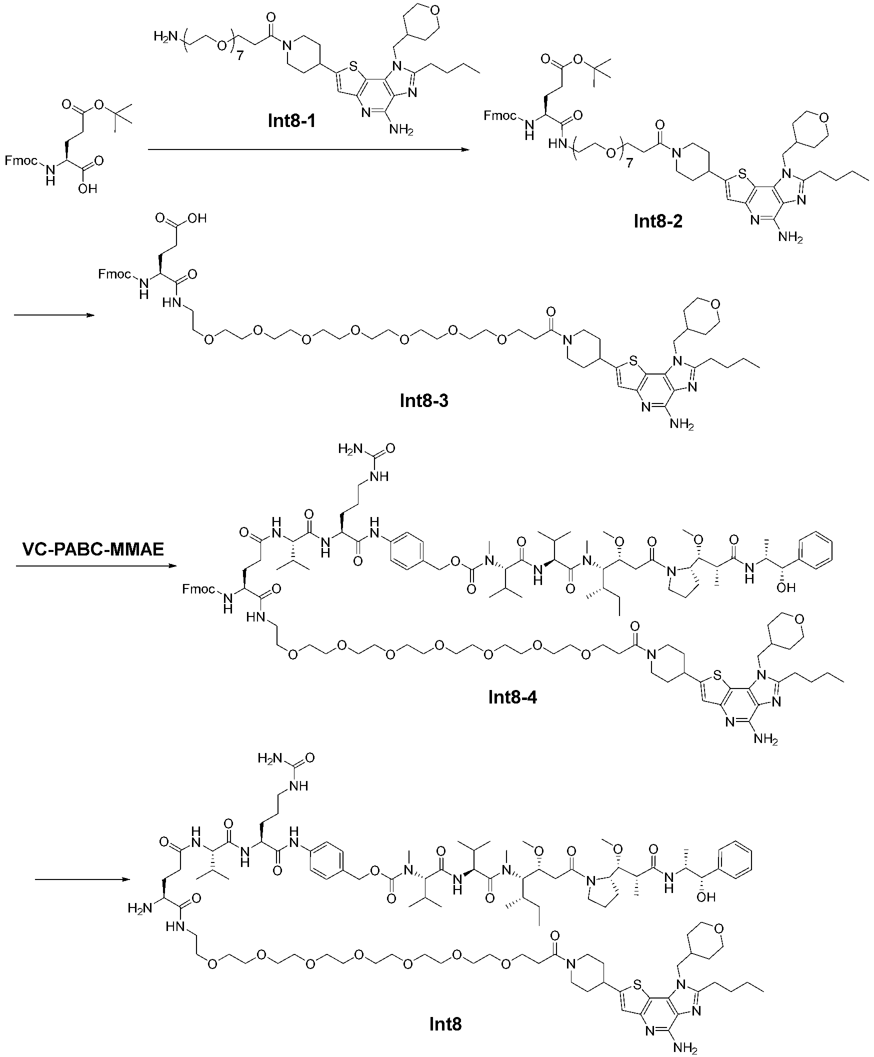

### Step 1: Preparation of Compound Int8-2

(*S*)-2-((((9*H*-Fluoren-9-yl)methoxy)carbonyl)amino)-5-(tert-butyloxy)-5-oxopentanoic acid (276.97 mg, 644.45 µmol), *N,N*-diisopropylethylamine (209.93 mg, 1610 µmol), 2-(7-azobenzotriazole)-*N*,*N*,*N*',*N*'-tetramethyluronium hexafluorophosphate (296.84 mg, 773.34 µmol) and Compound **Int8-1** (604 mg, 644.45 µmol) were added to anhydrous *N,N*-dimethylformamide (4 mL), and stirred at 0°C for 2 hours. The reaction mixture was directly purified by reverse-phase column chromatography (Preparation method B, elution gradient: A% = 25% to 35%) to obtain the title compound of this step (747.9 mg, yield: 95.6%).

MS m/z (ESI: 1214.6 [M+H]⁺.

### Step 2: Preparation of Compound Int8-3

Compound **Int8-2** (460 mg, 378.75 µmol) was dissolved in a mixed solvent of anhydrous dichloromethane (6 mL) and trifluoroacetic acid (3 mL), and stirred at 25°C for 2 hours. The reaction mixture was concentrated to obtain the title compound of this step (400 mg, 91.2% yield).

MS m/z (ESI): 1158.6 [M+H]⁺.

### Step 3: Preparation of Compound Int8-4

Compound **Int8-3** (113.74 mg, 91.19 µmol), *N,N*-diisopropylethylamine (23.24 mg, 178.03 µmol), 2-(7-azobenzotriazole)-*N,N,N',N'*-tetramethyluronium hexafluorophosphate (51.28 mg, 133.52 µmol) and VC-PABC-MMAE (95 mg, 84.56 µmol) were added to anhydrous *N,N-*dimethylformamide (4 mL), and stirred at 0°C for 2 hours. The reaction mixture was directly purified by reverse-phase column chromatography (Preparation method B, elution gradient: A% = 55% to 65%) to obtain the title compound of this step (170 mg, yield: 93.5%).

MS m/z (ESI): 1132.7 [M/2+H]⁺.

### Step 4: Preparation of Compound Int8

Compound **Int8-4** (170 mg, 75.09 µmol) and diethylamine (27.74 mg, 375.47 µmol) were added to *N,N*-dimethylformamide (4 mL), and stirred at 25°C for 2 hours. The reaction mixture was directly purified by reverse-phase column chromatography (Preparation method B, elution gradient: A% = 55% to 65%) to obtain the title compound of this step (110 mg, yield: 71.9%).

MS m/z (ESI): 1021.6 [M/2+H]⁺.

### Intermediate Preparation Example 9: Preparation of Compound Int9

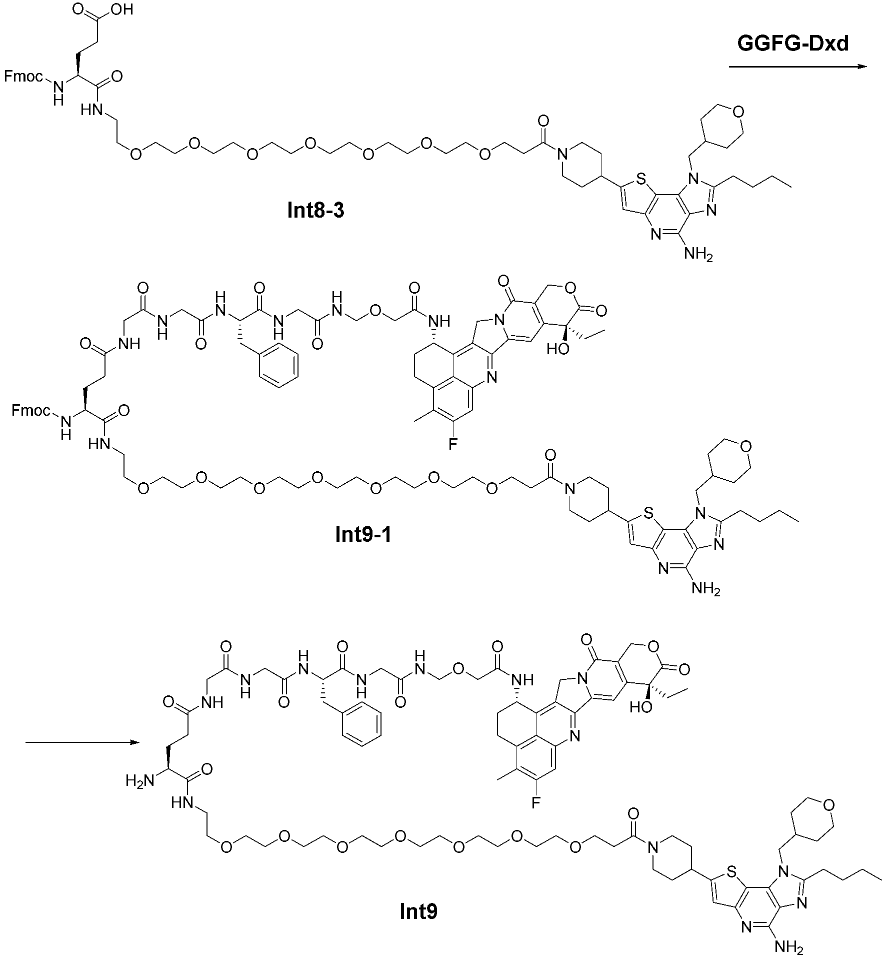

### Step 1: Preparation of Compound Int9-1

Compound **Int8-3** (440 mg, 379.83 µmol), *N,N*-diisopropylethylamine (98.18 mg, 759.66 µmol), 2-(7-azobenzotriazole)-*N*,*N*,*N*',*N*'-tetramethyluronium hexafluorophosphate (173.31 mg, 455.8 µmol) and GGFG-Dxd (319.38 mg, 379.83 µmol) were added to anhydrous *N,N-*dimethylformamide (6 mL), and reacted at 0°C for 2 hours. The reaction mixture was directly purified by reverse-phase column chromatography (Preparation method B, elution gradient: A% = 25% to 35%) to obtain the title compound of this step (250 mg, yield: 35.0%). MS m/z (ESI): 991.5 [M/2+H]⁺.

### Step 2: Preparation of Compound Int9

Compound **Int9-1** (205 mg, 103.47 µmol) and diethylamine (36.31 mg, 491.49 µmol) were added to *N,N*-dimethylformamide (2 mL), and reacted at 25°C for 2 hours. The reaction mixture was directly purified by reverse-phase column chromatography (Preparation method B, elution gradient: A% = 25% to 35%) to obtain the title compound of this step (160 mg, yield: 93.1%).

MS m/z (ESI): 586.9 [M/3+H]⁺.

### Intermediate Preparation Example 10: Preparation of Compound Int10

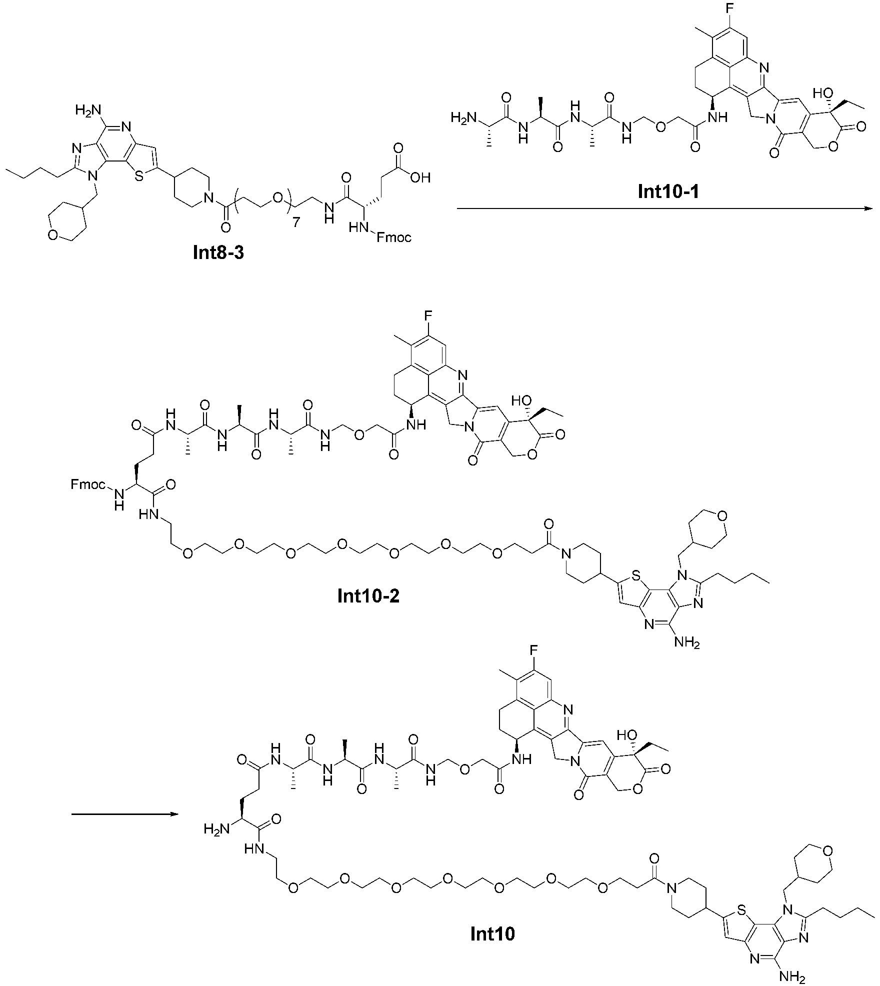

### Step 1: Preparation of Compound Int10-2

Compound **Int8-3** (75.54 mg, 61.78 µmol), *N,N*-diisopropylethylamine (15.97 mg, 123.56 µmol), 2-(7-azobenzotriazole)-*N,N,N',N'*-tetramethyluronium hexafluorophosphate (30.54 mg, 80.32 µmol) and Compound **Int10-1** (50 mg, 67.96 µmol) were added to anhydrous *N,N-*dimethylformamide (3 mL), and reacted at 0°C for 2 hours. The reaction mixture was directly purified by reverse-phase column chromatography (Preparation method B, elution gradient: A% = 25% to 35%) to obtain the title compound of this step (85 mg, yield: 77.2%).

MS m/z (ESI): 1876.9 [M+H]⁺.

### Step 2: Preparation of Compound Int10

Compound **Int10-2** (85 mg, 45.3 µmol) and diethylamine (15.9 mg, 215.2 µmol) were added to *N,N*-dimethylformamide (2 mL), and reacted at 25°C for 2 hours. The reaction mixture was directly purified by reverse-phase column chromatography (Preparation method B, elution gradient: A% = 45% to 55%) to obtain the title compound of this step (70 mg, yield: 98.3%).

MS m/z (ESI): 1653.8 [M+H]⁺.

### Intermediate Preparation Example 11: Preparation of Compound Int11

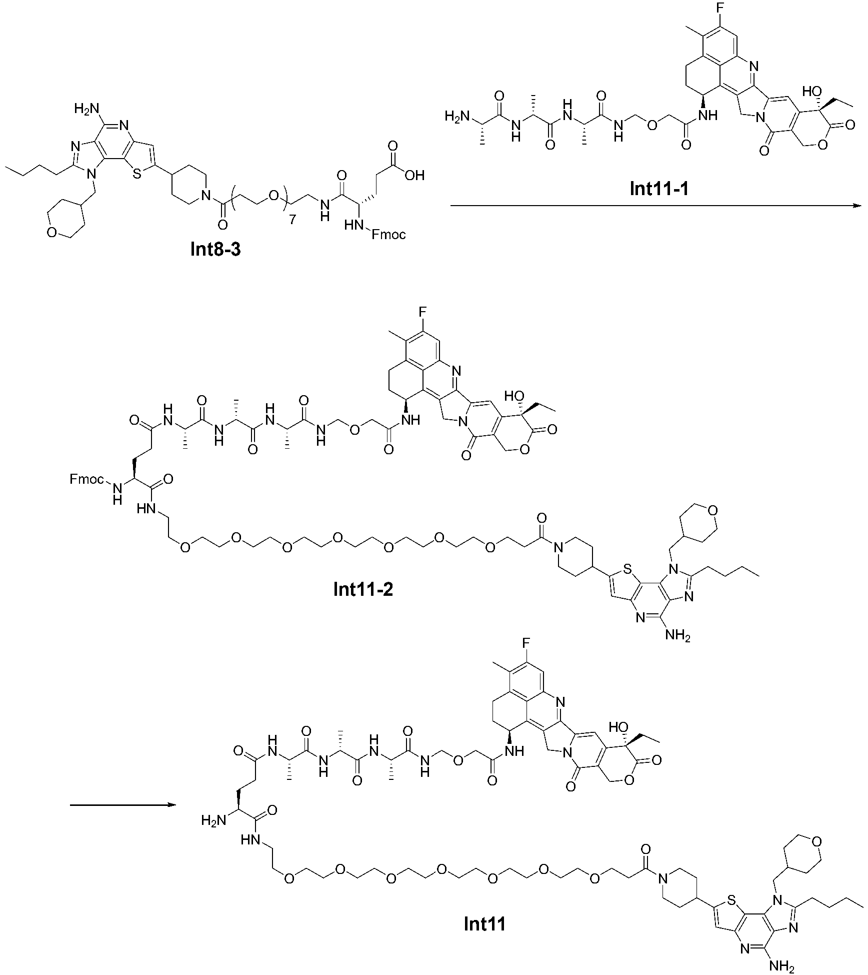

### Step 1: Preparation of Compound Int11-2

Compound Int8-3 (51.95 mg, 44.85 µmol), *N,N-*diisopropylethylamine (11.09 mg, 85.84 µmol), 2-(7-azobenzotriazole)-*N,N,N',N'*-tetramethyluronium hexafluorophosphate (22.85 mg, 60.08 µmol) and Compound **Int11-1** (30 mg, 40.77 µmol) were added sequentially to anhydrous *N,N-*dimethylformamide (3 mL), and reacted at 0°C for 2 hours. The reaction mixture was directly purified by reverse-phase column chromatography (Preparation method B, elution gradient: A% = 45% to 50%) to obtain the title compound of this step (56 mg, yield: 73.2%). MS m/z (ESI): 1876.9 [M+H]⁺.

### Step 2: Preparation of Compound Int11

Compound **Int11-2** (57 mg, 30.38 µmol) and diethylamine (11.22 mg, 151.91 µmol) were added to *N,N*-dimethylformamide (2 mL), and reacted at 25°C for 2 hours. The reaction mixture was directly purified by reverse-phase column chromatography (Preparation method B, elution gradient: A% = 45%-55%) to obtain the title compound of this step (30 mg, yield: 59.7%).

MS m/z (ESI): 827.4 [M/2+H]⁺.

### Intermediate Preparation Example 12: Preparation of Compound Int12

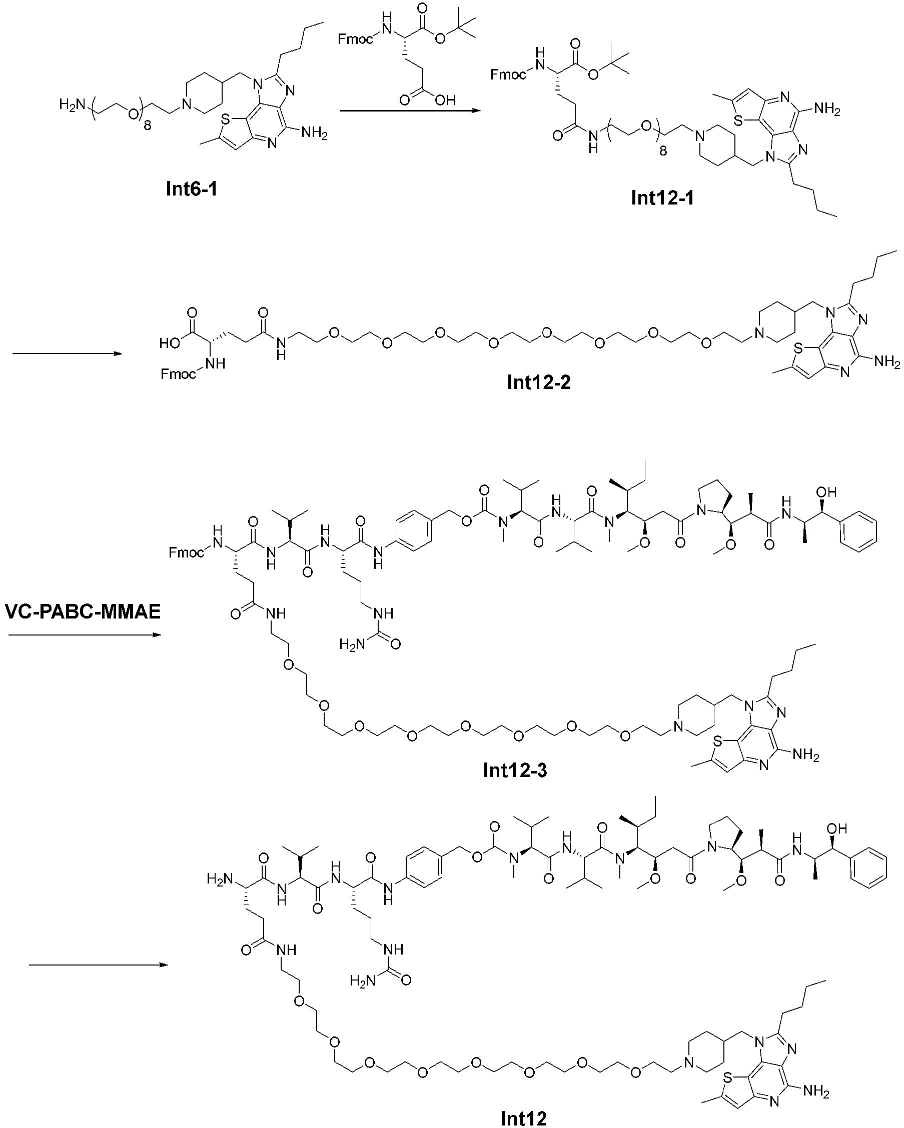

### Step 1: Preparation of Compound Int12-1

(*S*)-2-((((9*H*-Fluoren-9-yl)methoxy)carbonyl)amino)-5-(tert-butyloxy)-5-oxopentanoic acid (37.29 mg, 87.65 µmol), *N,N*-diisopropylethylamine (14.16 mg, 109.56 µmol), 2-(7-azobenzotriazole)-*N*,*N*,*N*',*N*'-tetramethyluronium hexafluorophosphate (38.88 mg, 102.25 µmol) and Compound **Int6-1** (55 mg, 73.04 µmol) were added sequentially to anhydrous *N,N-*dimethylformamide (5 mL), and reacted at 0°C for 2 hours. The reaction mixture was directly purified by reverse-phase column chromatography (Preparation method B, elution gradient: A% = 25% to 35%) to obtain the title compound of this step (80 mg, yield: 99.4%).

MS m/z (ESI): 1160.6 [M+H]⁺.

### Step 2: Preparation of Compound Int12-2

Compound **Int12-1** (50 mg, 43.09 µmol) was dissolved in a mixed solvent of anhydrous dichloromethane (3 mL) and trifluoroacetic acid (1.5 mL), and reacted at 25°C for 4 hours. The reaction mixture was directly purified by reverse-phase column chromatography (Preparation method B, elution gradient: A% = 35% to 45%) to obtain the title compound of this step (45 mg, yield: 62.2%).

MS m/z (ESI): 1104.6 [M+H]⁺.

### Step 3: Preparation of Compound Int12-3

Compound **Int12-2** (45 mg, 40.74 µmol), *N,N*-diisopropylethylamine (7.58 mg, 58.07 µmol), 2-(7-azobenzotriazole)-*N*,*N*,*N*',*N*'-tetramethyluronium hexafluorophosphate (20.81 mg, 54.19 µmol) and VC-PABC-MMAE (45.78 mg, 40.74 µmol) were added to anhydrous *N,N-*dimethylformamide (4 mL), and reacted at 0°C for 2 hours. The reaction mixture was directly purified by reverse-phase column chromatography (Preparation method B, elution gradient: A% = 20% to 30%) to obtain the title compound of this step (30 mg, yield: 35.1%).

MS m/z (ESI): 1105.7 [M/2+H]⁺.

### Step 4: Preparation of Compound Int12

Compound **Int12-3** (30 mg, 13.44 µmol) and diethylamine (4.58 mg, 53.76 µmol) were added to *N,N*-dimethylformamide (2 mL), and reacted at 25°C for 2 hours. The reaction mixture was directly purified by reverse-phase column chromatography (Preparation method B, elution gradient: A% = 35% to 45%) to obtain the title compound of this step (25 mg, yield: 93.6%).

MS m/z (ESI): 994.6 [M/2+H]⁺.

### Intermediate Preparation Example 13: Preparation of Compound Int13

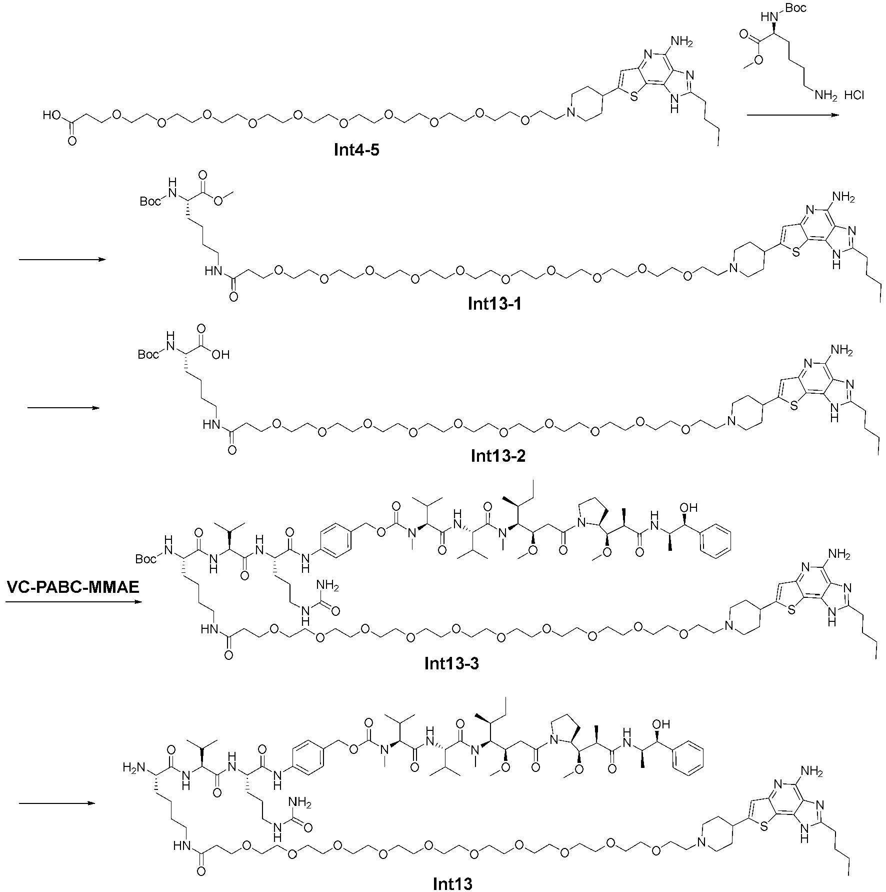

### Step 1: Preparation of Compound Int13-1

Compound **Int4-5** (150 mg, 169.23 µmol) and *N*-tert-butyloxycarbonyllysine methyl ester hydrochloride (46.37 mg, 169.23 µmol) were dissolved in anhydrous *N,N*-dimethylformamide (4 mL), then *N,N*-diisopropylethylamine (44.18 mg, 338.46 µmol) and 2-(7-azobenzotriazole)-*N*,*N*,*N*',*N*'-tetramethyluronium hexafluorophosphate (78.01 mg, 203.08 µmol) were added sequentially. The reaction system was reacted at 25°C for 2 hours. The reaction mixture was concentrated under reduced pressure to obtain a residue, which was purified by silica gel column chromatography (eluent: dichloromethane/methanol = 6/1 (v/v)) to obtain the title compound of this step (160 mg, yield: 95.8%).

MS m/z (ESI): 1084.6 [M+H]⁺.

### Step 2: Preparation of Compound Int13-2

Compound **Int13-1** (185 mg, 162.08 µmol) was dissolved in a mixed solvent of tetrahydrofuran (1 mL), methanol (1 mL) and water (1 mL). Lithium hydroxide monohydrate (28.63 mg, 648.31 µmol) was added, and the reaction system was reacted at 25°C for 3 hours. The reaction mixture was concentrated, and *N,N*-dimethylformamide (1 mL) was added. The resulting solution was purified by reverse-phase column chromatography (Preparation method A, elution gradient: A% = 25% to 35%) to obtain the title compound of this step (160 mg, yield: 87.6%).

MS m/z (ESI): 1070.6 [M+H]⁺.

### Step 3: Preparation of Compound Int13-3

Compound **Int13-2** (50 mg, 42.04 µmol), VC-PABC-MMAE (52.48 mg, 42.04 µmol), *N,N-*diisopropylethylamine (13.70 mg, 105.11 µmol) and 2-(7-azobenzotriazole)-*N,N,N',N'-*tetramethyluronium hexafluorophosphate (19.37 mg, 50.45 µmol) were added sequentially to *N,N-*dimethylformamide (1.5 mL), and reacted at room temperature for 1.5 hours. The reaction mixture was directly purified by reverse-phase column chromatography (Preparation method B, elution gradient: A% = 45% to 55%) to obtain the title compound of this step (41 mg, yield: 40.3%).

MS m/z (ESI): 1088.7 [M/2+H]⁺.

### Step 4: Preparation of Compound Int13

Compound **Int13-3** (38 mg, 15.72 µmol) and anhydrous zinc bromide (17.88 mg, 78.59 µmol) were added to anhydrous dichloromethane (3 mL), and reacted at room temperature for 5 hours. The reaction mixture was concentrated to obtain a crude product, which was purified by reverse-phase column chromatography (Preparation method B, elution gradient: A% = 20% to 30%) to obtain the title compound of this step (30 mg, yield: 82.8%).

MS m/z (ESI): 1038.6 [M/2+H]⁺.

### Intermediate Preparation Example 14: Preparation of Compound Int14

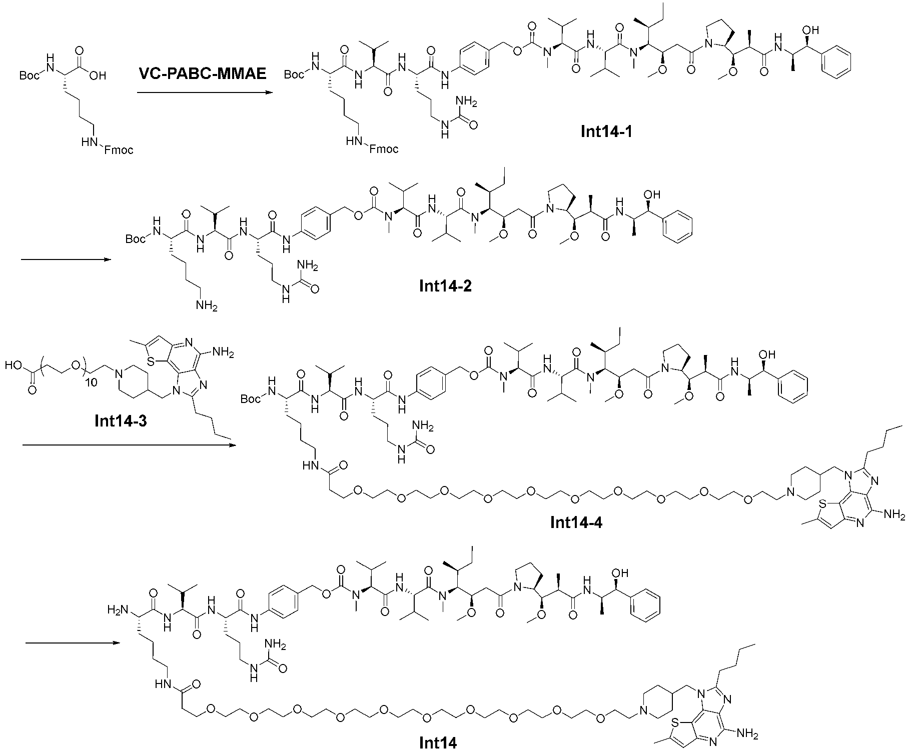

### Step 1: Preparation of Compound Int14-1

VC-PABC-MMAE (150 mg, 133.52 µmol), *N,N*-diisopropylethylamine (22.43 mg, 173.58 µmol), 2-(7-azobenzotriazole)-*N*,*N*,*N*',*N*'-tetramethyluronium hexafluorophosphate (66 mg, 173.58 µmol) and *N⁶*-(((9*H*-fluoren-9-yl)methoxy)carbonyl)-*N²*-(tert-butoxycarbonyl)-*L*-lysine (62.56 mg, 133.52 µmol) were added sequentially to anhydrous *N,N*-dimethylformamide (5 mL), and reacted at 0°C for 1 hour. The reaction mixture was directly purified by reverse-phase column chromatography (Preparation method B, elution gradient: A% = 60% to 70%) to obtain the title compound of this step (170 mg, yield: 85.1 %).

MS m/z (ESI): 1573.9 [M+H]⁺.

### Step 2: Preparation of Compound Int14-2

Compound **Int14-1** (85 mg, 53.46 µmol) and diethylamine (19.75 mg, 267.32 µmol) were added to *N,N*-dimethylformamide (4 mL), and reacted at 25°C for 2 hours. The reaction mixture was directly purified by reverse-phase column chromatography (Preparation method B, elution gradient: A% = 25% to 35%) to obtain the title compound of this step (73 mg, yield: 98.6%).

MS m/z (ESI): 1351.8 [M+H]⁺.

### Step 3: Preparation of Compound Int14-4

Compound **Int14-3** (55.52 mg, 63.81 µmol), *N,N*-diisopropylethylamine (10.32 mg, 79.071 µmol), 2-(7-azobenzotriazole)-*N,N,N',N'*-tetramethyluronium hexafluorophosphate (28.34 mg, 73.8 µmol) and Compound **Int14-2** (75 mg, 55.48 µmol) were added to anhydrous *N,N-*dimethylformamide (4 mL), and reacted at 0°C for 1 hour. The reaction mixture was directly purified by reverse-phase column chromatography (Preparation method B, elution gradient: A% = 25% to 40%) to obtain the title compound of this step (38 mg, yield: 32.7%).

MS m/z (ESI): 1102.7 [M/2+H]⁺.

### Step 4: Preparation of Compound Int14

Compound **Int14-4** (38 mg, 17.24 µmol) and zinc bromide (29.81 mg, 131.05 µmol) were added to anhydrous dichloromethane (3 mL), and reacted at 25°C for 3 hours. The reaction mixture was concentrated to obtain a crude product, which was purified by reverse-phase column chromatography (Preparation method B, elution gradient: A% = 20% to 30%) to obtain the title compound of this step (61 mg, yield: 78.8%).

MS m/z (ESI): 702.1 [M/3+H]⁺.

### Intermediate Preparation Example 15: Preparation of Compound Int15

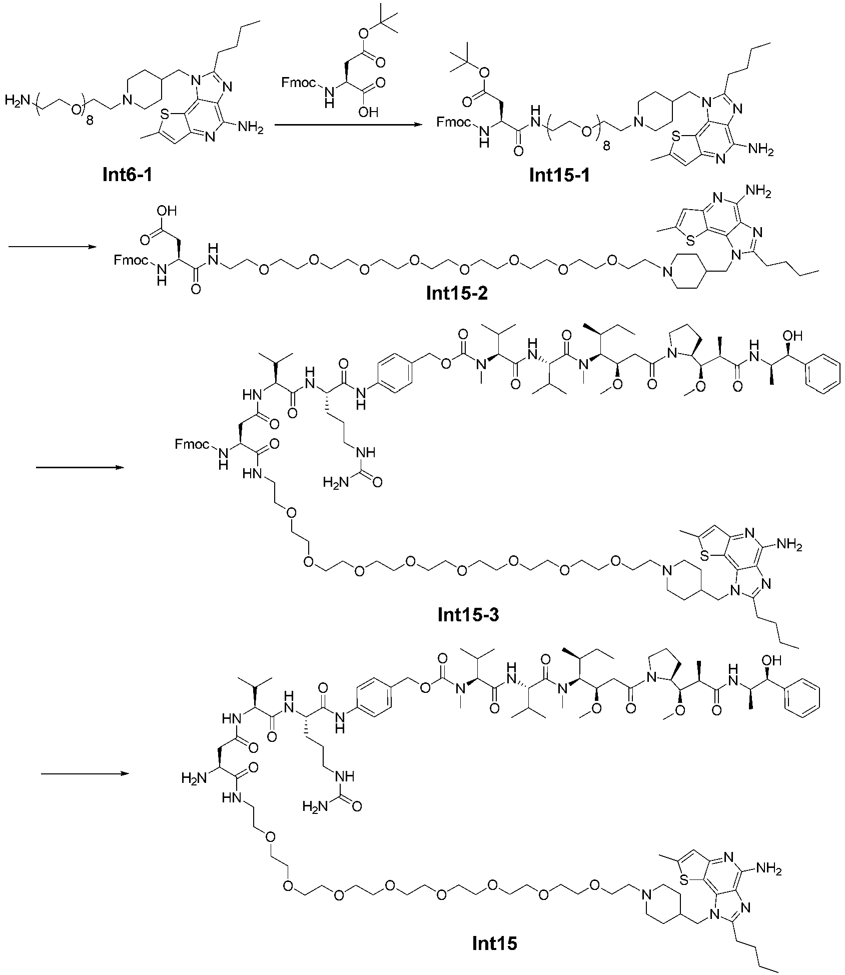

### Step 1: Preparation of Compound Int15-1

Compound **Int6-1** (60 mg, 79.78 µmol), (*S*)-2-((((9*H*-fluoren-9-yl)methoxy)carbonyl) amino)-4-(tert-butyloxy)-4-oxobutanoic acid (32.78 mg, 79.78 µmol), *N,N*-diisopropylethylamine (25.73 mg, 199.46 µmol) and 2-(7-azobenzotriazole)-*N,N,N',N'*-tetramethyluronium hexafluorophosphate (36.38 mg, 95.73 µmol) were added sequentially to *N,N*-dimethylformamide (1 mL), and stirred at room temperature for 1 hour. The reaction mixture was directly purified by reverse-phase column chromatography (Preparation method B, elution gradient: A% = 35% to 40%) to obtain the title compound of this step (52 mg, yield: 56.9%).

MS m/z (ESI): 1146.7 [M+H]⁺.

### Step 2: Preparation of Compound Int15-2

Compound **Int15-1** (50 mg, 43.60 µmol) and 48% hydrobromic acid aqueous solution (0.5 mL) were added to acetonitrile (1 mL), and stirred at room temperature for 3 hours. The reaction mixture was concentrated to obtain the title compound of this step (35 mg, yield: 73.4%).

MS m/z (ESI): 1090.6 [M+H]⁺.

### Step 3: Preparation of Compound Int15-3

Compound **Int15-2** (35 mg, 32.09 mmol), VC-PABC-MMAE (36.04 mg, 32.09 µmol), *N,N-*diisopropylethylamine (10.34 mg, 80.22 µmol) and 2-(7-azobenzotriazole)-*N,N,N',N'-*tetramethyluronium hexafluorophosphate (14.63 mg, 38.50 µmol) were added sequentially to *N,N-*dimethylformamide (1 mL), and stirred at room temperature for 2 hours. The reaction mixture was directly purified by reverse-phase column chromatography (Preparation method B, elution gradient: A% = 40% to 45%) to obtain the title compound of this step (63 mg, yield: 89.3%).

MS m/z (ESI): 1098.6 [M/2+H]⁺.

### Step 4: Preparation of Compound Int15

Compound **Int15-3** (63 mg, 28.7 µmol) and piperidine (4.82 mg, 57.40 µmol) were added to *N,N*-dimethylformamide (1 mL) and stirred at room temperature for 2 hours. The reaction mixture was directly purified by reverse-phase column chromatography (Preparation method B, elution gradient: A% = 25% to 35%) to obtain the title compound of this step (28 mg, yield: 49.5%).

MS m/z (ESI): 1974.2 [M+H]⁺.

### Intermediate Preparation Example 16: Preparation of Compound Int16

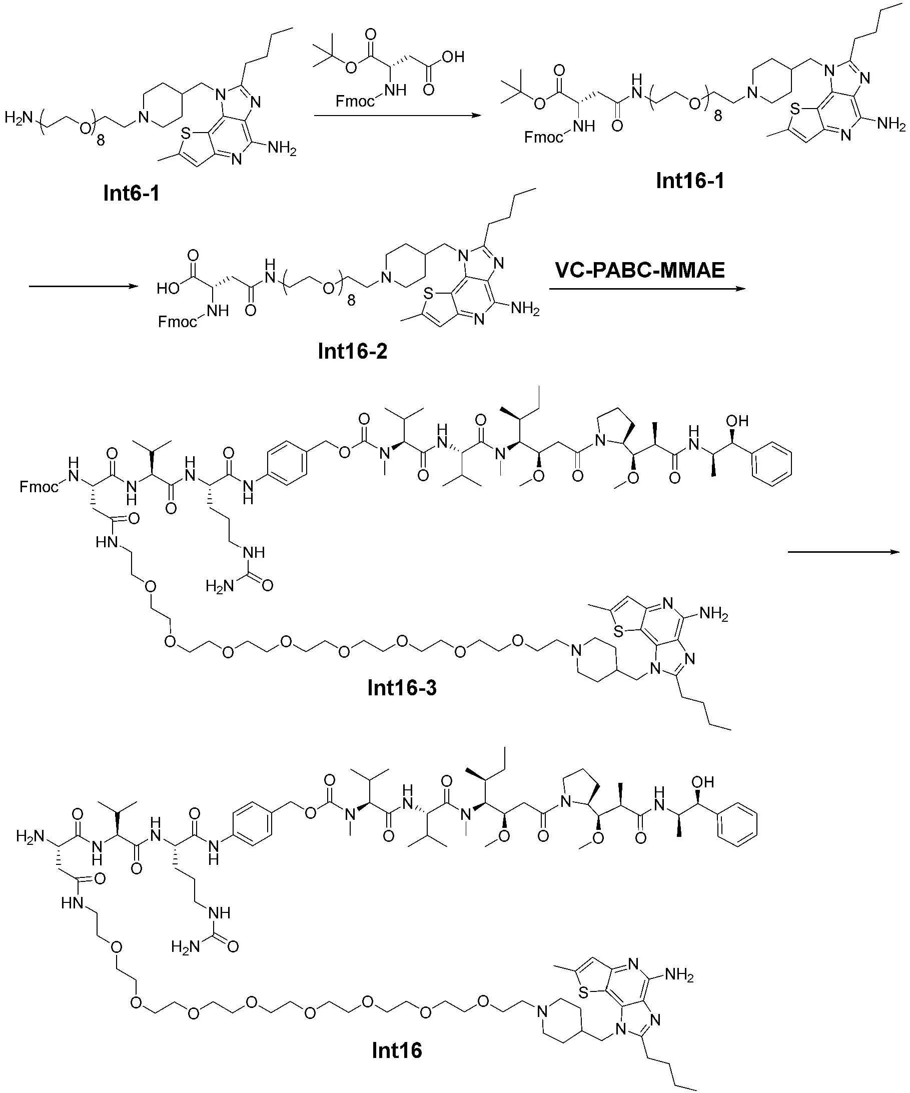

### Step 1: Preparation of Compound Int16-1

(*S*)-3-((((9*H*-Fluoren-9-yl)methoxy)carbonyl)amino)-4-(tert-butyloxy)-4-oxobutanoic acid (35.52 mg, 86.33 µmol), *N,N*-diisopropylethylamine (14.16 mg, 109.56 µmol), 2-(7-azobenzotriazole)-*N*,*N*,*N*',*N*'-tetramethyluronium hexafluorophosphate (38.88 mg, 102.25 µmol) and Compound **Int6-1** (65 mg, 86.33 µmol) were added sequentially to anhydrous *N,N-*dimethylformamide (5 mL), and reacted at 0°C for 2 hours. The reaction mixture was directly purified by reverse-phase column chromatography (Preparation method B, elution gradient: A% = 25% to 35%) to obtain the title compound of this step (50 mg, yield: 53.2%).

MS m/z (ESI): 1146.6 [M+H]⁺.

### Step 2: Preparation of Compound Int16-2

Compound **Int16-1** (50 mg, 43.18 µmol) was dissolved in a mixed solvent of anhydrous dichloromethane (3 mL) and trifluoroacetic acid (1.5 mL), and reacted at 25°C for 4 hours. The reaction mixture was directly purified by reverse-phase column chromatography (Preparation method B, elution gradient: A% = 35% to 45%) to obtain the title compound of this step (45 mg, yield: 89.2%).

MS m/z (ESI): 1090.5 [M+H]⁺.

### Step 3: Preparation of Compound Int16-3

Compound **Int16-2** (41.53 mg, 38.09 µmol), *N,N*-diisopropylethylamine (6.82 mg, 52.37 µmol), 2-(7-azobenzotriazole)-*N,N,N',N'*-tetramethyluronium hexafluorophosphate (18.07 mg, 47.05 µmol) and VC-PBAC-MMAE (42.8 mg, 38.09 µmol) were added sequentially to anhydrous *N,N*-dimethylformamide (3 mL), and reacted at 0°C for 2 hours. The reaction mixture was directly purified by reverse-phase column chromatography (Preparation method B, elution gradient: A% = 20% to 25%) to obtain the title compound of this step (52 mg, yield: 65.4%).

MS m/z (ESI): 1098.6 [M/2+H]⁺.

### Step 4: Preparation of Compound Int16

Compound **Int16-3** (52 mg, 23.69 µmol) and piperidine (6.52 mg, 76.54 µmol) were added to *N,N*-dimethylformamide (2 mL) and reacted at 25°C for 2 hours. The reaction mixture was directly purified by reverse-phase column chromatography (Preparation method B, elution gradient: A% = 35% to 45%) to obtain the title compound of this step (31 mg, yield: 82.9%).

MS m/z (ESI): 658.7 [M/3+H]⁺.

### Intermediate Preparation Example 17: Preparation of Compound Int17

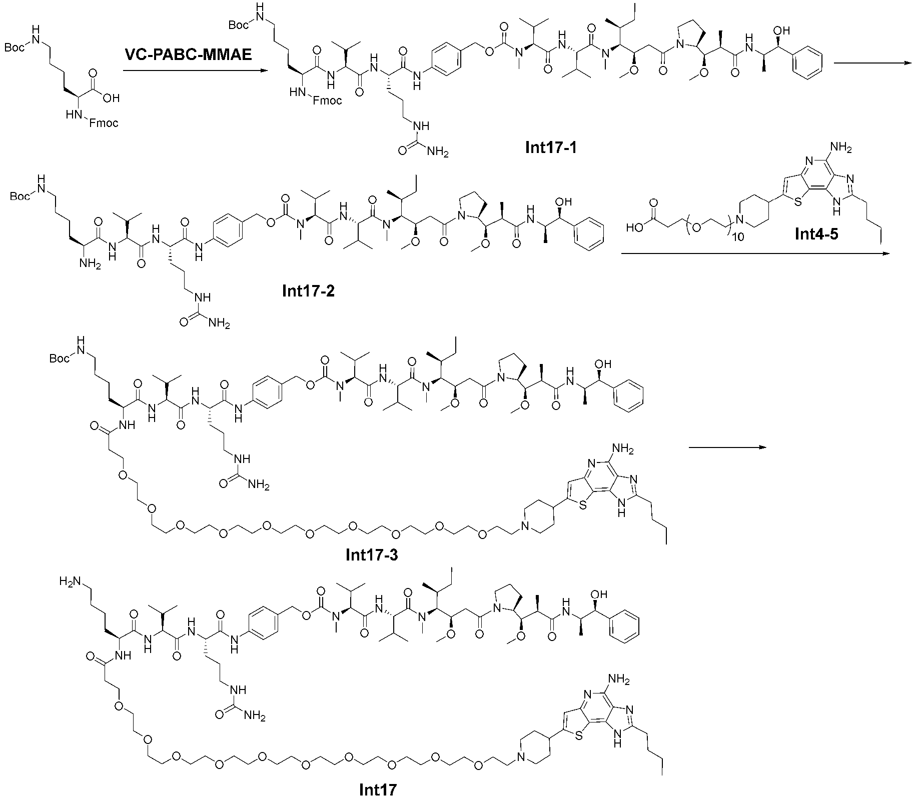

### Step 1: Preparation of Compound Int17-1

VC-PABC-MMAE (60 mg, 53.41 µmol), *N²*-(((9*H*-fluoren-9-yl)methoxy)carbonyl)- *N⁶-*(tert-butyloxycarbonyl)-L-lysine (25 mg, 53.41 µmol), *N,N*-diisopropylethylamine (17.2 mg, 133.52 µmol) and 2-(7-azobenzotriazole)-*N*,*N*,*N*',*N*'-tetramethyluronium hexafluorophosphate (24.35 mg, 64.09 µmol) were added sequentially to *N,N*-dimethylformamide (1 mL), and reacted at room temperature for 1 hour. The reaction system was poured into ice water (10 mL) and extracted with ethyl acetate (5 mL x 3). The organic phases were combined and backwashed with saturated brine (3 mL x 2). The resultant organic phase was dried over anhydrous sodium sulfate and concentrated to obtain the title compound of this step (82 mg, crude).

MS m/z (ESI): 1574.0 [M+H]⁺.

### Step 2: Preparation of Compound Int17-2

Compound **Int17-1** (82.00 mg, crude) and piperidine (8.07 mg, 93.78 µmol) were added sequentially to *N,N*-dimethylformamide (1 mL) and reacted at room temperature for 1 hour. The reaction mixture was directly purified by reverse-phase column chromatography (Preparation method B, elution gradient: A% = 25% to 35%) to obtain the title compound of this step (41 mg, total yield for two steps: 56.8%).

MS m/z (ESI): 1351.9 [M+H]⁺.

### Step 3: Preparation of Compound Int17-3

Compound **Int17-2** (41.00 mg, 30.32 µmol), Compound **Int4-5** (25.52 mg, 30.32 µmol), *N,N-*diisopropylethylamine (9.78 mg, 75.81 µmol) and 2-(7-azobenzotriazole)-*N,N,N,N-*tetramethyluronium hexafluorophosphate (17.28 mg, 45.48 µmol) were added sequentially to *N,N-*dimethylformamide (1 mL), and reacted at room temperature for 2 hours. The reaction mixture was directly purified by reverse-phase column chromatography (Preparation method B, elution gradient: A% = 45% to 50%) to obtain the title compound of this step (35 mg, yield: 53.1 %).

MS m/z (ESI): 1088.7 [M/2+H]⁺.

### Step 4: Preparation of Compound Int17

Compound **Int17-3** (35 mg, 16.09 µmol) and anhydrous zinc bromide (14.28 mg, 64.36 µmol) were added to dichloromethane (2 mL) and reacted at room temperature for 3 hours. The reaction mixture was directly purified by reverse-phase column chromatography (Preparation method B, elution gradient: A% = 25% to 30%) to obtain the title compound of this step (25 mg, yield: 74.9%).

MS m/z (ESI): 1038.6 [M/2+H]⁺.

### Intermediate Preparation Example 18: Preparation of Compound Int18

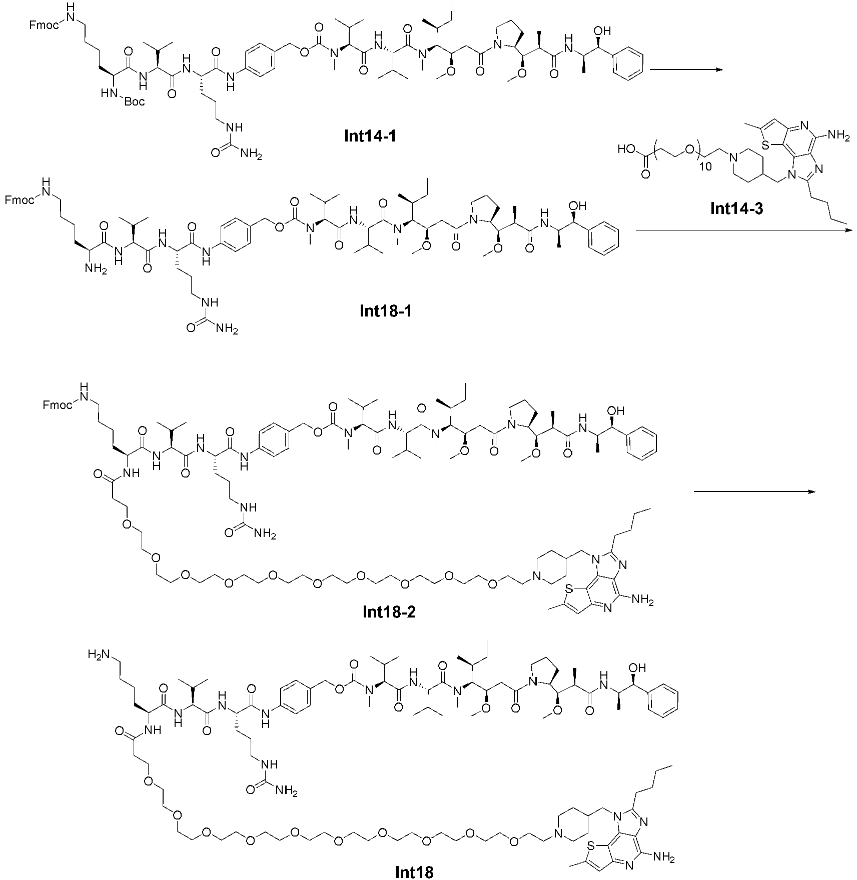

### Step 1: Preparation of Compound Int18-1

Compound **Int14-1** (87 mg, 55.27 µmol) and zinc bromide (95.56 mg, 420.09 µmol) were added to anhydrous dichloromethane (3 mL) and reacted at 25°C for 3 hours. The reaction mixture was directly purified by reverse-phase column chromatography (Preparation method B, elution gradient: A% = 35% to 45%) to obtain the title compound of this step (61 mg, yield: 78.8%).

MS m/z (ESI): 1473.8 [M+H]⁺.

### Step 2: Preparation of Compound Int18-2

Compound **Int14-3** (21.25 mg, 24.42 µmol), *N,N-*diisopropylethylamine (4.54 mg, 34.81 µmol), 2-(7-azobenzotriazole)-*N,N,N,N*-tetramethyluronium hexafluorophosphate (12.48 mg, 32.49 µmol) and Compound **Int18-1** (36 mg, 24.42 µmol) were added sequentially to anhydrous N,N-dimethylformamide (3 mL), and reacted at 0°C for 1 hour. The reaction mixture was directly purified by reverse-phase column chromatography (Preparation method B, elution gradient: A% = 20% to 35%) to obtain the title compound of this step (45 mg, yield: 83.4%).

MS m/z (ESI): 1163.7 [M/2+H]⁺.

### Step 3: Preparation of Compound Int18

Compound **Int18-2** (40 mg, 17.03 µmol) and diethylamine (6.29 mg, 85.13 µmol) were added to N,N-dimethylformamide (4 mL) and reacted at 25°C for 2 hours. The reaction mixture was directly purified by reverse-phase column chromatography (Preparation method B, elution gradient: A% = 25% to 35%) to obtain the title compound of this step (30 mg, yield: 83.8%).

MS m/z (ESI): 1052.7 [M/2+H]⁺.

### Intermediate Preparation Example 19: Preparation of Compound Int19

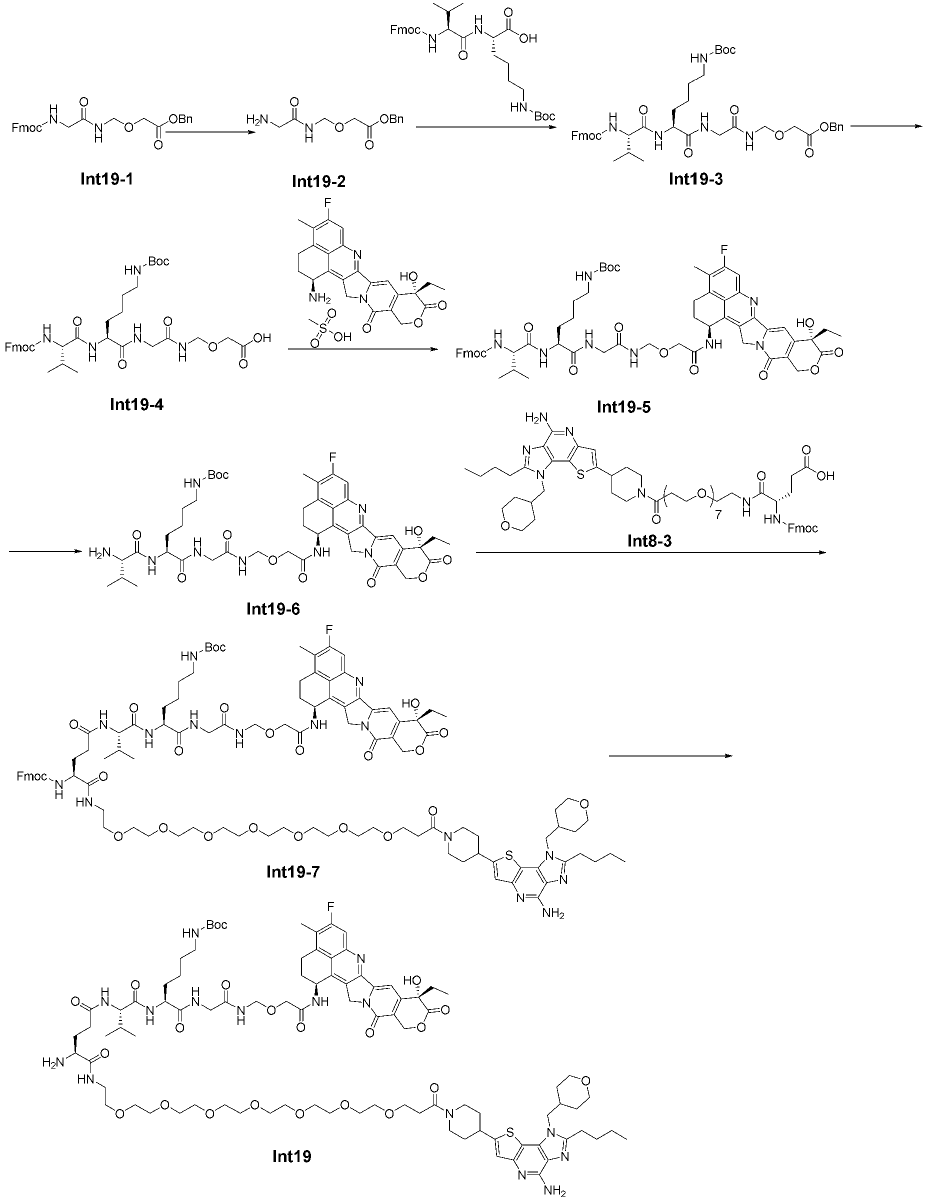

### Step 1: Preparation of Compound Int19-2

Compound **Int19-1** (800 mg, 1.67 mmol) and piperidine (287.11 mg, 3.34 mmol) were added to N,N-dimethylformamide (15 mL) and reacted at room temperature for 2 hours. After concentration, methyl tert-butyl ether (20 mL) was added. After sonication for 5 minutes, the reaction mixture was allowed to stand, filtered, and the resultant solid was dried to obtain the title compound of this step (315 mg, yield: 73.5%).

MS m/z (ESI): 253.1 [M+H]⁺.

### Step 2: Preparation of Compound Int19-3

Compound **Int19-2** (90 mg, 321.09 µmol), *N²*-((((9H-fluoren-9-yl)methoxy)carbonyl)-*L-*valine)-*N*⁶-(tert-butyloxycarbonyl)-*L*-lysine (202.53 mg, 321.09 µmol), *N,N-*diisopropylethylamine (104.60 mg, 802.72 µmol), 1-hydroxybenzotriazole (49.23 mg, 353.20 µmol) and 1-(3-dimethylaminopropyl)-3-ethylcarbodiimide hydrochloride (77.43 mg, 401.36 µmol) were added to *N,N*-dimethylformamide (3 mL), and reacted at room temperature for 3 hours. The reaction mixture was directly purified by reverse-phase column chromatography (Preparation method B, elution gradient: A% = 50% to 55%) to obtain the title compound of this step (142 mg, yield: 47.4%).

MS m/z (ESI): 802.4 [M+H]⁺.

### Step 3: Preparation of Compound Int19-4

Compound **Int19-3** (142 mg, 159.37 µmol), ammonium formate (50.75 mg, 796.84 µmol) and 10% palladium-on-carbon (15 mg) were added to anhydrous methanol (4 mL), and reacted at room temperature for 3 hours. The reaction mixture was directly purified by reverse-phase column chromatography (Preparation method B, elution gradient: A% = 30% to 35%) to obtain the title compound of this step (101 mg, yield: 80.1%).

MS m/z(ESI): 712.4 [M+H]⁺.

### Step 4: Preparation of Compound Int19-5

Compound **Int19-4** (95 mg, 133.46 µmol), exatecan mesylate (70.94 mg, 133.46 µmol), *N,N-*diisopropylethylamine (43.04 mg, 333.66 µmol) and bis(2-oxo-3-oxazolidinyl)phosphinoyl chloride (50.85 mg, 200.20 µmol) were added to N,N-dimethylformamide (1 mL), and reacted at room temperature for 3 hours. The reaction mixture was directly purified by reverse-phase column chromatography (Preparation method B, elution gradient: A% = 45% to 55%) to obtain the title compound of this step (105 mg, yield: 69.7%).

MS m/z (ESI): 1129.5 [M+H]⁺.

### Step 5: Preparation of Compound Int19-6

Compound **Int19-5** (105 mg, 92.98 µmol) and piperidine (15.83 mg, 185.97 µmol) were added to *N,N*-dimethylformamide (1 mL) and reacted at room temperature for 2 hours. The reaction mixture was directly purified by reverse-phase column chromatography (Preparation method A, elution gradient: A% = 27% to 33%) to obtain the title compound of this step (46 mg, yield: 54.5%).

MS m/z (ESI): 907.4 [M+H]⁺.

### Step 6: Preparation of Compound Int19-7

Compound **Int8-3** (50 mg, 41.85 µmol), Compound **Int19-6** (37.95 mg, 41.85 µmol), *N,N-*diisopropylethylamine (13.63 mg, 104.61 µmol), and 2-(7-azobenzotriazole)-*N,N,N,N-*tetramethyluronium hexafluorophosphate (19.27 mg, 50.21 µmol) were added sequentially to *N,N-*dimethylformamide (1 mL), and reacted at room temperature for 2 hours. The reaction mixture was directly purified by reverse-phase column chromatography (Preparation method A, elution gradient: A% = 40% to 48%) to obtain the title compound of this step (43 mg, yield: 50.2%).

MS m/z (ESI): 1024.5 [M/2+H]⁺.

### Step 7: Preparation of Compound Int19

Compound **Int19-7** (40 mg, 19.54 µmol) and piperidine (3.33 mg, 39.07 µmol) were added to N,N-dimethylformamide (1 mL) and reacted at room temperature for 2 hours. After concentration, the reaction mixture, methyl tert-butyl ether (5 mL) was added. After sonication for 5 minutes, the resultant reaction mixture was allowed to stand, filtered, and the resultant solid was dried to obtain the title compound of this step (28 mg, yield: 70.7%).

MS m/z (ESI): 1824.9 [M+H]⁺.

### Intermediate Preparation Example 20: Preparation of Compound Int20

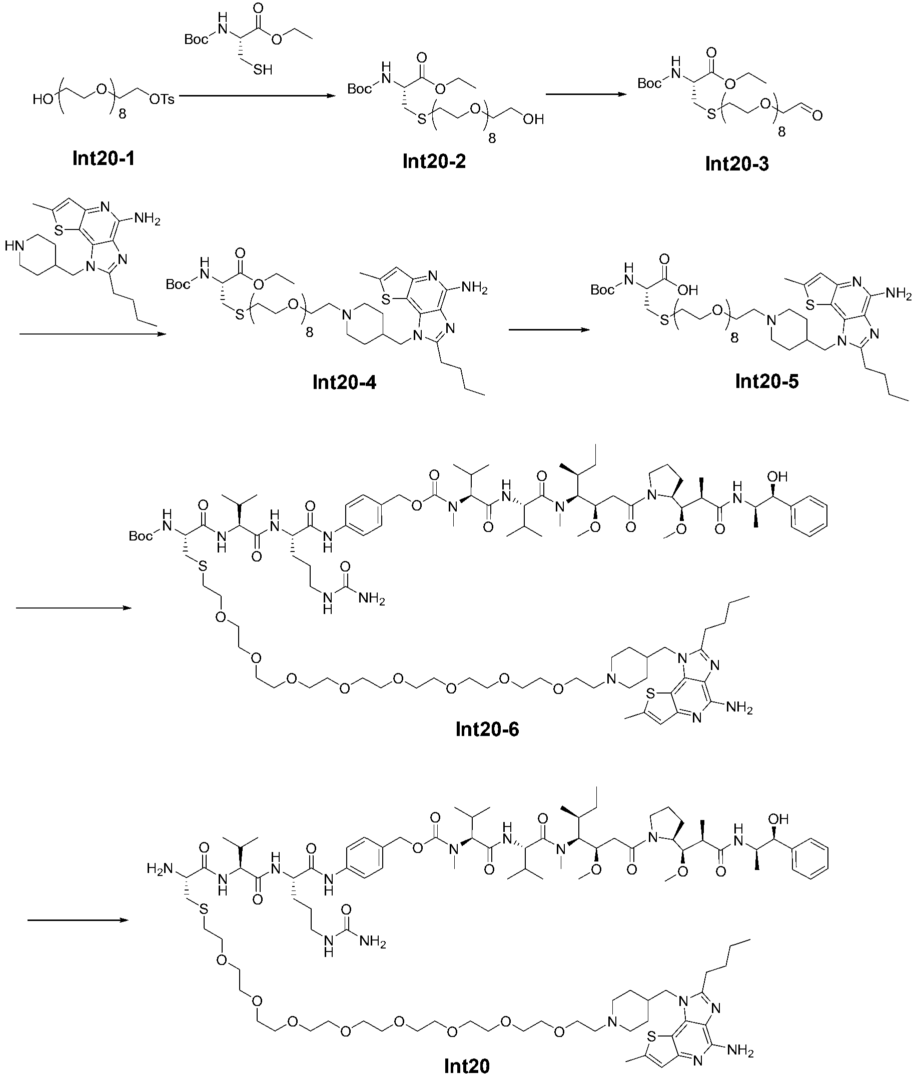

### Step 1: Preparation of Compound Int20-2

Compound **Int20-1** (460 mg, 808.9 µmol) and N-tert-butyloxycarbonyl-*L*-cysteine ethyl ester (242 mg, 970.67 µmol) were dissolved in *N*,*N*-dimethylformamide (6 mL). Then potassium iodide (13.43 mg, 80.89 µmol) and *N,N*-diisopropylethylamine (150 mg, 1152.68 µmol) were added, and reacted at 25°C for 10 hours. The reaction mixture was concentrated, and the resulting residue was purified by silica gel column chromatography (eluent: dichloromethane/methanol = 5/1 (v/v)) to obtain the title compound of this step (320 mg, yield: 64.5%).

MS m/z (ESI): 646.1 [M+H]⁺.

### Step 2: Preparation of Compound Int20-3

Compound **Int20-2** (220 mg, 337.26 µmol) and Dess-Martin periodinane (216.66 mg, 505.89 µmol) were added to dichloromethane (15 mL) and reacted at 25°C for 10 hours. Saturated sodium thiosulfate solution (5 mL) and saturated sodium bicarbonate aqueous solution (10 mL) were added to the reaction mixture and stirred until clear. The reaction mixture was then extracted three times with dichloromethane, washed with saturated brine, dried over anhydrous sodium sulfate, and filtered. The filtrate was concentrated, and the resulting residue was purified by silica gel column chromatography (eluent: dichloromethane/methanol = 20/1 (v/v)) to obtain the title compound of this step (185 mg, yield: 85.2%).

MS m/z (ESI): 644.3 [M+H]⁺.

### Step 3: Preparation of Compound Int20-4

Compound **Int20-3** (146.31 mg, 227.26 µmol), 2-butyl-7-methyl-1-(piperidin-4-ylmethyl)-1*H*-imidazo[4,5-*d*]thieno[3,2-*b*]pyridin-4-amine (65 mg, 181.81 µmol), sodium cyanoborohydride (31.56 mg, 502.2 mmol) and glacial acetic acid (31.4 mg, 518.16 mmol) were added sequentially to a mixed solvent of 1,2-dichloroethane (4 mL) and methanol (2 mL), and reacted at 25°C for 4 hours. The reaction mixture was concentrated to obtain a crude product, which was dissolved in methanol (3 mL) and purified by reverse-phase column chromatography (Preparation method B, elution gradient: A% = 20% to 30%) to obtain the title compound of this step (109 mg, yield: 63.8%).

MS m/z (ESI): 985.5 [M+H]⁺.

### Step 4: Preparation of Compound Int20-5

Compound **Int20-4** (100 mg, 101.49 µmol) was dissolved in methanol (4 mL). Then lithium hydroxide monohydrate (21.31 mg, 507.46 µmol) aqueous solution (1 mL) was added, and reacted at 25°C for 4 hours. The reaction mixture was directly purified by reverse-phase column chromatography (Preparation method B, elution gradient: A% = 35% to 45%) to obtain the title compound of this step (85 mg, yield: 87.5%).

MS m/z (ESI): 957.5 [M+H]⁺.

### Step 5: Preparation of Compound Int20-6

Compound **Int20-5** (65 mg, 67.91 µmol), *N,N*-diisopropylethylamine (12.63 mg, 96.76 µmol, 16.15 µL), 2-(7-azobenzotriazole)-*N*,*N*,*N*',*N*'-tetramethyluronium hexafluorophosphate (34.69 mg, 90.31 µmol) and VC-PABC-MMAE (76.28 mg, 67.91 µmol) were added sequentially to anhydrous *N,N*-dimethylformamide (3 mL), and reacted at 0°C for 2 hours. The reaction mixture was directly purified by reverse-phase column chromatography (Preparation method B, elution gradient: A% = 30% to 45%) to obtain the title compound of this step (85 mg, yield: 63.9%).

MS m/z (ESI): 1032.1 [M/2+H]⁺.

### Step 6: Preparation of Compound Int20

Compound **Int20-6** (38 mg, 18.42 µmol) and zinc bromide (31.85 mg, 140.01 µmol) were added to anhydrous dichloromethane (2 mL), and reacted at 25°C for 4 hours. The reaction mixture was concentrated to obtain a crude product, which was purified by reverse-phase column chromatography (Preparation method B, elution gradient: A% = 20% to 30%) to obtain the title compound of this step (30 mg, yield: 87.0%).

MS m/z (ESI): 982.1 [M/2+H]⁺.

### Drug-Linker Preparation Example 1-1: Preparation of Compound C-1

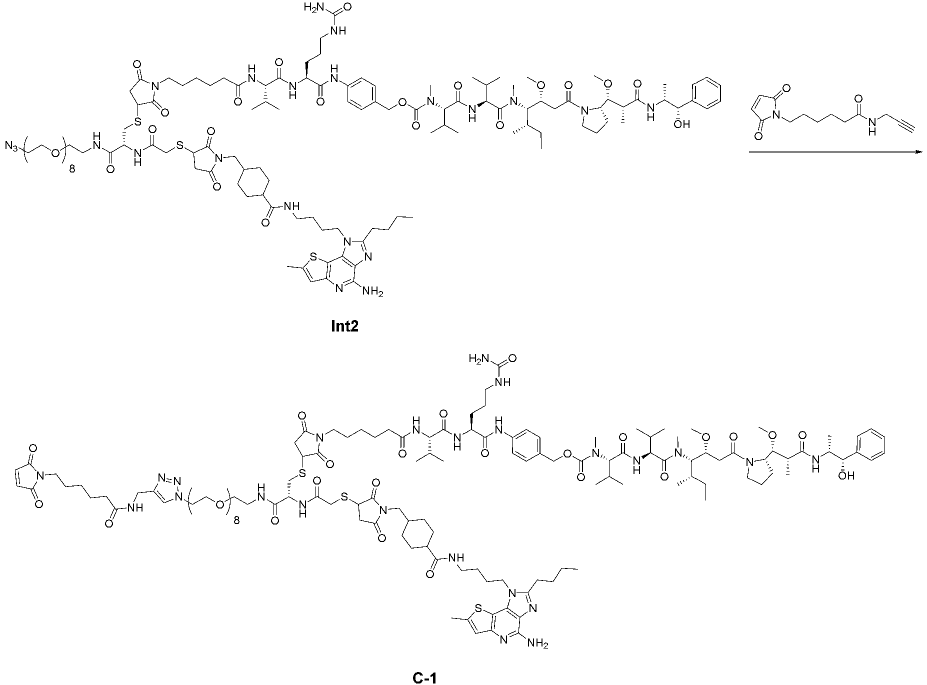

Compound **Int2** (50 mg, 0.02 mmol) was dissolved in DMSO (6 mL) and water (0.6 mL). Under the protection of nitrogen atmosphere, 6-(2,5-dioxo-2,5-dihydro-1*H*-pyrrol-1-yl)-*N*-(prop-2-yn-1-yl)hexanamide (5.99 mg, 0.024 mmol), sodium ascorbate (5.94 mg, 0.03 mmol) and anhydrous copper sulfate (6.4 mg, 0.04 mmol) were added, and stirred at room temperature for 1 hour. The reaction mixture was extracted by adding water and ethyl acetate, and the organic phase was dried and concentrated to obtain a crude product, which was purified by preparative HPLC to obtain the title compound of this step (15 mg, yield: 27.2%).

MS m/z (ESI): 1365.7 [M/2+H]⁺.

### Drug-Linker Preparation Example 1-2: Preparation of Compound C-2

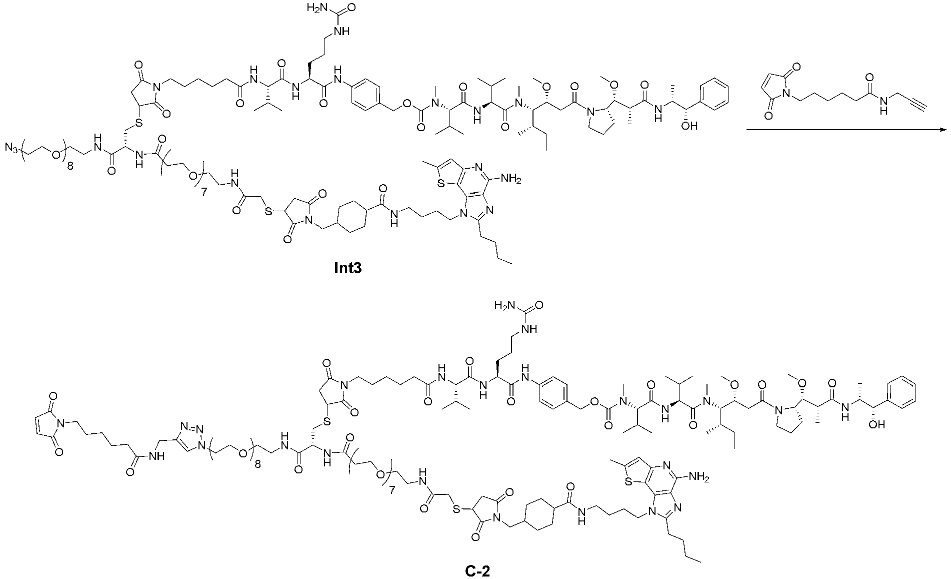

By using the synthetic route of Example 1-1, replacing the starting Compound **Int2** with Compound **Int3,** the title compound of this step was obtained (10 mg, yield: 6%).

MS m/z (ESI): 1555.3 [M/2+H]⁺.

### Drug-linker Preparation Example 1-3: Preparation of Compound C-3

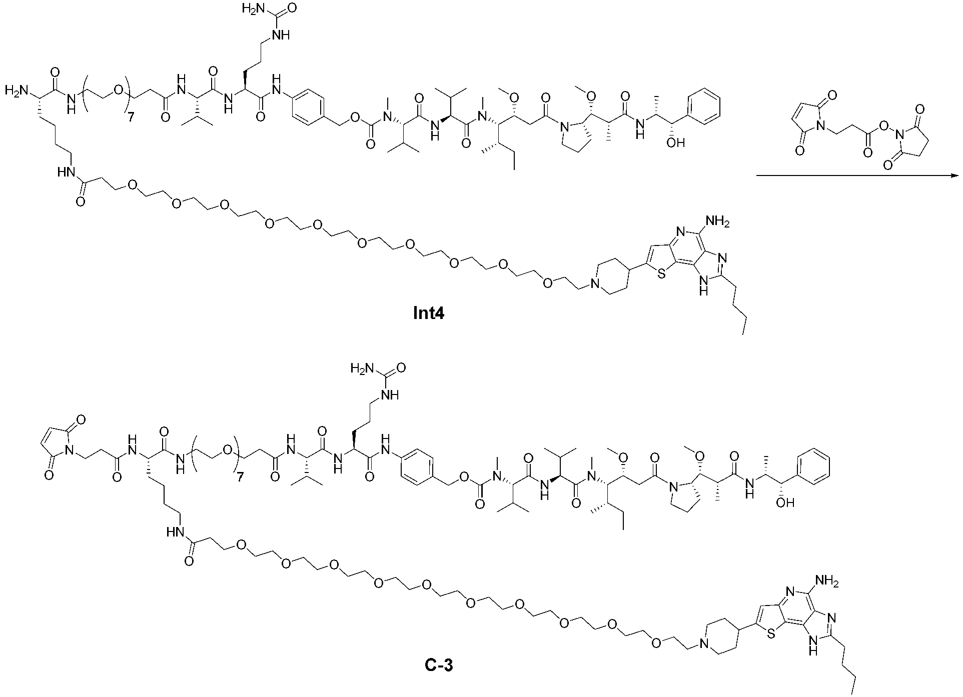

Compound **Int4** (20 mg, 7.74 µmol), 2,5-dioxopyrrolidin-1-yl 3-(2,5-dioxo-2,5-dihydro-1*H-*pyrrol-1-yl)propionate (2.17 mg, 7.74 µmol) and *N,N*-diisopropylethylamine (1.52 mg, 11.61 µmol) were added sequentially to anhydrous *N,N*-dimethylformamide (1.5 mL), and reacted at 25°C for 3 hours. The reaction mixture was purified by preparative HPLC to obtain the title compound of this step (0.64 mg, yield: 3%).

MS m/z (ESI): 1303.8 [M/2+H]⁺.

### Drug-linker Preparation Example 1-4: Preparation of Compound C-4

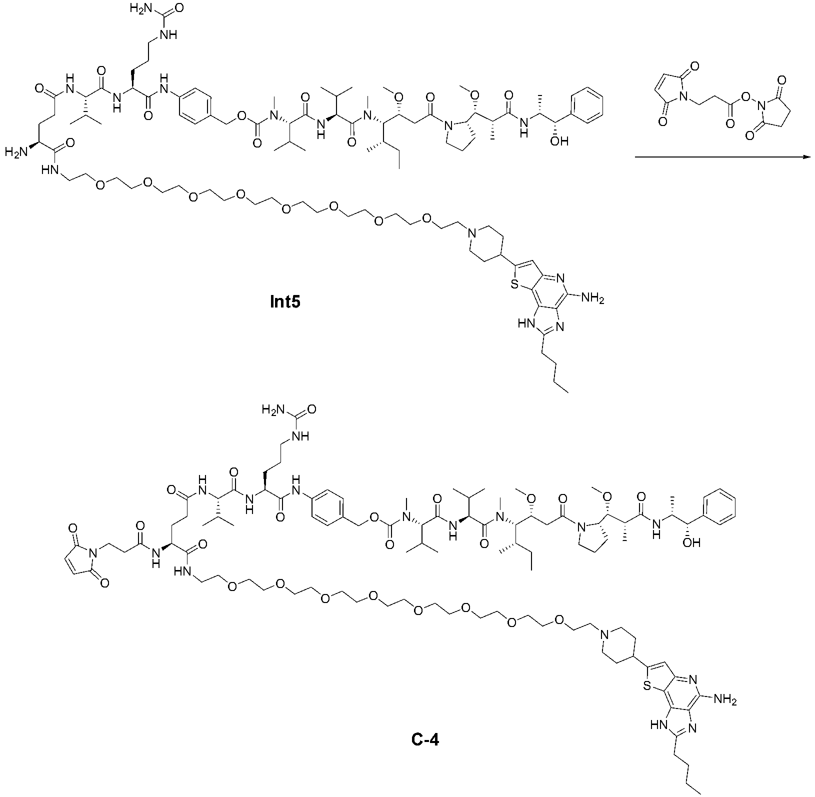

Compound **Int5** (50 mg, 24.24 µmol), 2,5-dioxopyrrolidin-1-yl 3-(2,5-dioxo-2,5-dihydro-1*H*-pyrrol-1-yl)propionate (20.38 mg, 72.72 µmol) and *N,N*-diisopropylethylamine (4.75 mg, 36.36 µmol) were added sequentially to anhydrous *N,N*-dimethylformamide (2 mL), and reacted at 0°C for 2 hours. The reaction mixture was purified by preparative HPLC to obtain the title compound of this step (11 mg, yield: 20.2%). MS m/z (ESI): 1056.1 [M/2+H]⁺.

### Drug-linker Preparation Example 1-5: Preparation of Compound C-5

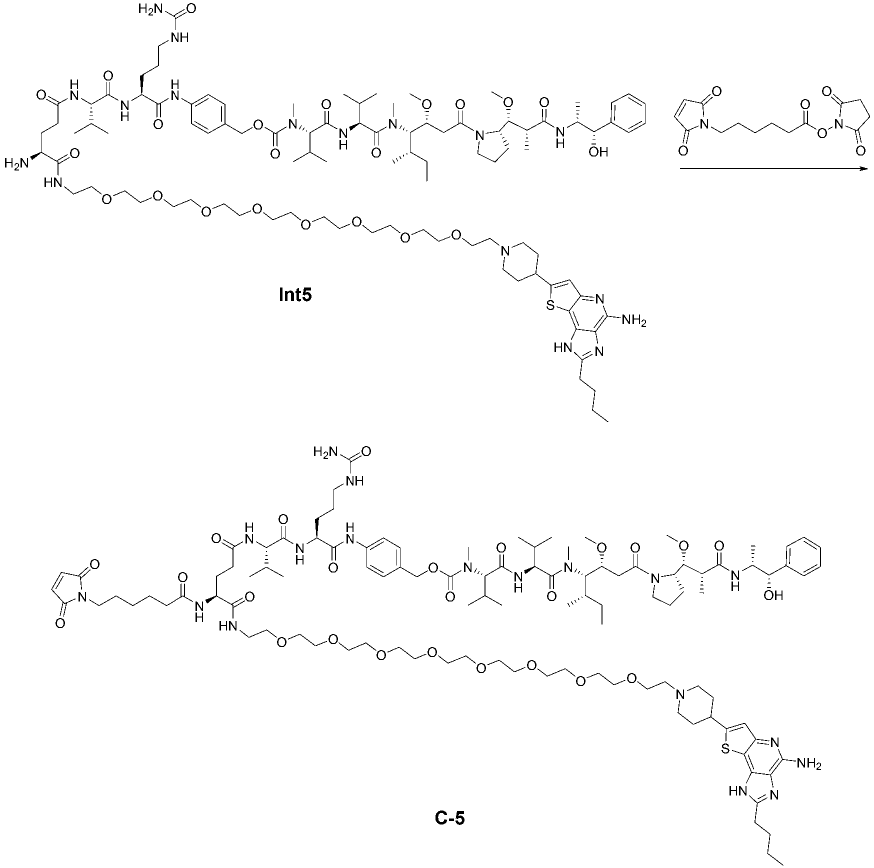

Compound **Int5** (60 mg, 29.09 µmol), 2,5-dioxopyrrolidin-1-yl 6-(2,5-dioxo-2,5-dihydro-1*H*-pyrrol-1-yl)hexanoate (9.44 mg, 09.09 µmol) and *N,N*-diisopropylethylamine (45.70 mg, 43.63 µmol) were dissolved in anhydrous *N,N*-dimethylformamide (2 mL), and reacted at 0°C for 2 hours. The reaction mixture was purified by preparative HPLC to obtain the title compound of this step (11 mg, yield: 16.3%).

MS m/z (ESI): 1077.1 [M/2+H]⁺

### Drug-linker Preparation Example 1-6: Preparation of Compound C-6

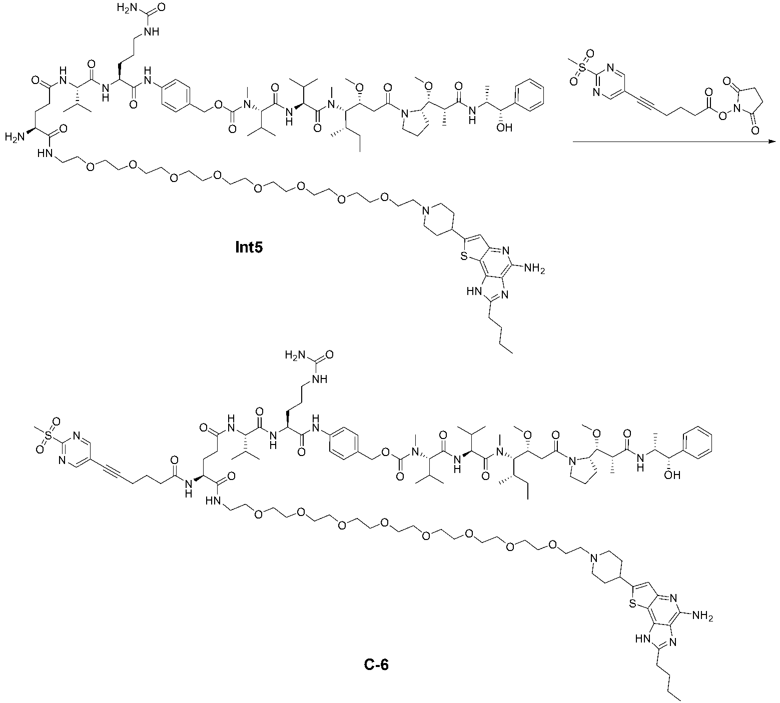

Compound **Int5** (60 mg, 29.09 µmol), 2,5-dioxopyrrolidin-1-yl 6-(2-(methylsulfonyl)pyrimidin-5-yl)hex-5-ynoate (22.38 mg, 58.18 µmol), *N,N-*diisopropylethylamine (5.7 mg, 43.63 µmol) and 1-hydroxybenzotriazole (5.09 mg, 14.55 µmol) were added sequentially to anhydrous *N,N*-dimethylformamide (2 mL), and reacted at 25°C for 2 hours. The reaction mixture was purified by preparative HPLC to obtain the title compound of this step (4 mg, yield: 5.8%).

MS m/z (ESI): 1105.6 [M/2+H]⁺.

### Drug-linker Preparation Example 1-7: Preparation of Compound C-7

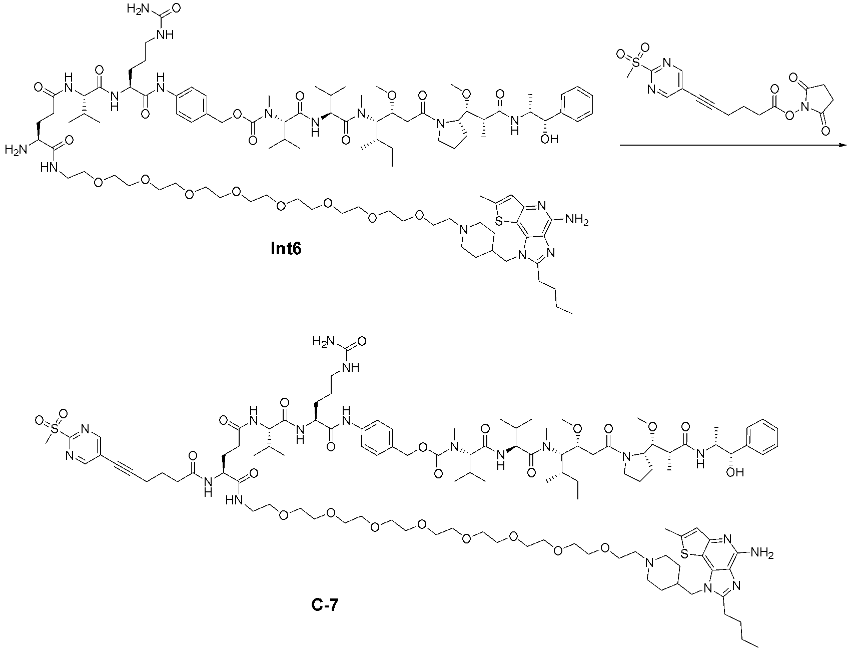

Compound **Int6** (47 mg, 23.66 µmol), 2,5-dioxopyrrolidin-1-yl 6-(2-(methylsulfonyl)pyrimidin-5-yl)hex-5-ynoate (12.99 mg, 35.49 µmol), and *N,N-*diisopropylethylamine (5.55 mg, 42.57 µmol) were added sequentially to *N,N*-dimethylformamide (0.5 mL), and reacted at room temperature for 1.5 hours. The reaction mixture was directly purified by reverse-phase column chromatography (Preparation method B, elution gradient: A% = 20% to 35%) to obtain the title compound of this step (37.38 mg, yield: 70.6%).

MS m/z (ESI): 1119.6 [M/2+H]⁺.

### Drug-linker Preparation Example 1-8: Preparation of Compound C-8

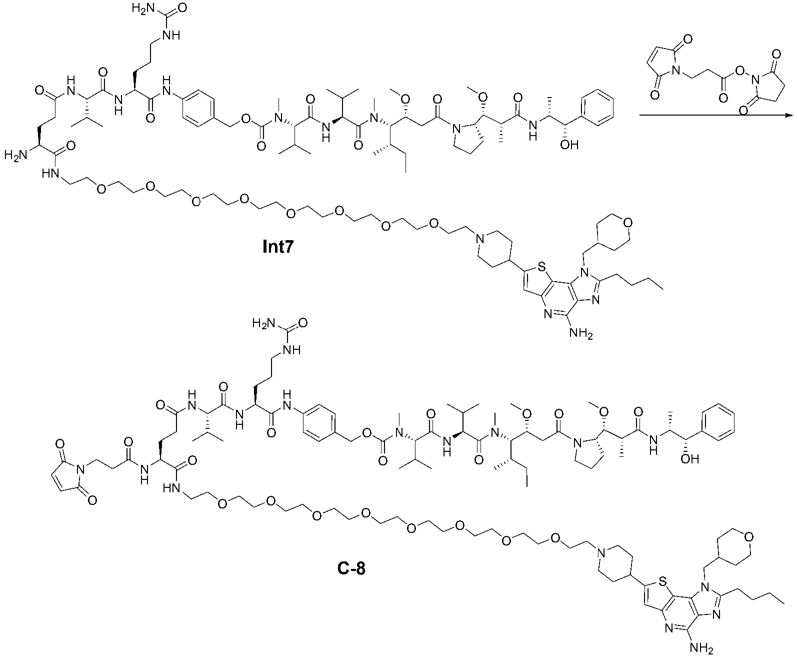

Compound **Int7** (50 mg, 24.31 µmol), 2,5-dioxopyrrolidin-1-yl 3-(2,5-dioxo-2,5-dihydro-1*H*-pyrrol-1-yl)propionate (7.76 mg, 29.16 µmol), and *N,N*-diisopropylethylamine (4.52 mg, 34.63 µmol) were added sequentially to anhydrous *N,N*-dimethylformamide (2 mL), and reacted at 0°C for 2 hours. The reaction mixture was purified by preparative HPLC to obtain the title compound of this step (21.5 mg, yield: 41.3%).

MS m/z (ESI): 1105.2 [M/2+H]⁺.

### Drug-linker Preparation Example 1-9: Preparation of Compound C-9

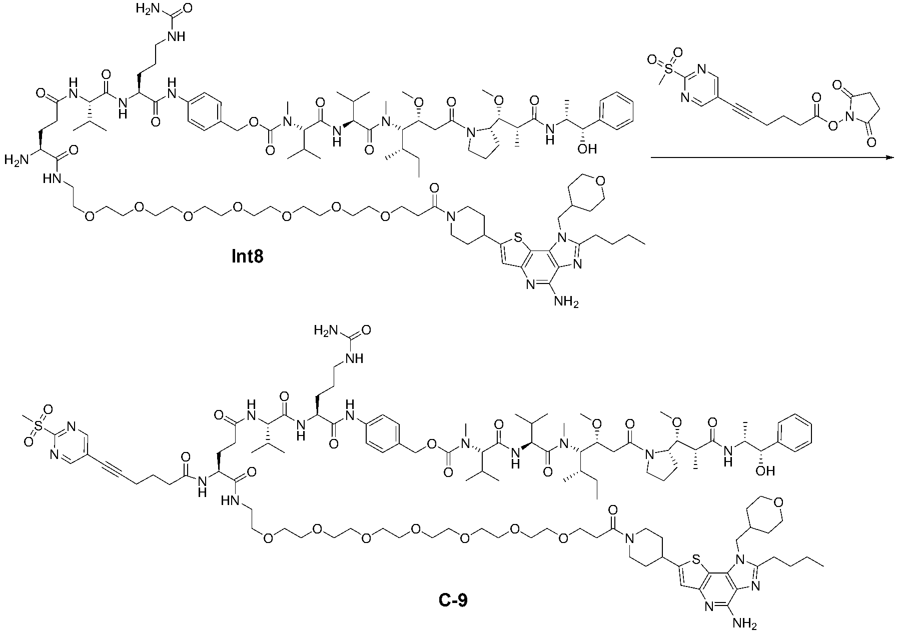

Compound **Int8** (113.67 mg, 55.68 µmol), 2,5-dioxopyrrolidin-1-yl 6-(2-(methylsulfonyl)pyrimidin-5-yl)hex-5-ynoate (22.27 mg, 60.95 µmol), and *N,N-*diisopropylethylamine (11.63 mg, 89.08 µmol) were added sequentially to anhydrous *N,N-*dimethylformamide (2 mL), and reacted at 0°C for 2 hours. The reaction mixture was purified by preparative HPLC to obtain the title compound of this step (55 mg, yield: 53.9%).

MS m/z (ESI): 1146.6 [M/2+H]⁺.

### Drug-linker Preparation Example 1-10: Preparation of Compound C-10

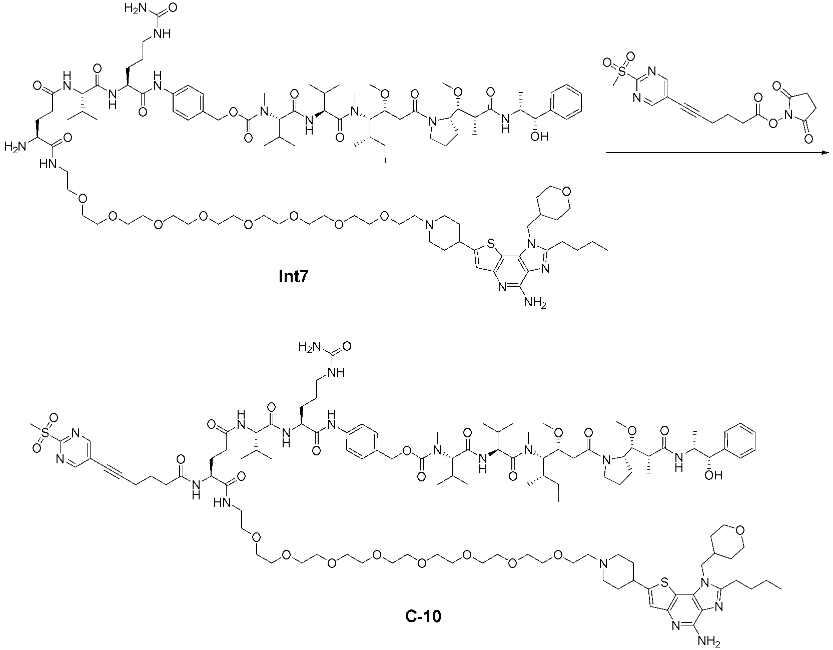

Compound **Int7** (70 mg, 30.62 µmol), 2,5-dioxopyrrolidin-1-yl 6-(2-(methylsulfonyl)pyrimidin-5-yl)hex-5-ynoate (18.64 mg, 45.93 µmol) and *N,N-*diisopropylethylamine (7.98 mg, 61.24 µmol) were added to anhydrous N,N-dimethylformamide (1 mL), and reacted at 25°C for 2 hours. The reaction mixture was purified by preparative HPLC to obtain the title compound of this step (42 mg, 55.4% yield).

MS m/z (ESI): 1154.7 [M/2+H]⁺.

### Drug-linker Preparation Example 1-11: Preparation of Compound C-11

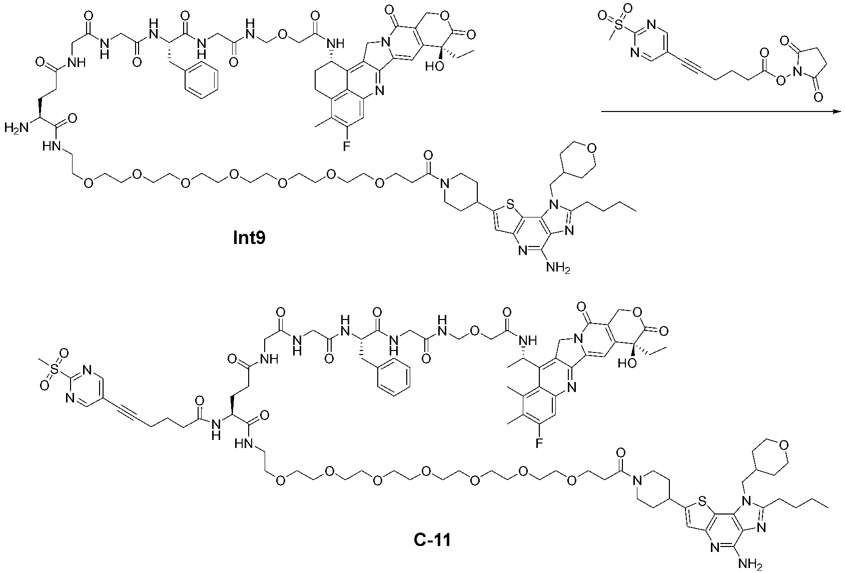

Compound **Int9** (90 mg, 51.17 µmol), 2,5-dioxopyrrolidin-1-yl 6-(2-(methylsulfonyl)pyrimidin-5-yl)hex-5-ynoate (23.02 mg, 63.01 µmol) and *N,N-*diisopropylethylamine (12.02 mg, 92.1 µmol) were added sequentially to anhydrous *N,N-*dimethylformamide (2 mL) and reacted at 0°C for 2 hours. The reaction mixture was purified by preparative HPLC to obtain the title compound of this step (27 mg, yield: 29.2%).

MS m/z (ESI): 1005.5 [M/2+H]⁺.

### Drug-linker Preparation Example 1-12: Preparation of Compound C-12

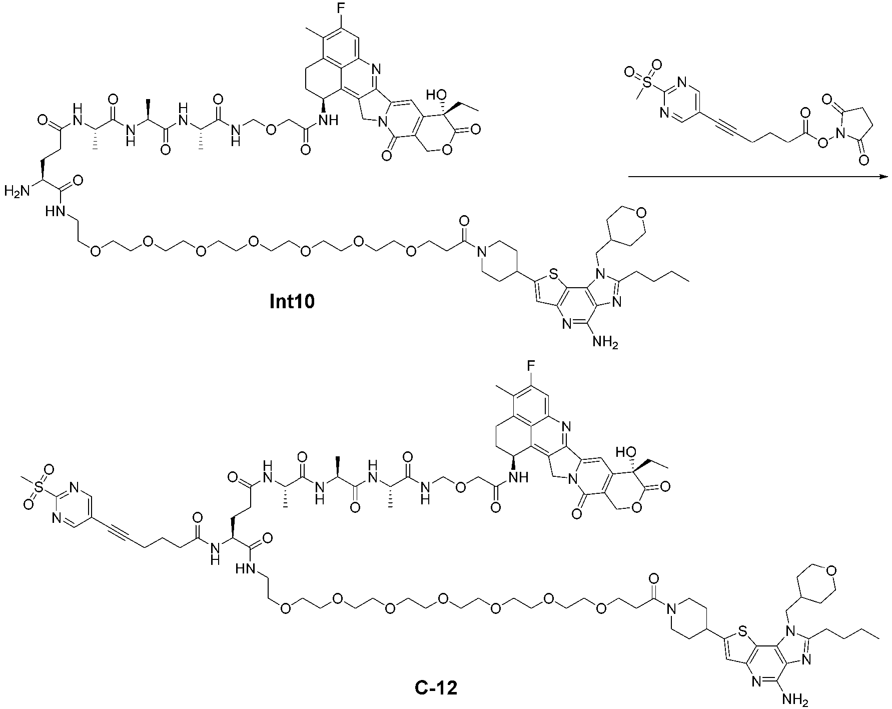

Compound **Int10** (70 mg, 42.33 µmol), 2,5-dioxopyrrolidin-1-yl 6-(2-(methylsulfonyl)pyrimidin-5-yl)hex-5-ynoate (20.1 mg, 55.02 µmol) and *N,N-*diisopropylethylamine (7.87 mg, 60.31 µmol) were added sequentially to anhydrous *N,N-*dimethylformamide (2 mL) and reacted at 0°C for 2 hours. The reaction mixture was purified by preparative HPLC to obtain the title compound of this step (2.53 mg, yield: 7.1%).

MS m/z (ESI): 1903.8 [M+H]⁺.

### Drug-linker Preparation Example 1-13: Preparation of Compound C-13

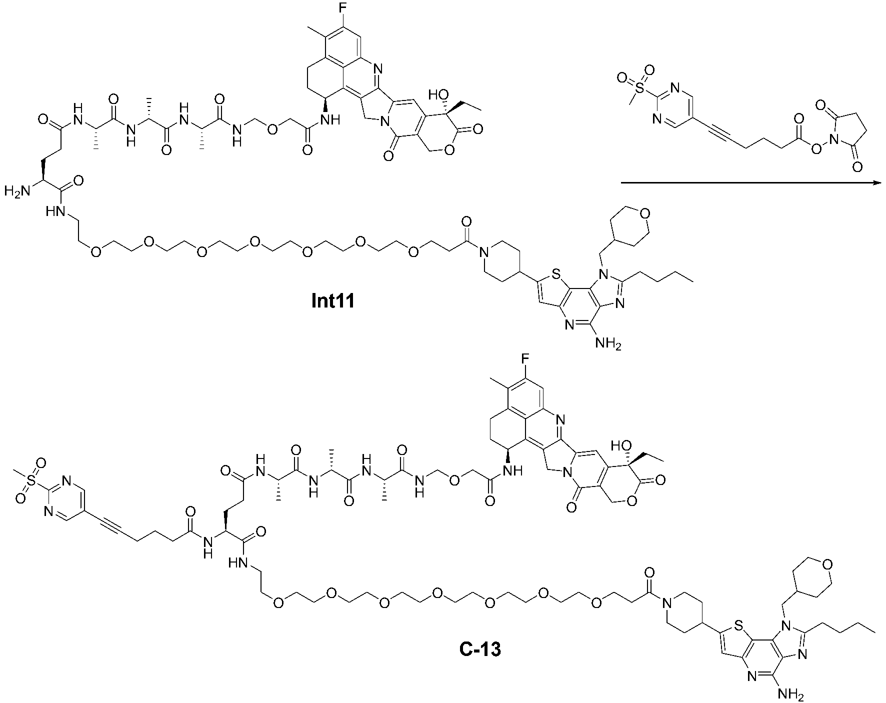

Compound **Int11** (30 mg, 18.14 µmol), 2,5-dioxopyrrolidin-1-yl 6-(2-(methylsulfonyl)pyrimidin-5-yl)hex-5-ynoate (8.62 mg, 23.58 µmol) and *N,N-*diisopropylethylamine (3.55 mg, 27.21 µmol) were added sequentially to anhydrous *N,N-*dimethylformamide (2 mL), and reacted at 0°C for 2 hours. The reaction mixture was purified by preparative HPLC to obtain the title compound of this step (11 mg, yield: 31.8%).

MS m/z (ESI): 952.4 [M/2+H]⁺.

### Drug-linker Preparation Example 1-14: Preparation of Compound C-14

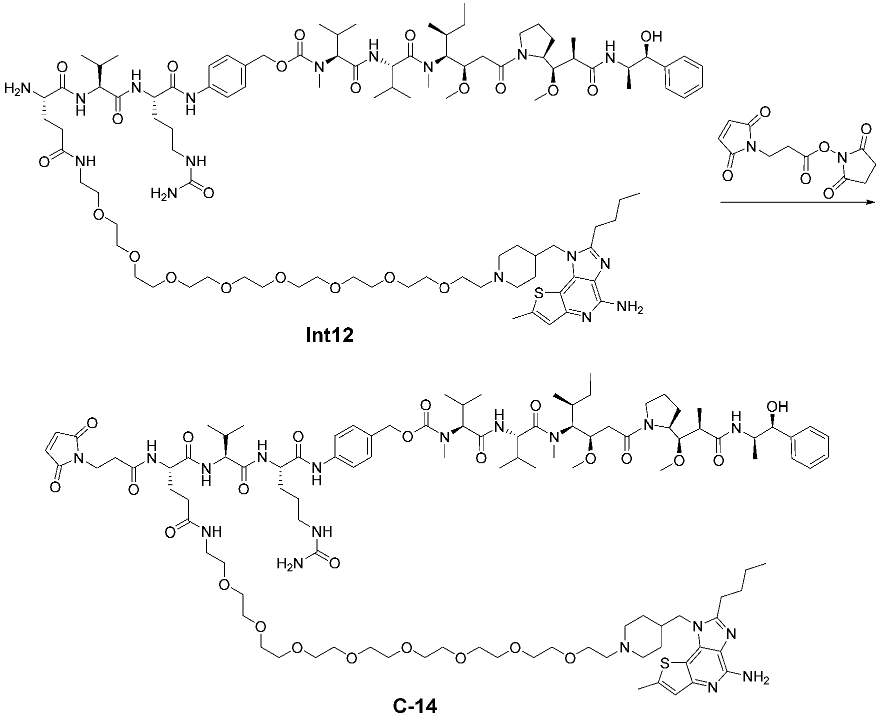

Compound **Int12** (25 mg, 12.58 µmol), 2,5-dioxopyrrolidin-1-yl 3-(2,5-dioxo-2,5-dihydro-1*H*-pyrrol-1-yl)propionate (10.05 mg, 37.74 µmol) and *N,N*-diisopropylethylamine (2.34 mg, 17.92 µmol) were added sequentially to anhydrous *N,N*-dimethylformamide (2 mL) and reacted at 0°C for 2 hours. The reaction mixture was purified by preparative HPLC to obtain the title compound of this step (8 mg, yield: 31.3%).

MS m/z (ESI): 1070.1 [M/2+H]⁺.

### Drug-linker Preparation Example 1-15: Preparation of Compound C-15

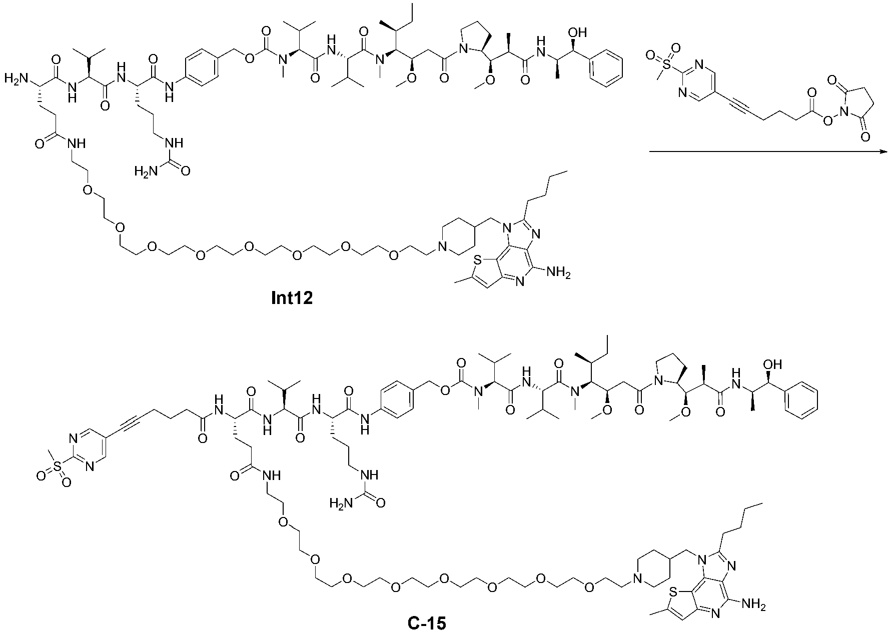

Compound **Int12** (28.80 mg, 14.49 µmol), 2,5-dioxopyrrolidin-1-yl 6-(2-(methylsulfonyl)pyrimidin-5-yl)hex-5-ynoate (5.88 mg, 14.49 µmol) and *N,N-*diisopropylethylamine (3.78 mg, 28.98 µmol) were added sequentially to *N,N*-dimethylformamide (0.5 mL) and reacted at room temperature for 2 hours. The reaction mixture was directly purified by reverse-phase column chromatography (Preparation method B, elution gradient: A% = 20% to 35%) to obtain the title compound of this step (13.57 mg, yield: 41.8%).

MS m/z (ESI): 1119.6 [M/2+H]⁺.

### Drug-linker Preparation Example 1-16: Preparation of Compound C-16

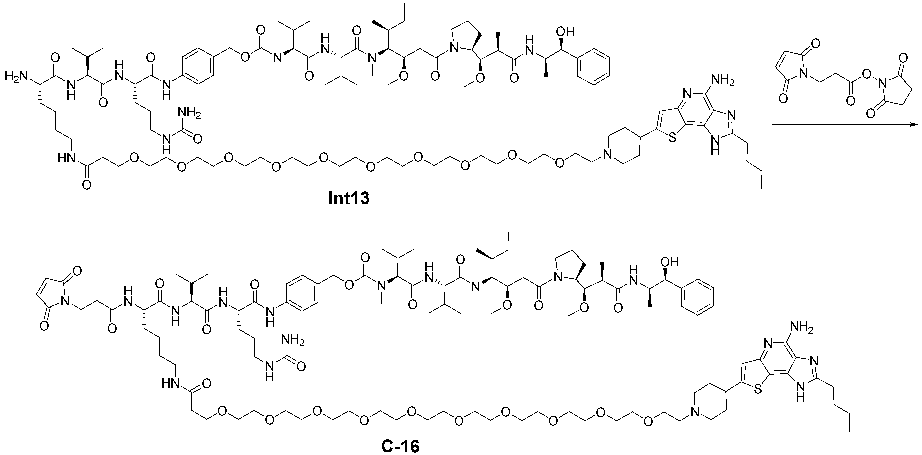

Compound **Int13** (30 mg, 13.01 µmol), 2,5-dioxopyrrolidin-1-yl 3-(2,5-dioxo-2,5-dihydro-1*H*-pyrrol-1-yl)propionate (5.25 mg, 19.51 µmol) and *N,N*-diisopropylethylamine (2.54 mg, 19.51 µmol) were added sequentially to *N,N*-dimethylformamide (1 mL) and reacted at room temperature for 1.5 hours. The reaction mixture was directly purified by reverse-phase column chromatography (Preparation method B, elution gradient: A% = 25% to 40%) to obtain the title compound of this step (9.81 mg, yield: 31.5%).

MS m/z (ESI): 1114.2 [M/2+H]⁺.

### Drug-linker Preparation Example 1-17: Preparation of Compound C-17

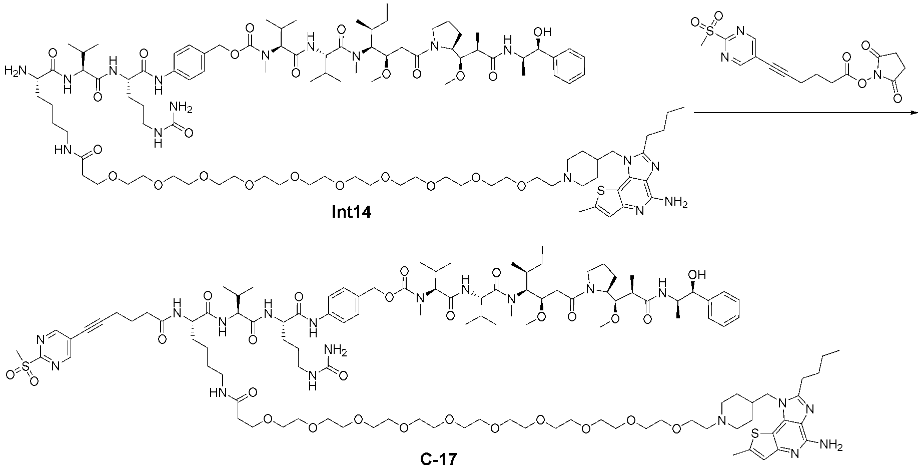

Compound **Int14** (20 mg, 9.50 µmol), 2,5-dioxopyrrolidin-1-yl 6-(2-(methylsulfonyl)pyrimidin-5-yl)hex-5-ynoate (5.56 mg, 15.21 µmol) and *N,N-*diisopropylethylamine (1.77 mg, 13.67 µmol) were added sequentially to anhydrous *N,N-*dimethylformamide (2 mL) and reacted at 0°C for 2 hours. The reaction mixture was purified by preparative HPLC to obtain the title compound of this step (6 mg, yield: 28.2%).

MS m/z (ESI): 1177.7 [M/2+H]⁺.

### Drug-linker Preparation Example 1-18: Preparation of Compound C-18

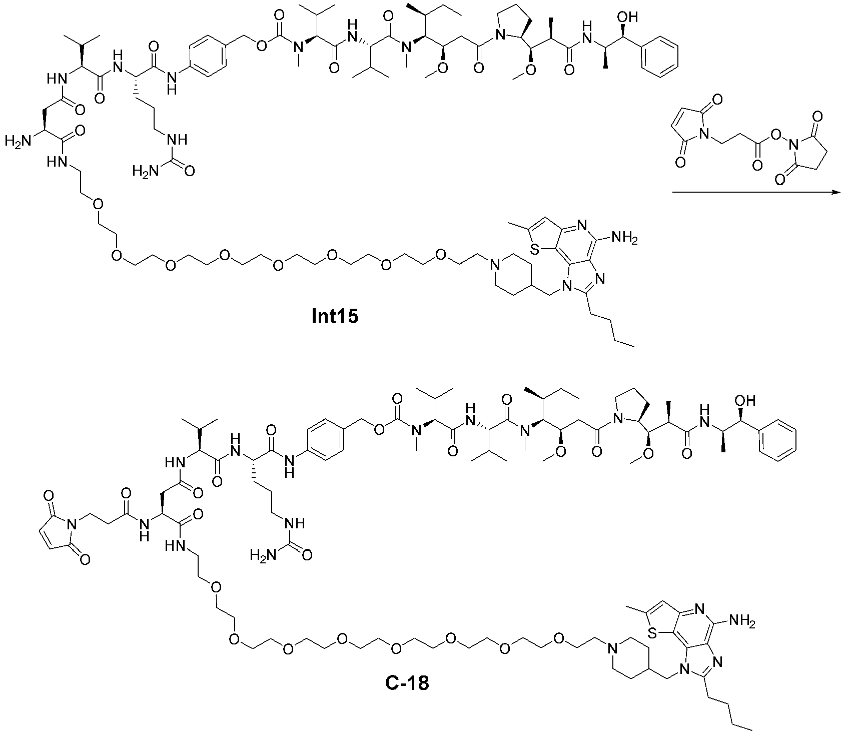

Compound **Int15** (28 mg, 14.19 µmol), 2,5-dioxopyrrolidin-1-yl 3-(2,5-dioxo-2,5-dihydro-1*H*-pyrrol-1-yl)propionate (5.66 mg, 21.28 µmol) and *N,N*-diisopropylethylamine (2.75 mg, 21.28 µmol) were added sequentially to *N,N*-dimethylformamide (0.5 mL) and reacted at room temperature for 2 hours. The reaction mixture was directly purified by reverse-phase column chromatography (Preparation method B, elution gradient: A% = 20% to 35%) to obtain the title compound of this step (7.64 mg, yield: 25.3%).

MS m/z (ESI): 1063.1 [M/2+H]⁺.

### Drug-linker Preparation Example 1-19: Preparation of Compound C-19

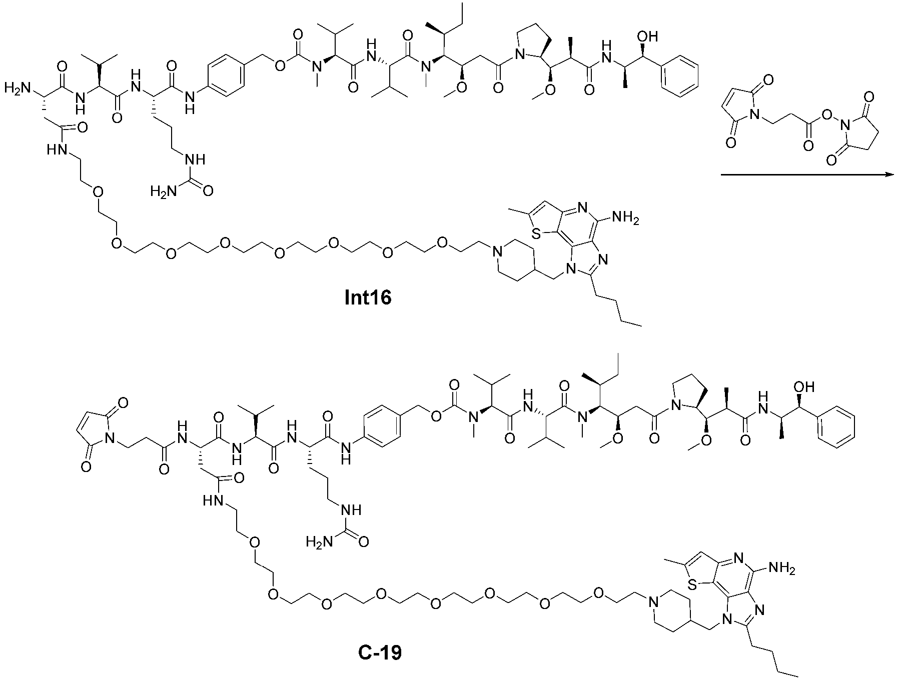

Compound **Int16** (23.15 mg, 11.73 µmol), 2,5-dioxopyrrolidin-1-yl 3-(2,5-dioxo-2,5-dihydro-1*H*-pyrrol-1-yl)propionate (6.25 mg, 23.46 µmol) and *N,N*-diisopropylethylamine (2.18 mg, 16.72 µmol) were dissolved in anhydrous *N,N*-dimethylformamide (2 mL) and reacted at 0°C for 2 hours. The reaction mixture was purified by preparative HPLC to obtain the title compound of this step (12 mg, yield: 50.7%).

MS m/z (ESI): 1063.1 [M/2+H]⁺.

### Drug-linker Preparation Example 1-20: Preparation of Compound C-20

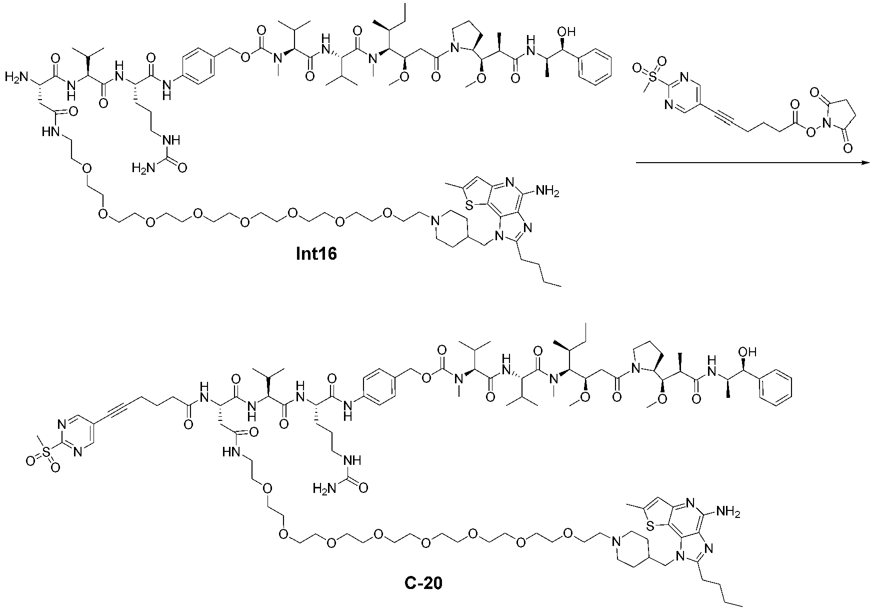

Compound **Int16** (24.7 mg, 12.52 µmol), 2,5-dioxopyrrolidin-1-yl 6-(2-(methylsulfonyl)pyrimidin-5-yl)hex-5-ynoate (5.08 mg, 12.52 µmol) and *N,N-*diisopropylethylamine (3.26 mg, 25.03 µmol) were added sequentially to *N,N*-dimethylformamide (1 mL) and reacted at room temperature for 2 hours. The reaction mixture was directly purified by reverse-phase column chromatography (Preparation method B, elution gradient: A% = 20% to 35%) to obtain the title compound of this step (17.16 mg, yield: 61.7%).

MS m/z (ESI): 1112.6 [M/2+H]⁺.

### Drug-linker Preparation Example 1-21: Preparation of Compound C-21

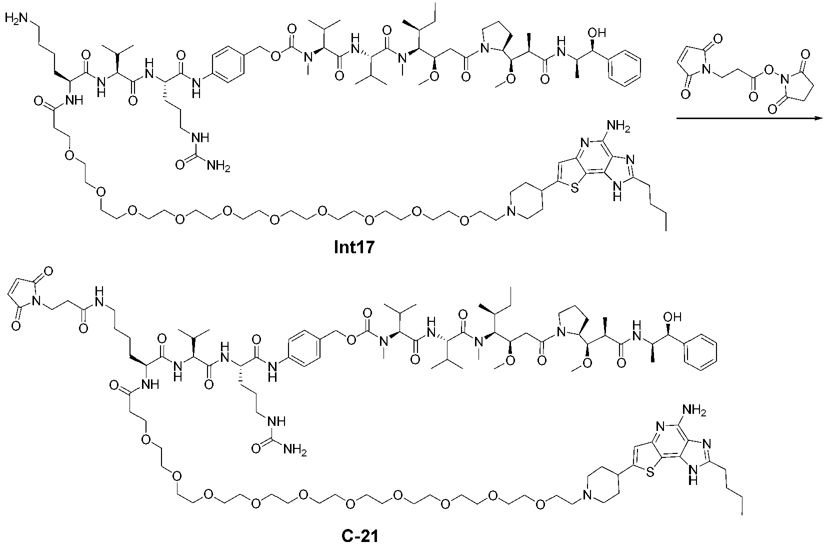

Compound **Int17** (25 mg, 12.05 µmol), 2,5-dioxopyrrolidin-1-yl 3-(2,5-dioxo-2,5-dihydro-1*H*-pyrrol-1-yl)propionate (4.37 mg, 16.26 µmol) and *N,N*-diisopropylethylamine (3.12 mg, 24.10 µmol) were added sequentially to *N,N*-dimethylformamide (1 mL) and reacted at room temperature for 2 hours. The reaction mixture was directly purified by reverse-phase column chromatography (Preparation method B, elution gradient: A% = 30% to 45%) to obtain the title compound of this step (11.1 mg, yield: 41.4%).

MS m/z (ESI): 1114.2 [M/2+H]⁺.

### Drug-linker Preparation Example 1-22: Preparation of Compound C-22

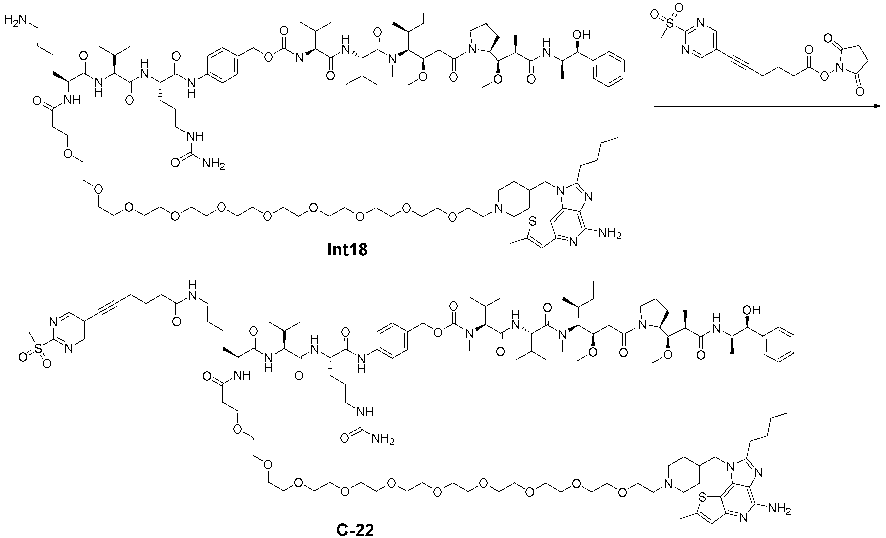

Compound **Int18** (20 mg, 9.50 µmol), 2,5-dioxopyrrolidin-1-yl 6-(2-(methylsulfonyl)pyrimidin-5-yl)hex-5-ynoate (5.56 mg, 15.21 µmol) and *N,N-*diisopropylethylamine (1.77 mg, 13.67 µmol) were added sequentially to anhydrous *N,N-*dimethylformamide (2 mL) and reacted at 0°C for 2 hours. The reaction mixture was purified by preparative HPLC to obtain the title compound of this step (8 mg, yield: 37.6%).

MS m/z (ESI): 1177.7 [M/2+H]⁺.

### Drug-linker Preparation Example 1-23: Preparation of Compound C-23

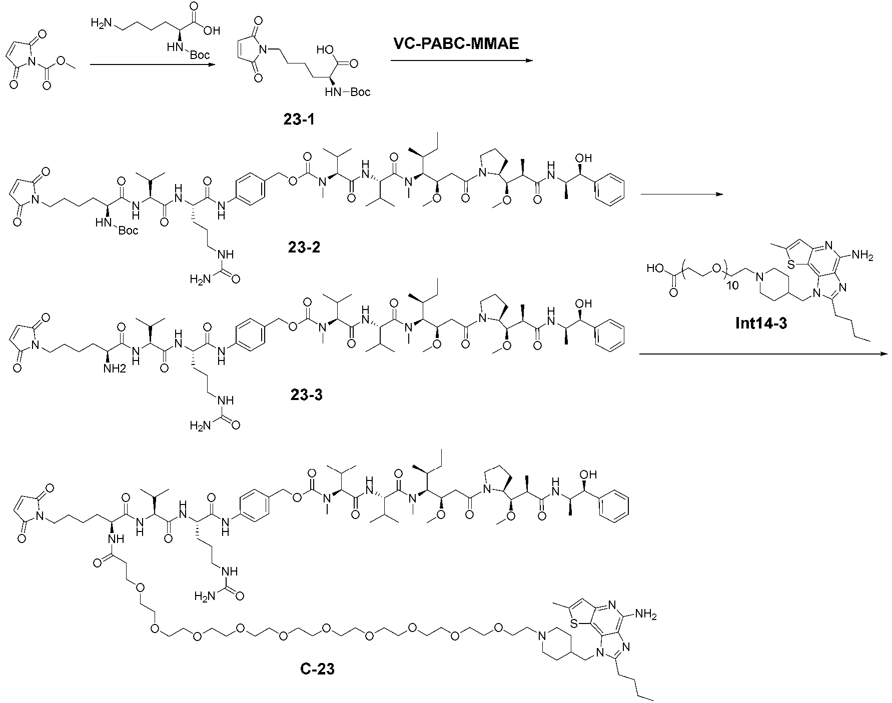

### Step 1: Preparation of Compound 23-1

(Tert-butoxycarbonyl)-*L*-lysine (500 mg, 1.93 mmol) was dissolved in saturated sodium bicarbonate aqueous soution (10 mL) at 0°C. Methyl 2,5-dioxo-2,5-dihydro-1*H*-pyrrole-1-carboxylate (314.87 mg, 1.93 mmol) was added, and reacted at 0°C for 40 minutes, and then at 25°C for 50 minutes. The reaction mixture was cooled to 0°C, adjusted with concentrated sulfuric acid to approximately pH 3.0, and extracted with ethyl acetate. The organic phases were combined and washed with brine. The resultant organic phase was concentrated to obtain a crude product, which was purified by silica gel column chromatography (eluent: dichloromethane/methanol = 5/1 (v/v)) to obtain the title compound of this step (320 mg, yield: 50.9%).

MS m/z (ESI): 349.1 [M+Na]+.

### Step 2: Preparation of Compound 23-2

Compound **23-1** (23.75 mg, 72.78 µmol), VC-PABC-MMAE (81.76 mg, 72.78 µmol), *N,N-*diisopropylethylamine (14.25 mg, 109.25 µmol) and 2-(7-azobenzotriazole)-*N,N,N,N-*tetramethyluronium hexafluorophosphate (39.13 mg, 101.89 µmol) were added sequentially to anhydrous *N,N*-dimethylformamide (3 mL), and reacted at 0°C for 2 hours. The reaction mixture was directly purified by reverse-phase column chromatography (Preparation method B, elution gradient: A% = 30% to 35%) to obtain the title compound of this step (80 mg, yield: 72.9%).

MS m/z (ESI): 1431.8 [M+H]⁺.

### Step 3: Preparation of Compound 23-3

Compound **23-2** (56 mg, 39.11 µmol) was dissolved in anhydrous dichloromethane (3 mL). Then zinc bromide (71.18 mg, 2312.9 µmol) was added, and reacted at 25°C for 2 hours. The reaction mixture was concentrated, and the resultant residue was dissolved in methanol and purified by reverse-phase column chromatography (Preparation method B, elution gradient: A% = 30% to 35%) to obtain the title compound of this step (28.5 mg, yield: 54.7%).

MS m/z (ESI): 1331.7 [M+H]⁺.

### Step 4: Preparation of Compound C-23

Compound **Int14-3** (9.31 mg, 10.7 µmol), Compound **23-3** (14.25 mg, 10.7 µmol), *N,N-*diisopropylethylamine (2.1 mg, 16.05 µmol), and 2-(7-azobenzotriazole)-N,N,N,N-tetramethyluronium hexafluorophosphate (5.34 mg, 13.91 µmol) were added sequentially to anhydrous *N,N*-dimethylformamide (2 mL), and reacted at 0°C for 2 hours. The reaction mixture was directly purified by reverse-phase column chromatography (Preparation method B, elution gradient: A% = 20% to 30%) to obtain the title compound of this step (15 mg, yield: 64.2%).

MS m/z (ESI): 1092.7 [M/2+H]⁺.

### Drug-linker Preparation Example 1-24: Preparation of Compound C-24

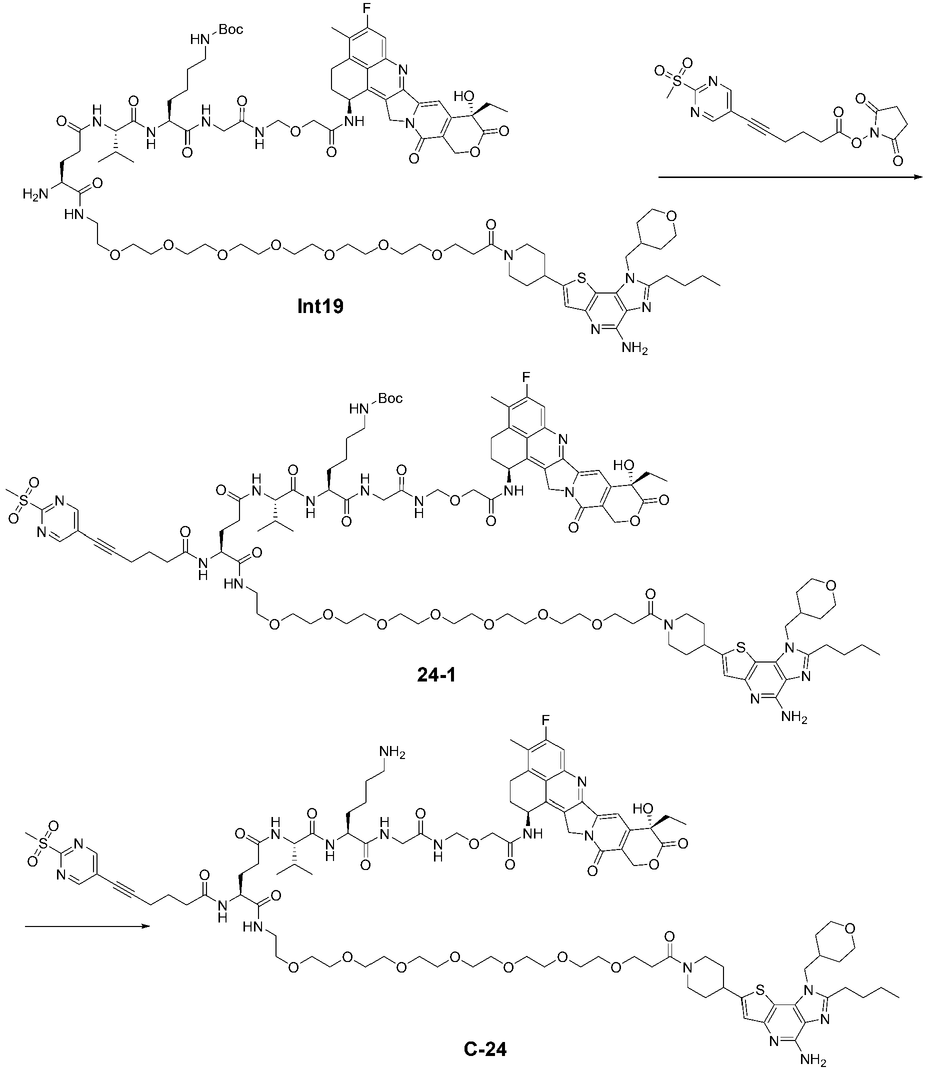

### Step 1: Preparation of Compound 24-1

Compound **Int19** (28 mg, 15.34 µmol), 2,5-dioxopyrrolidin-1-yl 6-(2-(methylsulfonyl)pyrimidin-5-yl)hex-5-ynoate (8.41 mg, 23.01 µmol) and *N,N-*diisopropylethylamine (4.00 mg, 30.68 µmol) were added sequentially to *N,N*-dimethylformamide (1 mL), and reacted at room temperature for 2 hours. The reaction mixture was directly purified by reverse-phase column chromatography (Preparation method A, elution gradient: A% = 35% to 40%) to obtain the title compound of this step (30 mg, yield: 94.2%).

MS m/z (ESI): 1038.5 [M/2+H]⁺.

### Step 2: Preparation of Compound C-24

Compound **24-1** (30 mg, 13.01 µmol) and anhydrous zinc bromide (11.72 mg, 52.04 µmol) were added to anhydrous dichloromethane (2 mL) and reacted at room temperature for 2 hours. The reaction mixture was directly purified by reverse-phase column chromatography (Preparation method A, elution gradient: A% = 25% to 35%) to obtain the title compound of this step (4.65 mg, yield: 16.8%).

MS m/z (ESI): 1975.9 [M+H]⁺.

### Drug-linker Preparation Example 1-25: Preparation of Compound C-25

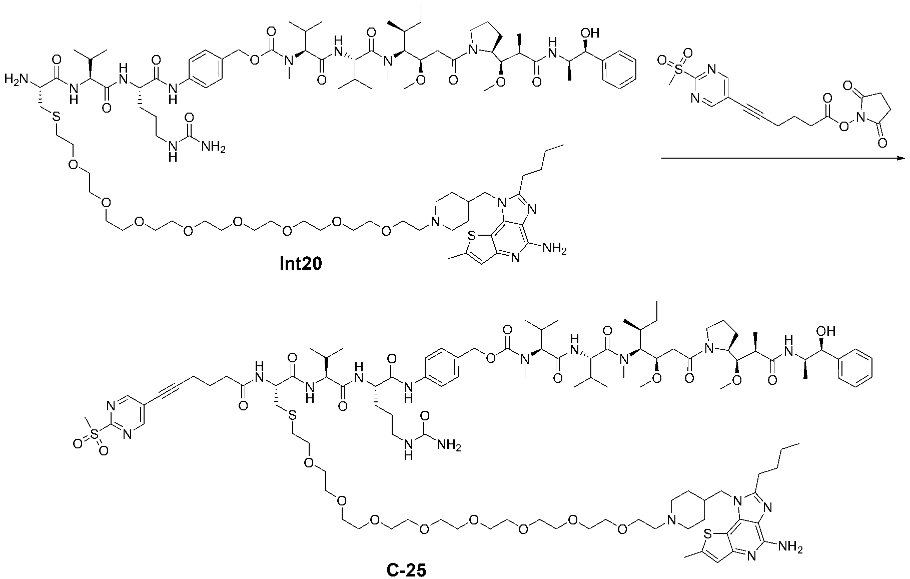

Compound **Int20** (30 mg, 15.28 µmol), 2,5-dioxopyrrolidin-1-yl 6-(2-(methylsulfonyl)pyrimidin-5-yl)hex-5-ynoate (8.94 mg, 24.46 µmol) and *N,N-*diisopropylethylamine (2.84 mg, 21.75 µmol, 3.64 µL) were dissolved in anhydrous *N,N-*dimethylformamide (2 mL) and reacted at 0°C for 2 hours. The reaction mixture was purified by preparative HPLC to obtain the title compound of this step (20 mg, yield: 62.2%).

MS m/z (ESI): 1107.1 [M/2+H]⁺.

### II. Preparation of immune-stimulating antibody-drug conjugate (iADC)

The B7H3 monoclonal antibody (Ab-01) stock solution was adjusted to the target pH using 1 M sodium hydrogen phosphate solution. 0.1 M EDTA and 10 mM TCEP were added to the resulting B7H3 monoclonal antibody (Ab-01) buffer solution, shaken, and allowed to react at room temperature for 2 hours. Then, 10 mM DMSO solutions of drug-linkers **C-1** to **C-25** were added, respectively, shaken, and allowed to react at room temperature for 2 hours. The reaction mixtures were then purified using NAP-5 gel column and washed with histidine hydrochloride buffer at pH 6.0 to 6.1. The resultant filtrates were collected to obtain the immune-stimulating antibody-drug conjugates (iADCs) **iADC-1** to **iADC-25** in histidine hydrochloride buffer, which were stored at -20°C.

### III. Determination of drug/antibody ratio of immune-stimulating antibody-drug conjugate (iADC): DAR value

The molecular weights of the iADCs were determined by LC-MS, and the drug/antibody ratio (DAR) values were calculated.

### Chromatographic conditions:

Liquid chromatography column: Thermo MAbPac RP 3.0*100 mm;
Mobile phase A: 0.1% FA/H₂O; Mobile phase B: 0.1% FA/ACN;
Flow rate: 0.25 ml/min; Sample chamber temperature: 8°C; Column temperature: 60°C; Injection volume: 1 µL;

| Time (min) | 2 | 20 | 22 | 25 | 26 | 30 |
|---|---|---|---|---|---|---|
| Mobile phase A (volume %) | 75 | 60 | 5 | 5 | 75 | 75 |
| Mobile phase B (volume %) | 25 | 40 | 95 | 95 | 25 | 25 |

### Mass spectrometry conditions:

Mass spectrometer model: AB Sciex Triple TOF 5600+;
GS1 35; GS2 35; CUR 30; TEM 350; ISVF 5500; DP 250; CE 10; Accumulation time: 0.5 s; m/z 600-4000; Time bins to sum 40.

The DAR values of immune-stimulating antibody-drug conjugates (iADCs) were calculated using CE-SDS, as shown in Table 1:

**Table 1. Average DAR values of immune-stimulating antibody-drug conjugates (iADCs)**

| iADC name | drug-linker number | Conjugation pH value | TCEP equivalents | Drug-linker equivalents | Average DAR value |
|---|---|---|---|---|---|
| **iADC-1** | **C-1** | 7.49 | 5.5 | 10 | 2.55 |
| **iADC-2** | **C-2** | 7.69 | 2.5 | 10 | 2.39 |
| **iADC-3** | **C-3** | 6.51 | 2.5 | 5 | 3.87 |
| **iADC-4** | **C-4** | 6.51 | 2.5 | 5.5 | 3.62 |
| **iADC-5** | **C-5** | 6.51 | 2.5 | 5.5 | 3.32 |
| **iADC-6** | **C-6** | 6.51 | 2.5 | 5.5 | 2.97 |
| **iADC-7** | **C-7** | 6.55 | 2.5 | 5.5 | 2.94 |
| **iADC-8** | **C-8** | 6.50 | 2.5 | 5.5 | 3.15 |
| **iADC-9** | **C-9** | 7.67 | 3 | 6 | 4.74 |
| **iADC-10** | **C-10** | 7.67 | 1.5 | 3 | 1.97 |
| **iADC-11** | **C-11** | 7.66 | 2.5 | 5.5 | 3.48 |
| **iADC-12** | **C-12** | 7.66 | 2.5 | 5.5 | 1.64 |
| **iADC-13** | **C-13** | 7.60 | 2.5 | 5.5 | 3.68 |
| **iADC-14** | **C-14** | 6.54 | 2.5 | 5 | 2.07 |
| **iADC-15** | **C-15** | 6.55 | 2.5 | 5.5 | 3.11 |
| **iADC-16** | **C-16** | 6.58 | 2.5 | 5.5 | 2.94 |
| **iADC-17** | **C-17** | 6.55 | 2.5 | 5.5 | 2.48 |
| **iADC-18** | **C-18** | 6.55 | 2.5 | 5.5 | 2.30 |
| **iADC-19** | **C-19** | 6.49 | 2.5 | 6 | 3.29 |
| **iADC-20** | **C-20** | 6.55 | 2.5 | 5.5 | 2.89 |
| **iADC-21** | **C-21** | 6.58 | 2.5 | 6 | 2.92 |
| **iADC-22** | **C-22** | 6.55 | 2.5 | 5.5 | 2.88 |
| **iADC-23** | **C-23** | 6.55 | 2.5 | 5.5 | 3.78 |
| **iADC-24** | **C-24** | 7.60 | 2.5 | 5.5 | 1.14 |
| **iADC-25** | **C-25** | 6.55 | 2.5 | 5.5 | 3.38 |

### Biological Assays

Experimental Example 1: Inhibitory effect of immunomodulatory antibody-drug conjugate (iADC) on proliferation of B7H3-positive tumor cells

### Experimental steps:

1. B7H3-positive tumor cells (HCC1954 (Connotech) and NCI-N87 (ATCC)) in the logarithmic growth phase were harvested and counted.
2. The two tumor cells were seeded into 96-well plates (Coming) at a density of 2000 cells (HCC1954) or 3000 cells (NCI-N87) per well in a volume of 80 µL, and then incubated overnight in a 37°C, 5% CO₂ incubator to allow cell attachment (18 to 24 hours).
3. 5× Drug working solutions with gradient concentrations were prepared according to the experimental design. The 96-well plates were taken out, and 20 µL of the working solution was transferred from the compound plate to the cell plates. The cell plates were incubated in a 37°C, 5% CO₂ incubator for 72 hours.
4. Plate detection: The cell plates were taken out and observed for cell state under a microscope. After the observation, a detection solution was added to the cell plates according to the CellCounting-Lite^{®} 2.0 Kit (Novozymes) instructions, and the raw data were obtained using PHERA Star FS.
5. Calculation of inhibition rate: Inhibition rate (IR%) = 1 - (mean luminescence value of experimental wells / mean luminescence value of DMSO control wells) * 100%. Graphpad Prism 8.0 software was used for data analysis and graphing. IC₅₀ values were calculated by fourparameter curve fitting the logarithms of cell viability and drug concentration.

### Experimental results:

The inhibitory effect of each immune-stimulating antibody-drug conjugate (iADC) on tumor cell proliferation was determined using the aforementioned method, as shown in Tables 2 and 3.

**Table 2. Inhibitory effects of immune-stimulating antibody-drug conjugates (iADCs) on proliferation of HCC1954 tumor cells**

| iADC name | IC₅₀ (HCC954, nM) |
|---|---|
| **iADC-1** | 0.011 |
| **iADC-4** | 0.017 |
| **iADC-5** | 0.040 |
| **iADC-6** | 0.020 |
| **iADC-8** | 0.077 |
| **iADC-10** | 0.009 |
| **iADC-14** | 0.510 |
| **iADC-16** | 0.046 |
| **iADC-18** | 0.119 |
| **iADC-19** | 0.054 |
| **iADC-21** | 0.090 |
| **iADC-23** | 0.187 |
| **iADC-25** | 0.080 |

**Table 3. Inhibitory effects of immune-stimulating antibody-drug conjugates (iADCs) on Proliferation of NCI-N87 tumor cells**

| iADC name | IC₅₀ (NCI-N87, nM) |
|---|---|
| **iADC-1** | 3.07 |
| **iADC-4** | 7.27 |
| **iADC-5** | 2.24 |
| **iADC-6** | 3.44 |
| **iADC-8** | 2.22 |
| **iADC-10** | 7.00 |
| **iADC-16** | 1.04 |
| **iADC-18** | 1.49 |
| **iADC-19** | 1.14 |
| **iADC-23** | 1.17 |
| **iADC-25** | 1.31 |

The results showed that the iADCs exhibited potent inhibitory effects on the proliferation of HCC1954 cells and NCI-N87 cells.

Experimental Example 2: Inhibitory effect of immunomodulatory antibody-drug conjugate (iADC) on proliferation of B7H3-positive Calu-6 tumor cells with low HER2 expression

### Experimental steps:

1. B7H3-positive Calu-6 tumor cells with low HER2 expression (Nanjing Cobioer) in the logarithmic growth phase were harvested.
2. The tumor cells were seeded in a 96-well plate (Coming) at a density of 8,000 cells per well in a volume of 80 µL, and then incubated overnight in a 37°C, 5% CO₂ incubator to allow cell attachment (18 to 24 hours).
3. 5× drug working solutions with gradient concentrations were prepared according to the experimental design. The 96-well cell plate was taken out, 20 µL of the working solution was transferred from the compound plate to the cell plate. The cell plate was incubated in a 37°C, 5% CO₂ incubator for 72 hours.
4. Plate detection: The cell plate was taken out and observed for cell state under a microscope. After the observation, the detection solution was added to the cell plate according to the CellCounting-Lite^{®} 2.0 Kit (Novozymes) instructions, and the raw data were obtained using PHERA Star FS.

Calculation of inhibition rate: Inhibition rate (IR%) = 1 - (mean luminescence value of experimental wells / mean luminescence value of DMSO control wells) * 100%. Data analysis and graphing were performed using Graphpad Prism 8.0 software. IC₅₀ values were calculated by fourparameter curve fitting the logarithms of inhibition rate and drug concentration.

### Experimental results:

The inhibitory effect of each immune-stimulating antibody-drug conjugate (iADC) on the proliferation of Calu-6 tumor cells was determined using the aforementioned method. The results are shown in Table 4:

**Table 4. Inhibitory effects of immune-stimulating antibody-drug conjugates (iADCs) on proliferation of Calu-6 tumor cells**

| iADC name | IC₅₀ (Calu-6, nM) |
|---|---|
| **iADC-10** | 0.0100 |
| **iADC-19** | 0.0002 |
| **iADC-23** | 0.0040 |
| **iADC-25** | 0.0020 |

The results demonstrated that the iADCs exhibited potent inhibitory effects on proliferation of Calu-6 cells.

Experimental Example 3: Determination of ability of immunomodulatory antibody-drug conjugate (iADC) to stimulate TNF-α secretion from peripheral blood mononuclear cells (PBMCs) in the presence of tumor cells

### Experimental steps:

1. HCC1954 tumor cells (Connotech) in the logarithmic growth phase were harvested, washed twice with PBS, resuspended in RPMI1640+10% heat-inactivated FBS complete medium, and adjusted to a cell density of 2×10⁵ cells/mL. The HCC1954 tumor cells were seeded in a 96-well plate (Corning), 1×10⁴ cells per well.
2. PBMCs (SailyBio) were resuscitated, resuspended in RPMI1640+10% heat-inactivated FBS complete medium, and adjusted to a density of 6×10⁵ cells/mL. The cells were then added into the 96-well plate seeded with tumor cells, 3×10⁴ PBMCs per well.
3. 100 µL of the test drug at different concentrations was added to the plate, resulting in final concentrations of 500 nM, 166.7 nM, 55.6 nM, 18.52 nM, 6.17 nM, 2.06 nM, and 0.69 nM, respectively. 100 µL of RPMI1640+10% heat-inactivated FBS complete medium was added to blank wells.
4. The plate was incubated in a 5% CO₂, 37°C incubator. After incubation for 24 hours, 50 µL of supernatant was taken from each well for assay. According to the human TNF-α ELISA kit (Invitrogen) procedures, the primary antibody and HRP-labeled secondary antibody were incubated. After color development, absorbance was measured at 450 nm. TNF-α concentration was calculated according to the standard curve using Graphpad Prism 8.0 software.

### Experimental results:

The ability of each immune-stimulating antibody-drug conjugate (iADC) to stimulate TNF-α secretion from human peripheral blood mononuclear cells (PBMCs) in the presence of HCC1954 tumor cells was determined using the aforementioned method. The results are shown in Table 5:

**Table 5. Results of TNF-α secretion stimulated by immune-stimulating antibody-drug conjugates (iADCs)**

| iADC name | EC₅₀ (nM) |
|---|---|
| **iADC-4** | 8.19 |
| **iADC-5** | 10.79 |
| **iADC-6** | 42.34 |
| **iADC-14** | 6.38 |
| **iADC-16** | 10.25 |
| **iADC-19** | 6.42 |
| **iADC-21** | 47.51 |
| **iADC-25** | 75.30 |

The results demonstrated that all the iADCs could effectively stimulated TNF-α secretion from PBMCs in vitro when HCC1954 tumor cells and PBMCs were co-incubated. Other iADCs in the present application also exhibited similar abilities to stimulate PBMCs to secrete TNF-α. For example, **iADC-1, iADC-8, iADC-10, iADC-11, iADC-13, iADC-18, iADC-23,** and **iADC-24** all had EC₅₀ values less than 600 nM.

### Experimental Example 4: In vivo efficacy study of immune-stimulating antibody-drug conjugate (iADC) in CT26-hB7H3 subcutaneous xenograft tumor model

### Experimental steps:

1. Each Balb/c mouse (Chengdu GemPharmatech) was subcutaneously inoculated with 3×10⁵ CT26-hB7H3 cells (suspended in 0.1 ml PBS) in the right axilla.
2. When the mean tumor volume reached approximately 100 mm³, mice with irregular, excessively small, or excessively large tumor were excluded. The remaining mice were randomly divided into groups based on tumor volume and body weight, and were separately administered 0.9% sodium chloride injection (vehicle control), **iADC-8** (20 mg/kg, once per week, for a total of two doses), **iADC-9** (20 mg/kg, single dose), or **iADC-10** (23.81 mg/kg, single dose) via tail vein (i.v.) injection. The efficacy of the test compounds in this tumor-bearing mouse model and the animals' tolerance to the test compounds were observed.
3. During the experiment, the mice were weighed and tumor volumes were measured twice weekly, and the data were recorded.
4. Data statistics: Tumor volume (V) was calculated using the formula: V = 1/2 × a × b², wherein a and b represent length and width, respectively. Antitumor drug efficacy was evaluated using tumor growth inhibition rate (TGI) (%), which was calculated using the formula: TGI (%) _{(tumor volume)} = [1 - (Tvt - T_{V0})/(C_{Vt} - C_{V0})] × 100%, wherein, T_{V0} represents the mean tumor volume of the test compound group at the time of grouping and administration; T_{Vt} represents the mean tumor volume of the test compound group t days after administration; C_{V0} represents the mean tumor volume of the vehicle group at the time of grouping and administration; C_{Vt} represents the mean tumor volume of the vehicle group t days after administration. When tumor regression occurred, TGI (%) _{(tumor volume)} = 100% - (T_{Vt} - T_{V0}) / T_{V0} × 100%. If the tumor was smaller than its initial volume (i.e., Vₜ < V₀), it was defined as partial tumor regression (PR); if the tumor completely disappeared, it was defined as complete tumor regression (CR).

### Experimental results:

After administration, all test compounds significantly inhibited tumor growth. Compared with the vehicle control group, the TGI of **iADC-8** at 17 days post-administration was 60.72% (P < 0.05); the TGI of **iADC-9** at 18 days post-administration was 145.74% (P < 0.001); and the TGI of **iADC-10** at 18 days post-administration was 98.02% (P < 0.01) (Table 6). These results demonstrated that **iADC-8, iADC-9,** and **iADC-10** exhibited significant tumor inhibition effect in the CT26-hB7H3 subcutaneous xenograft tumor model. Furthermore, no deaths occurred, and no significant body weight loss was observed in any of the test groups (Table 7), indicating that all test compounds were well tolerated by the mice.

**Table 6. Efficacy of immune-stimulating antibody-drug conjugates in CT26-hB7H3 cell subcutaneous xenograft tumors**

| iADC name | Tumor growth inhibition rate (TGI) % | | | | Tumor regression |
|---|---|---|---|---|---|
| | Day 7 | Day 14 | Day 17 | Day 18 | |
| **iADC-8** | 66.77% | 63.61% | 60.72% | / | 2/7 PR |
| **iADC-9** | 137.78% | 155.52% | / | 145.74% | 5/6 PR |
| **iADC-10** | 106.58% | 109.42% | / | 98.02% | 4/6 PR |

| | | | | | |
|---|---|---|---|---|---|
| PR: partial regression. | | | | | |

**Table 7. Changes in mouse body weight in in vivo efficacy study of CT26-hB7H3 cell subcutaneous xenograft tumors**

| iADC name | Change rate of body weight in mice | | | |
|---|---|---|---|---|
| | Day 7 | Day 14 | Day 17 | Day 18 |
| **iADC-8** | + 0.73% | + 5.29% | + 8.58% | / |
| **iADC-10** | + 0.08% | + 2.64% | / | + 5.47% |

| | | | | |
|---|---|---|---|---|
| +: body weight gain; -: body weight loss. | | | | |

### Experimental Example 5: In vivo efficacy study of immune-stimulating antibody-drug conjugate (iADC) in MC38-hB7H3 cell subcutaneous xenograft model.

### Experimental steps:

1. Each C57BL/6J mouse (Chengdu GemPharmatech) was subcutaneously inoculated with 1×10⁶ MC38-hB7H3 cells (suspended in 0.1 ml PBS) in the right axilla.
2. When the mean tumor volume reached approximately 100 mm³, mice with irregular, excessively small, or excessively large tumor were excluded. The remaining mice were randomly divided into groups based on tumor volume and body weight and were administered 0.9% sodium chloride injection (vehicle control), **iADC-11** (10 mg/kg, single dose), or **iADC-12** (21.2 mg/kg, single dose) via tail vein (i.v.) injection. The efficacy of the test compounds in this tumor-bearing mouse model and the animals' tolerance to the test compounds were observed.
3. During the experiment, the mice were weighed and tumor volumes were measured twice weekly, and the data were recorded.
4. Data statistics: Tumor volume (V) was calculated using the formula: V = 1/2 × a × b², wherein a and b represent length and width, respectively. Antitumor drug efficacy was evaluated using tumor growth inhibition rate (TGI) (%), which was calculated using the formula: TGI (%) _{(tumor volume)} = [1 - (T_{Vt} - T_{V0})/(C_{Vt} - C_{V0})] × 100%, wherein, T_{V0} represents the mean tumor volume of the test compound group at the time of grouping and administration; T_{Vt} represents the mean tumor volume of the test compound group t days after administration; C_{V0} represents the mean tumor volume of the vehicle group at the time of grouping and administration; C_{Vt} represents the mean tumor volume of the vehicle group t days after administration. When tumor regression occurred, TGI (%) _{(tumor volume)} = 100% - (Tvt - T_{V0}) / T_{V0} × 100%. If the tumor was smaller than its starting volume (i.e., Vₜ < V₀), it was defined as partial tumor regression (PR); if the tumor completely disappeared, it was defined as complete tumor regression (CR).

### Experimental results:

After administration, both **iADC-11** and **iADC-12** significantly inhibited tumor growth. Compared with the vehicle control group, the TGI values of **iADC-11** and **iADC-12** at 32 days post-administration were 94.33% and 60.18%, respectively (P < 0.01) (Table 8). These results demonstrated that **iADC-11** and **iADC-12** exhibited antitumor effects in the MC38-hB7H3 cell subcutaneous xenograft tumor model. Furthermore, no mice deaths occurred in any of the experimental groups, and no significant body weight loss was observed (Table 9), indicating that all test compounds were well tolerated by the mice.

**Table 8. Efficacy of immune-stimulating antibody-drug conjugates in MC38-hB7H3 cell subcutaneous xenograft tumors**

| Name of iADC | Tumor growth inhibition rate (TGI) % | | | Tumor regression |
|---|---|---|---|---|
| | Day 7 | Day 18 | Day 32 | |
| iADC-11 | 113.74% | 115.99% | 94.33% | 2/6 CR+2/6 PR |
| iADC-12 | 76.52% | 38.78% | 60.18% | 1/6 PR |

| | | | | |
|---|---|---|---|---|
| PR: partial regression; CR: complete regression. | | | | |

**Table 9. Changes in mouse body weight in in vivo efficacy study of CT26-hB7H3 cell subcutaneous xenograft tumors**

| Name of iADC | Change rate of body weight in mice | | |
|---|---|---|---|
| | Day 7 | Day 18 | Day 32 |
| iADC-11 | + 2.67% | + 5.47% | + 9.43% |
| iADC-12 | + 2.82% | + 7.59% | + 11.89% |

| | | | |
|---|---|---|---|
| +: body weight gain; -: body weight loss. | | | |

In addition to those described herein, various modifications of the present invention will be apparent to those skilled in the art from the foregoing description. Such modifications are intended to fall within the scope of the appended claims.

## Claims

1. An antibody-drug conjugate represented by Formula (I): 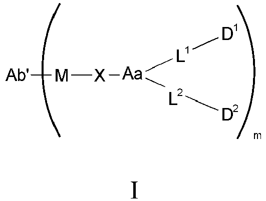 wherein,
Ab' represents an antibody or antigen-binding fragment thereof that specifically binds to B7H3;
M represents a linking site connected to the antibody or antigen-binding fragment thereof;
X represents a linker connecting M and Aa;
Aa is an amino acid fragment or a peptide fragment formed from two or more amino acids;
L¹ represents a linker connecting Aa and D¹;
L² represents a linker connecting Aa and D²;
D¹ represents a cytotoxic drug moiety;
D² represents a TLR agonist moiety;
m ranges from 1 to 10, for example, 1, 2, 3, 4, 5, 6, 7, 8, 9, or 10.

2. The antibody-drug conjugate according to claim 1, wherein the antibody or antigen-binding fragment thereof comprises:
(1) the following heavy chain variable region (VH) and/or light chain variable region (VL):
a heavy chain variable region (VH) comprising the following 3 CDRs: a CDR-H1 having a sequence as set forth in SEQ ID NO: 11 or a variant thereof, a CDR-H2 having a sequence as set forth in SEQ ID NO: 12 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(2) the following heavy chain variable region (VH) and/or light chain variable region (VL):
a heavy chain variable region (VH) comprising the following 3 CDRs: a CDR-H1 having a sequence as set forth in SEQ ID NO: 14 or a variant thereof, a CDR-H2 having a sequence as set forth in SEQ ID NO: 15 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(3) the following heavy chain variable region (VH) and/or light chain variable region (VL):
a heavy chain variable region (VH) comprising the following 3 CDRs: a CDR-H1 having a sequence as set forth in SEQ ID NO: 8 or a variant thereof, a CDR-H2 having a sequence as set forth in SEQ ID NO: 9 or a variant thereof, and a CDR-H3 having a sequence as set forth in SEQ ID NO: 10 or a variant thereof; and/or, a light chain variable region (VL) comprising the following 3 CDRs: a CDR-L1 having a sequence as set forth in SEQ ID NO: 3 or a variant thereof, a CDR-L2 having a sequence as set forth in SEQ ID NO: 4 or a variant thereof, and a CDR-L3 having a sequence as set forth in SEQ ID NO: 5 or a variant thereof; or
(4) the following heavy chain variable region (VH) and/or light chain variable region (VL):
a heavy chain variable region (VH) comprising the following 3 CDRs: a CDR-H1 having a sequence as set forth in SEQ ID NO: 16 or a variant thereof, a CDR-H2 having a sequence as set forth in SEQ ID NO: 17 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof.

3. The antibody-drug conjugate according to claim 1 or 2, wherein the antibody or antigen-binding fragment thereof comprises:
a VH as set forth in SEQ ID NO: 23 or 1 or a variant thereof, and/or, a VL as set forth in SEQ ID NO: 2 or a variant thereof;
preferably, the antibody or antigen-binding fragment thereof comprises: a heavy chain comprising a VH as set forth in SEQ ID NO: 23 or 1 or a variant thereof, and a heavy chain constant region (CH) as set forth in SEQ ID NO: 18 or a variant thereof, and/or, a light chain comprising a VL as set forth in SEQ ID NO: 2 or a variant thereof, and a light chain constant region (CL) as set forth in SEQ ID NO: 19 or a variant thereof;
preferably, the amino acid sequence of the heavy chain of the antibody or antigen-binding fragment thereof is set forth in SEQ ID NO: 22 or 20, and the amino acid sequence of the light chain of the antibody or antigen-binding fragment thereof is set forth in SEQ ID NO: 21;
optionally, the N-terminal glutamine of the heavy chain or heavy chain variable region and/or the light chain or light chain variable region undergoes cyclization to form a pyroglutamic acid or pyroglutamate; and/or, the heavy chain or heavy chain constant region (CH) or variant thereof lacks a C-terminal lysine.

4. The antibody-drug conjugate according to any one of claims 1 to 3, wherein M is a covalent bond or is selected from the group consisting of the following structures: 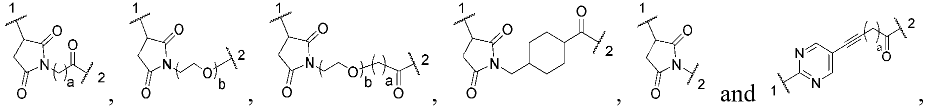
wherein, each a is independently an integer ranging from 0 to 6, b is an integer ranging from 1 to 10, and the position 1 of M is connected to Ab', and the position 2 is connected to X;
preferably, M is a covalent bond or is selected from the group consisting of the following structures:

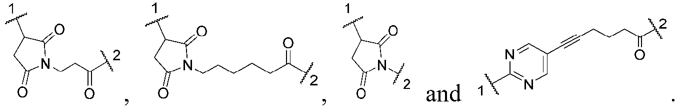

5. The antibody-drug conjugate according to any one of claims 1 to 4, wherein X is a covalent bond or is selected from the group consisting of C₁₋₆ alkylene, -NH-(CH₂)_{c}-C(O)-, 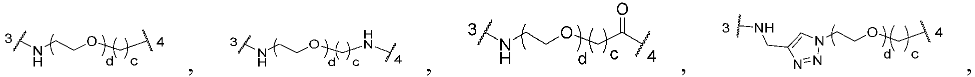 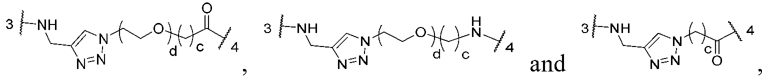 preferably a covalent bond or 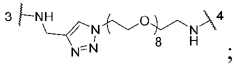 wherein, each c is independently an integer ranging from 0 to 6, and each d is independently an integer ranging from 1 to 10; the position 3 of X is connected to M, and the position 4 is connected to Aa.

6. The antibody-drug conjugate according to any one of claims 1 to 5, wherein Aa is selected from the group consisting of the following amino acid fragments: Gly, Phe, Ala, Val, Cys, Asp, Glu, Lys, Nle, and Arg;
preferably, Aa is selected from the group consisting of the following structures: 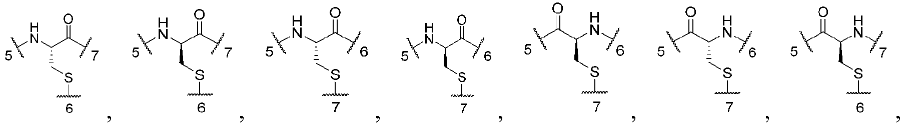 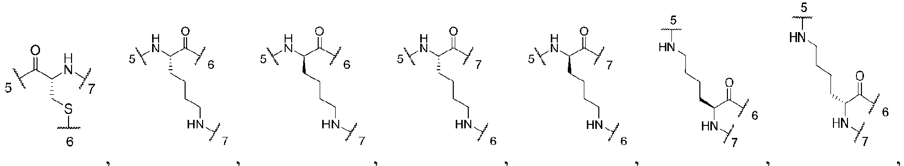 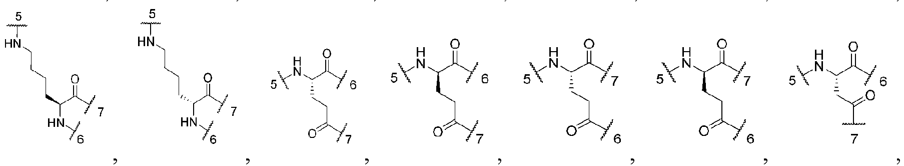 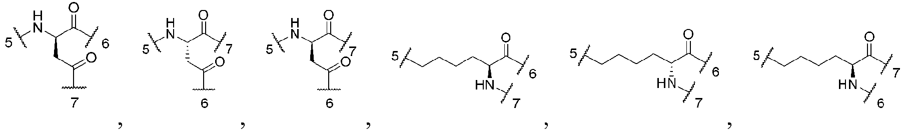 and 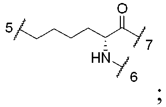
wherein, the position 5 of Aa is connected to X, the position 6 is connected to L¹, and the position 7 is connected to L².

7. The antibody-drug conjugate according to any one of claims 1 to 6, wherein L¹ is a covalent bond or is selected from the group consisting of non-cleavable linkers and cleavable linkers, wherein the cleavable linker is cleavable by an enzyme present in a pathological environment, and the enzyme is selected from the group consisting of a protease, a phosphatase, a pyrophosphatase, *β*-glucuronidase, *β*-galactosidase, and a sulfatase;
preferably, L¹ is -L^{a}-L^{b}-L^{c}-, wherein:
L^{a} is a covalent bond or is selected from the group consisting of C₁₋₆ alkylene, -NH-(CH₂)ₑ-C(O)-, 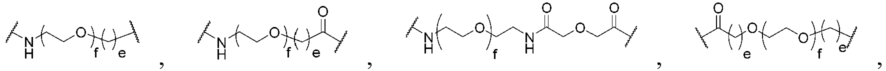 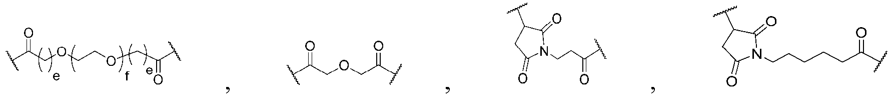 and 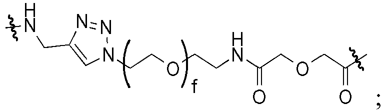 wherein, each e is independently an integer ranging from 0 to 6, and each f is independently an integer ranging from 1 to 10;
L^{b} is a covalent bond or is an amino acid fragment or a peptide fragment formed by two or more amino acids, wherein the amino acid is selected from the group consisting of Val, Cit, Glu, Lys, Arg, Phe, Leu, Gly, Ala, and Asn;
L^{c} is a covalent bond or is selected from the group consisting of -NH-CH₂- and the following structures: 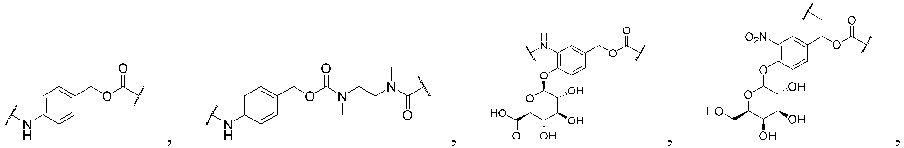 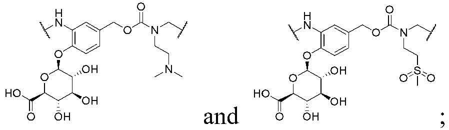
preferably, L^{a} is a covalent bond or is selected from the group consisting of C₁₋₆ alkylene, - NH-(CH₂)ₑ-C(O)-, 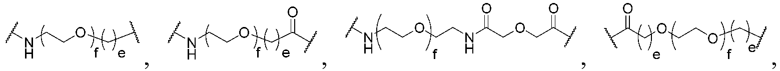 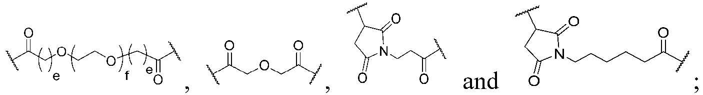
preferably, L^{b} is a covalent bond or is selected from the group consisting of the following structures:

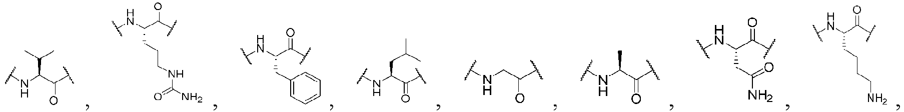

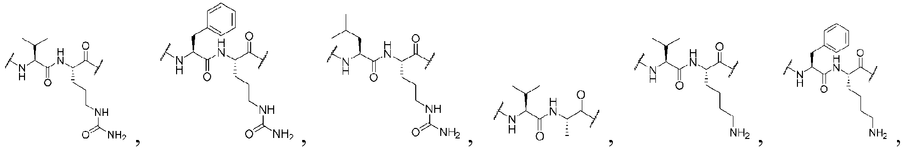

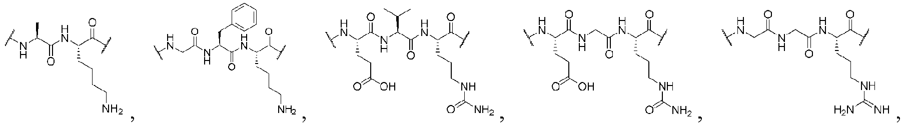

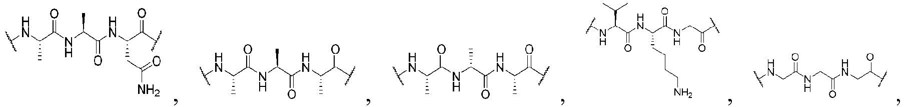

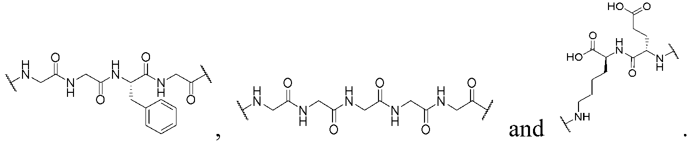

8. The antibody-drug conjugate according to any one of claims 1 to 7, wherein L¹ is a covalent bond or is selected from the group consisting of the following structures: 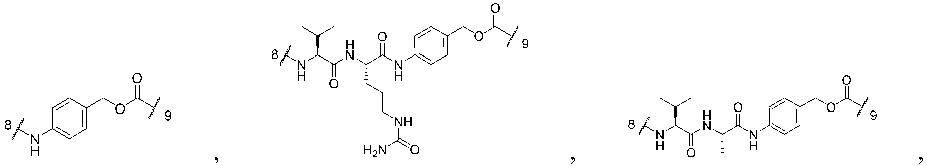 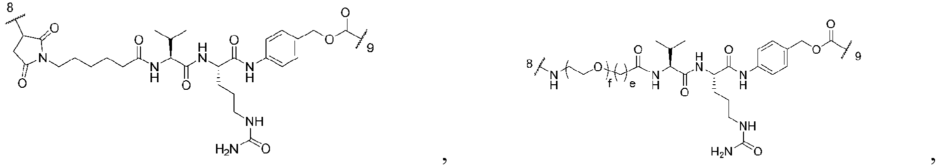 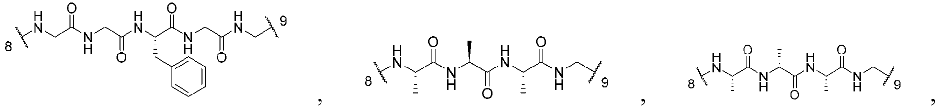 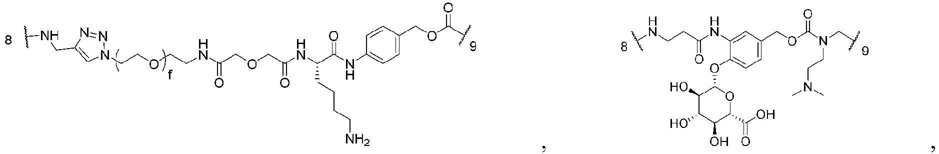 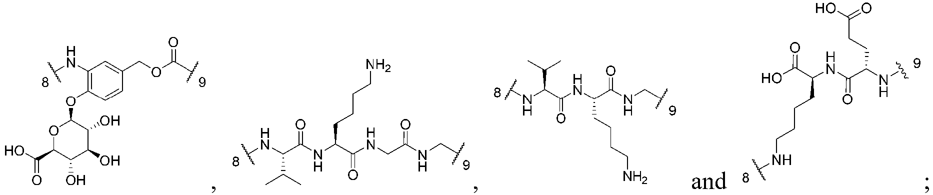
wherein, each e is independently an integer ranging from 0 to 6, and each f is independently an integer ranging from 1 to 10; the position 8 of L¹ is connected to Aa, and the position 9 is connected to D¹;
preferably, L¹ is: 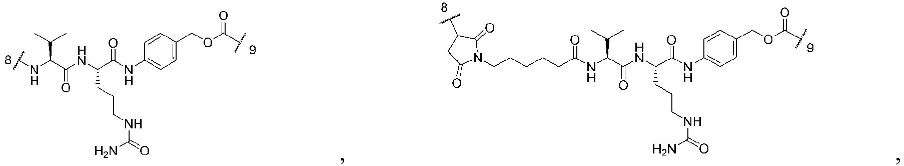 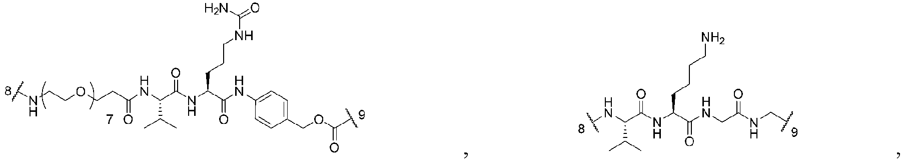 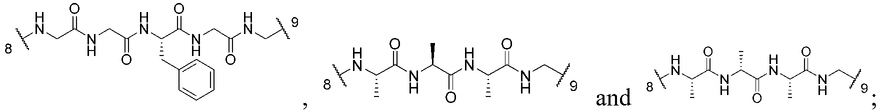
wherein, the position 8 of L¹ is connected to Aa, and the position 9 is connected to D¹.

9. The antibody-drug conjugate according to any one of claims 1 to 8, wherein L² is selected from the group consisting of non-cleavable linkers and cleavable linkers, the cleavable linker is cleavable by an enzyme present in a pathological environment, and the enzyme is selected from the group consisting of a protease, a phosphatase, a pyrophosphatase, *β*-glucuronidase, *β-*galactosidase, and a sulfatase.

10. The antibody-drug conjugate according to any one of claims 1 to 9, wherein L² is selected from the group consisting of the following structures: 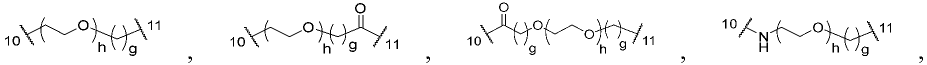 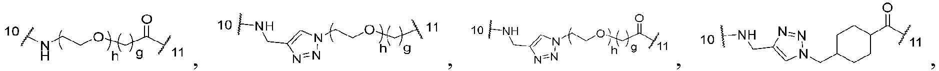 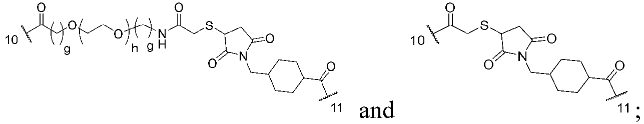
wherein each g is independently an integer ranging from 0 to 6, and each h is independently an integer ranging from 1 to 10; the position 10 of L² is connected to Aa, and the position 11 is connected to D²;
preferably, L² is selected from the group consisting of the following structures: 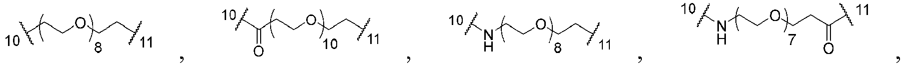 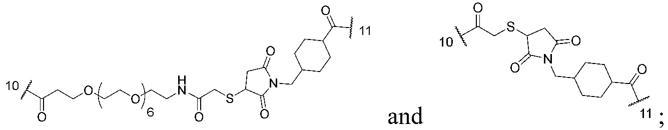
wherein, the position 10 of L² is connected to Aa, and the position 11 is connected to D².

11. The antibody-drug conjugate according to any one of claims 1 to 10, wherein D¹ is selected from the group consisting of cytotoxic drug moieties, wherein the cytotoxic drug includes tubulin inhibitors, DNA damaging agents, and topoisomerase inhibitors; the tubulin inhibitors include dolastatin and auristatins, maytansines, tubulysins, and cryptomycins; the DNA damaging agents include pyrrolobenzodiazepines, duocarmycins, and calicheamicins; and the topoisomerase inhibitors include camptothecin and derivatives thereof, epipodophyllotoxins, anthracyclines, and anthraquinones;
preferably, the tubulin inhibitor is selected from the group consisting of dolastatin 10, MMAE, MMAF, maytansine, DM1, DM3, and DM4; and the topoisomerase inhibitor is selected from the group consisting of camptothecin, SN-38, exatecan, topotecan, belotecan, rubitecan, diflomotecan, 10-hydroxycamptothecin, 9-aminocamptothecin, Dxd, T030, doxorubicin, epirubicin, and PNU-159682;
preferably, D¹ is selected from the group consisting of the following structures:

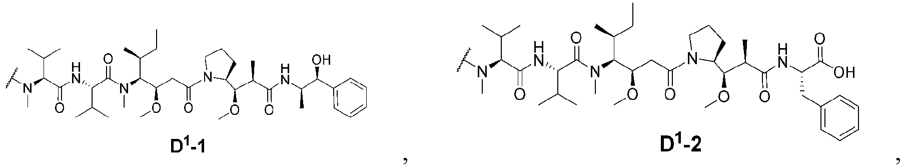

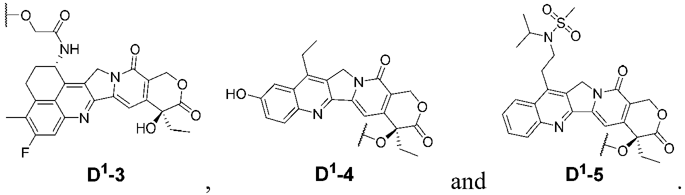

12. The antibody-drug conjugate according to any one of claims 1 to 11, wherein D² is a TLR agonist moiety, and the TLR agonist is selected from the group consisting of a TLR2 agonist, a TLR4 agonist, a TLR6 agonist, a TLR7 agonist, a TLR8 agonist, a TLR7/8 agonist, and a TLR9 agonist;
preferably, the TLR agonist is selected from the group consisting of a TLR7 agonist, a TLR8 agonist, and a TLR7/8 agonist;
preferably, D² is selected from the group consisting of the following structures:

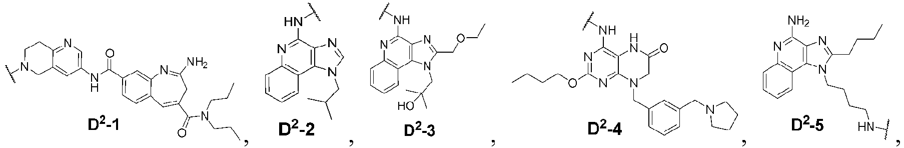

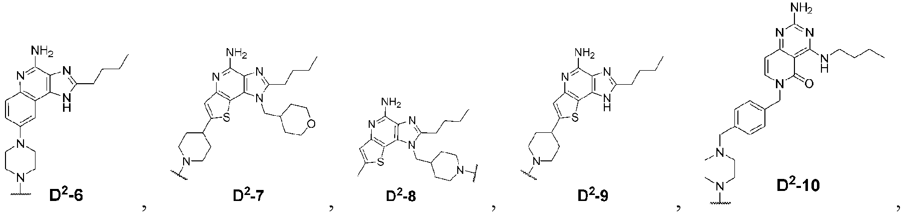

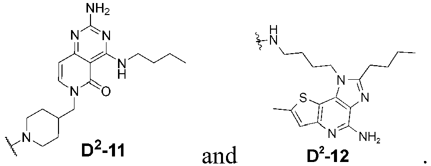

13. The antibody-drug conjugate according to any one of claims 1 to 12, wherein 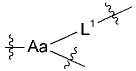 is selected from the group consisting of: 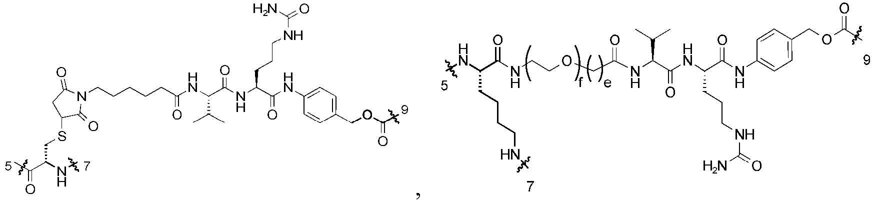 (for example 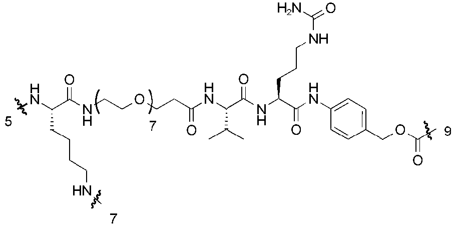 ), 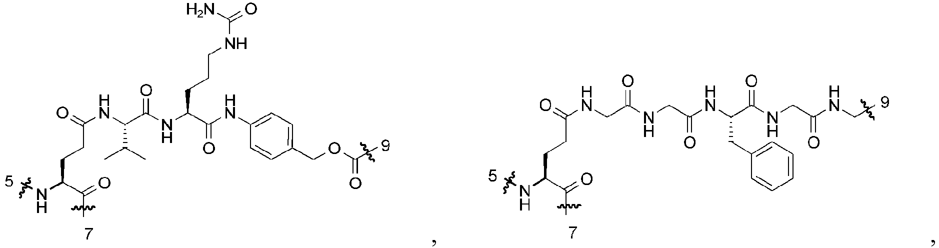 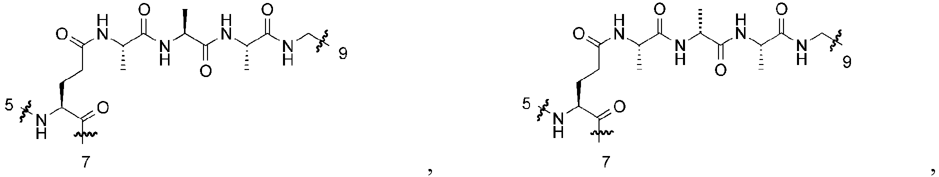 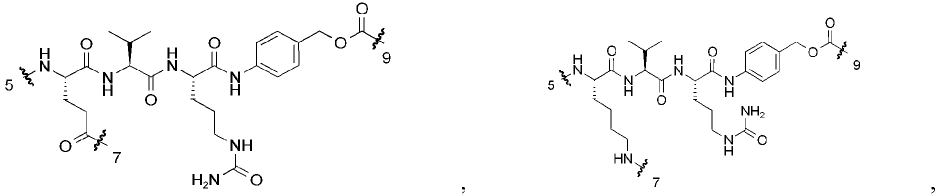 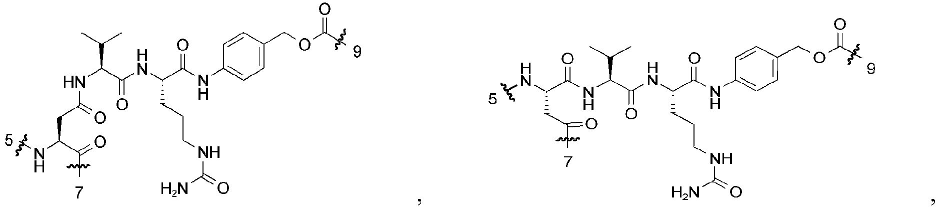 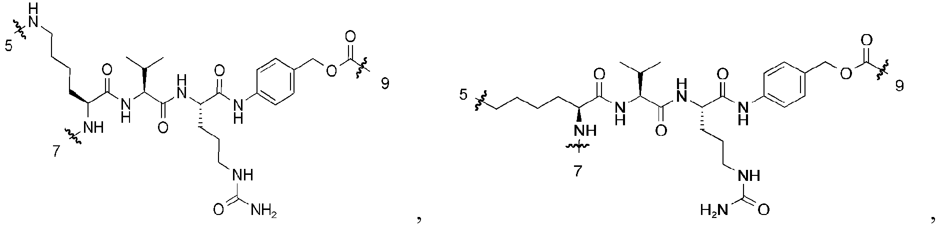 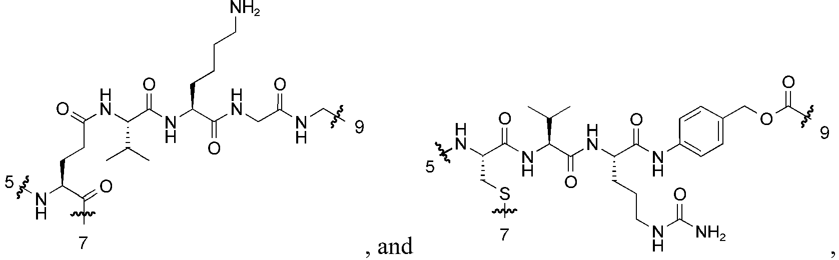 wherein, e is an integer ranging from 0 to 6, and f is an integer ranging from 1 to 10; the position 5 is connected to X, the position 7 is connected to L², and the position 9 is connected to D¹.

14. The antibody-drug conjugate according to any one of claims 1 to 13, wherein 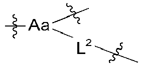 is selected from the group consisting of: 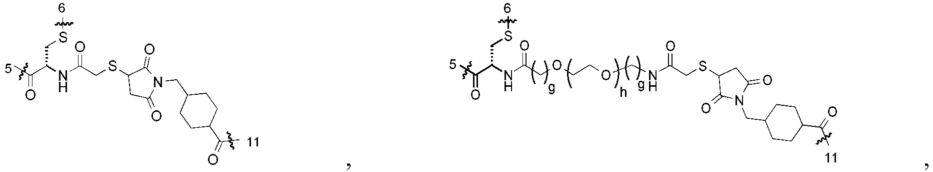 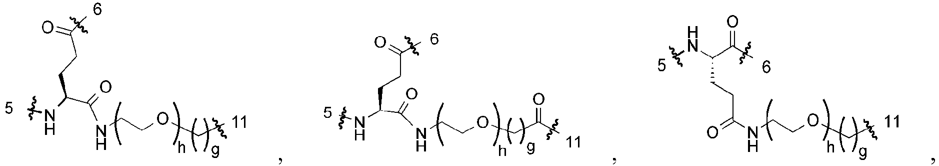 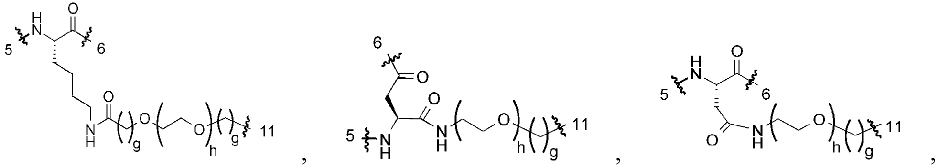 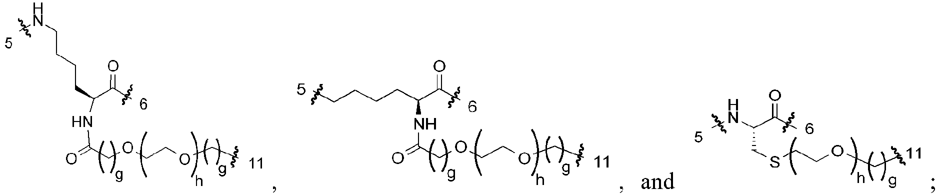 wherein, each g is independently an integer ranging from 0 to 6, and each h is independently an integer ranging from 1 to 10;
preferably, 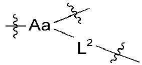 is selected from the group consisting of: 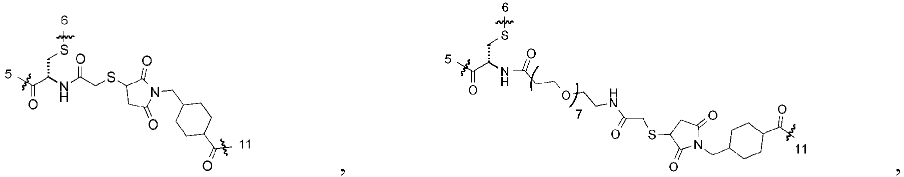 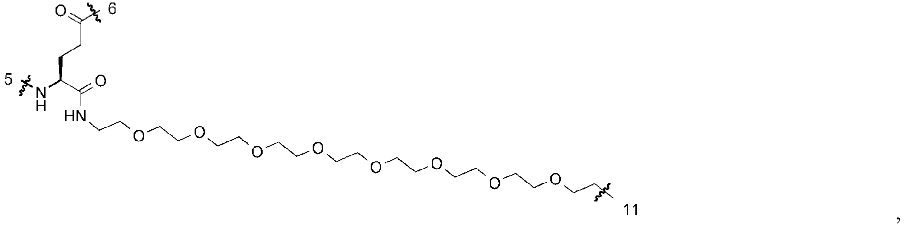 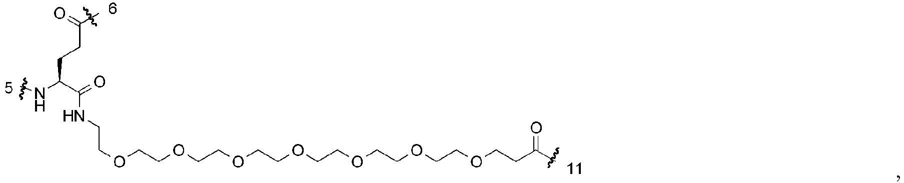 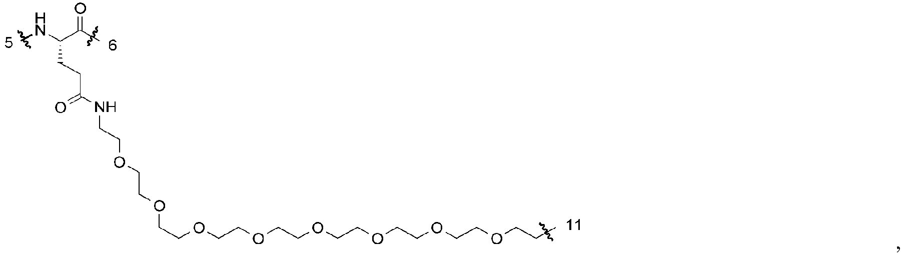 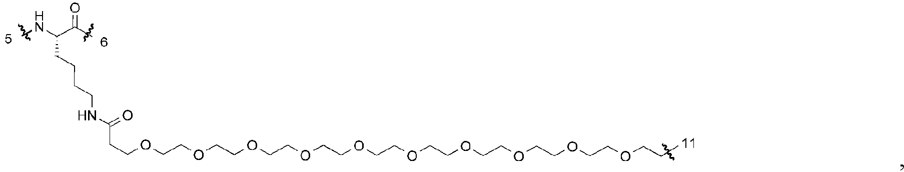 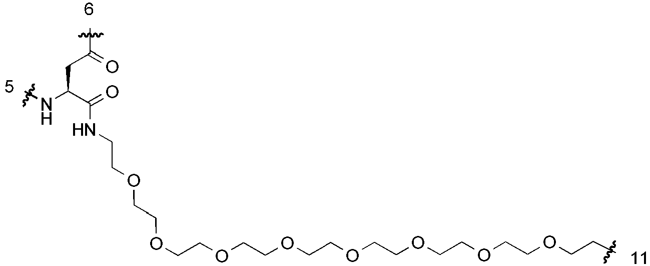 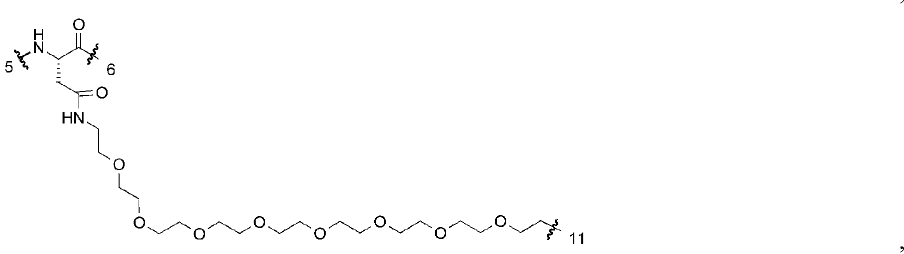 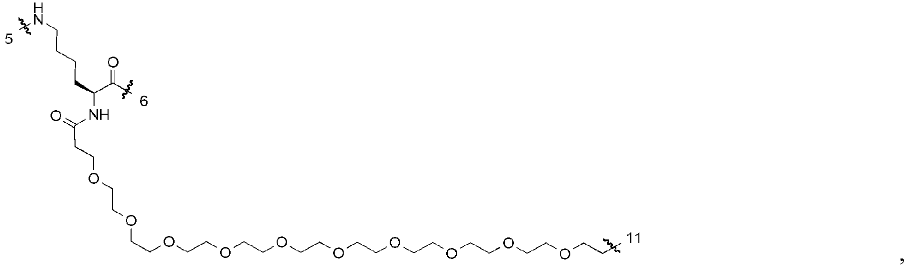 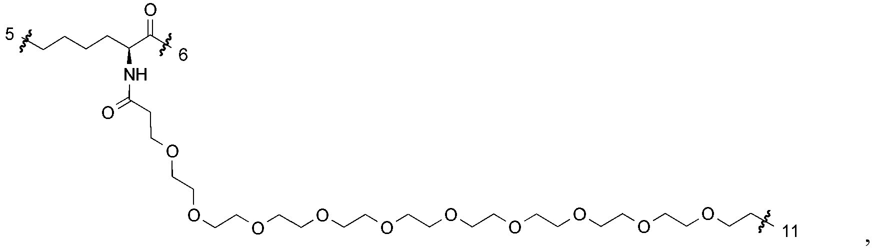 and 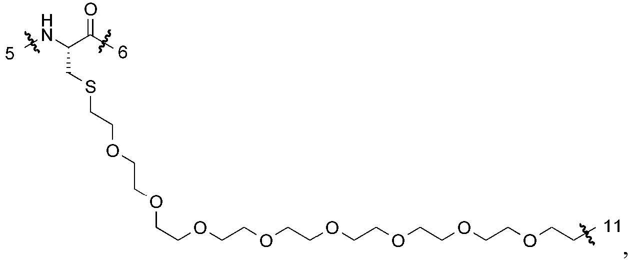
wherein, the position 5 is connected to X, the position 6 is connected to L¹, and the position 11 is connected to D².

15. The antibody-drug conjugate according to any one of claims 1 to 14, wherein 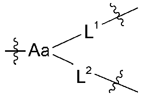 is selected from the group consisting of: 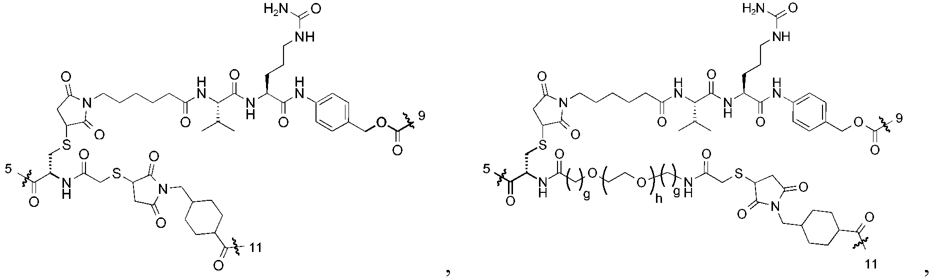 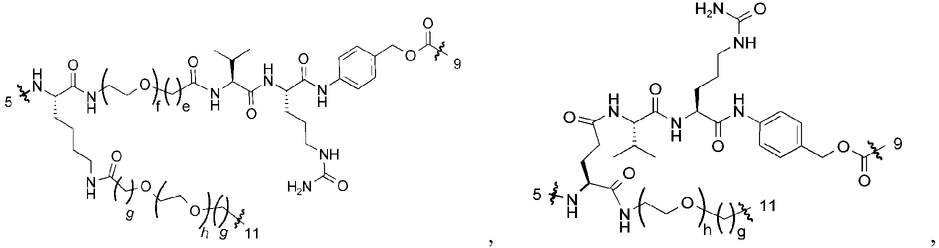 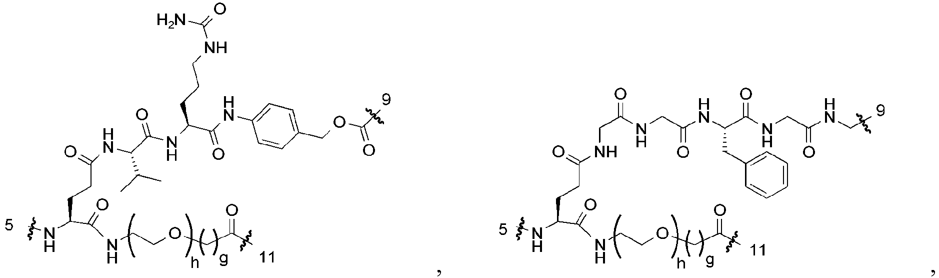 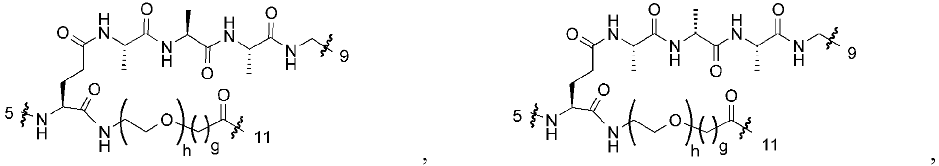 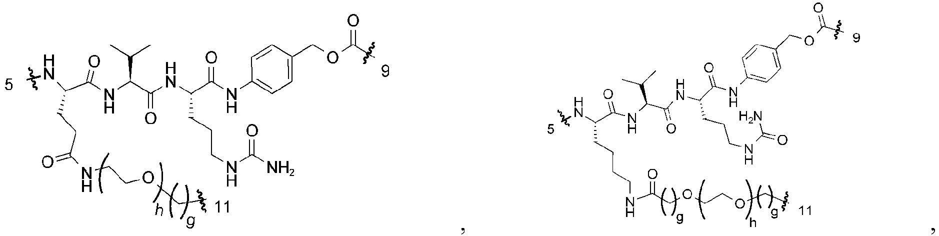 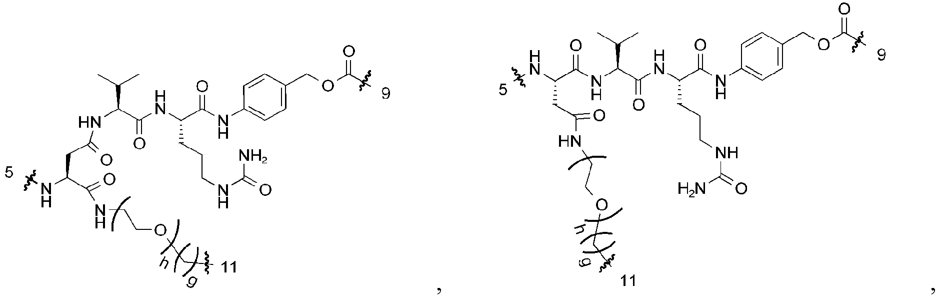 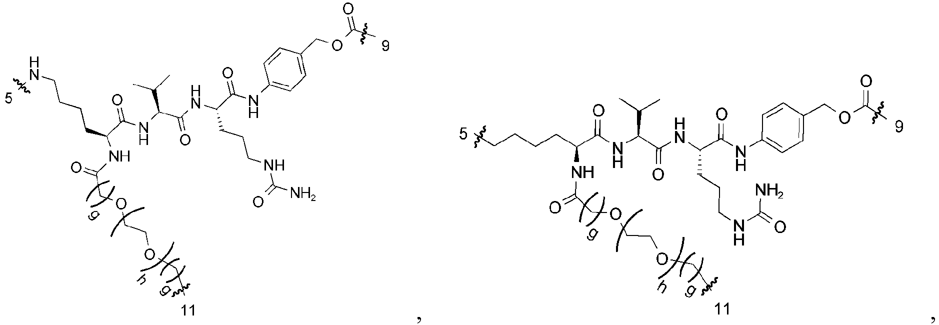 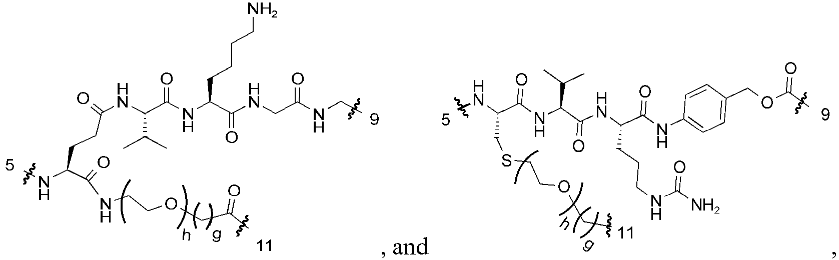 wherein, each g is independently an integer ranging from 0 to 6, and each h is independently an integer ranging from 1 to 10;
preferably, 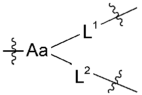 is selected from the group consisting of: 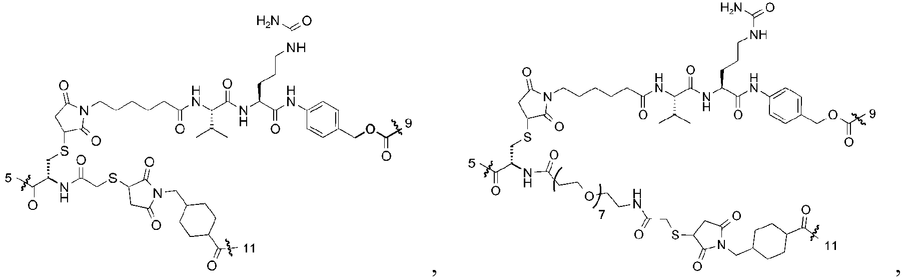 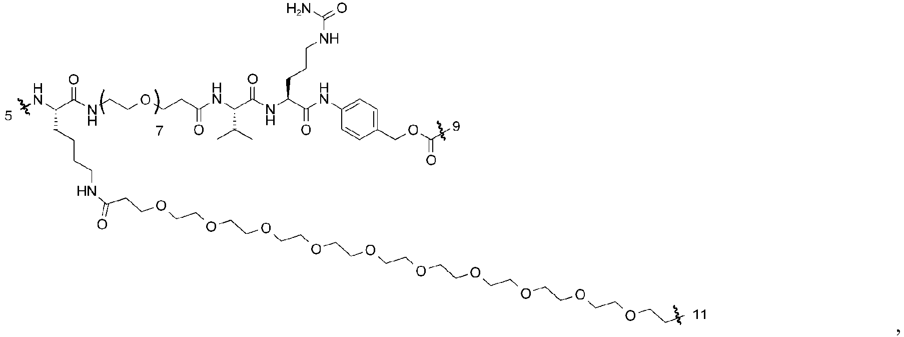 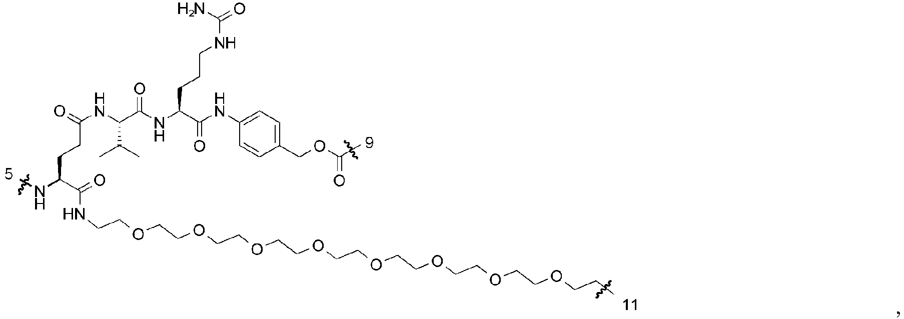 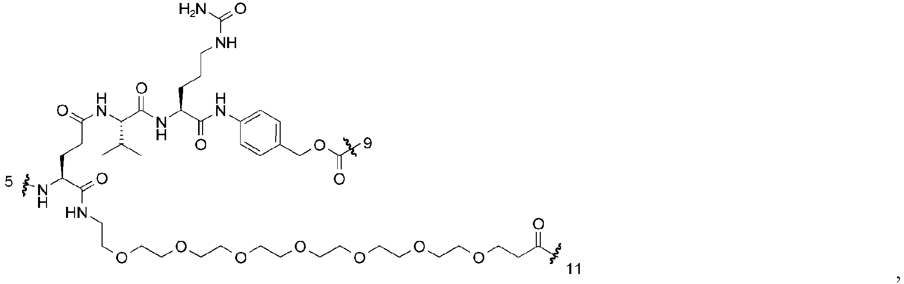 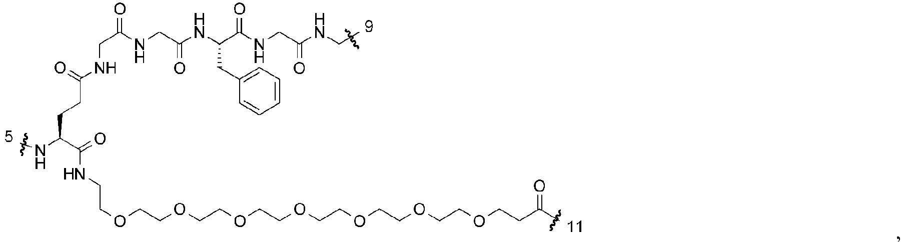 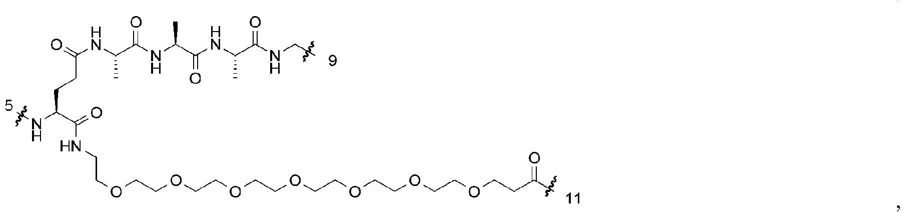 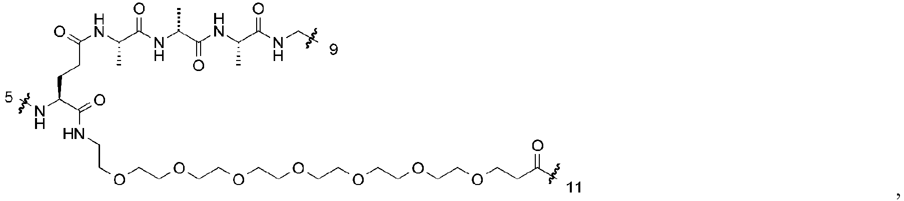 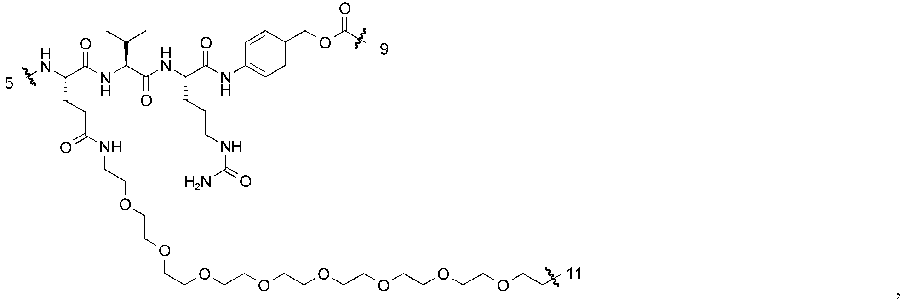 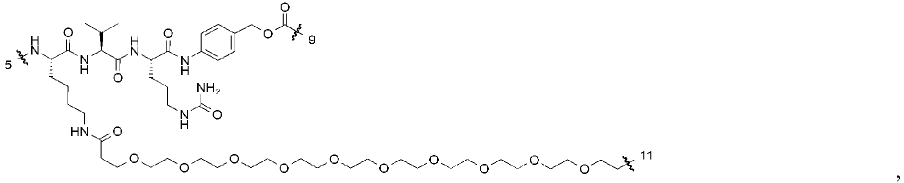 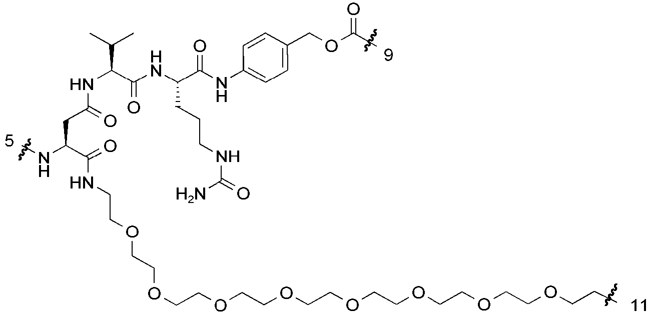 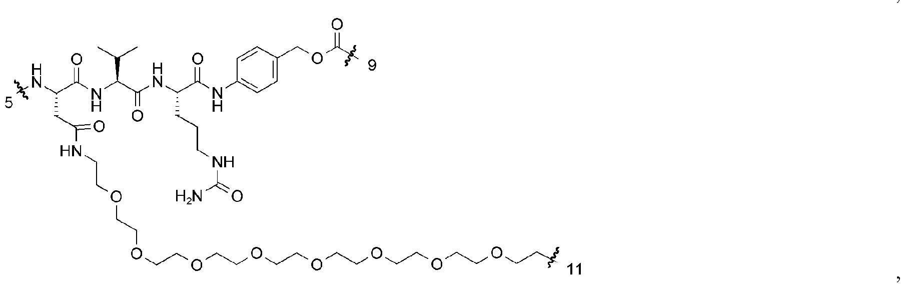 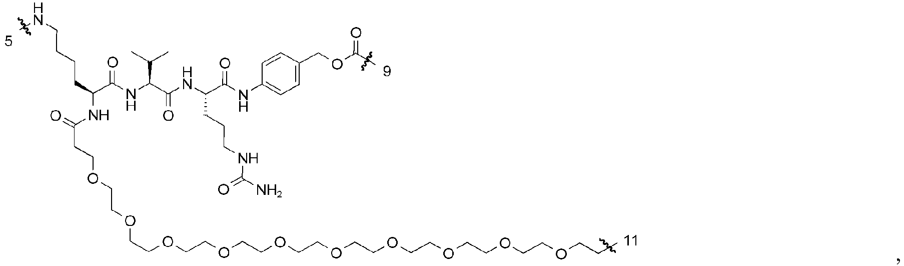 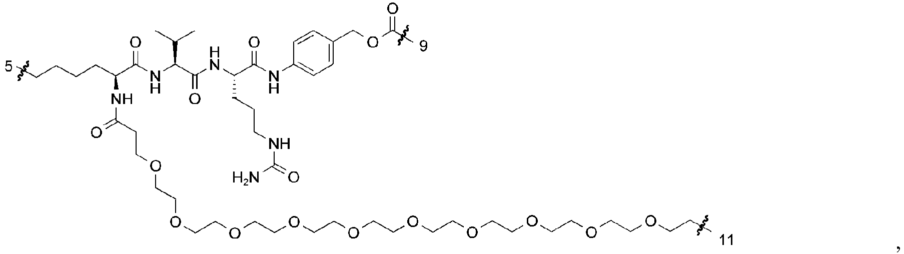 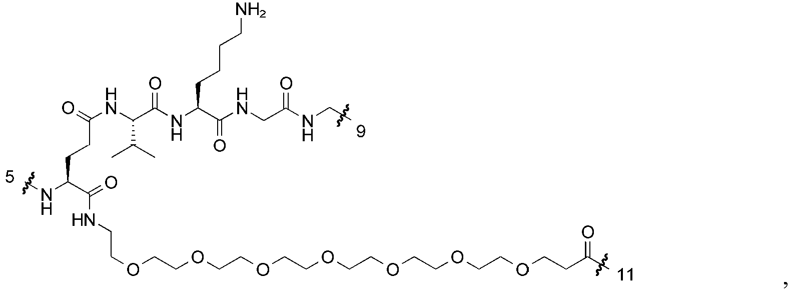 and 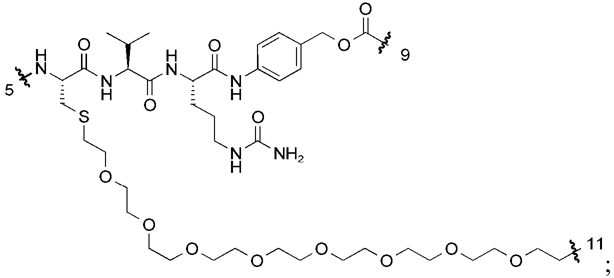
wherein, the position 5 is connected to X, the position 9 is connected to D¹, and the position 11 is connected to D².

16. The antibody-drug conjugate according to any one of claims 1 to 15, wherein 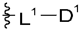 is selected from the group consisting of: 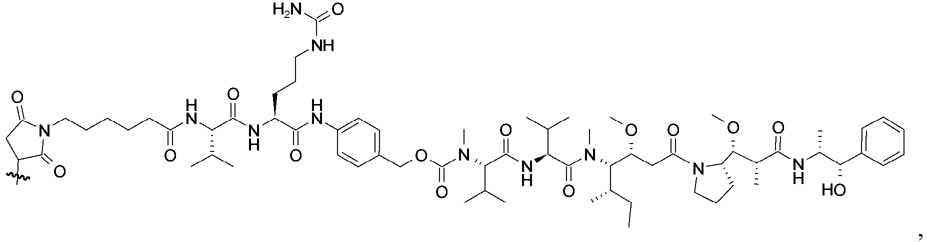 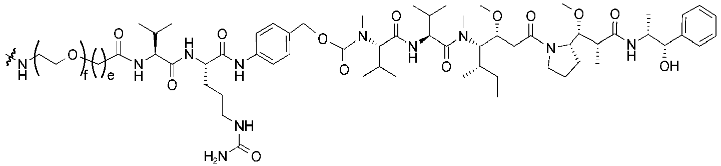 (for example 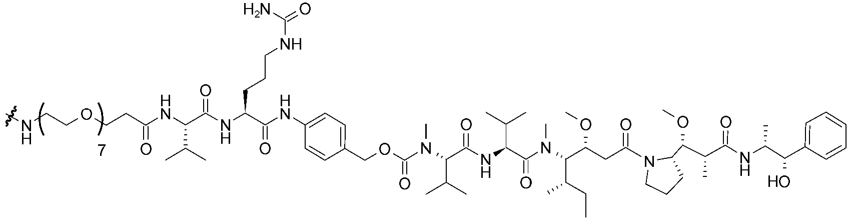 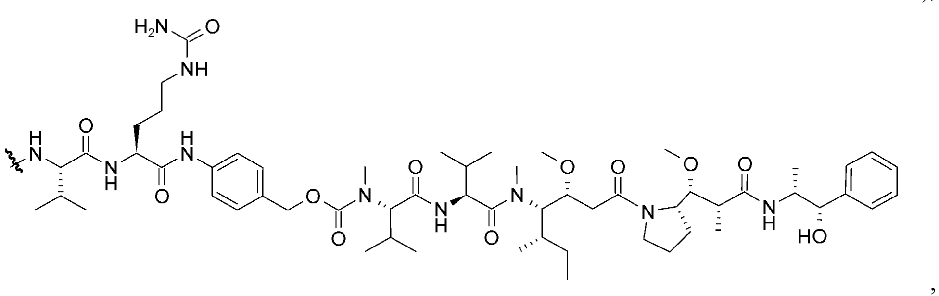 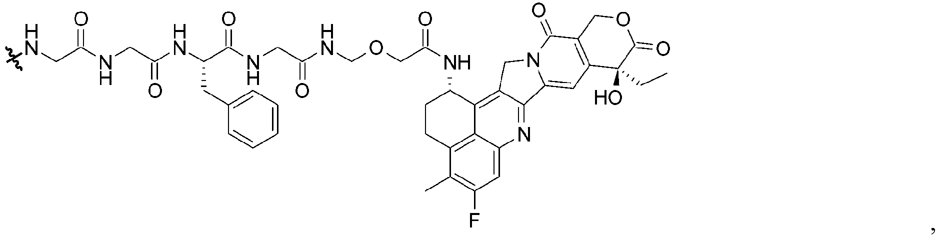 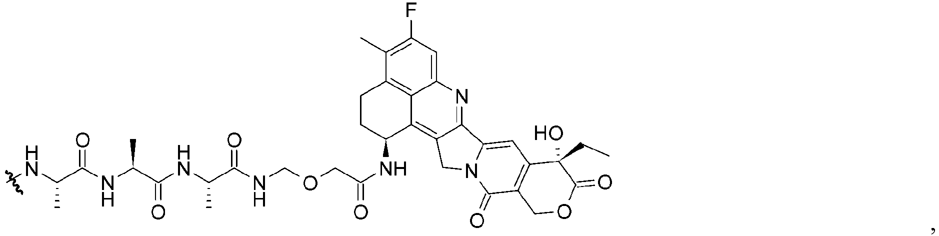 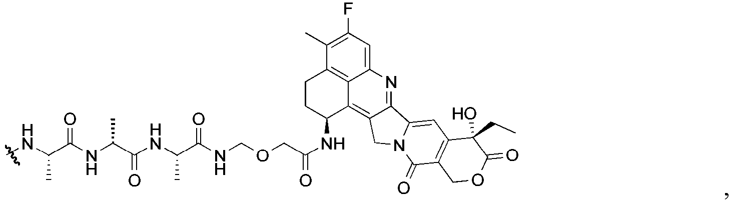 and 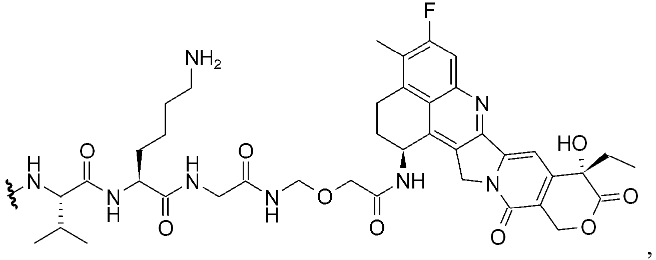 wherein, each e is independently an integer ranging from 0 to 6, and each f is independently an integer ranging from 1 to 10.

17. The antibody-drug conjugate according to any one of claims 1 to 16, wherein 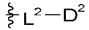 is selected from the group consisting of: 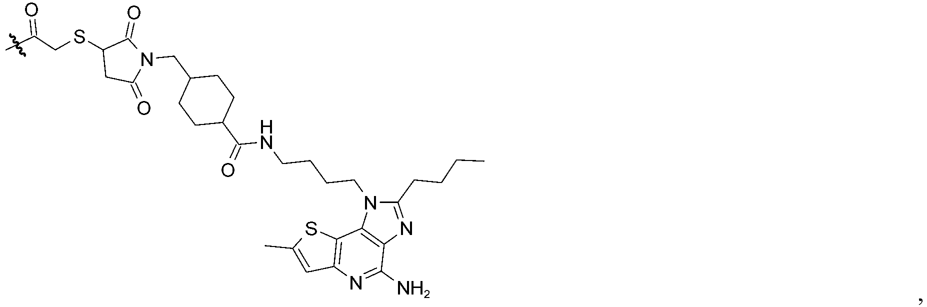 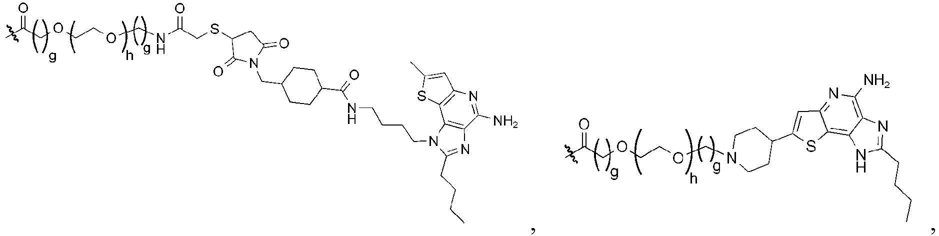 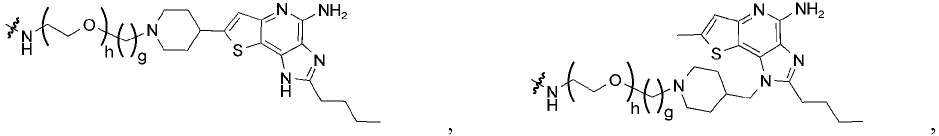 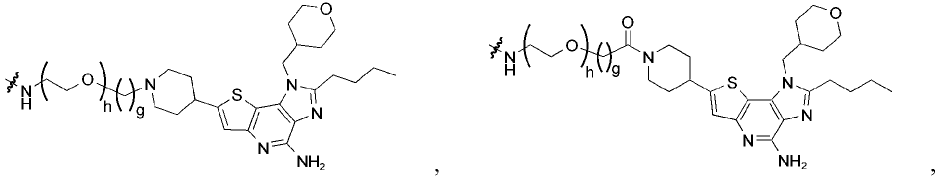 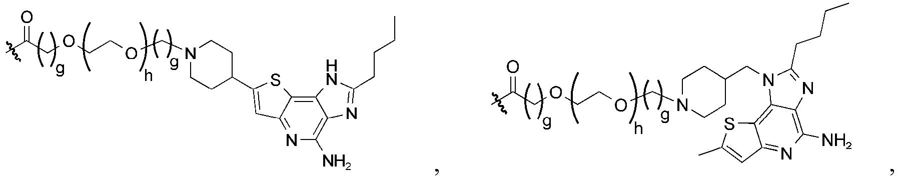 and 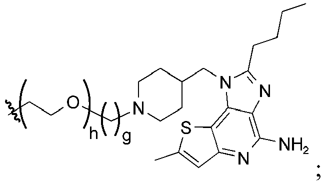 wherein, each g is independently an integer ranging from 0 to 6, and each h is independently an integer ranging from 1 to 10;
preferably, 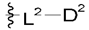 conjugate is selected from the group consisting of: 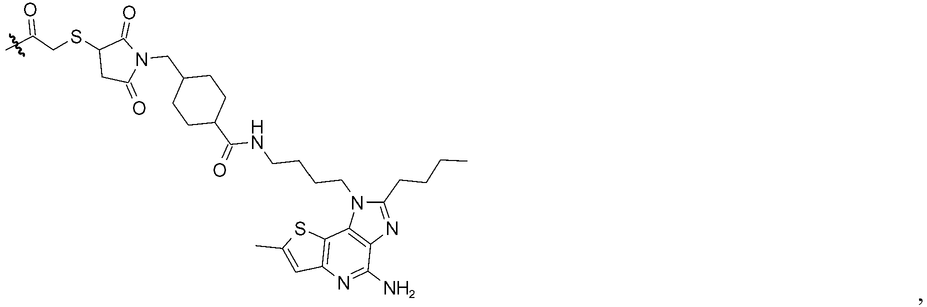 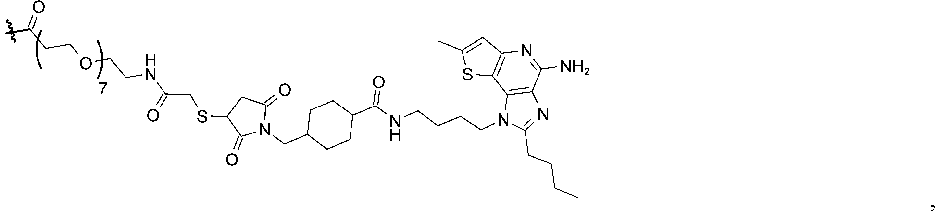 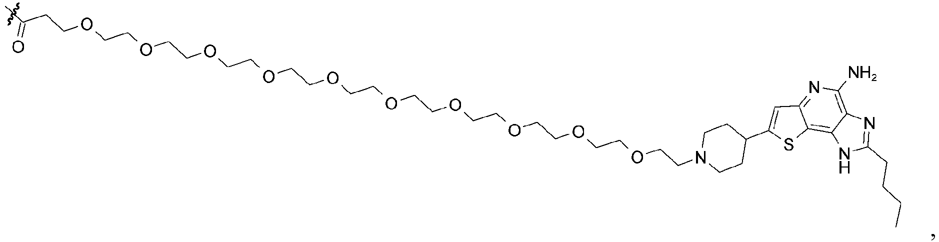 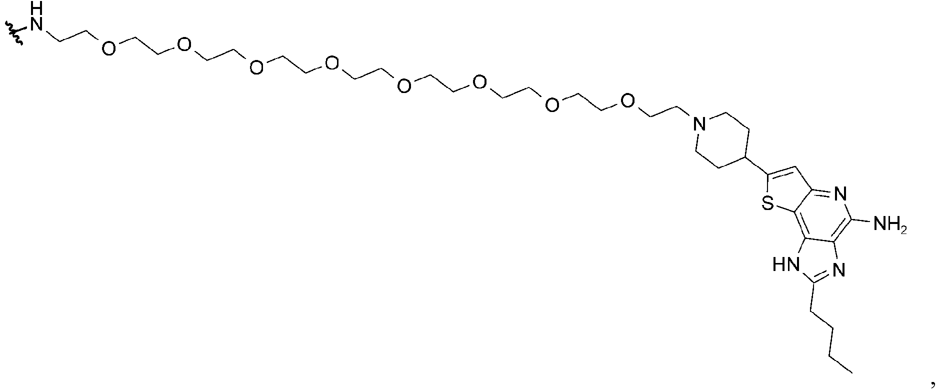 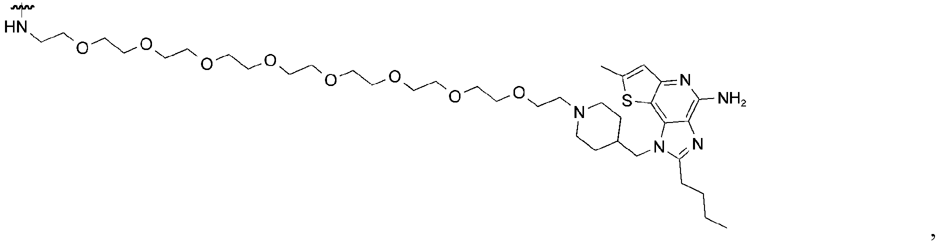 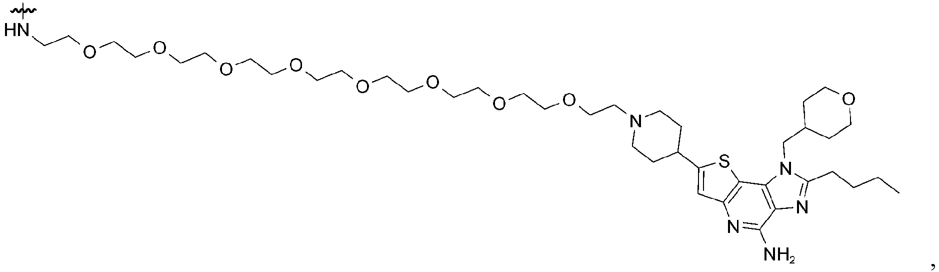 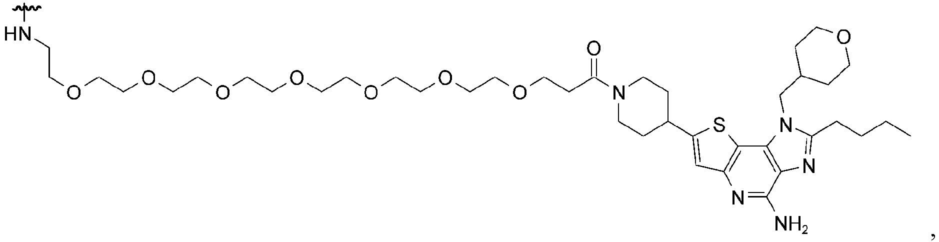 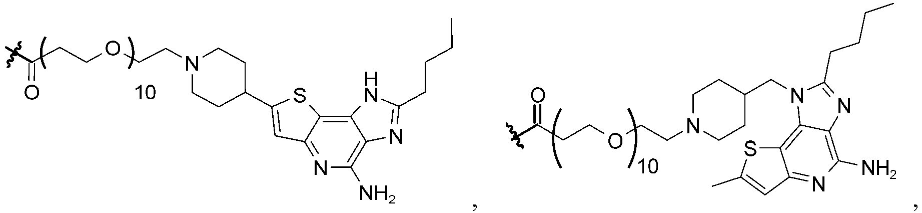 and

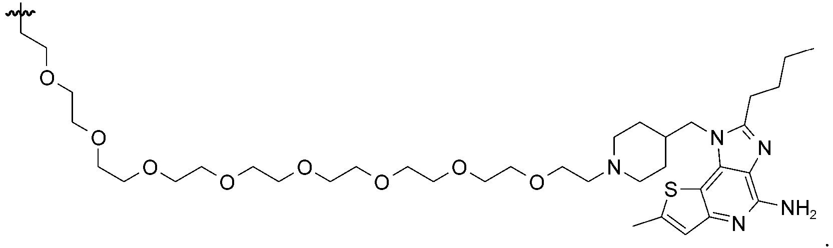

18. The antibody-drug conjugate according to any one of claims 1 to 17, wherein 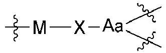 is selected from the group consisting of: 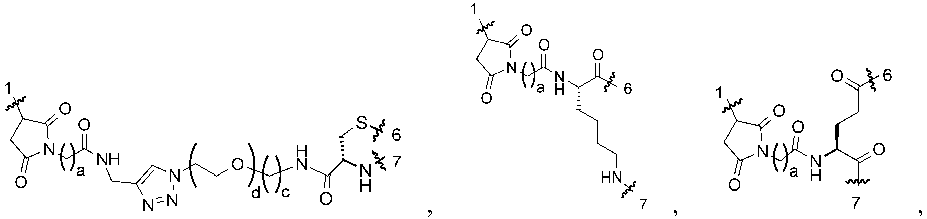 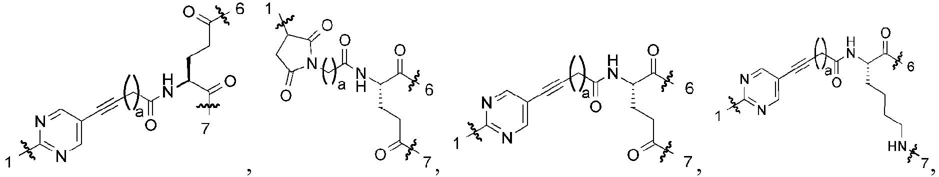 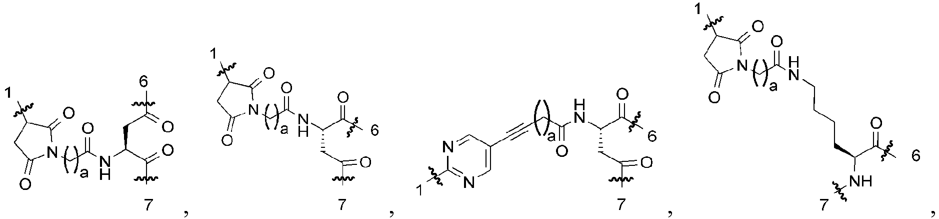 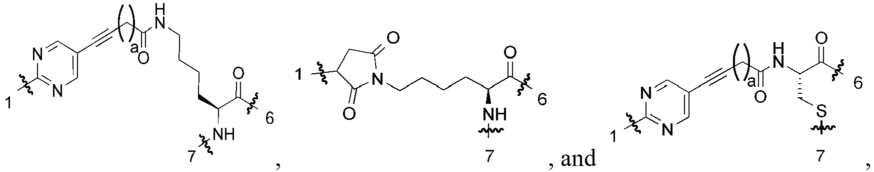
wherein each a is independently an integer ranging from 0 to 6, each c is independently an integer ranging from 0 to 6, and each d is independently an integer ranging from 1 to 10;
preferably, 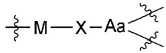
is selected from the group consisting of: 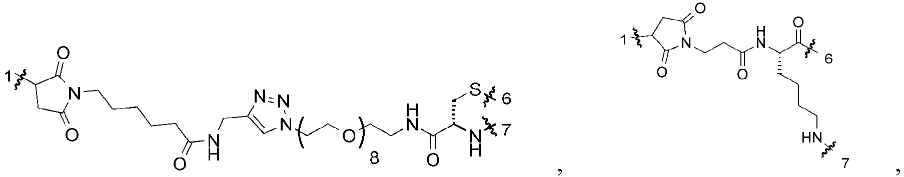 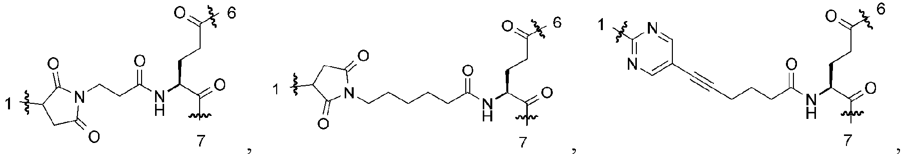 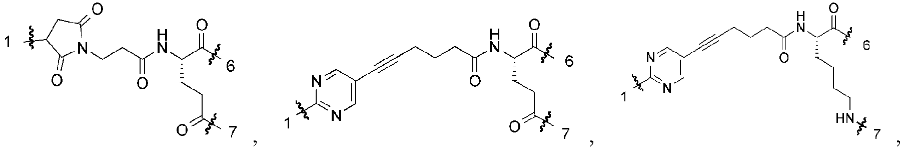 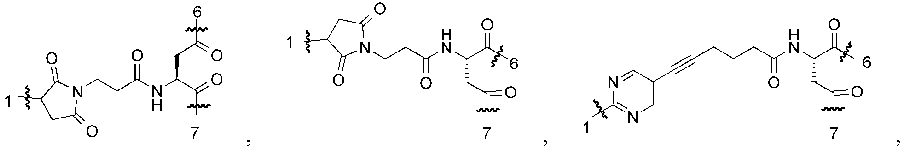 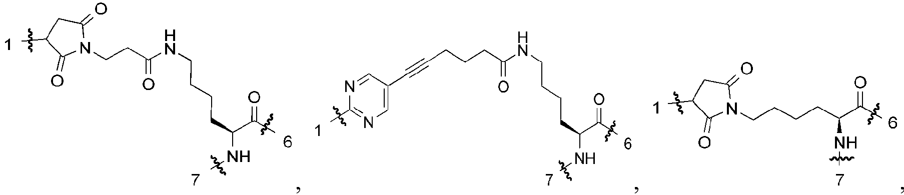
and 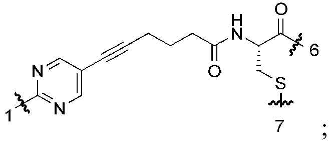
wherein the position 1 is connected to Ab', the position 6 is connected to L¹, and the position 7 is connected to L².

19. The antibody-drug conjugate according to any one of claims 1 to 18, wherein 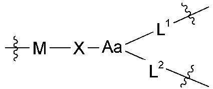 is selected from the group consisting of: 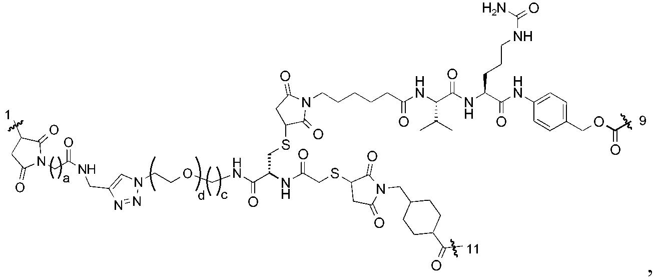 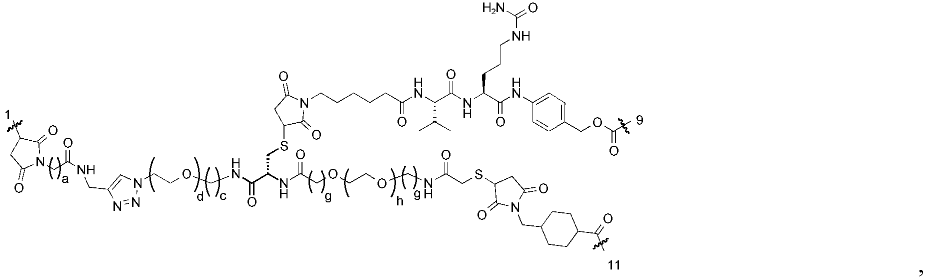 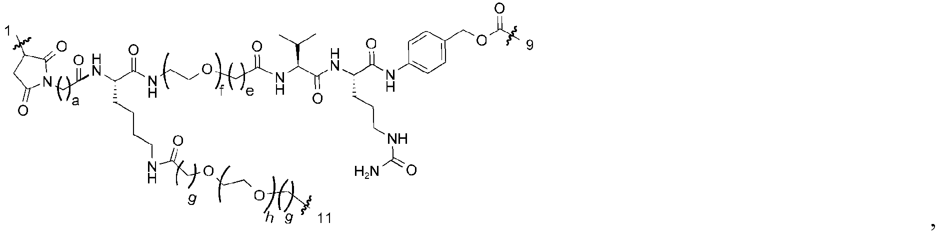 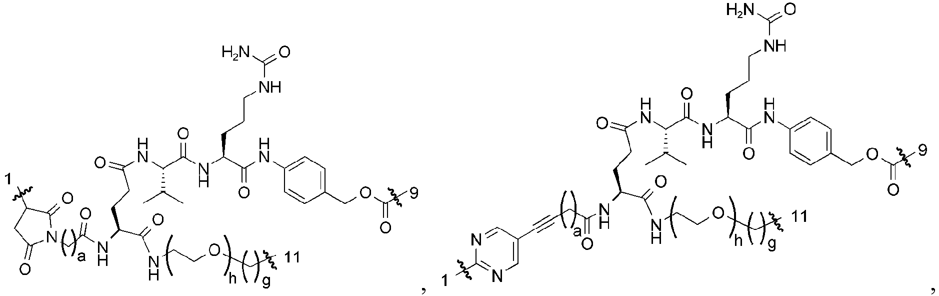 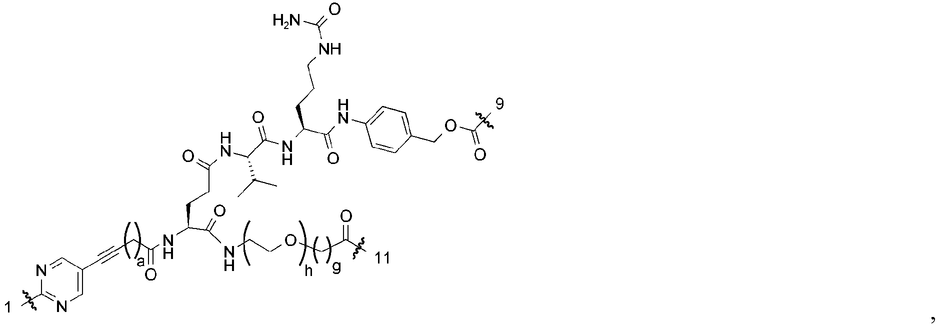 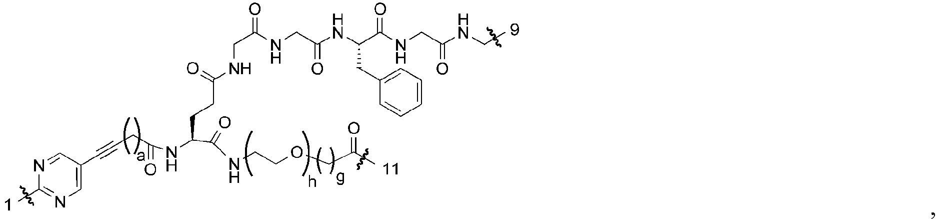 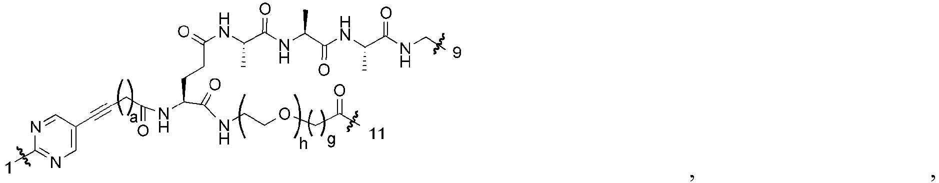 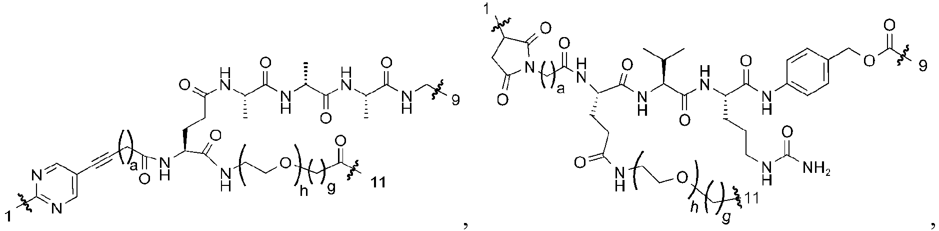 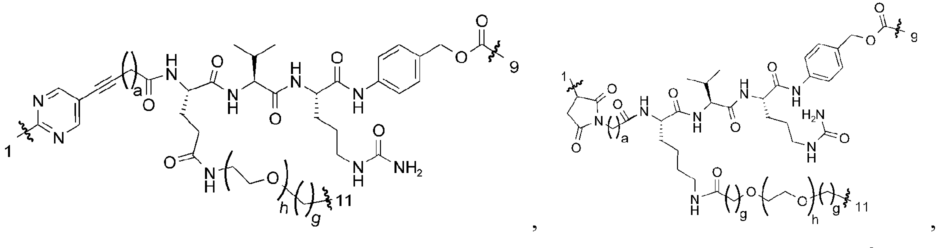 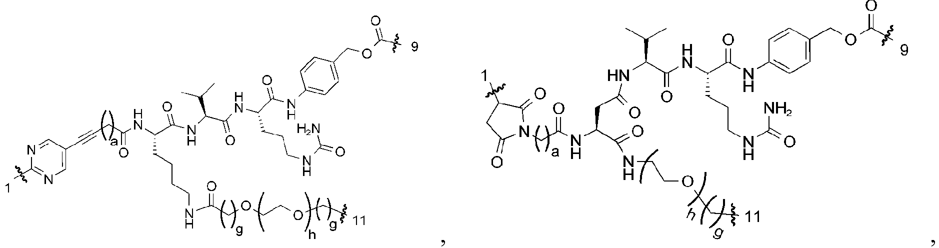 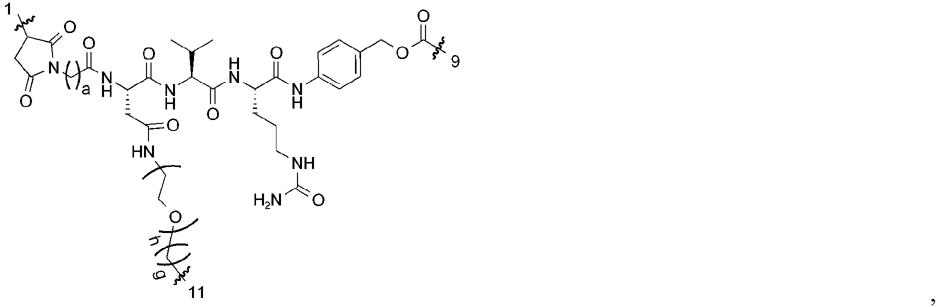 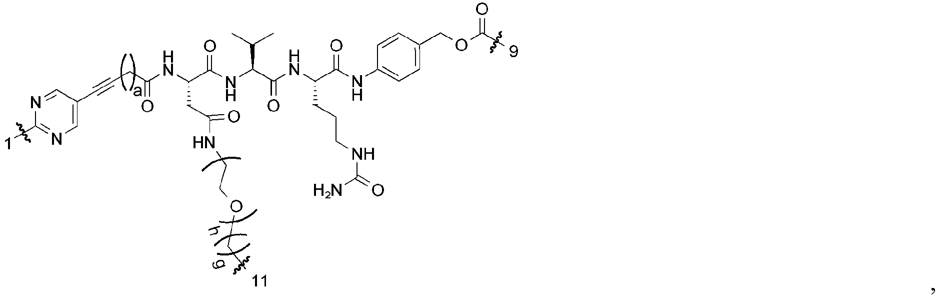 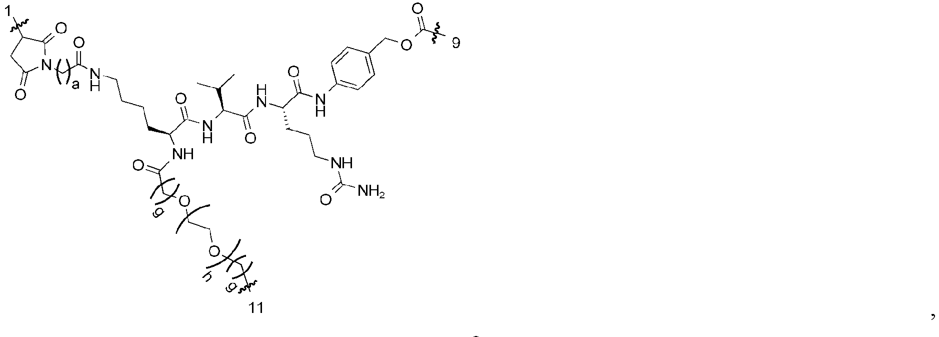 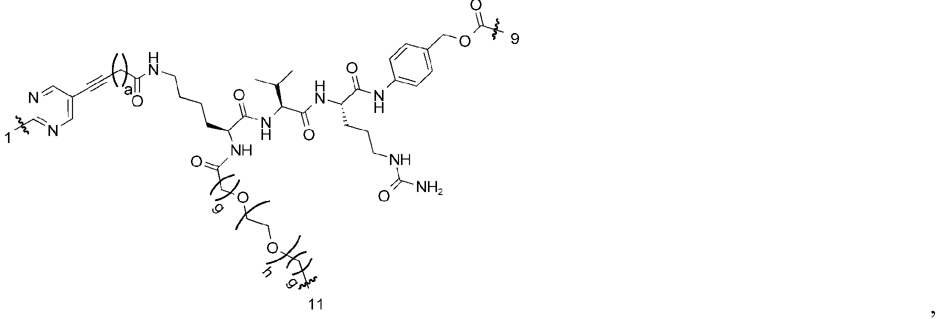 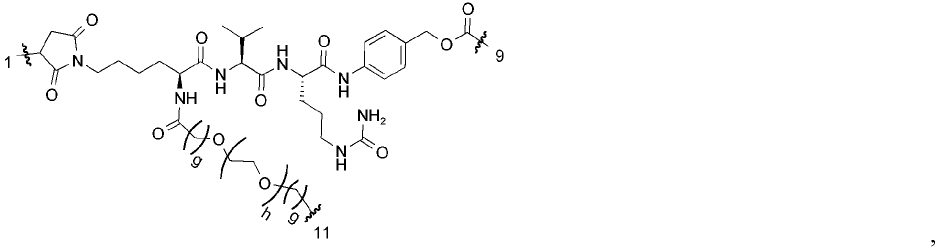 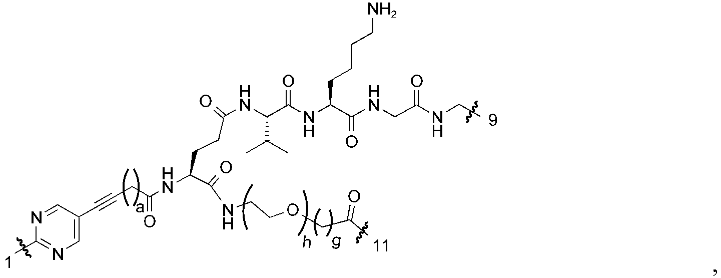 and 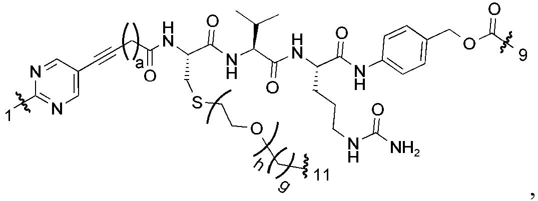 wherein, each a is independently an integer ranging from 0 to 6, each c is independently an integer ranging from 0 to 6, each d is independently an integer ranging from 1 to 10, each e is independently an integer ranging from 0 to 6, each f is independently an integer ranging from 1 to 10, each g is independently an integer ranging from 0 to 6, and each h is independently an integer ranging from 1 to 10;
preferably, 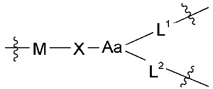 is selected from the group consisting of: 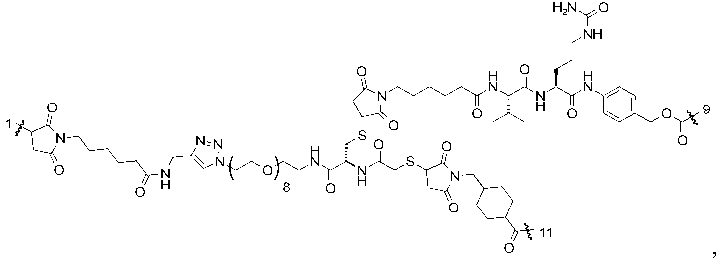 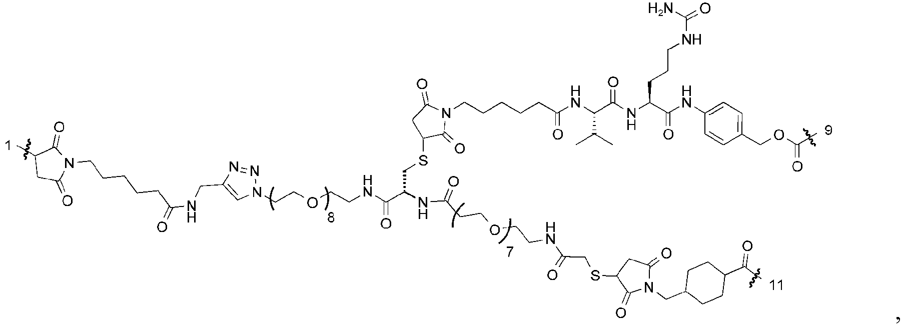 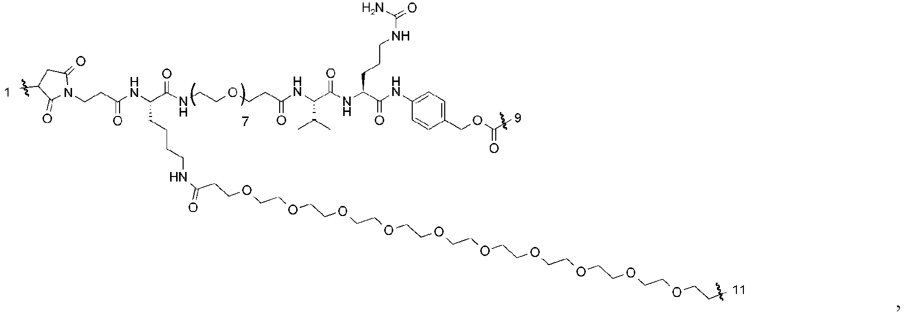 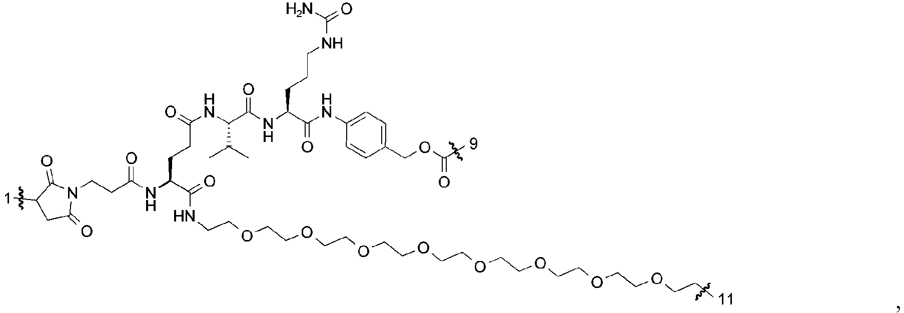 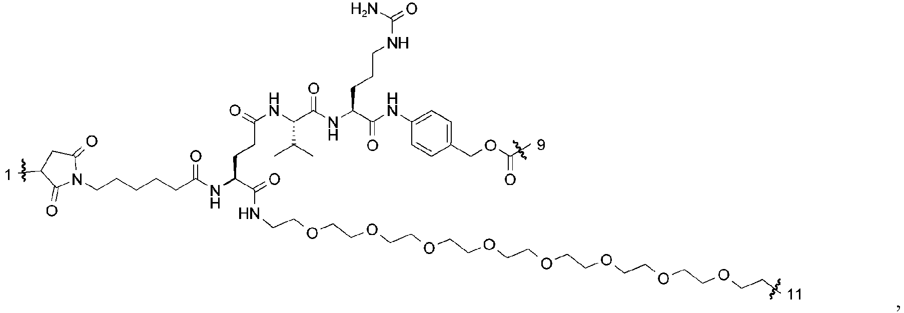 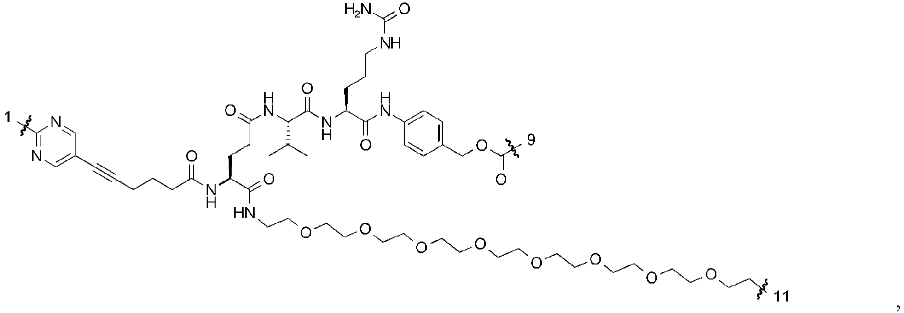 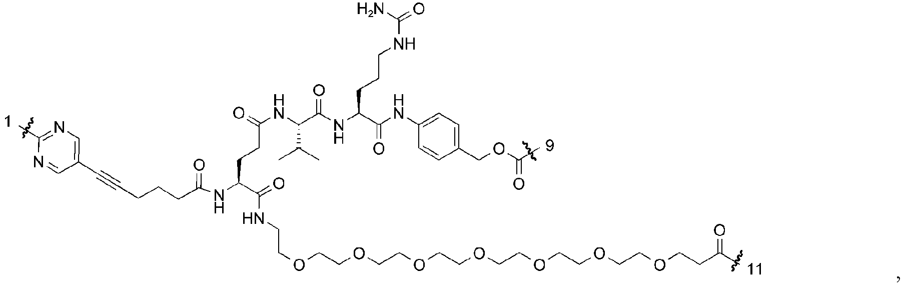 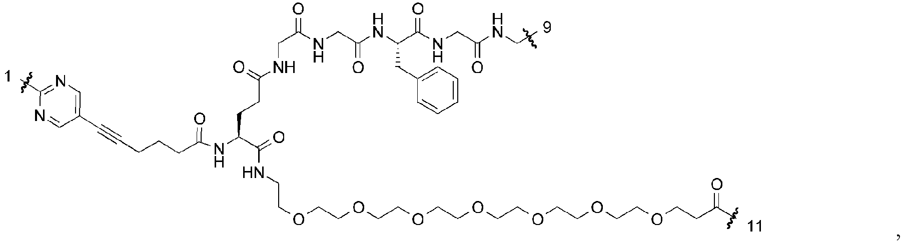 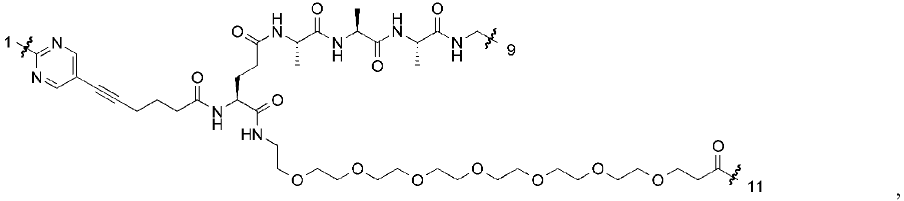 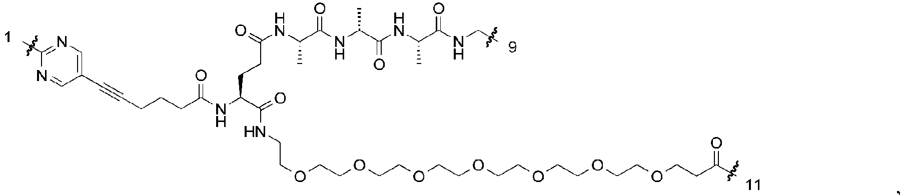 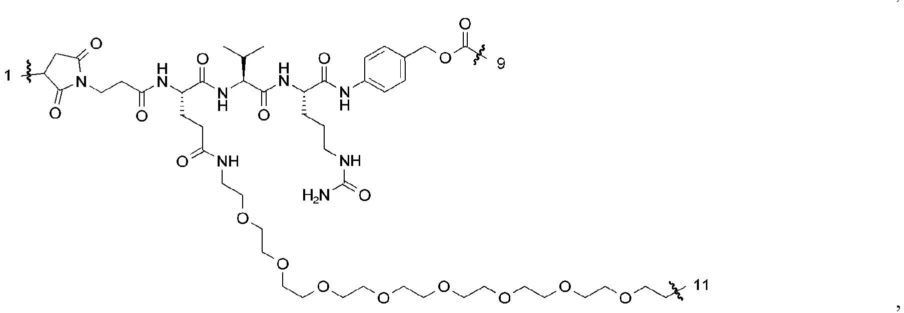 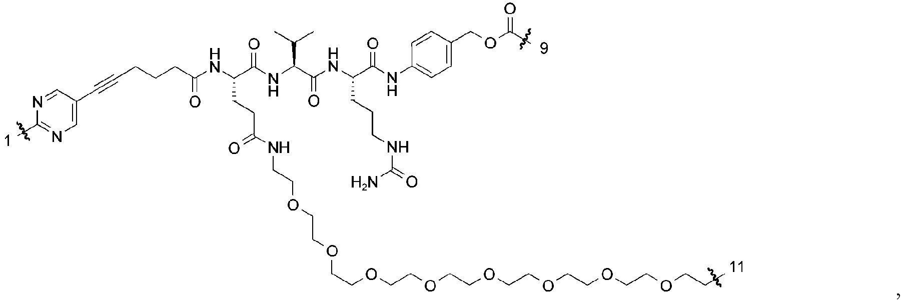 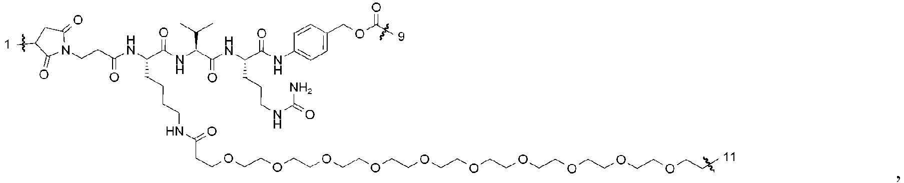 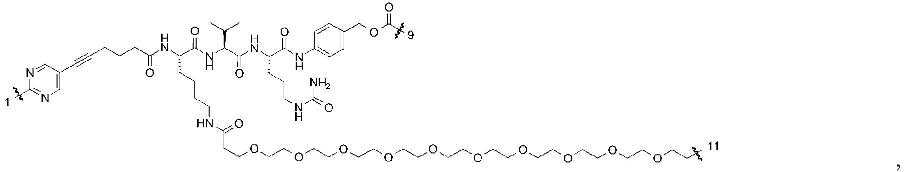 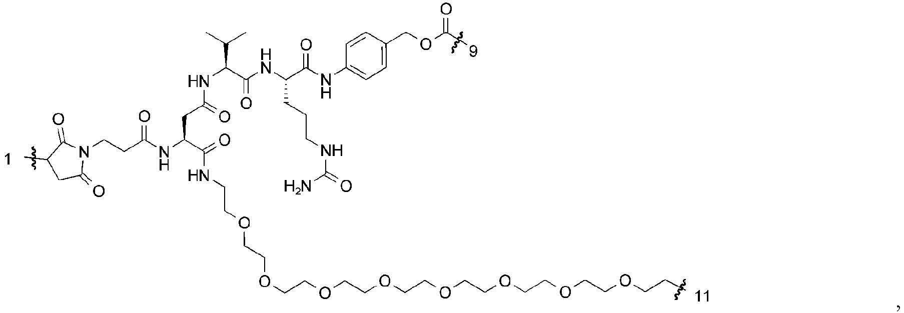 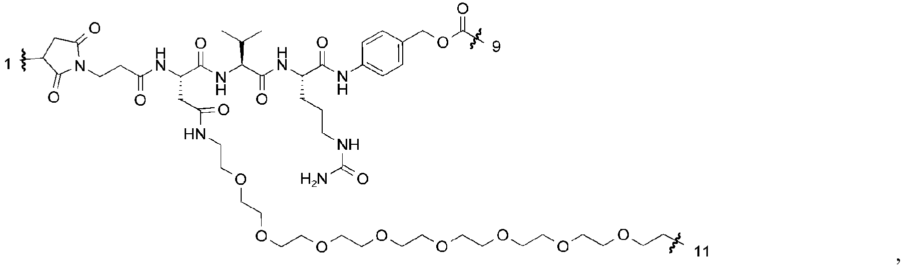 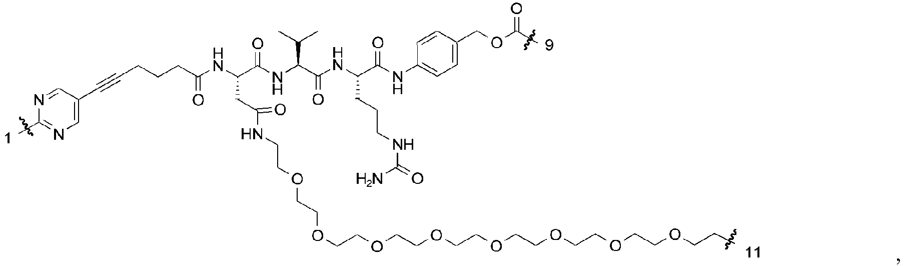 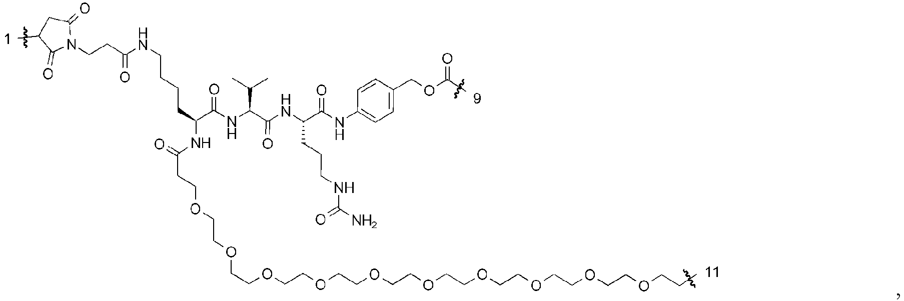 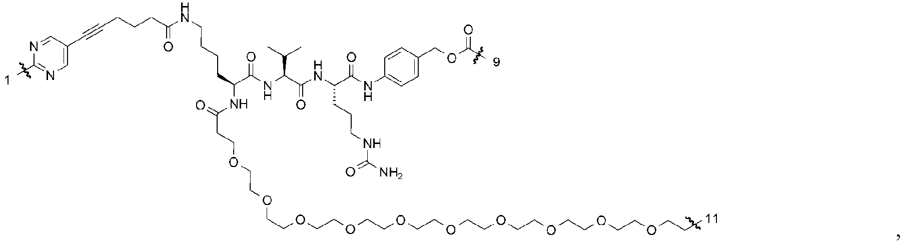 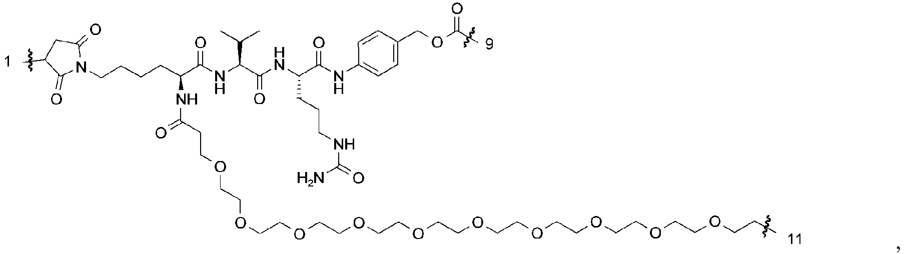 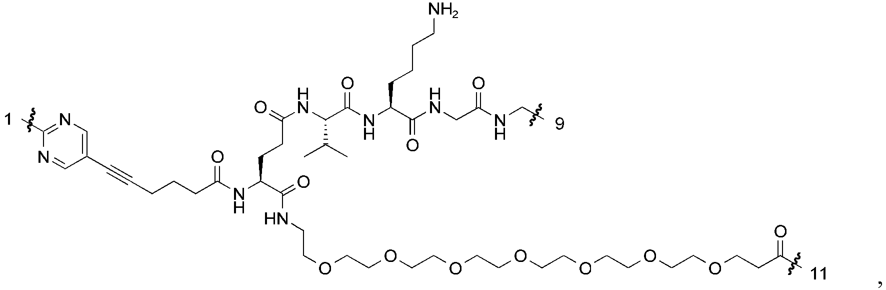 and 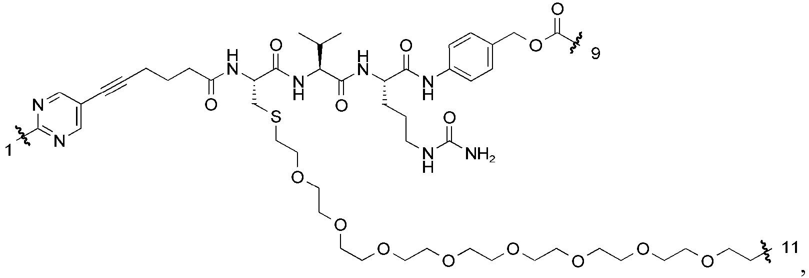
wherein, the position 1 is connected to Ab', the position 9 is connected to D¹, and the position 11 is connected to D².

20. The antibody-drug conjugate according to any one of claims 1 to 19, wherein 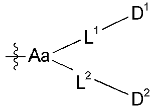 is selected from the group consisting of: 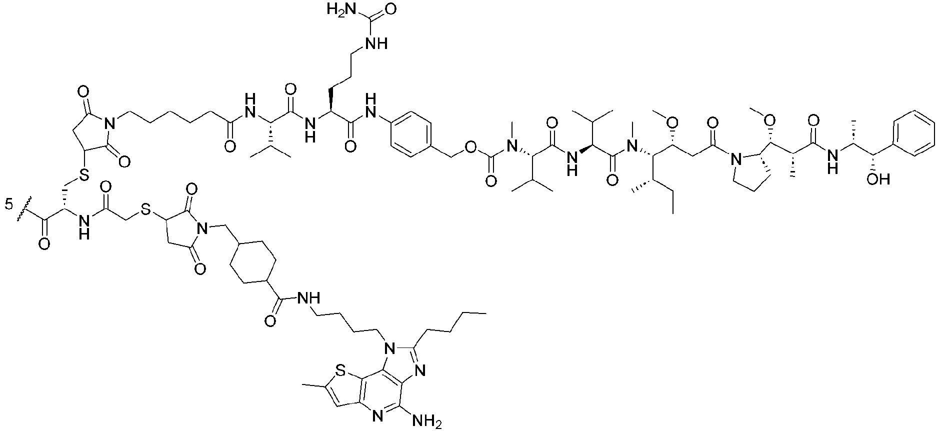 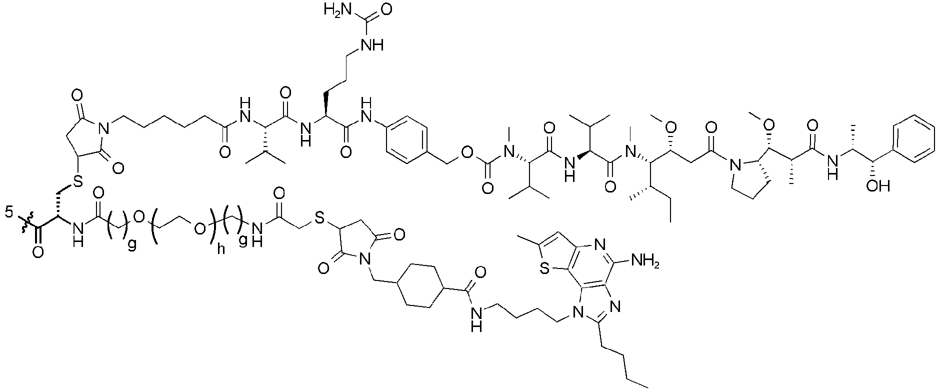 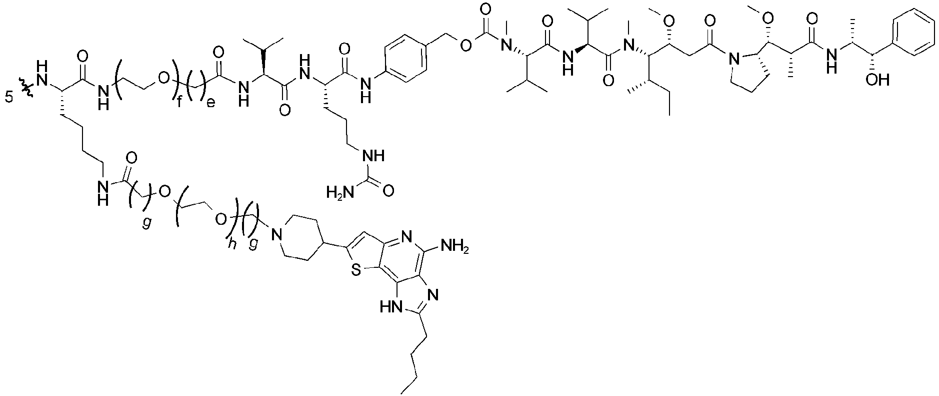 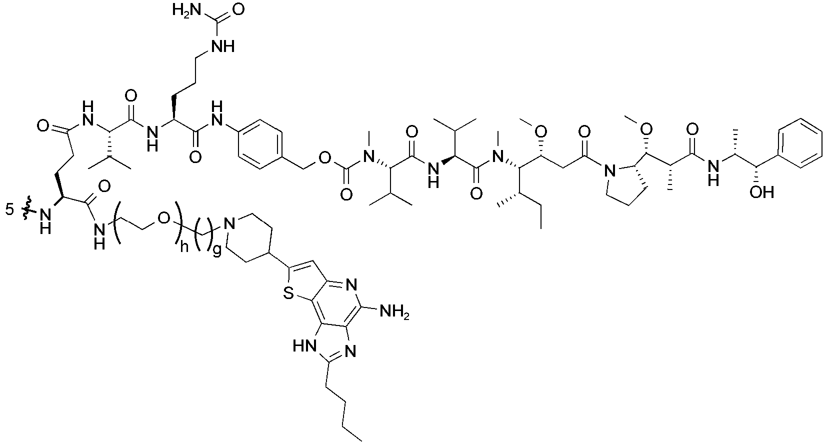 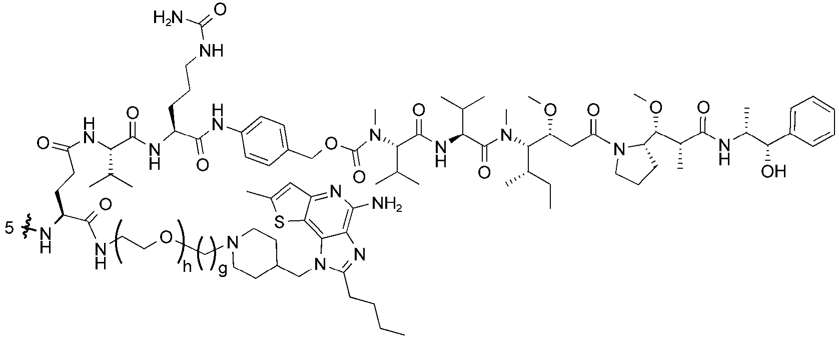 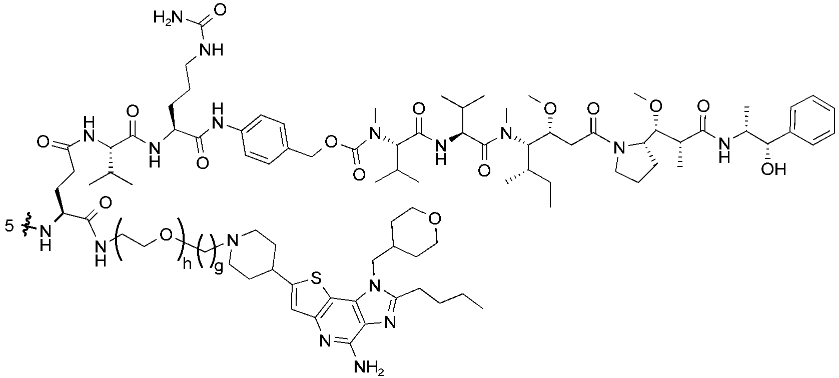 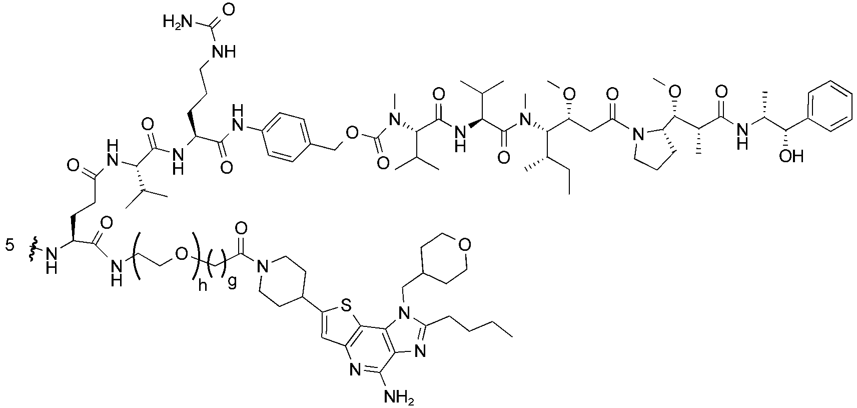 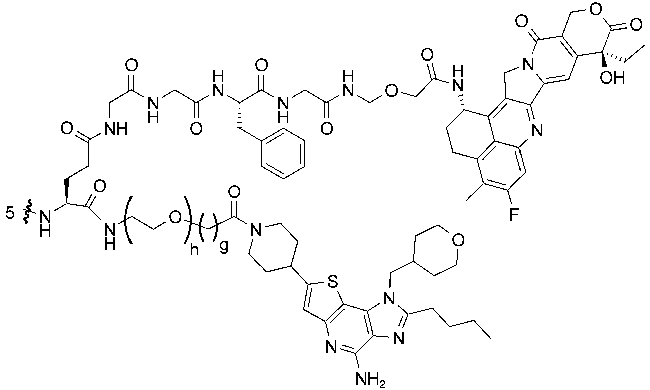 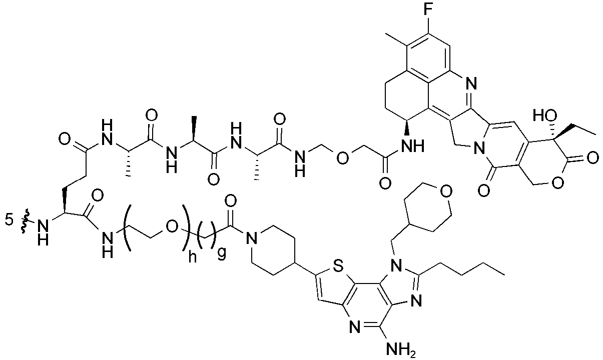 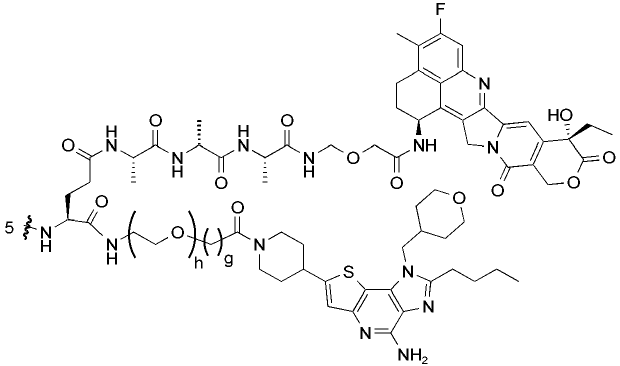 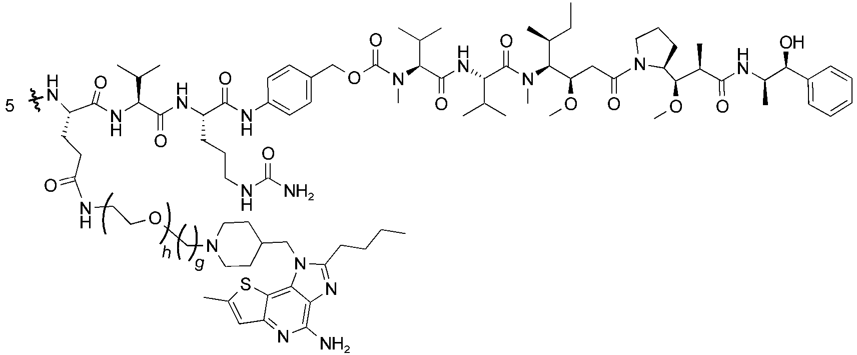 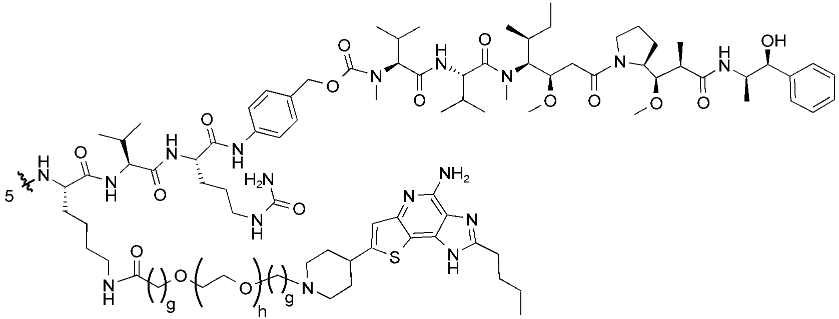 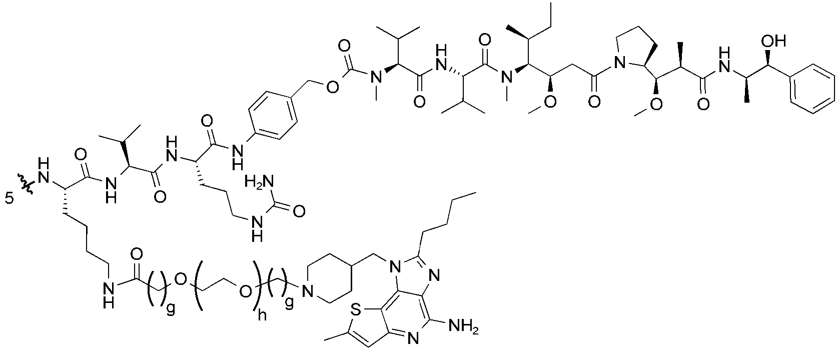 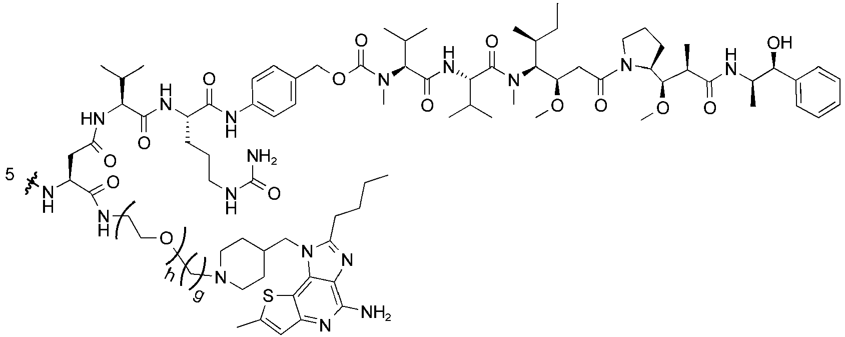 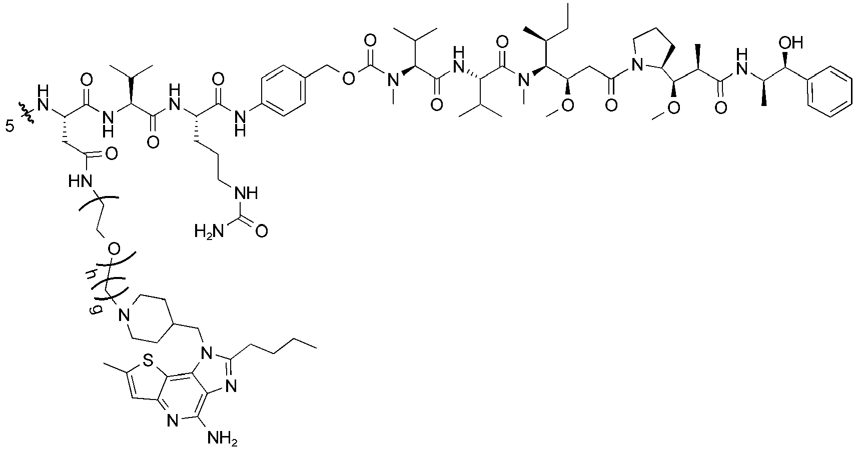 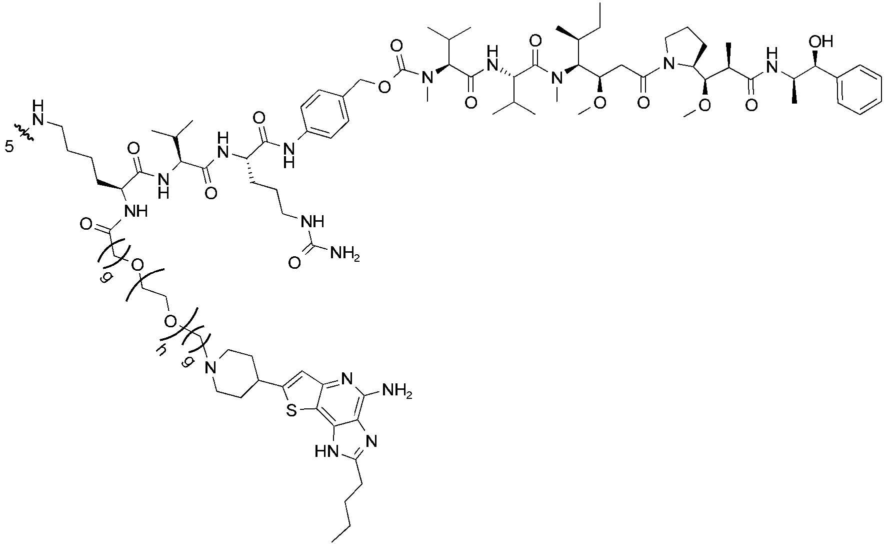 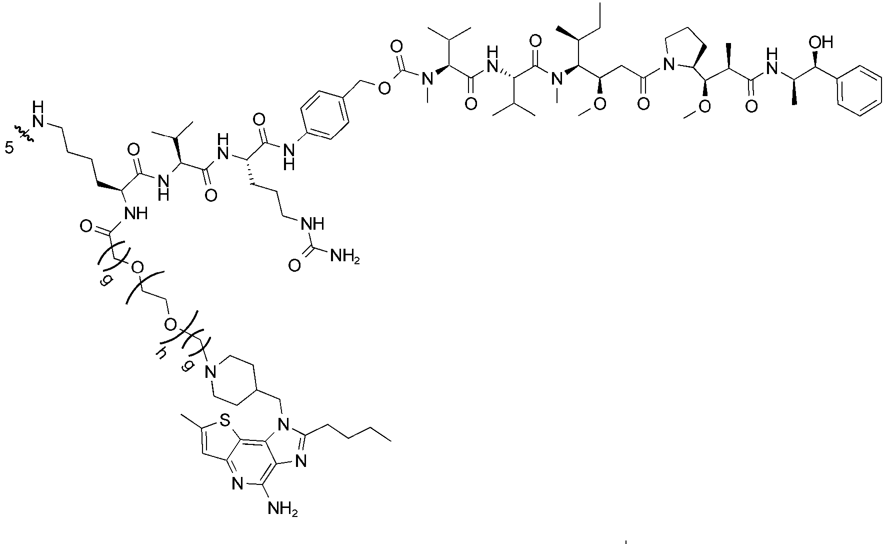 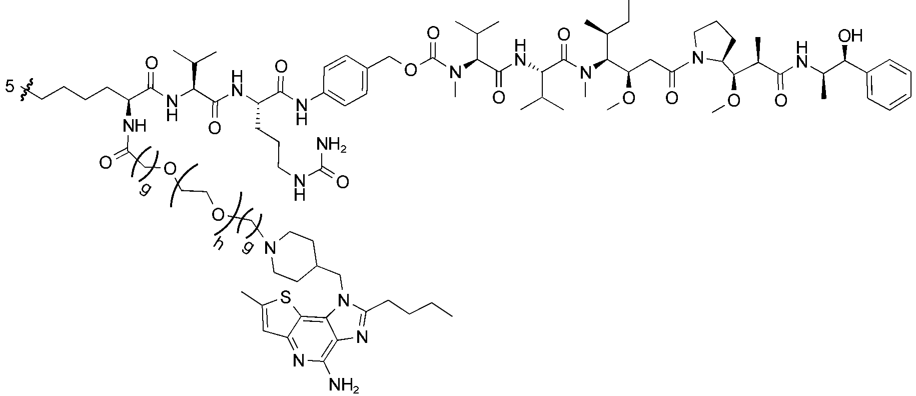 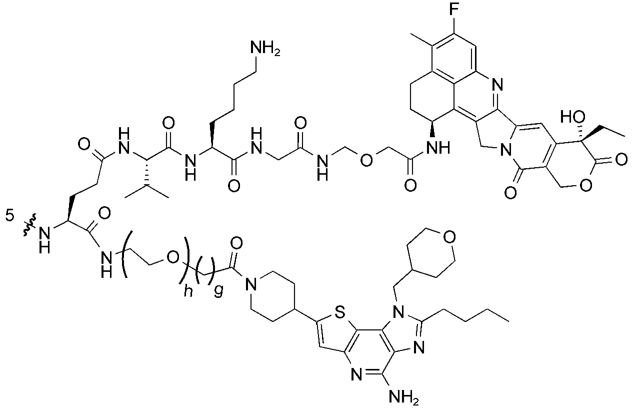 and 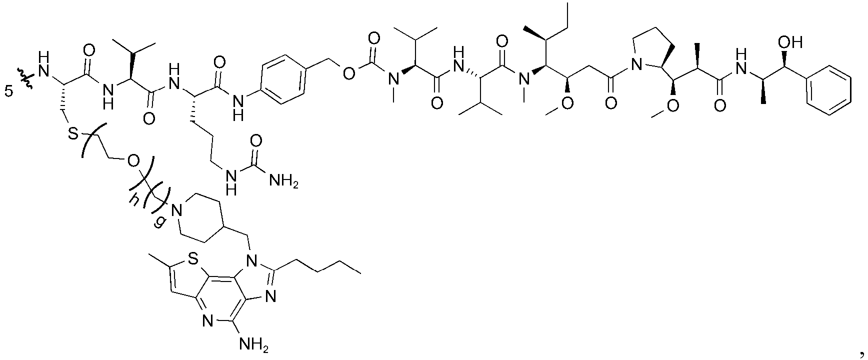
wherein, each g is independently an integer ranging from 0 to 6, and each h is independently an integer ranging from 1 to 10;
preferably, 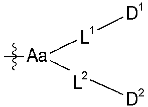
is selected from the group consisting of: 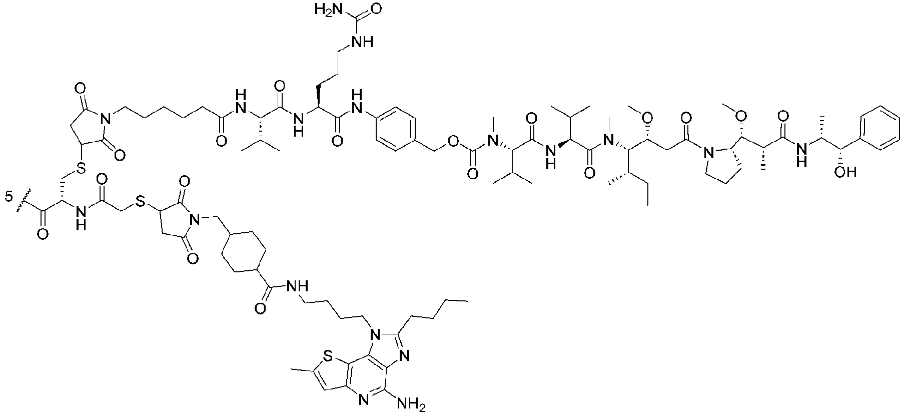 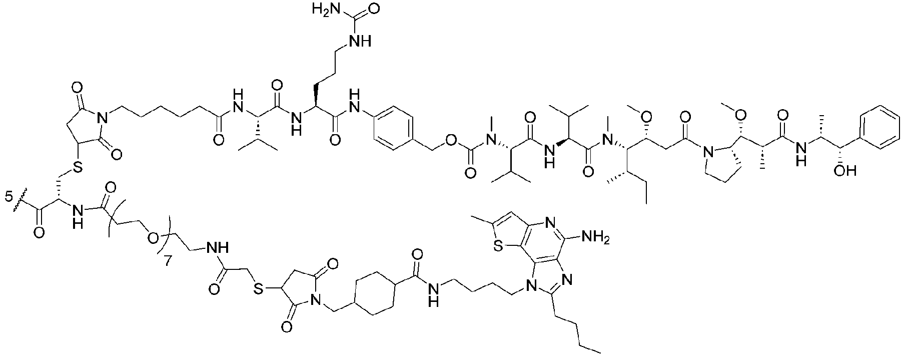 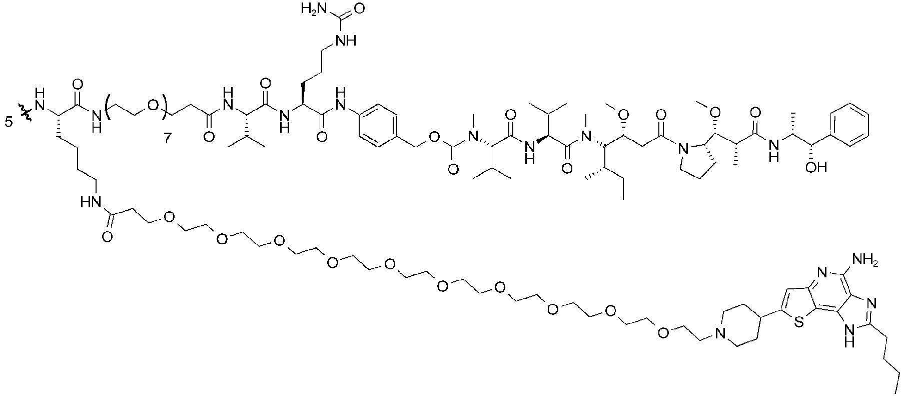 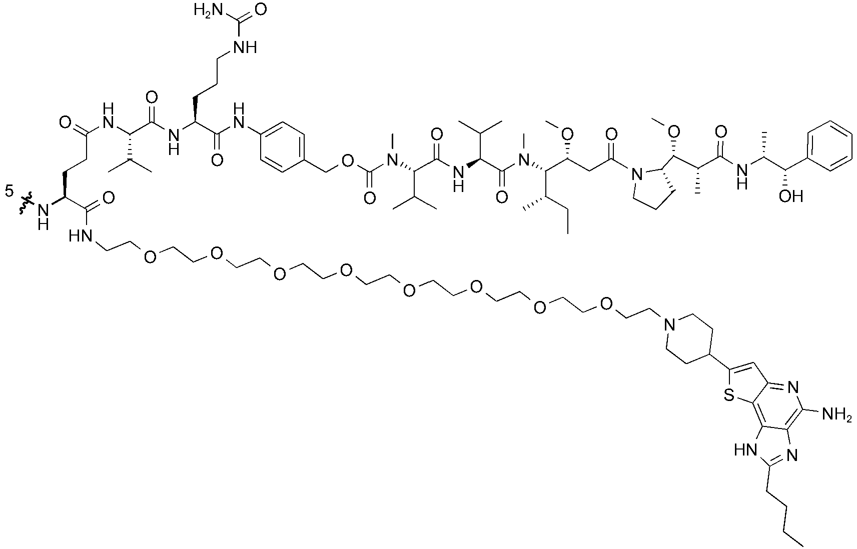 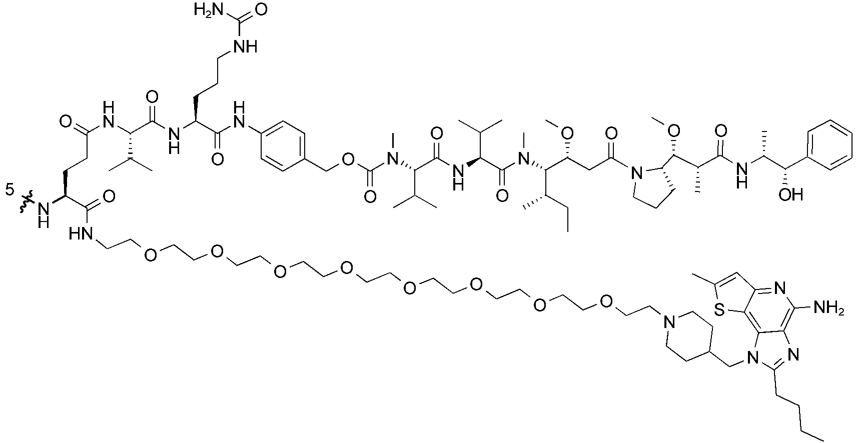 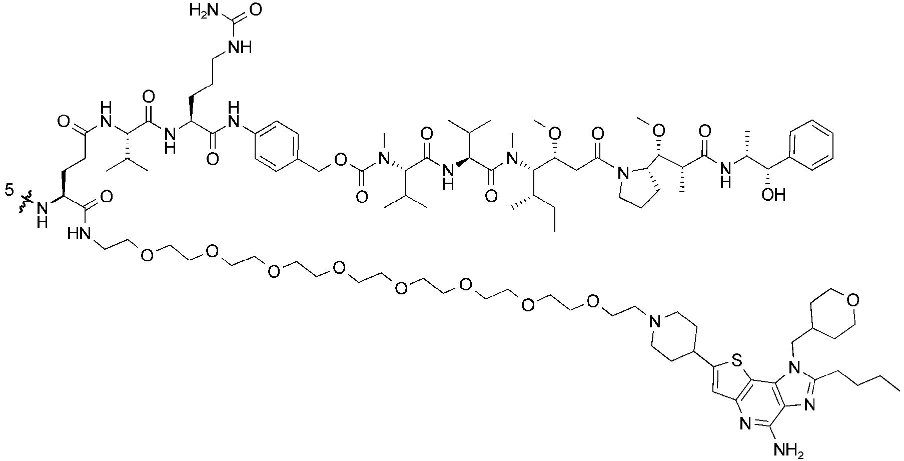 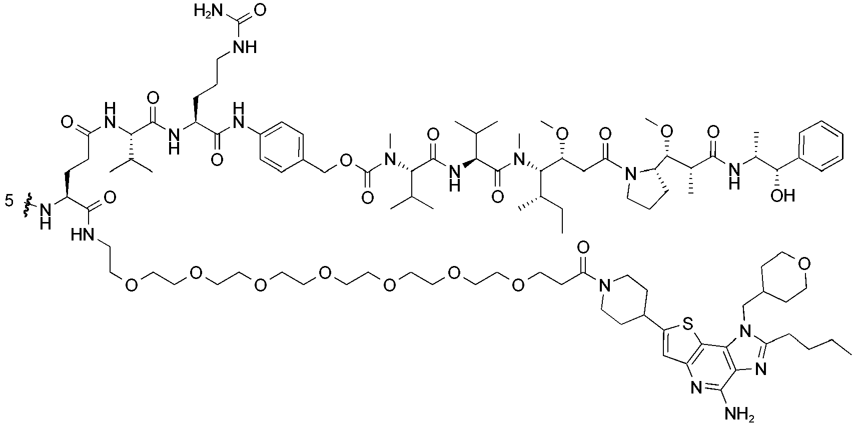 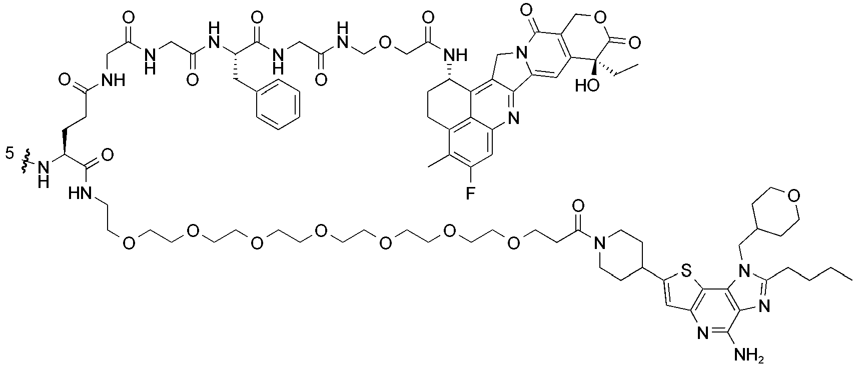 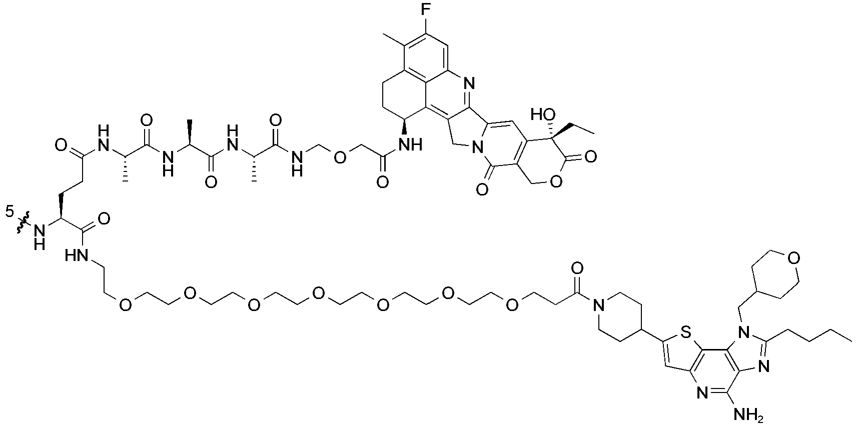 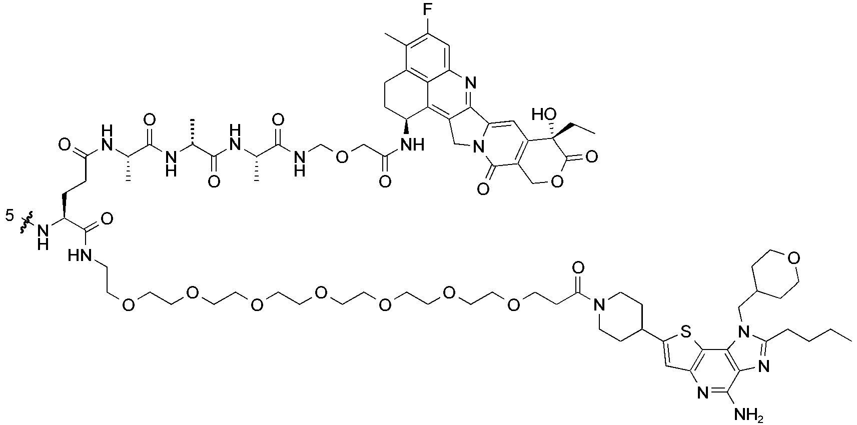 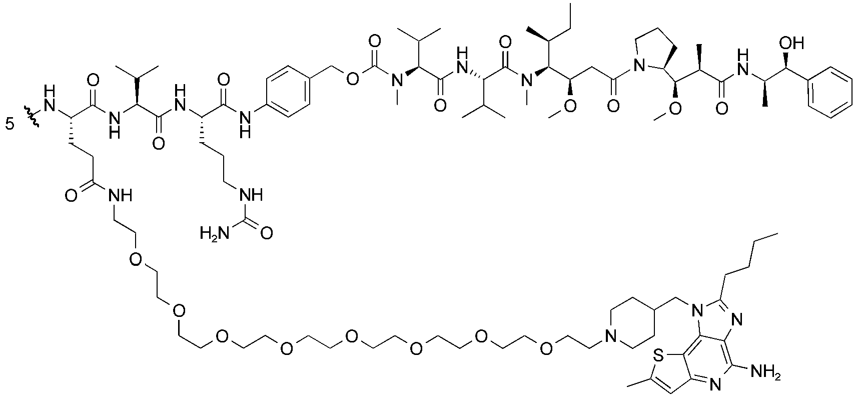 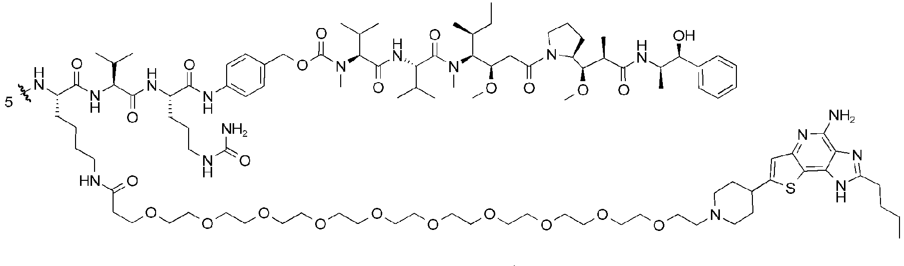 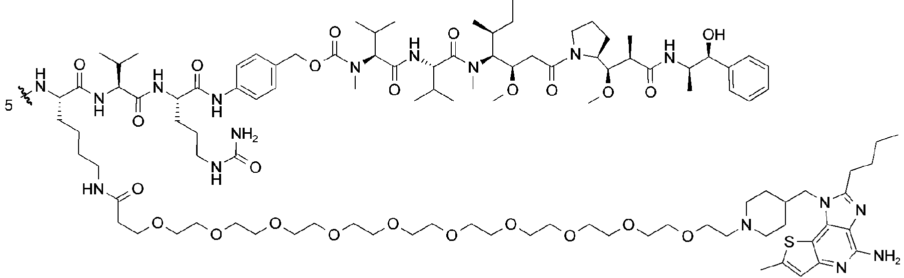 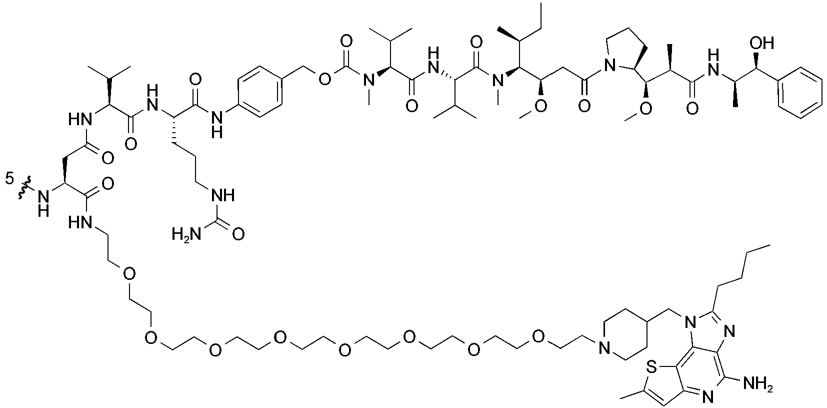 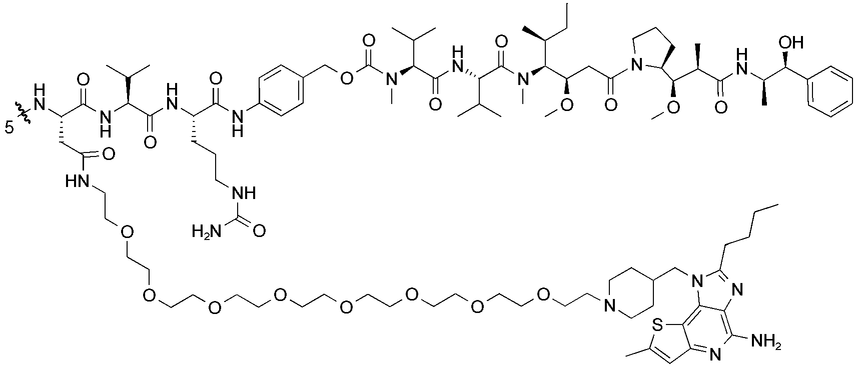 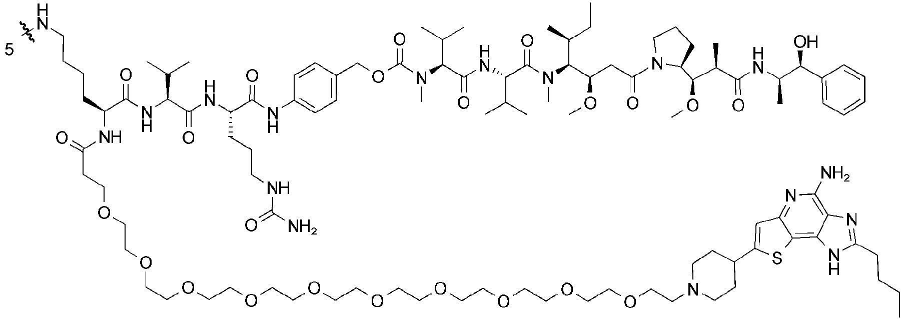 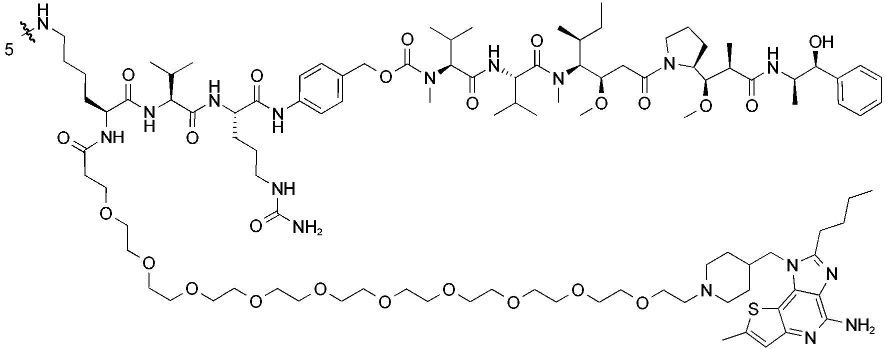 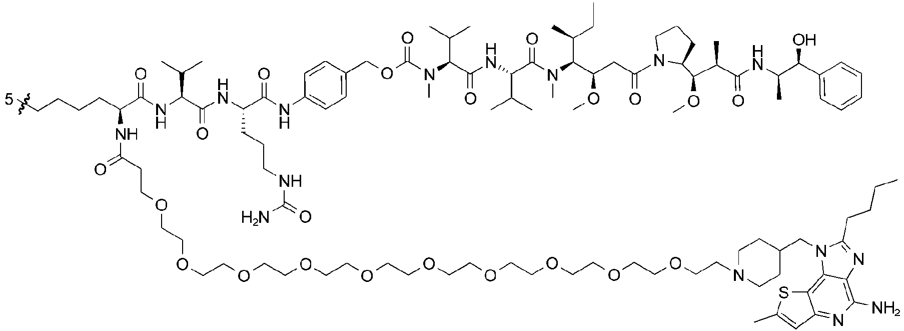 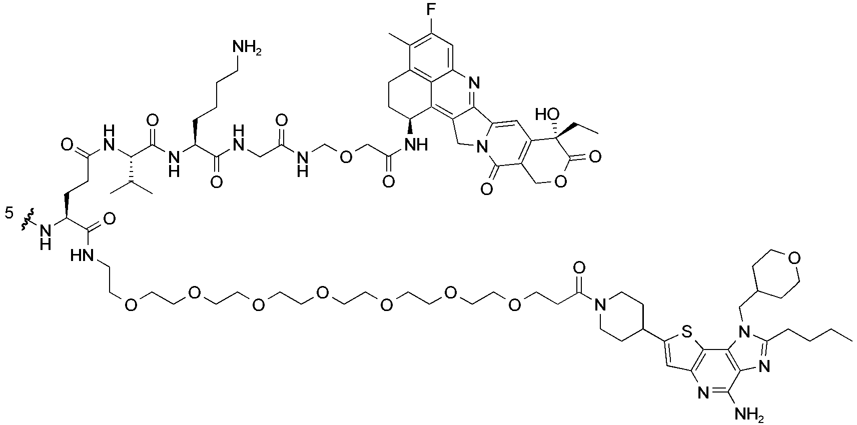 and 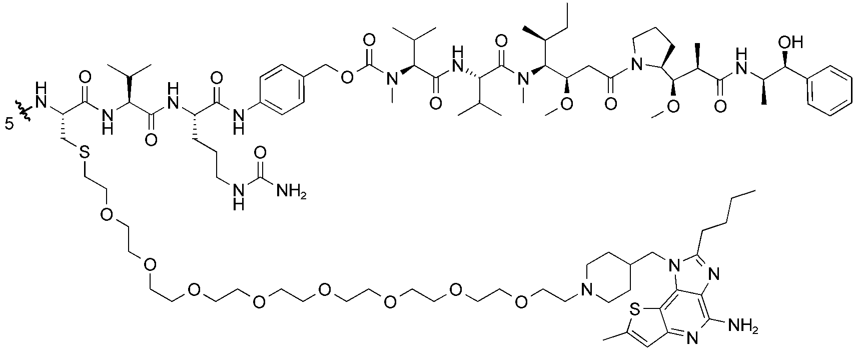
wherein, the position 5 is connected to X.

21. The antibody-drug conjugate according to any one of claims 1 to 20, which has a structure selected from the group consisting of: 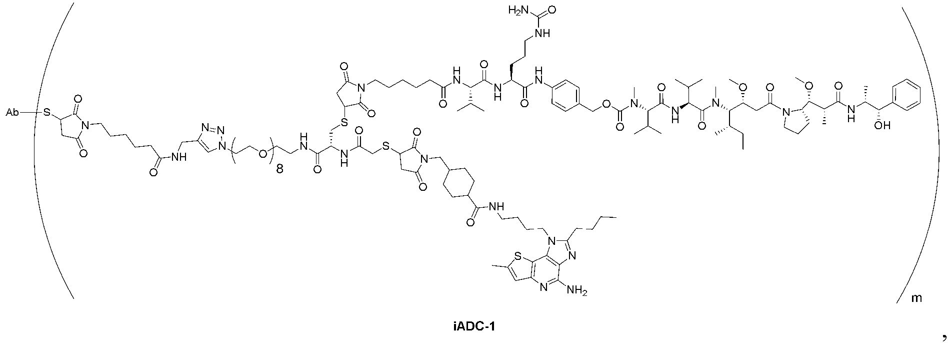 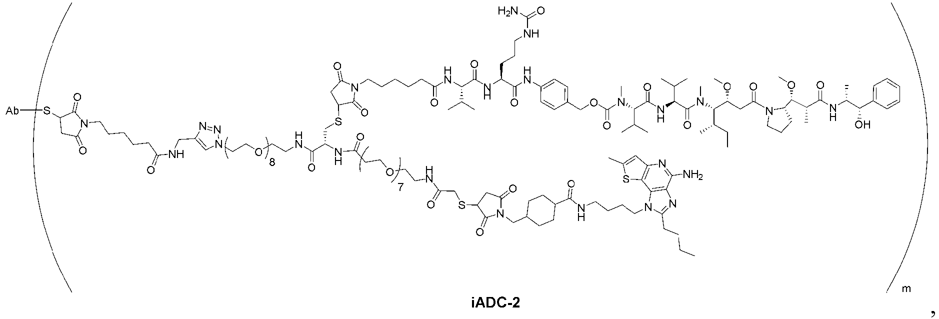 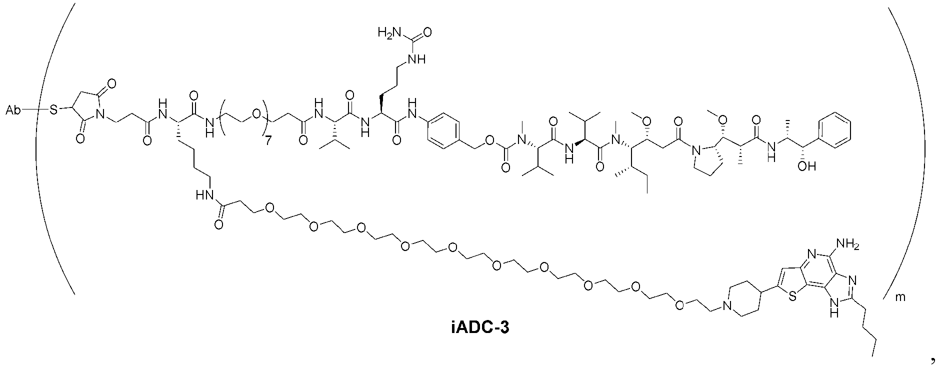 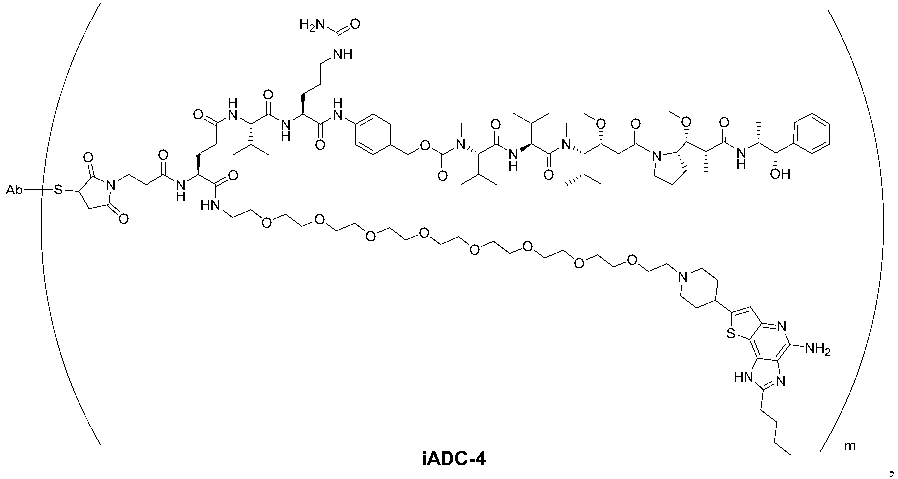 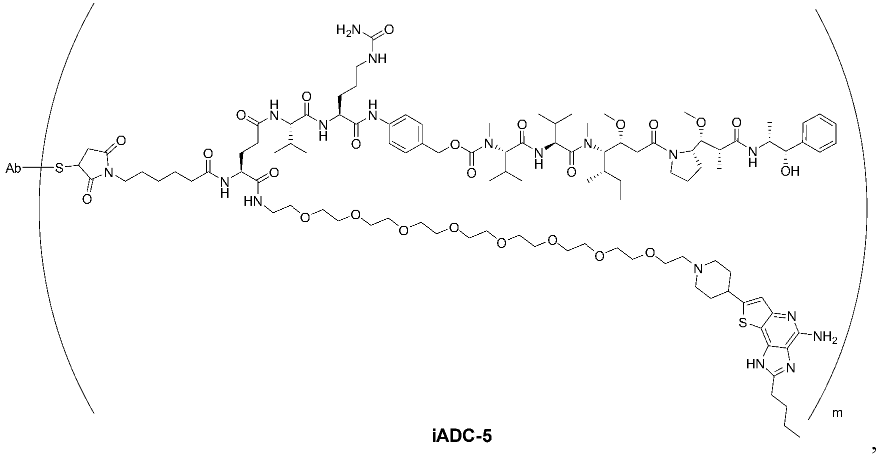 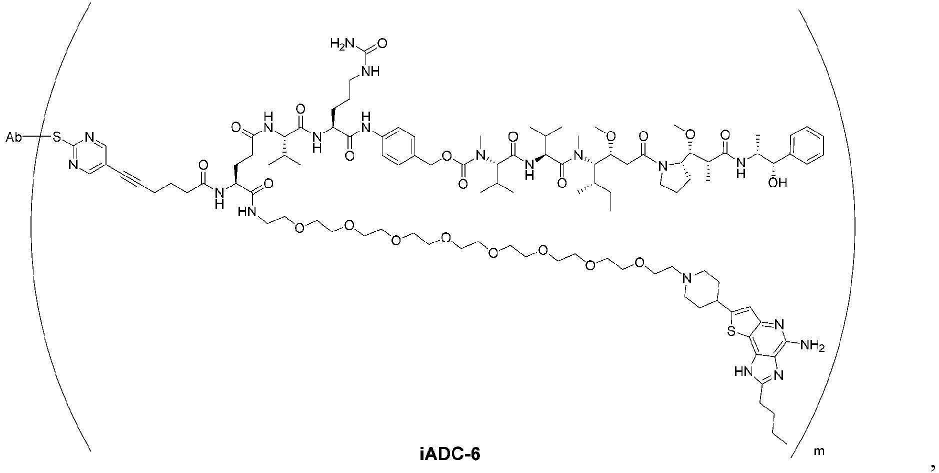 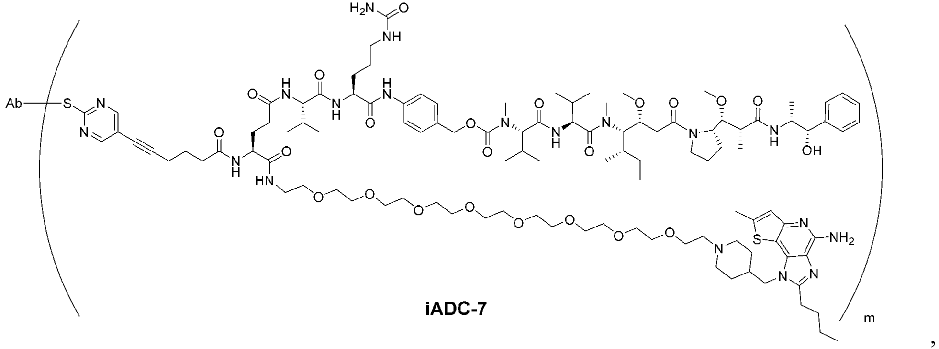 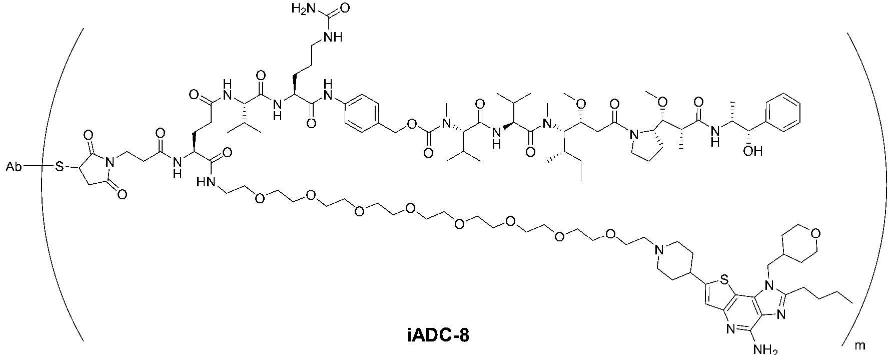 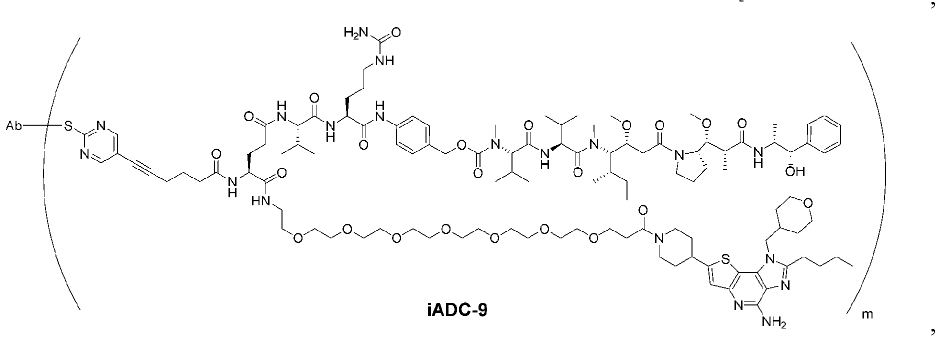 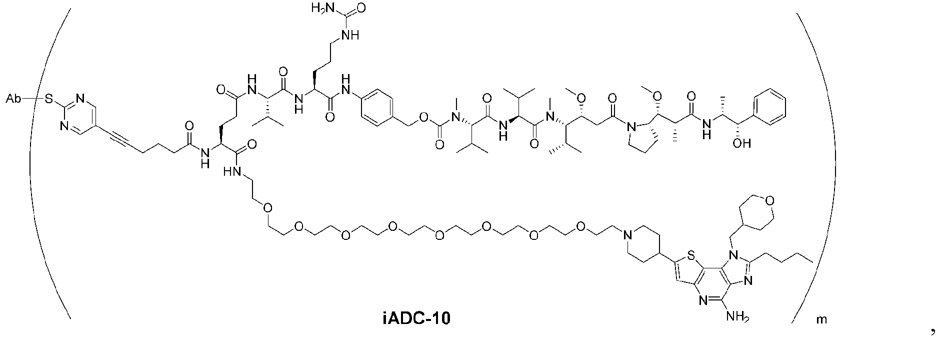 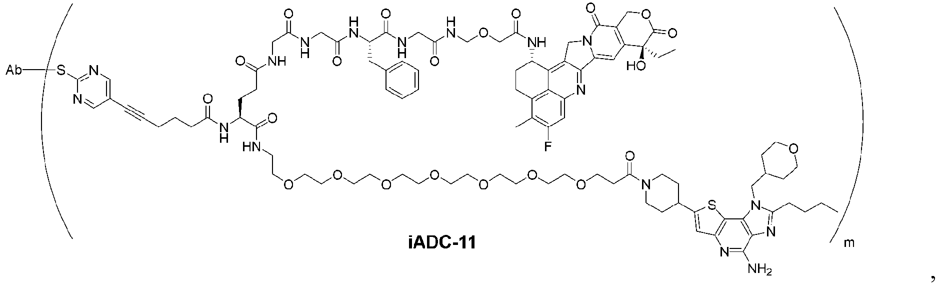 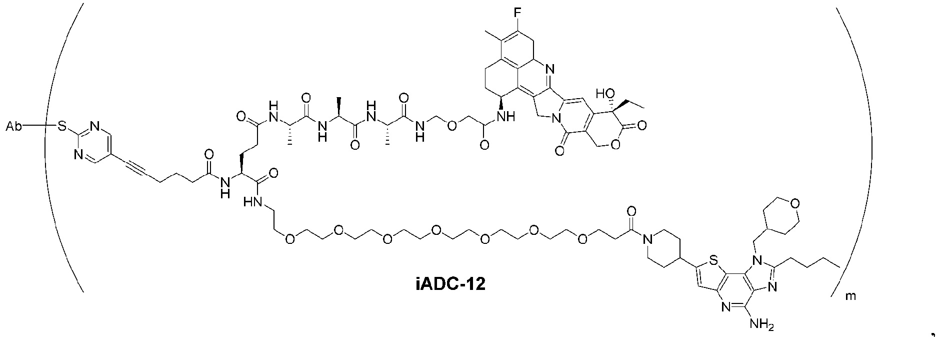 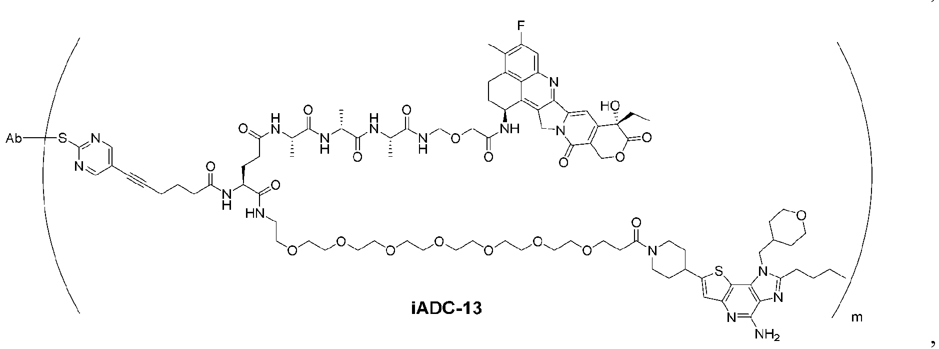 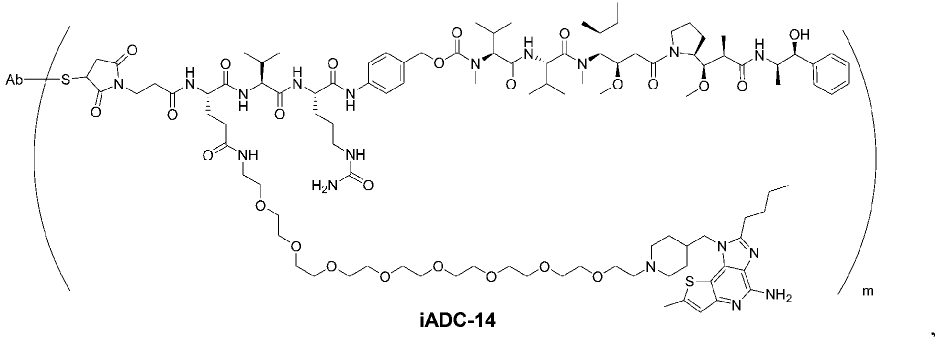 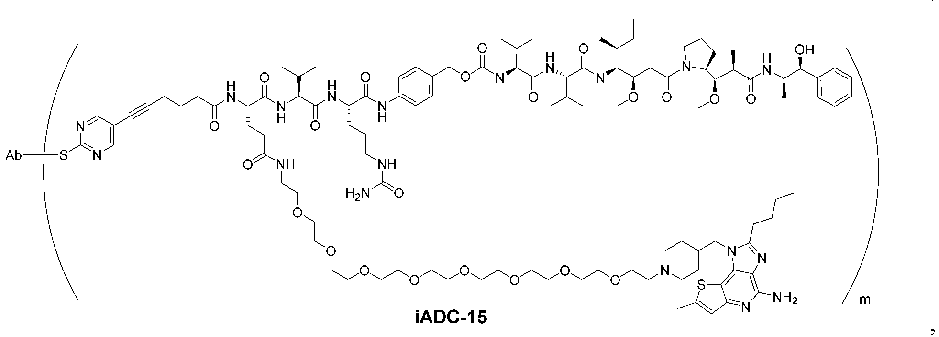 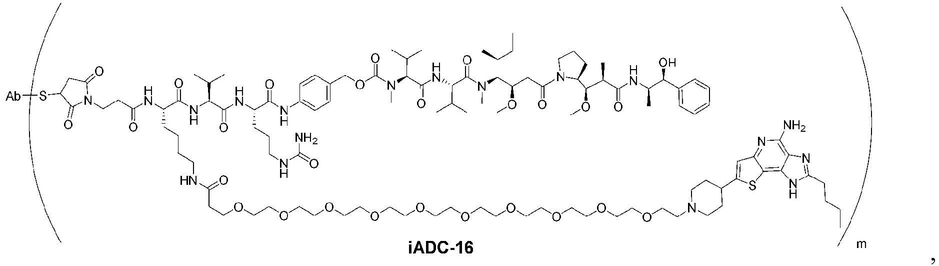 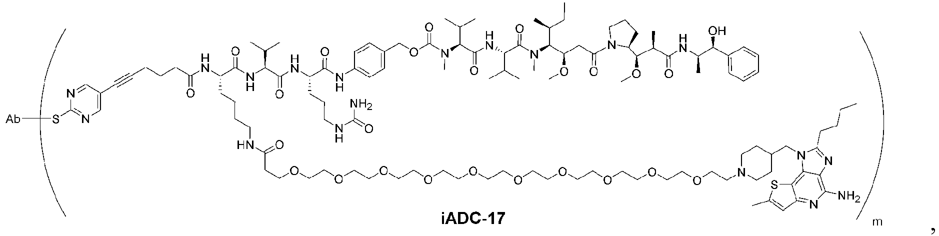 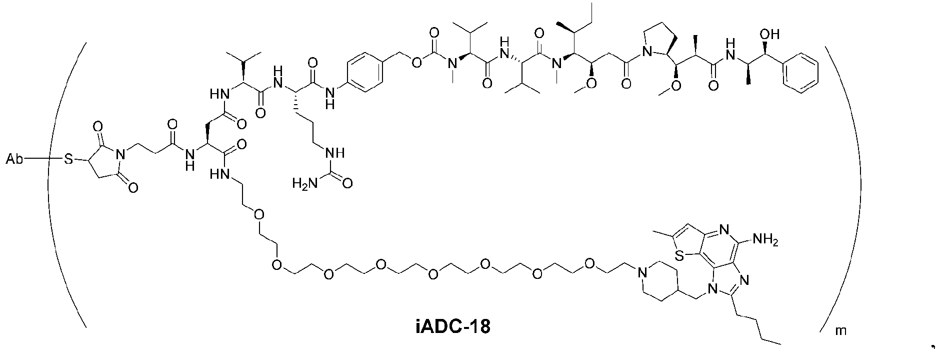 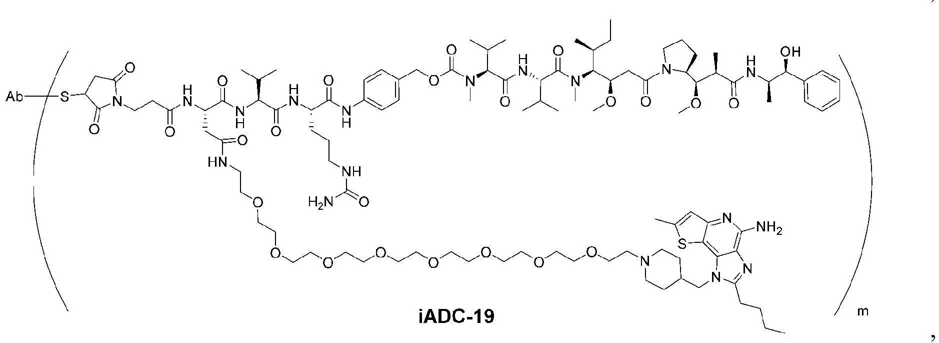 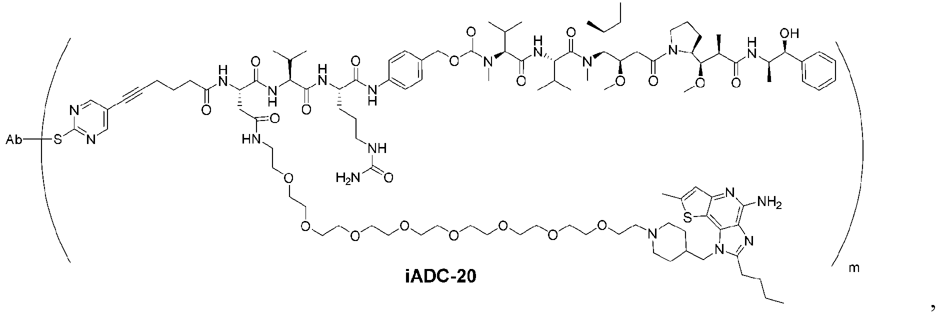 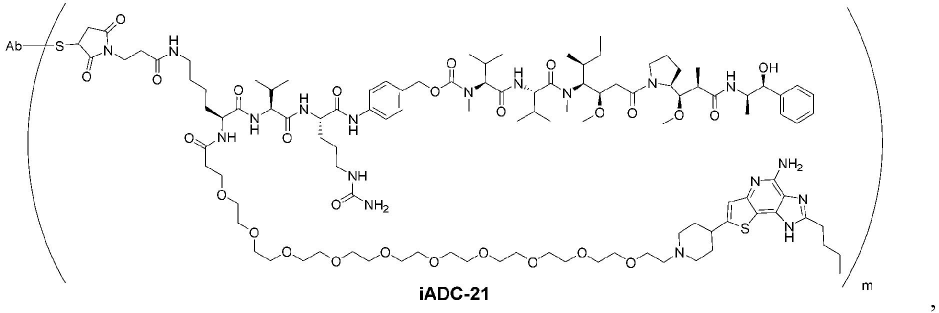 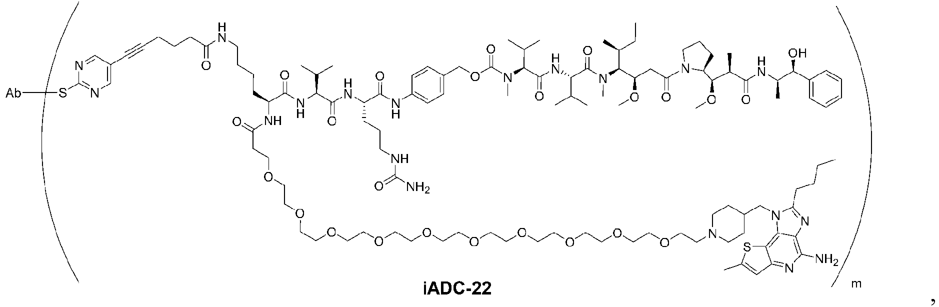 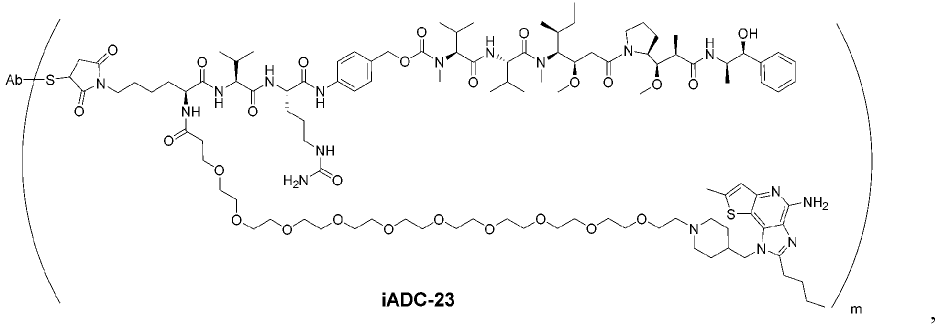 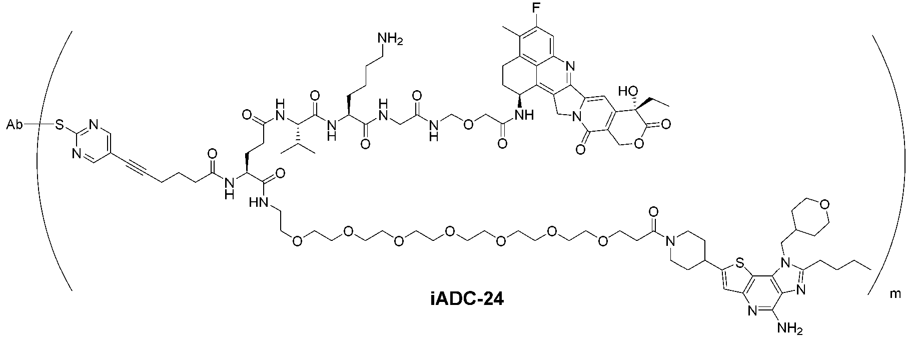 and 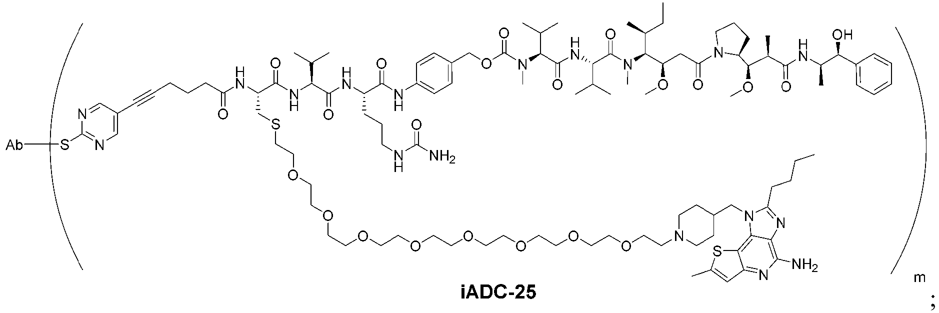
each m independently ranges from 1 to 10, for example, 1, 2, 3, 4, 5, 6, 7, 8, 9 or 10; Ab-(Sis the antibody or antigen-binding fragment thereof as defined in any one of claims 1 to 20;
preferably, Ab-(S- in each antibody-drug conjugate represents an antibody or antigen-binding fragment thereof comprising a VH as set forth in SEQ ID NO: 23 or a VH as set forth in SEQ ID NO: 1 and a VL as set forth in SEQ ID NO: 2, for example, Ab-01 (which comprises a heavy chain as set forth in SEQ ID NO: 22 and a light chain as set forth in SEQ ID NO: 21), for example, Ab-02 (which comprises a heavy chain as set forth in SEQ ID NO: 20 and a light chain as set forth in SEQ ID NO: 21); optionally, the N-terminal glutamine of the heavy chain or heavy chain variable region and/or the light chain or light chain variable region undergoes cyclization to form a pyroglutamic acid or pyroglutamate; and/or, the heavy chain or heavy chain constant region (CH) or a variant thereof lacks C-terminal lysine.

22. A composition, which comprises the antibody-drug conjugate according to any one of claims 1 to 21, wherein the composition has a DAR value of 1.0 to 10.0, preferably 1.0 to 5.0, for example, 1.0 to 1.5, 1.0 to 2.0, 1.0 to 2.5, 1.0 to 3.0, 1.0 to 3.5, 1.0 to 4.0, 1.0 to 4.5, 1.0 to 5.0, 2.0 to 2.5, 2.0 to 3.0, 2.0 to 3.5, 2.0 to 4.0, 2.0 to 4.5, 2.0 to 5.0, 2.5 to 3.0, 2.5 to 3.5, 2.5 to 4.0, 2.5 to 4.5, 2.5 to 5.0, 3.0 to 3.5, 3.0 to 4.0, 3.0 to 4.5, 3.0 to 5.0, 3.5 to 4.0, 3.5 to 4.5, 3.5 to 5.0, 4.0 to 4.5, 4.0 to 5.0, 4.5 to 5.0, for example, about 1.0, about 1.01, about 1.02, about 1.03, about 1.04, about 1.05, about 1.06, about 1.07, about 1.08, about 1.09, about 1.1, about 1.11, about 1.12, about 1.13, about 1.14, about 1.15, about 1.16, about 1.17, about 1.18, about 1.19, about 1.2, about 1.21, about 1.22, about 1.23, about 1.24, about 1.25, about 1.26, about 1.27, about 1.28, about 1.29, about 1.30, about 1.31, about 1.32, about 1.33, about 1.34, about 1.35, about 1.36, about 1.37, about 1.38, about 1.39, about 1.40, about 1.41, about 1.42, about 1.43, about 1.44, about 1.45, about 1.46, about 1.47, about 1.48, about 1.49, about 1.5, about 1.51, about 1.52, about 1.53, about 1.54, about 1.55, about 1.56, about 1.57, about 1.58, about 1.59, about 1.6, about 1.61, about 1.62, about 1.63, about 1.64, about 1.65, about 1.66, about 1.67, about 1.68, about 1.69, about 1.7, about 1.71, about 1.72, about 1.73, about 1.74, about 1.75, about 1.76, about 1.77, about 1.78, about 1.79, about 1.8, about 1.81, about 1.82, about 1.83, about 1.84, about 1.85, about 1.86, about 1.87, about 1.88, about 1.89, about 1.9, about 1.91, about 1.92, about 1.93, about 1.94, about 1.95, about 1.96, about 1.97, about 1.98, about 1.99, about 2.0, about 2.01, about 2.02, about 2.03, about 2.04, about 2.05, about 2.06, about 2.07, about 2.08, about 2.09, about 2.1, about 2.11, about 2.12, about 2.13, about 2.14, about 2.15, about 2.16, about 2.17, about 2.18, about 2.19, about 2.2, about 2.21, about 2.22, about 2.23, about 2.24, about 2.25, about 2.26, about 2.27, about 2.28, about 2.29, about 2.3, about 2.31, about 2.32, about 2.33, about 2.34, about 2.35, about 2.36, about 2.37, about 2.38, about 2.39, about 2.4, about 2.41, about 2.42, about 2.43, about 2.44, about 2.45, about 2.46, about 2.47, about 2.48, about 2.49, about 2.5, about 2.51, about 2.52, about 2.53, about 2.54, about 2.55, about 2.56, about 2.57, about 2.58, about 2.59, about 2.6, about 2.61, about 2.62, about 2.63, about 2.64, about 2.65, about 2.66, about 2.67, about 2.68, about 2.69, about 2.7, about 2.71, about 2.72, about 2.73, about 2.74, about 2.75, about 2.76, about 2.77, about 2.78, about 2.79, about 2.8, about 2.81, about 2.82, about 2.83, about 2.84, about 2.85, about 2.86, about 2.87, about 2.88, about 2.89, about 2.9, about 2.91, about 2.92, about 2.93, about 2.94, about 2.95, about 2.96, about 2.97, about 2.98, about 2.99, about 3.0, about 3.01, about 3.02, about 3.03, about 3.04, about 3.05, about 3.06, about 3.07, about 3.08, about 3.09, about 3.1, about 3.11, about 3.12, about 3.13, about 3.14, about 3.15, about 3.16, about 3.17, about 3.18, about 3.19, about 3.2, about 3.21, about 3.22, about 3.23, about 3.24, about 3.25, about 3.26, about 3.27, about 3.28, about 3.29, about 3.3, about 3.31, about 3.32, about 3.33, about 3.34, about 3.35, about 3.36, about 3.37, about 3.38, about 3.39, about 3.4, about 3.41, about 3.42, about 3.43, about 3.44, about 3.45, about 3.46, about 3.47, about 3.48, about 3.49, about 3.5, about 3.51, about 3.52, about 3.53, about 3.54, about 3.55, about 3.56, about 3.57, about 3.58, about 3.59, about 3.6, about 3.61, about 3.62, about 3.63, about 3.64, about 3.65, about 3.66, about 3.67, about 3.68, about 3.69, about 3.7, about 3.71, about 3.72, about 3.73, about 3.74, about 3.75, about 3.76, about 3.77, about 3.78, about 3.79, about 3.8, about 3.81, about 3.82, about 3.83, about 3.84, about 3.85, about 3.86, about 3.87, about 3.88, about 3.89, about 3.9, about 3.91, about 3.92, about 3.93, about 3.94, about 3.95, about 3.96, about 3.97, about 3.98, about 3.99, about 4.0, about 4.01, about 4.02, about 4.03, about 4.04, about 4.05, about 4.06, about 4.07, about 4.08, about 4.09, about 4.1, about 4.11, about 4.12, about 4.13, about 4.14, about 4.15, about 4.16, about 4.17, about 4.18, about 4.19, about 4.2, about 4.21, about 4.22, about 4.23, about 4.24, about 4.25, about 4.26, about 4.27, about 4.28, about 4.29, about 4.3, about 4.31, about 4.32, about 4.33, about 4.34, about 4.35, about 4.36, about 4.37, about 4.38, about 4.39, about 4.4, about 4.41, about 4.42, about 4.43, about 4.44, about 4.45, about 4.46, about 4.47, about 4.48, about 4.49, about 4.5, about 4.51, about 4.52, about 4.53, about 4.54, about 4.55, about 4.56, about 4.57, about 4.58, about 4.59, about 4.6, about 4.61, about 4.62, about 4.63, about 4.64, about 4.65, about 4.66, about 4.67, about 4.68, about 4.69, about 4.7, about 4.71, about 4.72, about 4.73, about 4.74, about 4.75, about 4.76, about 4.77, about 4.78, about 4.79, about 4.8, about 4.81, about 4.82, about 4.83, about 4.84, about 4.85, about 4.86, about 4.87, about 4.88, about 4.89, about 4.9, about 4.91, about 4.92, about 4.93, about 4.94, about 4.95, about 4.96, about 4.97, about 4.98, about 4.99, or about 5.0.

23. A compound represented by Formula (II) or a pharmaceutically acceptable salt, ester, stereoisomer, tautomer, polymorph, solvate, N-oxide, isotope-labeled compound, metabolite or prodrug thereof: 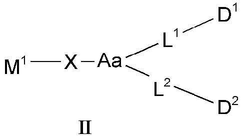 wherein,
X, Aa, L¹, L², D¹, and D² are as defined in any one of claims 1 to 21;
M¹ is a precursor of the linking site that is attached to an antibody or antigen-binding fragment thereof;
preferably, the antibody or antigen-binding fragment thereof is as defined in any one of claims 1 to 21;
preferably, M¹ is selected from the group consisting of the following structures: 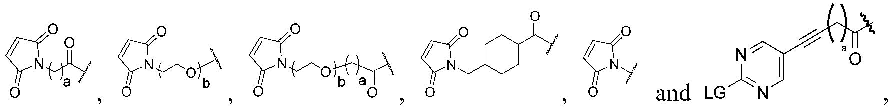
more preferably, M¹ is selected from the group consisting of the following structures: 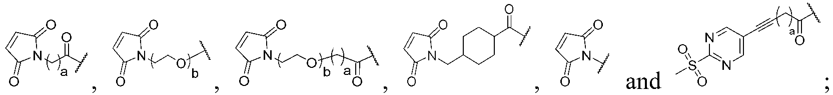
more preferably, M¹ is selected from the group consisting of the following structures: 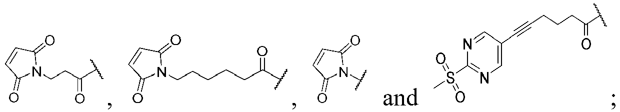
wherein, each a is independently an integer ranging from 0 to 6, and b is an integer ranging from 1 to 10;
LG represents a leaving group,
preferably, each LG is independently selected from the group consisting of halogen (e.g., F, Cl, Br, I), halogenated C₁₋₆ alkyl, C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, hydroxyl, sulfhydryl group, amino, nitro, azido, cyano, alkenyl, alkynyl, and alkynyl-containing structural fragment, wherein the halogenated C₁₋₆ alkyl, halogenated C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, alkenyl, alkynyl, and alkynyl-containing structural fragment are optionally substituted with one or more suitable substituents;
preferably, LG is independently selected from the group consisting of halogen (e.g., F, Cl, Br, I), halogenated C₁₋₆ alkyl, C₁₋₆ alkylsulfonyl, halogenated C₁₋₆ alkylsulfonyl, halogenated sulfonyl, C₁₋₆ alkylsulfonate group, halogenated C₁₋₆ alkylsulfonate group, C₁₋₆ alkylsulfinate group, C₁₋₆ alkylsulfoxide group, halogenated phenoxy, hydroxyl, sulfhydryl group, amino, nitro, azido, cyano, alkenyl, alkynyl, and alkynyl-containing structural fragment;
more preferably, LG is C₁₋₆ alkylsulfonyl, such as methylsulfonyl.

24. The compound or the pharmaceutically acceptable salt, ester, stereoisomer, tautomer, polymorph, solvate, N-oxide, isotope-labeled compound, metabolite or prodrug thereof according to claim 23, wherein:
M¹ is 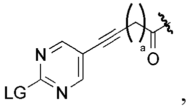 preferably 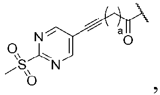 more preferably 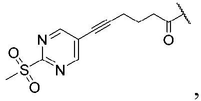 a is an integer ranging from 0 to 6;
L² is 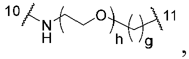 preferably 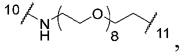 wherein g is an integer ranging from 0 to 6, h is an integer ranging from 1 to 10, and the position 10 of L² is connected to Aa, and the position 11 is connected to D²;
D² is

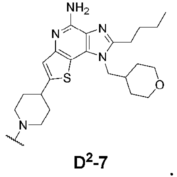

25. The compound or the pharmaceutically acceptable salt, ester, stereoisomer, tautomer, polymorph, solvate, N-oxide, isotope-labeled compound, metabolite or prodrug thereof according to claim 23 or 24, wherein 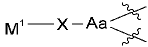 is selected from 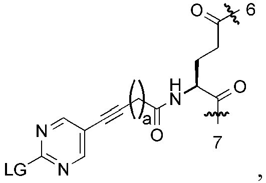 preferably 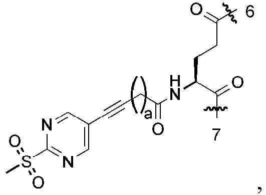 more preferably 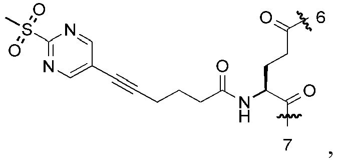 wherein, a is an integer ranging from 0 to 6; the position 6 is connected to L¹, and the position 7 is connected to L².

26. The compound or the pharmaceutically acceptable salt, ester, stereoisomer, tautomer, polymorph, solvate, N-oxide, isotope-labeled compound, metabolite or prodrug thereof according to any one of claims 23 to 25, wherein 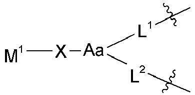 is selected from 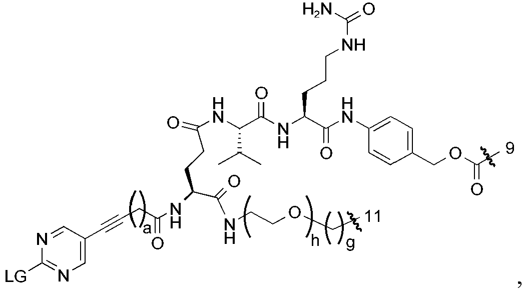
preferably 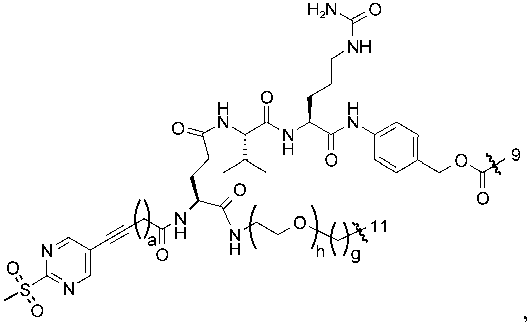
more preferably 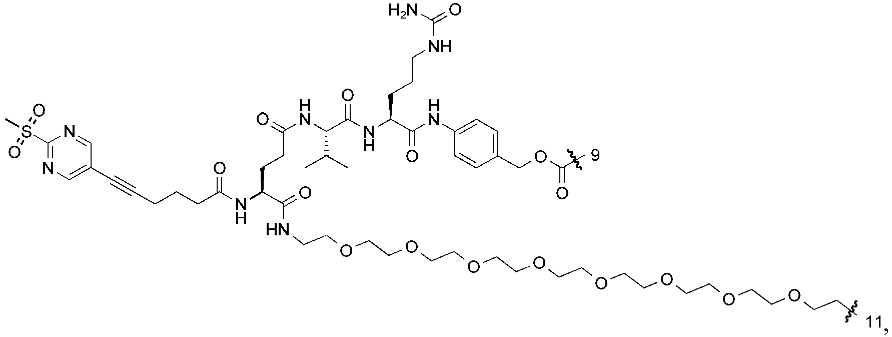
wherein, a is an integer ranging from 0 to 6, g is an integer ranging from 0 to 6, and h is an integer ranging from 1 to 10; the position 9 is connected to D¹, and the position 11 is connected to D².

27. The compound or the pharmaceutically acceptable salt, ester, stereoisomer, tautomer, polymorph, solvate, N-oxide, isotope-labeled compound, metabolite or prodrug thereof according to any one of claims 23 to 26, wherein the compound is selected from the group consisting of the following structures: 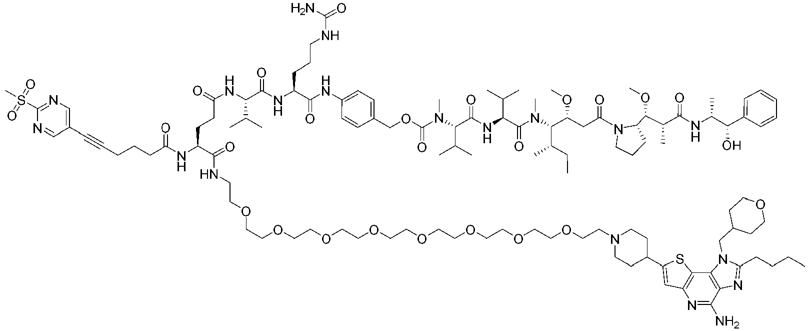 and

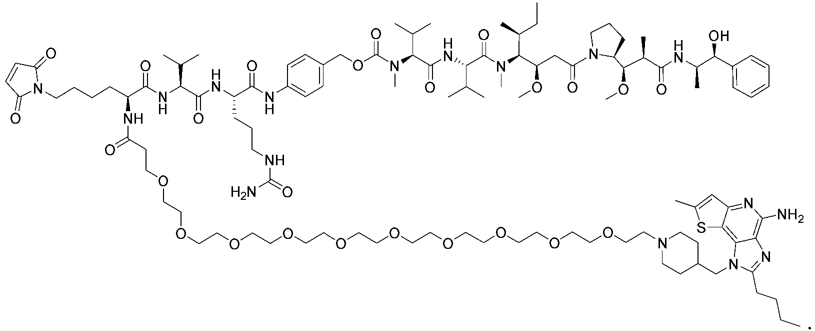

28. An antibody or antigen-binding fragment thereof that specifically binds to B7H3, wherein the antibody or antigen-binding fragment thereof comprises:
(1) the following heavy chain variable region (VH) and/or light chain variable region (VL):
a heavy chain variable region (VH) comprising the following 3 CDRs: a CDR-H1 having a sequence as set forth in SEQ ID NO: 11 or a variant thereof, a CDR-H2 having a sequence as set forth in SEQ ID NO: 12 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(2) the following heavy chain variable region (VH) and/or light chain variable region (VL):
a heavy chain variable region (VH) comprising the following 3 CDRs: a CDR-H1 having a sequence as set forth in SEQ ID NO: 14 or a variant thereof, a CDR-H2 having a sequence as set forth in SEQ ID NO: 15 or a variant thereof, and a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof;
(3) the following heavy chain variable region (VH) and/or light chain variable region (VL):
a heavy chain variable region (VH) comprising the following 3 CDRs: a CDR-H1 having a sequence as set forth in SEQ ID NO: 8 or a variant thereof, a CDR-H2 having a sequence as set forth in SEQ ID NO: 9 or a variant thereof, a CDR-H3 having a sequence as set forth in SEQ ID NO: 10 or a variant thereof; and/or, a light chain variable region (VL) comprising the following 3 CDRs: a CDR-L1 having a sequence as set forth in SEQ ID NO: 3 or a variant thereof, a CDR-L2 having a sequence as set forth in SEQ ID NO: 4 or a variant thereof, and a CDR-L3 having a sequence as set forth in SEQ ID NO: 5 or a variant thereof; or
(4) the following heavy chain variable region (VH) and/or light chain variable region (VL):
a heavy chain variable region (VH) comprising the following 3 CDRs: a CDR-H1 having a sequence as set forth in SEQ ID NO: 16 or a variant thereof, a CDR-H2 having a sequence as set forth in SEQ ID NO: 17 or a variant thereof, a CDR-H3 having a sequence as set forth in SEQ ID NO: 13 or a variant thereof; and/or, a light chain variable region (VL) comprising the following 3 CDRs: a CDR-L1 having a sequence as set forth in SEQ ID NO: 6 or a variant thereof, a CDR-L2 having a sequence as set forth in SEQ ID NO: 7 or a variant thereof, and a CDR-L3 having a sequence as set forth in SEQ ID NO: 5 or a variant thereof.
preferably, the antibody or antigen-binding fragment thereof comprises: a VH as set forth in SEQ ID NO: 23 or 1 or a variant thereof, and/or, a VL as set forth in SEQ ID NO: 2 or a variant thereof;
preferably, the antibody or antigen-binding fragment thereof comprises: a heavy chain comprising a VH as set forth in SEQ ID NO: 23 or 1 or a variant thereof and a heavy chain constant region (CH) as set forth in SEQ ID NO: 18 or a variant thereof, and/or, a light chain comprising a VL as set forth in SEQ ID NO: 2 or a variant thereof and a light chain constant region (CL) as set forth in SEQ ID NO: 19 or a variant thereof;
preferably, the amino acid sequence of the heavy chain of the antibody or antigen-binding fragment thereof is set forth in SEQ ID NO: 22 or 20, and the amino acid sequence of the light chain of the antibody or antigen-binding fragment thereof is set forth in SEQ ID NO: 21;
optionally, the N-terminal glutamine of the heavy chain or heavy chain variable region and/or the light chain or light chain variable region undergoes cyclization to form a pyroglutamic acid or pyroglutamate; and/or, the heavy chain or heavy chain constant region (CH) or variant thereof lacks a C-terminal lysine;
preferably, the variant has a sequence identity of at least 80%, at least 85%, at least 90%, at least 91%, at least 92%, at least 93%, at least 94%, at least 95%, at least 96%, at least 97%, at least 98%, at least 99%, or 100% as compared with the sequence from which it is derived, or has a substitution, deletion, or addition of one or more amino acids (e.g., a substitution, deletion, or addition of 1, 2, 3, 4, or 5 amino acids) as compared with the sequence from which it is derived; preferably, the substitution is a conservative substitution.

29. A pharmaceutical composition, which comprises the antibody-drug conjugate according to any one of claims 1 to 21, the composition according to claim 22, the compound or the pharmaceutically acceptable salt, ester, stereoisomer, tautomer, polymorph, solvate, *N*-oxide, isotope-labeled compound, metabolite or prodrug thereof according to any one of claims 23 to 27 or the antibody or antigen-binding fragment thereof according to claim 28, and one or more pharmaceutical excipients.

30. Use of the compound or the pharmaceutically acceptable salt, ester, stereoisomer, tautomer, polymorph, solvate, N-oxide, isotope-labeled compound, metabolite or prodrug thereof according to any one of claims 23 to 27 or the antibody or antigen-binding fragment thereof according to claim 28, in the manufacture of an antibody-drug conjugate (e.g., the antibody-drug conjugate according to any one of claims 1 to 21).

31. Use of the antibody-drug conjugate according to any one of claims 1-21, the composition according to claim 22, the compound or the pharmaceutically acceptable salt, ester, stereoisomer, tautomer, polymorph, solvate, *N*-oxide, isotope-labeled compound, metabolite or prodrug thereof according to any one of claims 23 to 27, the antibody or antigen-binding fragment thereof according to claim 28 or the pharmaceutical composition according to claim 29, in the manufacture of a medicament, particularly a medicament for treating and/or preventing a cancer (e.g., a cancer associated with B7H3 expression);
preferably, the cancer is a B7H3-positive cancer;
preferably, the cancer is selected from the group consisting of breast cancer (e.g., breast ductal carcinoma), gastric cancer, lung cancer (e.g., lung adenocarcinoma), and colon cancer.

32. A method for treating and/or preventing cancer (e.g., cancer associated with B7H3 expression), comprising administering to a subject in need thereof a therapeutically and/or prophylactically effective amount of the antibody-drug conjugate according to any one of claims 1-21, the composition according to claim 22, the compound or the pharmaceutically acceptable salt, ester, stereoisomer, tautomer, polymorph, solvate, N-oxide, isotope-labeled compound, metabolite or prodrug thereof according to any one of claims 23 to 27, the antibody or antigen-binding fragment thereof according to claim 28 or the pharmaceutical composition according to claim 29;
preferably, the cancer is a B7H3-positive cancer;
preferably, the cancer is selected from the group consisting of breast cancer (e.g., breast ductal carcinoma), gastric cancer, lung cancer (e.g., lung adenocarcinoma), and colon cancer.

33. A compound as shown below, or a salt, ester, stereoisomer, tautomer, polymorph, solvate, *N*-oxide, isotope-labeled compound, metabolite or prodrug thereof: 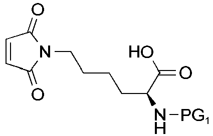 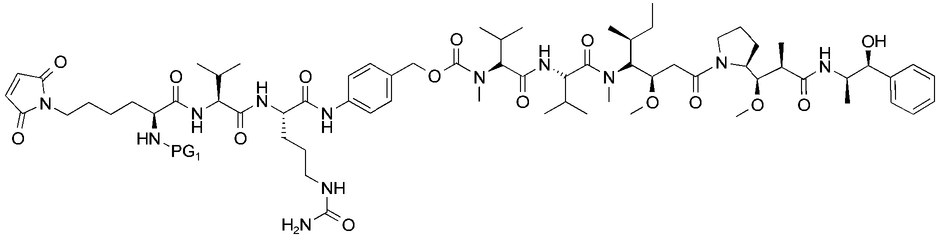 wherein, PG₁ is each independently H or an amino-protecting group, preferably an alkoxycarbonyl amino-protecting group, such as benzyloxycarbonyl (Cbz), tert-butyloxycarbonyl (Boc), methyloxycarbonyl (Fmoc), allyloxycarbonyl (Alloc), trimethylsilylethoxycarbonyl (Teoc), methyl(or ethyl)oxycarbonyl; an acyl amino-protecting group, such as phthaloyl (Pht), tosyl (Tos), trifluoroacetyl (Tfa), o-(p-)nitrobenzenesulfonyl (Ns), pivaloyl, benzoyl, tert-butyloxycarbonyl, 9-fluorenylmethyloxycarbonyl, allyloxycarbonyl, trichloroethoxycarbonyl, trimethylsilylethoxycarbonyl, benzyloxycarbonyl, p-methylbenzenesulfonyl, p-nitrobenzenesulfonyl, trifluoroacetyl, methoxycarbonyl, or ethoxycarbonyl; an alkyl amino-protecting group, such as trityl (Trt), 2,4-dimethoxybenzyl (Dmb), 4-methoxybenzyl (PMB), or benzyl (Bn).

34. A compound as shown below, or a salt, ester, stereoisomer, tautomer, polymorph, solvate, N-oxide, isotope-labeled compound, metabolite or prodrug thereof: 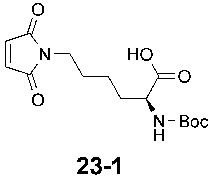 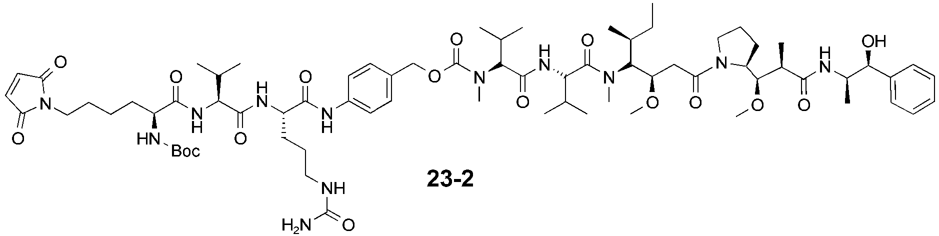 or

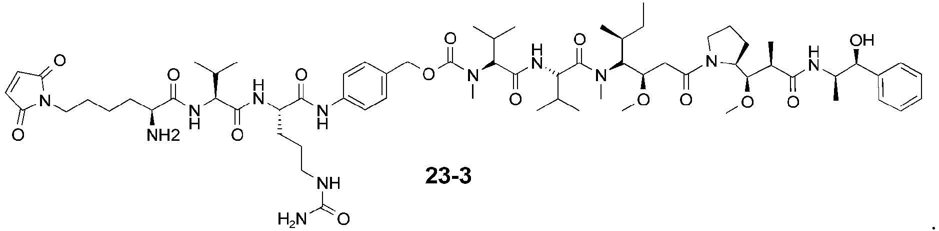

35. A compound represented by Formula (III), or a salt, ester, stereoisomer, tautomer, polymorph, solvate, *N*-oxide, isotope-labeled compound, metabolite or prodrug thereof: 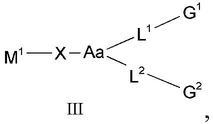 wherein,
X, Aa, L¹, L², and M¹ are as defined in any one of claims 23 to 27;
G¹ is p-nitrophenoxy or C₁₋₆ alkanoyloxy;
G² is hydroxy or oxo;
preferably, the compound is selected from the group consisting of: 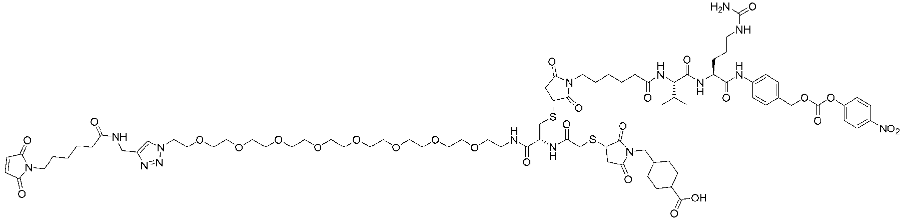 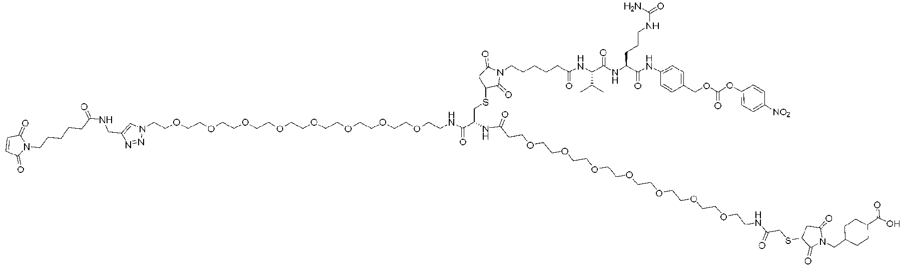 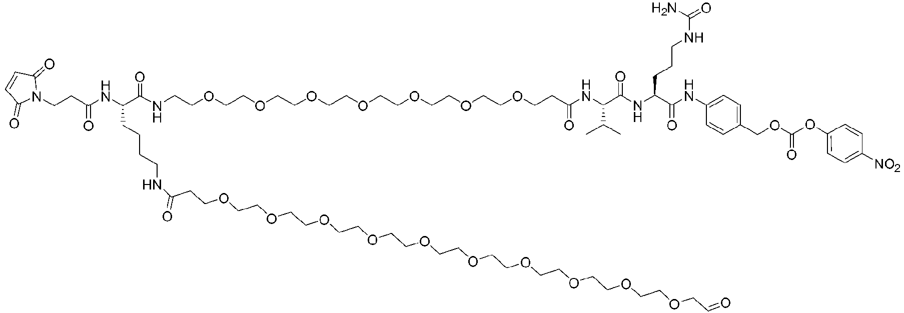 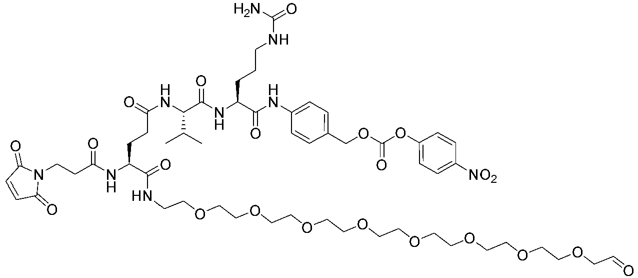 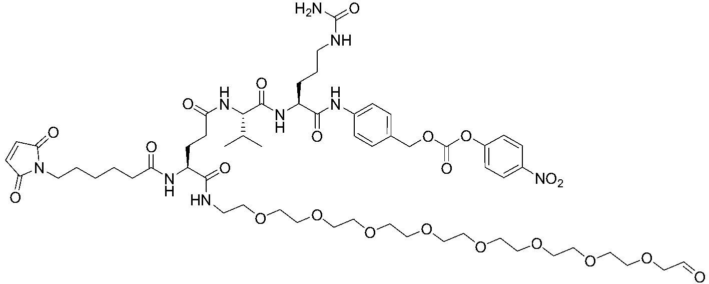 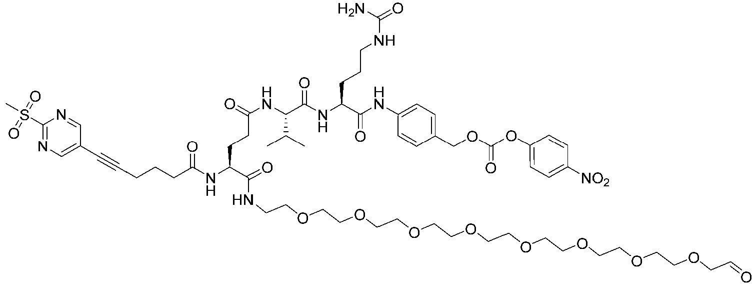 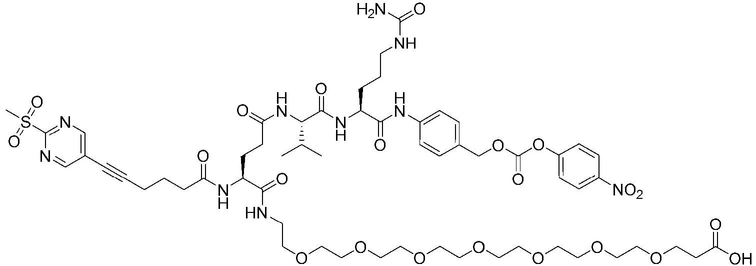 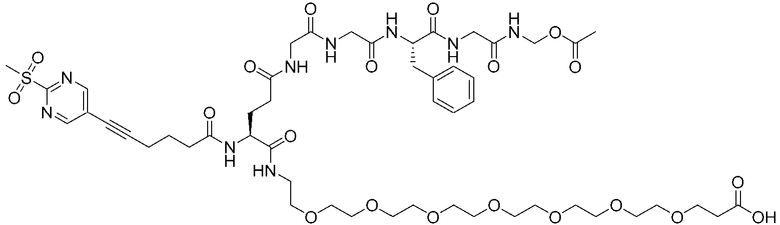 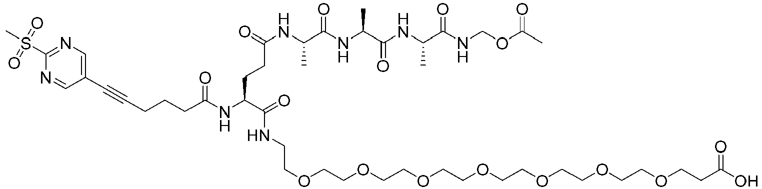 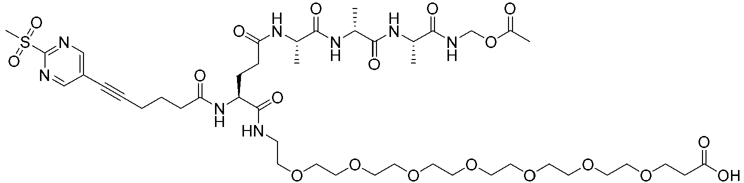 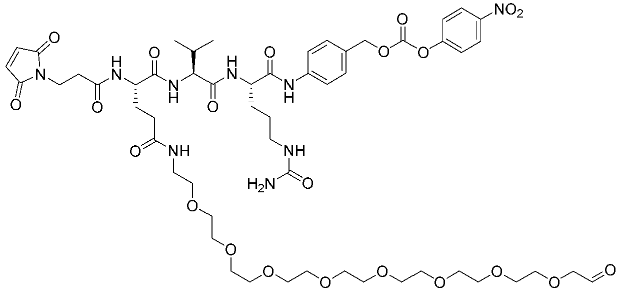 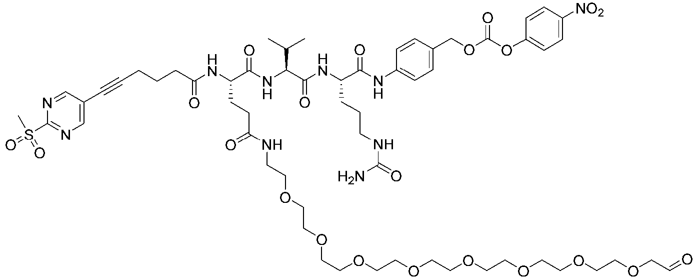 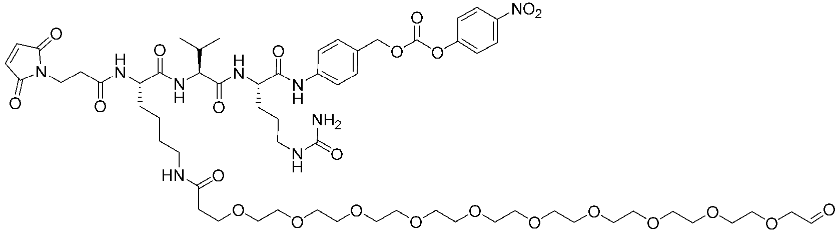 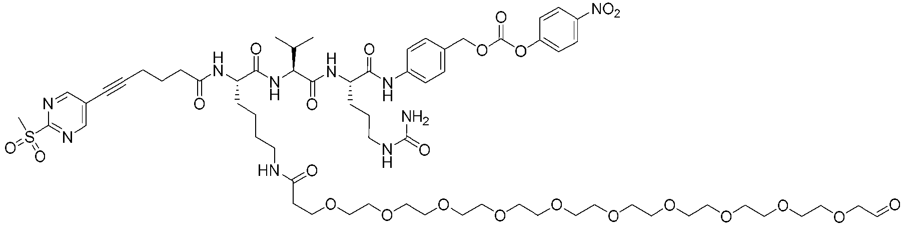 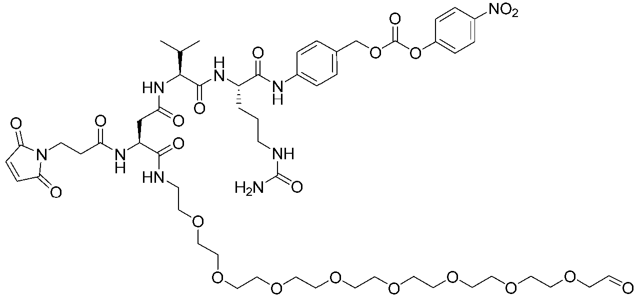 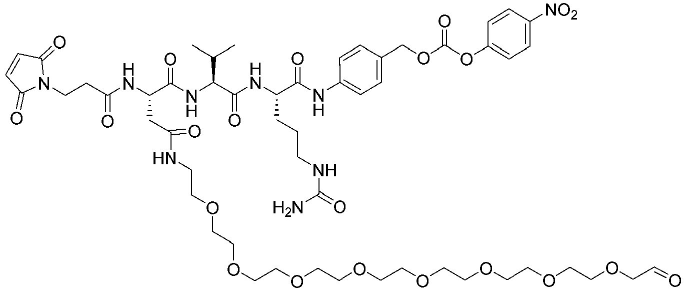 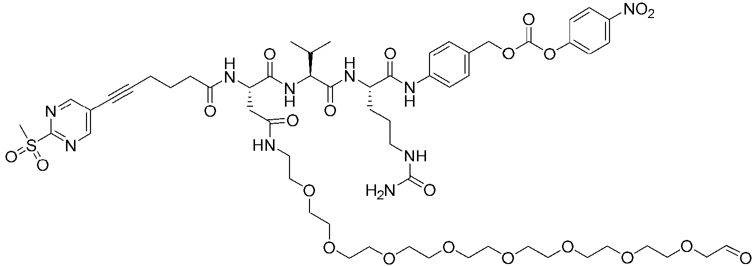 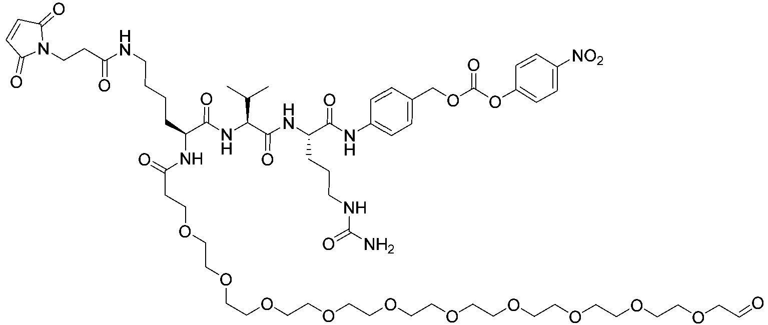 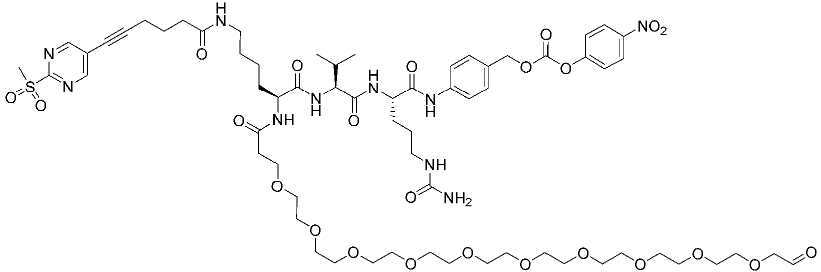 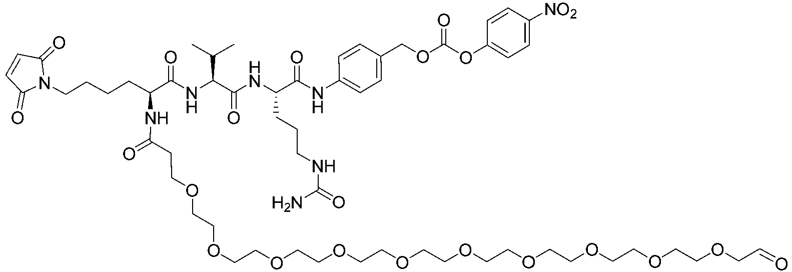 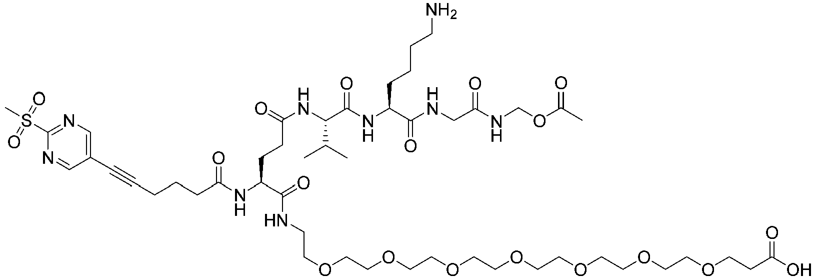 and

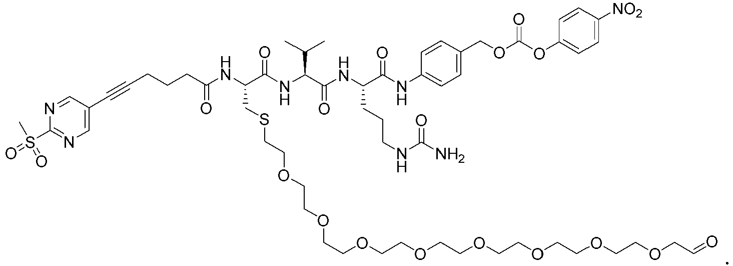

36. A linker unit which is a compound having a structure represented by Formula (IV), or a salt, ester, stereoisomer, tautomer, polymorph, solvate, *N*-oxide, isotope-labeled compound, metabolite or prodrug thereof: 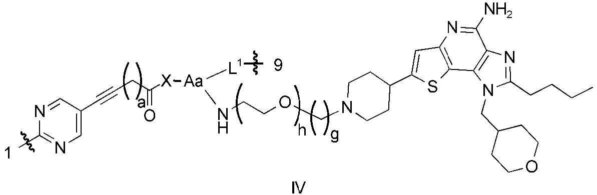 wherein, X, Aa, L¹, a, g, and h are as defined in any one of claims 1 to 21.

37. A compound or a pharmaceutically acceptable salt, ester, stereoisomer, tautomer, polymorph, solvate, *N*-oxide, isotope-labeled compound, metabolite or prodrug thereof, which comprises the linker unit according to claim 36 and at least one of LG or D¹, wherein the linker unit is connected to LG or D¹, wherein D¹ is connected to the position 9 of the linker unit; and LG is connected to the position 1 of the linker unit; wherein D¹ is as defined in any one of claims 1 to 21, and LG is as defined in any one of claims 23 to 27.

38. An antibody-drug conjugate, which comprises the compound or the pharmaceutically acceptable salt, ester, stereoisomer, tautomer, polymorph, solvate, *N*-oxide, isotope-labeled compound, metabolite or prodrug thereof according to any one of claims 23 to 27, and/or, the antibody or antigen-binding fragment thereof according to claim 28.
